# EUROPEAN PATENT SPECIFICATION

(11) **EP 4 126 879 B1**
(45) Date of publication and mention of the grant of the patent: **21.01.2026**
(21) Application number: 21780725.4
(22) Date of filing: 01.04.2021
(51) Int. Cl.: C07D 487/04, A61K 31/395, A61K 31/437, A61K 31/444, A61K 31/4545, A61K 31/4985, A61K 31/501, A61K 31/506, A61K 31/5377, A61K 31/5386, C07D 471/04, C07D 519/00, A61P 35/00

(54) **N-PHENYL SUBSTITUTED INDOLE DERIVATIVES AS MYT1 INHIBITORS FOR THE TREATMENT OF CANCER**
N-PHENYL SUBSTITUIERTE INDOL ABKÖMMLINGE ALS MYT1 HEMMSTOFFE ZUR BEHANDLUNG VON KREBS
N-PHÉNYLE INDOLES EN TANT QU'INHIBITEURS DE MYT1 POUR LE TRAITEMENT DES MALADIES DE CANCER

(30) Priority: 01.04.2020 US 202063003745 P
(43) Date of publication of application: 08.02.2023
(73) Proprietor: Repare Therapeutics Inc., St-Laurent QC H4S 2A1 (CA)
(72) Inventor: SZYCHOWSKI, Janek, Saint-Laurent, Québec H4S 2A1 (CA); LIU, Bingcan, Saint-Laurent, Québec H4S 2A1 (CA); DIETRICH, Evelyne, Saint-Laurent, Québec H4S 2A1 (CA); VALLÉE, Frédéric, Saint-Laurent, Québec H4S 2A1 (CA); PERRYMAN, Alexander, Saint-Laurent, Québec H4S 2A1 (CA); TRUCHON, Jean-François, Saint-Laurent, Québec H4R 3H8 (CA); PAPP, Robert, Saint-Laurent, Québec H4S 2A1 (CA); BEAULIEU, Patrick, Québec G6W 7V6 (CA)
(74) Representative: Hoffmann Eitle
(86) International application number: PCT/CA2021/050443
(87) International publication number: WO 2021/195781

(56) References cited:
- WO-A1-00/33842
- WO-A1-01/64680
- WO-A1-2015/073528
- US-A1- 2016 075 712

## Description

### FIELD OF THE INVENTION

The invention relates to compounds and pharmaceutical compositions, their preparation and their use in the treatment of a disease or condition, e.g., cancer, and, in particular, those diseases or conditions (e.g., cancers that harbor CCNE1 amplification/overexpression or FBXW7-mutated cancers) which depend on the activity of membrane-associated tyrosine and threonine-specific cdc2-inhibitory kinase (Myt1) (Gene name PKMYT1).

### BACKGROUND

DNA is continuously subjected to both endogenous insults (e.g., stalled replication forks, reactive oxygen species) and exogenous insults (UV, ionizing radiation, chemical) that can lead to DNA damage. As a result, cells have established sophisticated mechanisms to counteract these deleterious events that would otherwise compromise genomic integrity and lead to genomic instability diseases such as cancer. These mechanisms are collectively referred to as the DNA damage response (DDR). One component of the overall DDR is the activation of various checkpoint pathways that modulate specific DNA-repair mechanisms throughout the various phases of the cell cycle, which includes the G1, S, G2 and Mitosis checkpoints. A majority of cancer cells have lost their G1 checkpoint owing to p53 mutations and as such, rely on the G2 checkpoint to make the necessary DNA damage corrections prior to committing to enter mitosis and divide into 2 daughter cells.

There is a need for new anti-cancer therapeutic approaches, e.g., those utilizing small-molecules, especially therapies allowing for targeted cancer treatment.

### SUMMARY OF THE INVENTION

In one aspect, the invention provides a compound of formula (I): 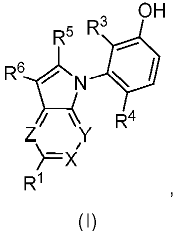 or a pharmaceutically acceptable salt thereof,
wherein
each of X, Y, and Z is independently N or CR²;
R¹ and each R² are independently hydrogen, optionally substituted C₁₋₆ alkyl, optionally substituted C₂₋₆ alkenyl, optionally substituted C₂₋₆ alkynyl, optionally substituted C₃₋₈ cycloalkyl, optionally substituted C₃₋₈ cycloalkenyl, optionally substituted C₂₋₉ heterocyclyl, optionally substituted C₂₋₉ heterocyclyl C₁₋₆ alkyl, optionally substituted C₆₋₁₀ aryl, optionally substituted C₁₋₉ heteroaryl, optionally substituted C₁₋₉ heteroaryl C₁₋₆ alkyl, halogen, cyano, -N(R⁷)₂, -OR⁷, - C(O)N(R⁸)₂, -SO₂N(R⁸)₂, -SO₂R^{7A}, or -Q-R^{7B}; or R¹ combines with one R² that is *vicinal* to R¹ to form an optionally substituted C₃₋₆ alkylene;
each of R³ and R⁴ is independently optionally substituted C₁₋₆ alkyl or halogen;
R⁵ is H or -N(R⁷)₂;
R⁶ is -C(O)NH(R⁸), -C(O)R^{7A}, or -SO₂R^{7A};
each R⁷ is independently hydrogen, optionally substituted C₁₋₆ alkyl, optionally substituted C₆₋₁₀ aryl C₁₋₆ alkyl, optionally substituted C₃₋₈ cycloalkyl, optionally substituted C₆₋₁₀ aryl, optionally substituted C₂₋₉ heterocyclyl, optionally substituted C₁₋₉ heteroaryl, optionally substituted C₁₋₉ heteroaryl C₁₋₆ alkyl, or -SO₂R^{7A}; or two R⁷ groups, together with the atom to which both are attached, combine to form an optionally substituted C₂₋₉ heterocyclyl;
each R^{7A} is independently optionally substituted C₁₋₆ alkyl, optionally substituted C₃₋₈ cycloalkyl, or optionally substituted C₆₋₁₀ aryl;
each R^{7B} is independently hydroxyl, optionally substituted C₁₋₆ alkyl, optionally substituted C₆₋₁₀ aryl, optionally substituted C₂₋₉ heterocyclyl, optionally substituted C₁₋₉ heteroaryl, -N(R⁷)₂, - C(O)N(R⁸)₂, -SO₂N(R⁸)₂, -SO₂R^{7A}, or optionally substituted alkoxy;
each R⁸ is independently hydrogen, optionally substituted C₁₋₆ alkyl, optionally substituted C₂₋₆ alkoxyalkyl, optionally substituted C₆₋₁₀ aryl C₁₋₆ alkyl, optionally substituted C₆₋₁₀ aryl, optionally substituted C₃₋₈ cycloalkyl, or optionally substituted C₁₋₉ heteroaryl; or two R⁸, together with the atom to which they are attached, combine to form an optionally substituted C₂₋₉ heterocyclyl;
Q is optionally substituted C₁₋₆ alkylene, optionally substituted C₂₋₆ alkenylene, optionally substituted C₂₋₆ alkynylene, optionally substituted C₃₋₈ cycloalkylene, optionally substituted C₃₋₈ cycloalkenylene optionally substituted C₆₋₁₀ arylene, optionally substituted C₂₋₉ heterocyclylene, or optionally substituted C₁₋₉ heteroarylene.

In some embodiments, the compound is enriched for the atropisomer of formula (IA):

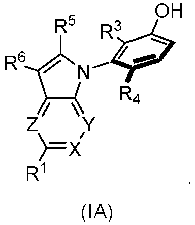

In some embodiments, X is CR². In some embodiments, the compound is of formula (II):

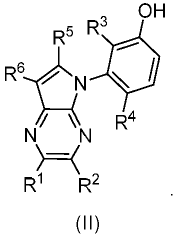

In some embodiments, the compound is enriched for the atropisomer of formula (IIA):

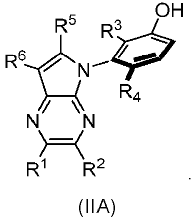

In some embodiments, the compound is of formula (III): 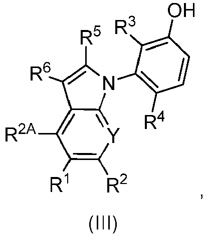 wherein R^{2A} is hydrogen, optionally substituted C₁₋₆ alkyl, optionally substituted C₂₋₆ alkenyl, optionally substituted C₂₋₆ alkynyl, optionally substituted C₃₋₈ cycloalkyl, optionally substituted C₃₋₈ cycloalkenyl, optionally substituted C₂₋₉ heterocyclyl, optionally substituted C₂₋₉ heterocyclyl C₁₋₆ alkyl, optionally substituted C₆₋₁₀ aryl, optionally substituted C₁₋₉ heteroaryl, optionally substituted C₁₋₉ heteroaryl C₁₋₆ alkyl, halogen, -N(R⁷)₂, -OR⁷, -C(O)N(R⁸)₂, -SO₂N(R⁸)₂, -SO₂R^{7A}, or -Q-R^{7B}. In some embodiments, the compound is enriched for the atropisomer of formula (IIIA):

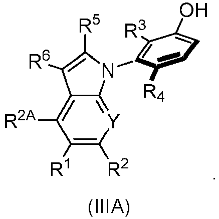

In some embodiments, R^{2A} is hydrogen, optionally substituted C₁₋₆ alkyl, or halogen.

In some embodiments, R³ is optionally substituted C₁₋₆ alkyl. In some embodiments, R³ is halogen. In some embodiments, R⁴ is optionally substituted C₁₋₆ alkyl. In some embodiments, R⁴ is halogen (e.g., chlorine).

In some embodiments, R² is hydrogen. In some embodiments, R² is optionally substituted C₁₋₆ alkyl. In some embodiments, R² is optionally substituted methyl or optionally substituted isopropyl. R² is halogen.

In some embodiments, R¹ is hydrogen. In some embodiments, R¹ is halogen. In some embodiments, R¹ is chlorine or bromine. In some embodiments, R¹ is optionally substituted C₁₋₆ alkyl. In some embodiments, R¹ is optionally substituted methyl, optionally substituted ethyl, optionally substituted isopropyl, or optionally substituted butyl. In some embodiments, R¹ is optionally substituted C₁₋₉ heteroaryl. In some embodiments, R¹ is 1,3-thiazolyl, 1,2-thiazolyl, 1,3-oxazolyl, benzo-1,3-thiazolyl, benzo-1,3-oxazolyl, indolyl, benzimidazolyl, pyridyl, imidazolyl, pyrimidyl, pyrazinyl, pyridazinyl, or pyrazolyl, wherein R¹ is optionally substituted with substituents as defined for optionally substituted C₁₋₉ heteroaryl. In some embodiments, R¹ is optionally substituted C₃₋₈ cycloalkyl. In some embodiments, R¹ is cyclopropyl, cyclobutyl, cyclopentyl, or cyclohexyl, wherein R¹ is optionally substituted with substituents as defined for optionally substituted C₃₋₈ cycloalkyl. In some embodiments, R¹ is optionally substituted C₂₋₉ heterocyclyl. In some embodiments, R¹ is 1,2,3,6-tetrahydropyridinyl, piperidinyl, morpholinyl, piperazinyl, thiomorpholinyl, oxa-aza-spiro[3,3]heptane, or oxa-aza-bicyclo[3.2.1]octane, wherein R¹ is optionally substituted with substituents as defined for optionally substituted C₂₋₉ heterocyclyl. In some embodiments, R¹ is optionally substituted C₃₋₈ cycloalkyl. In some embodiments, R¹ is optionally substituted cyclohexenyl or optionally substituted cyclopentenyl. In some embodiments, R¹ is optionally substituted C₆₋₁₀ aryl. In some embodiments, R¹ is optionally substituted phenyl.

In some embodiments, R¹ is -Q-R^{7B}. In some embodiments, Q is optionally substituted C₂₋₆ alkynylene. In some embodiments, Q is optionally substituted C₁₋₆ alkylene. In some embodiments, Q is optionally substituted C₆₋₁₀ arylene. In some embodiments, R^{7B} is optionally substituted C₂₋₉ heterocyclyl. In some embodiments, R^{7B} is optionally substituted C₆₋₁₀ aryl.

In some embodiments, R¹ is optionally substituted with one, two, or three groups independently selected from the group consisting of methyl, difluoromethyl, trifluoromethyl, fluorine, chlorine, bromine, amino, hydroxyl, cyano, oxo, -C(O)NH₂, -C(O)NH(Me), -C(O)N(Me)₂, - (CH₂)ₙ-C(O)OH, and -(CH₂)ₙ-C(O)Ot-Bu, wherein n is 0 or 1.

In some embodiments, R¹ is -N(R⁷)₂. In some embodiments, R¹ is diethylamino.

In some embodiments, R⁵ is hydrogen. In some embodiments, R⁵ is -N(R⁷)₂. In some embodiments, R⁵ is -NH₂. In some embodiments, R⁶ is -C(O)NH(R⁸). In some embodiments, R⁶ is -C(O)NH₂. In some embodiments, R⁶ is -C(O)NH(Me). In some embodiments, R⁶ is -SO₂R^{7A}. In some embodiments, R⁶ is -SO₂Me.

In some embodiments, the compound selected from the group consisting of compounds 1-328 (e.g., compounds 1-288) and pharmaceutically acceptable salts thereof.

In another aspect, the invention provides a pharmaceutical composition comprising the compound disclosed herein, or a pharmaceutically acceptable salt thereof, and a pharmaceutically acceptable excipient. In some embodiments, the composition is isotopically enriched in deuterium. The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

In yet another aspect, the invention provides a method of inhibiting Myt1 in a cell expressing Myt1, the method comprising contacting the cell with the compound disclosed herein.

In some embodiments, the cell is overexpressing CCNE1. In some embodiments, the cell is in a subject.

In still another aspect, the invention provides a method of treating a subject in need thereof comprising administering to the subject the compound disclosed herein, or a pharmaceutically acceptable salt thereof, or the pharmaceutical composition disclosed herein.

In some embodiments, the subject is suffering from, and is in need of a treatment for, a disease or condition having the symptom of cell hyperproliferation. In some embodiments, the disease or condition is a cancer. In some embodiments, the cancer is a cancer overexpressing CCNE1.

In still another aspect, the invention provides a method of treating a cancer in a subject, the method comprising administering to the subject in need thereof a therapeutically effective amount of a Myt1 inhibitor, wherein the cancer has been previously identified as a cancer overexpressing *CCNE1.*

In another aspect, the invention provides a method of treating a cancer in a subject, the method comprising administering to the subject in need thereof a therapeutically effective amount of a Myt1 inhibitor, wherein the cancer is a cancer overexpressing *CCNE1.*

In yet another aspect, the invention provides a method of inducing cell death in a cancer cell overexpressing *CCNE1,* the method comprising contacting the cell with an effective amount of a Myt1 inhibitor.

In some embodiments, the cell is in a subject. In some embodiments, the Myt1 inhibitor is the compound disclosed herein or a pharmaceutically acceptable salt thereof. In some embodiments, the cancer overexpressing CCNE1 is uterine cancer, ovarian cancer, breast cancer, stomach cancer, esophageal cancer, lung cancer, or endometrial cancer.

In still another aspect, the invention provides a method of treating a cancer in a subject, the method comprising administering to the subject in need thereof a therapeutically effective amount of a Myt1 inhibitor, wherein the cancer has been previously identified as a cancer having an inactivating mutation in the *FBXW7* gene.

In another aspect, the invention provides a method of treating a cancer in a subject, the method comprising administering to the subject in need thereof a therapeutically effective amount of a Myt1 inhibitor, wherein the cancer has an inactivating mutation in the *FBXW7* gene.

In yet another aspect, the invention provides a method of inducing cell death in an FBXW7-mutated cancer cell, the method comprising contacting the cell with an effective amount of a Myt1 inhibitor.

In some embodiments, the cell is in a subject. the cancer is uterine cancer, colorectal cancer, breast cancer, lung cancer, or esophageal cancer. In some embodiments, the Myt1 inhibitor is the compound disclosed herein, or a pharmaceutically acceptable salt thereof.

### ABBREVIATIONS

Abbreviations and terms that are commonly used in the fields of organic chemistry, medicinal chemistry, pharmacology, and medicine and are well known to practitioners in these fields are used herein. Representative abbreviations and definitions are provided below:
Ac is acetyl [CH₃C(O)-], Ac₂O is acetic anhydride; AcOH is acetic acid; APC is antigen-presenting cell; aq. is aqueous; 9-BBN is 9-borabicyclo[3.3.1]nonane; BINAP is (2,2'-bis(diphenylphosphino)-1,1'-binaphthyl); Bn is benzyl; BOC is tert Butyloxycarbonyl; CDI is carbonyldiimidazole; DCM is dichloromethane; DIAD is diisopropylazodicarboxylate; DIBAL is diisobutylaluminum hydride; DIPEA is diisoproplyethyl amine; DMA is dimethylacetamide; DMAP is 4-dimethylaminopyridine; DMF is N,N-dimethylformamide; DMSO is dimethyl sulfoxide; dppf is 1,1'-bis(diphenylphosphino)ferrocene; EDAC (or EDC) is 1-ethyl-3-[3-(dimethylamino)propyl]-carbodiimide HCl; ESI is electrospray ionization mass spectrometry; Et₂O is diethyl ether; Et₃N is triethylamine; Et is ethyl; EtOAc is ethyl acetate; EtOH is ethanol; 3-F-Ph is 3-fluorophenyl, HATU is (1-[bis(dimethylamino)methylene]-1H-1,2,3-triazolo[4,5-b]pyridinium 3-oxide hexafluorophosphate; HCl is hydrochloric acid; HOBt is 1-hydroxybenzotriazole; HPLC is high performance liquid chromatography; LCMS is HPLC with mass spectral detection; LiHMDS is lithium bis(trimethylsilyl)amide; LG is leaving group; M is molar; mCPBA is metachloroperbenzoic acid; mmol is millimole; Me is methyl; MeCN is acetonitrile; MeOH is methanol; Ms is methanesulfonyl; MS is mass spectrometry; N is normal; NaHMDS is sodium hexamethyldisiliazide; NaOAc is sodium acetate; NaOtBu is sodium tert-butoxide; NMO is N-methylmorpholine N-oxide; NMP is N-methyl pyrrolidinone; NMR is nuclear magnetic resonance spectroscopy; Pd₂(dba)₃ is tris(dibenzylideneacetone)dipalladium; PdCl₂(PPh₃)₂ is dichlorobis-(triphenylphosphene) palladium; PG Denotes an unspecified protecting group; Ph is phenyl; PhMe is toluene; PPh₃ is triphenylphosphine; PMB is para-methoxybenzyl; rt is room temperature; RBF is round-bottom flask; RuPhos Pd G1 is chloro-(2-Dicyclohexylphosphino-2',6'-diisopropoxy-1,1'-biphenyl)[2-(2-aminoethyl)phenyl]palladium(ll); SEM is [2-(trimethylsilyl)ethoxy]methyl; SFC is supercritical fluid chromatography; S_{N}Ar is nucleophilic aromatic substitution; TBAB is tetrabutyl ammonium bromide; TBAF is tetrabutyl ammonium fluoride; TBS is tert-butyldimethylsilyl; tBu is tert-butyl; Tf is triflate; TFA is trifluoroacetic acid; THF is tetrahydrofuran; THP is tetrahydropyran; TLC is thin layer chromatography; TMAD is tetramethylazodicarboxamide; TMS is trimethylsilyl; TPAP is tetrapropylammonium perruthenate; Ts is *p*-toluenesulfonyl; UPLC is ultra performance liquid chromatography.

### DEFINITIONS

The term "aberrant," as used herein, refers to different from normal. When used to describe activity, aberrant refers to activity that is greater or less than a normal control or the average of normal non-diseased control samples. Aberrant activity may refer to an amount of activity that results in a disease, where returning the aberrant activity to a normal or non-disease-associated amount (e.g. by administering a compound or using a method as described herein), results in reduction of the disease or one or more disease symptoms.

The term "acyl," as used herein, represents a group -C(=O)-R, where R is alkyl, alkenyl, alkynyl, cycloalkyl, cycloalkenyl, cycloalkynyl, aryl, heteroaryl, or heterocyclyl. Acyl may be optionally substituted as described herein for each respective R group.

The term "adenocarcinoma," as used herein, represents a malignancy of the arising from the glandular cells that line organs within an organism. Non-limiting examples of adenocarcinomas include non-small cell lung cancer, prostate cancer, pancreatic cancer, esophageal cancer, and colorectal cancer.

The term "alkanoyl," as used herein, represents a hydrogen or an alkyl group that is attached to the parent molecular group through a carbonyl group and is exemplified by formyl (i.e., a carboxyaldehyde group), acetyl, propionyl, butyryl, and iso-butyryl. Unsubstituted alkanoyl groups contain from 1 to 7 carbons. The alkanoyl group may be unsubstituted of substituted (e.g., optionally substituted C1-7 alkanoyl) as described herein for alkyl group. The ending "-oyl" may be added to another group defined herein, e.g., aryl, cycloalkyl, and heterocyclyl, to define "aryloyl," "cycloalkanoyl," and "(heterocyclyl)oyl." These groups represent a carbonyl group substituted by aryl, cycloalkyl, or heterocyclyl, respectively. Each of "aryloyl," "cycloalkanoyl," and "(heterocyclyl)oyl" may be optionally substituted as defined for "aryl," "cycloalkyl," or "heterocyclyl," respectively.

The term "alkenyl," as used herein, represents acyclic monovalent straight or branched chain hydrocarbon groups of containing one, two, or three carbon-carbon double bonds. Non-limiting examples of the alkenyl groups include ethenyl, prop-1-enyl, prop-2-enyl, 1-methylethenyl, but-1-enyl, but-2-enyl, but-3-enyl, 1-methylprop-1-enyl, 2-methylprop-1-enyl, and 1-methylprop-2-enyl. Alkenyl groups may be optionally substituted as defined herein for alkyl.

The term "alkenylene," as used herein, refers to a divalent alkenyl group. An optionally substituted alkenylene is an alkenylene that is optionally substituted as described herein for alkenyl.

The term "alkoxy," as used herein, represents a chemical substituent of formula -OR, where R is a C₁₋₆ alkyl group, unless otherwise specified. In some embodiments, the alkyl group can be further substituted as defined herein. The term "alkoxy" can be combined with other terms defined herein, e.g., aryl, cycloalkyl, or heterocyclyl, to define an "aryl alkoxy," "cycloalkyl alkoxy," and "(heterocyclyl)alkoxy" groups. These groups represent an alkoxy that is substituted by aryl, cycloalkyl, or heterocyclyl, respectively. Each of "aryl alkoxy," "cycloalkyl alkoxy," and "(heterocyclyl)alkoxy" may optionally substituted as defined herein for each individual portion.

The term "alkoxyalkyl," as used herein, represents a chemical substituent of formula -L-O-R, where L is C₁₋₆ alkylene, and R is C₁₋₆ alkyl. An optionally substituted alkoxyalkyl is an alkoxyalkyl that is optionally substituted as described herein for alkyl.

The term "alkyl," as used herein, refers to an acyclic straight or branched chain saturated hydrocarbon group, which, when unsubstituted, has from 1 to 12 carbons, unless otherwise specified. In certain preferred embodiments, unsubstituted alkyl has from 1 to 6 carbons. Alkyl groups are exemplified by methyl; ethyl; n- and iso-propyl; n-, sec-, iso- and tert-butyl; neopentyl, and the like, and may be optionally substituted, valency permitting, with one, two, three, or, in the case of alkyl groups of two carbons or more, four or more substituents independently selected from the group consisting of: amino; alkoxy; aryl; aryloxy; azido; cycloalkyl; cycloalkoxy; cycloalkenyl; cycloalkynyl; halo; heterocyclyl; (heterocyclyl)oxy; heteroaryl; hydroxy; nitro; thiol; silyl; cyano; alkylsulfonyl; alkylsulfinyl; alkylsulfenyl; =O; =S; -C(O)R or -SO₂R, where R is amino; and =NR', where R' is H, alkyl, aryl, or heterocyclyl. Each of the substituents may itself be unsubstituted or, valency permitting, substituted with unsubstituted substituent(s) defined herein for each respective group.

The term "alkylene," as used herein, refers to a divalent alkyl group. An optionally substituted alkylene is an alkylene that is optionally substituted as described herein for alkyl.

The term "alkylamino," as used herein, refers to a group having the formula -N(R^{N1})₂ or - NHR^{N1}, in which R^{N1} is alkyl, as defined herein. The alkyl portion of alkylamino can be optionally substituted as defined for alkyl. Each optional substituent on the substituted alkylamino may itself be unsubstituted or, valency permitting, substituted with unsubstituted substituent(s) defined herein for each respective group.

The term "alkylsulfenyl," as used herein, represents a group of formula -S-(alkyl). Alkylsulfenyl may be optionally substituted as defined for alkyl.

The term "alkylsulfinyl," as used herein, represents a group of formula -S(O)-(alkyl). Alkylsulfinyl may be optionally substituted as defined for alkyl.

The term "alkylsulfonyl," as used herein, represents a group of formula -S(O)2-(alkyl). Alkylsulfonyl may be optionally substituted as defined for alkyl.

The term "alkynyl," as used herein, represents monovalent straight or branched chain hydrocarbon groups of from two to six carbon atoms containing at least one carbon-carbon triple bond and is exemplified by ethynyl, 1-propynyl, and the like. The alkynyl groups may be unsubstituted or substituted (e.g., optionally substituted alkynyl) as defined for alkyl.

The term "alkynylene," as used herein, refers to a divalent alkynyl group. An optionally substituted alkynylene is an alkynylene that is optionally substituted as described herein for alkynyl.

The term "amino," as used herein, represents -N(R^{N1})₂, where, if amino is unsubstituted, both R^{N1} are H; or, if amino is substituted, each R^{N1} is independently H, -OH, -NO₂, -N(R^{N2})₂, - SO₂O_{R}^{N2}, -SO₂R^{N2}, -SOR^{N2}, -C(O)OR^{N2}, an N-protecting group, alkyl, alkenyl, alkynyl, alkoxy, aryl, arylalkyl, aryloxy, cycloalkyl, cycloalkenyl, heteroalkyl, or heterocyclyl, provided that at least one R^{N1} is not H, and where each R^{N2} is independently H, alkyl, or aryl. Each of the substituents may itself be unsubstituted or substituted with unsubstituted substituent(s) defined herein for each respective group. In some embodiments, amino is unsubstituted amino (i.e., -NH₂) or substituted amino (e.g., -NHR^{N1}), where R^{N1} is independently -OH, SO2OR^{N2}, -SO₂R^{N2}, -SOR^{N2}, -COOR^{N2}, optionally substituted alkyl, or optionally substituted aryl, and each R^{N2} can be optionally substituted alkyl or optionally substituted aryl. In some embodiments, substituted amino may be alkylamino, in which the alkyl groups are optionally substituted as described herein for alkyl. In some embodiments, an amino group is -NHR^{N1}, in which R^{N1} is optionally substituted alkyl.

The term "aryl," as used herein, represents a mono-, bicyclic, or multicyclic carbocyclic ring system having one or two aromatic rings. Aryl group may include from 6 to 10 carbon atoms. All atoms within an unsubstituted carbocyclic aryl group are carbon atoms. Non-limiting examples of carbocyclic aryl groups include phenyl, naphthyl, 1,2-dihydronaphthyl, 1,2,3,4-tetrahydronaphthyl, fluorenyl, indanyl, indenyl, etc. The aryl group may be unsubstituted or substituted with one, two, three, four, or five substituents independently selected from the group consisting of: alkyl; alkenyl; alkynyl; alkoxy; alkylsulfinyl; alkylsulfenyl; alkylsulfonyl; amino; aryl; aryloxy; azido; cycloalkyl; cycloalkoxy; cycloalkenyl; cycloalkynyl; halo; heteroalkyl; heterocyclyl; (heterocyclyl)oxy; hydroxy; nitro; thiol; silyl; -(CH₂)ₙ-C(0)OR^{A}; -C(O)R; and -SO₂R, where R is amino or alkyl, R^{A} is H or alkyl, and n is 0 or 1. Each of the substituents may itself be unsubstituted or substituted with unsubstituted substituent(s) defined herein for each respective group.

The term "aryl alkyl," as used herein, represents an alkyl group substituted with an aryl group. The aryl and alkyl portions may be optionally substituted as the individual groups as described herein.

The term "arylene," as used herein, refers to a divalent aryl group. An optionally substituted arylene is an arylene that is optionally substituted as described herein for aryl.

The term "aryloxy," as used herein, represents a chemical substituent of formula -OR, where R is an aryl group, unless otherwise specified. In optionally substituted aryloxy, the aryl group is optionally substituted as described herein for aryl.

The term "azido," as used herein, represents an -N₃ group.

The term "cancer," as used herein, refers to all types of cancer, neoplasm or malignant tumors found in mammals (e.g., humans).

The term "carbocyclic," as used herein, represents an optionally substituted C3-16 monocyclic, bicyclic, or tricyclic structure in which the rings, which may be aromatic or non-aromatic, are formed by carbon atoms. Carbocyclic structures include cycloalkyl, cycloalkenyl, cycloalkynyl, and certain aryl groups.

The term "carbonyl," as used herein, represents a -C(O)- group.

The term "carcinoma," as used herein, refers to a malignant new growth made up of epithelial cells tending to infiltrate the surrounding tissues and give rise to metastases.

The term "cyano," as used herein, represents -CN group.

The terms "CCNE1" and "cyclin E1," as used interchangeably herein, refer to G1/S specific cyclin E1 (Gene name: *CCNE1).* A cell overexpressing CCNE1 is one that exhibits a higher activity of CCNE1 than a cell normally expressing CCNE1. For example, a CCNE1-overexpressing cell is a cell that exhibits a copy number of at least 3 compared to a diploid normal cell with 2 copies. Thus, a cell exhibiting a copy number greater than 3 of CCNE1 is a cell overexpressing CCNE1. The CCNE1 overexpression may be measured by identifying the expression level of the gene product in a cell (e.g., CCNE1 mRNA transcript count or CCNE1 protein level).

The term "cycloalkenyl," as used herein, refers to a non-aromatic carbocyclic group having at least one double bond in the ring and from three to ten carbons (e.g., a C₃₋₁₀ cycloalkenyl), unless otherwise specified. Non-limiting examples of cycloalkenyl include cycloprop-1-enyl, cycloprop-2-enyl, cyclobut-1-enyl, cyclobut-1-enyl, cyclobut-2-enyl, cyclopent-1-enyl, cyclopent-2-enyl, cyclopent-3-enyl, norbornen-1-yl, norbornen-2-yl, norbornen-5-yl, and norbornen-7-yl. The cycloalkenyl group may be unsubstituted or substituted (e.g., optionally substituted cycloalkenyl) as described for cycloalkyl.

The term "cycloalkenyl alkyl," as used herein, represents an alkyl group substituted with a cycloalkenyl group, each as defined herein. The cycloalkenyl and alkyl portions may be substituted as the individual groups defined herein.

The term "cycloalkenylene," as used herein, represents a divalent cycloalkenyl group. An optionally substituted cycloalkenylene is a cycloalkenylene that is optionally substituted as described herein for cycloalkyl.

The term "cycloalkoxy," as used herein, represents a chemical substituent of formula -OR, where R is cycloalkyl group, unless otherwise specified. In some embodiments, the cycloalkyl group can be further substituted as defined herein.

The term "cycloalkyl," as used herein, refers to a cyclic alkyl group having from three to ten carbons (e.g., a C_{3-C10} cycloalkyl), unless otherwise specified. Cycloalkyl groups may be monocyclic or bicyclic. Bicyclic cycloalkyl groups may be of bicyclo[p.q.0]alkyl type, in which each of p and q is, independently, 1, 2, 3, 4, 5, 6, or 7, provided that the sum of p and q is 2, 3, 4, 5, 6, 7, or 8. Alternatively, bicyclic cycloalkyl groups may include bridged cycloalkyl structures, e.g., bicyclo[p.q.r]alkyl, in which r is 1, 2, or 3, each of p and q is, independently, 1, 2, 3, 4, 5, or 6, provided that the sum of p, q, and r is 3, 4, 5, 6, 7, or 8. The cycloalkyl group may be a spirocyclic group, e.g., spiro[p.q]alkyl, in which each of p and q is, independently, 2, 3, 4, 5, 6, or 7, provided that the sum of p and q is 4, 5, 6, 7, 8, or 9. Non-limiting examples of cycloalkyl include cyclopropyl, cyclobutyl, cyclopentyl, cyclohexyl, cycloheptyl, 1-bicyclo[2.2.1.]heptyl, 2-bicyclo[2.2.1.]heptyl, 5-bicyclo[2.2.1.]heptyl, 7-bicyclo[2.2.1.]heptyl, and decalinyl. The cycloalkyl group may be unsubstituted or substituted (e.g., optionally substituted cycloalkyl) with one, two, three, four, or five substituents independently selected from the group consisting of: alkyl; alkenyl; alkynyl; alkoxy; alkylsulfinyl; alkylsulfenyl; alkylsulfonyl; amino; aryl; aryloxy; azido; cycloalkyl; cycloalkoxy; cycloalkenyl; cycloalkynyl; halo; heteroalkyl; heterocyclyl; (heterocyclyl)oxy; heteroaryl; hydroxy; nitro; thiol; silyl; cyano; =O; =S; -SO₂R, where R is optionally substituted amino; =NR', where R' is H, alkyl, aryl, or heterocyclyl; and -CON(R^{A})₂, where each R^{A} is independently H or alkyl, or both R^{A}, together with the atom to which they are attached, combine to form heterocyclyl. Each of the substituents may itself be unsubstituted or substituted with unsubstituted substituent(s) defined herein for each respective group.

The term "cycloalkyl alkyl," as used herein, represents an alkyl group substituted with a cycloalkyl group, each as defined herein. The cycloalkyl and alkyl portions may be optionally substituted as the individual groups described herein.

The term "cycloalkylene," as used herein, represents a divalent cycloalkyl group. An optionally substituted cycloalkylene is a cycloalkylene that is optionally substituted as described herein for cycloalkyl.

The term "cycloalkynyl," as used herein, refers to a monovalent carbocyclic group having one or two carbon-carbon triple bonds and having from eight to twelve carbons, unless otherwise specified. Cycloalkynyl may include one transannular bond or bridge. Non-limiting examples of cycloalkynyl include cyclooctynyl, cyclononynyl, cyclodecynyl, and cyclodecadiynyl. The cycloalkynyl group may be unsubstituted or substituted (e.g., optionally substituted cycloalkynyl) as defined for cycloalkyl.

"Disease" or "condition" refer to a state of being or health status of a patient or subject capable of being treated with the compounds or methods provided herein.

The term "FBXW7," as used herein, refers to F-box/WD Repeat-Containing Protein 7 gene, transcript, or protein. An FBXW7-mutated gene, also described herein as an FBXW7 gene having an inactivating mutation, is one that fails to produce a functional FBXW7 protein or produces reduced quantities of FBXW7 protein in a cell.

The term "halo," as used herein, represents a halogen selected from bromine, chlorine, iodine, and fluorine.

The term "heteroalkyl," as used herein refers to an alkyl, alkenyl, or alkynyl group interrupted once by one or two heteroatoms; twice, each time, independently, by one or two heteroatoms; three times, each time, independently, by one or two heteroatoms; or four times, each time, independently, by one or two heteroatoms. Each heteroatom is, independently, O, N, or S. In some embodiments, the heteroatom is O or N. None of the heteroalkyl groups includes two contiguous oxygen or sulfur atoms. The heteroalkyl group may be unsubstituted or substituted (e.g., optionally substituted heteroalkyl). When heteroalkyl is substituted and the substituent is bonded to the heteroatom, the substituent is selected according to the nature and valency of the heteratom. Thus, the substituent bonded to the heteroatom, valency permitting, is selected from the group consisting of =O, -N(R^{N2})2, -SO2OR^{N3}, -SO₂R^{N2}, -SOR^{N3}, -COOR^{N3}, an N protecting group, alkyl, alkenyl, alkynyl, aryl, cycloalkyl, cycloalkenyl, cycloalkynyl, heterocyclyl, or cyano, where each R^{N2} is independently H, alkyl, cycloalkyl, cycloalkenyl, cycloalkynyl, aryl, or heterocyclyl, and each R^{N3} is independently alkyl, cycloalkyl, cycloalkenyl, cycloalkynyl, aryl, or heterocyclyl. Each of these substituents may itself be unsubstituted or substituted with unsubstituted substituent(s) defined herein for each respective group. When heteroalkyl is substituted and the substituent is bonded to carbon, the substituent is selected from those described for alkyl, provided that the substituent on the carbon atom bonded to the heteroatom is not Cl, Br, or I. It is understood that carbon atoms are found at the termini of a heteroalkyl group.

The term "heteroaryl alkyl," as used herein, represents an alkyl group substituted with a heteroaryl group, each as defined herein. The heteroaryl and alkyl portions may be optionally substituted as the individual groups described herein.

The term "heteroarylene," as used herein, represents a divalent heteroaryl. An optionally substituted heteroarylene is a heteroarylene that is optionally substituted as described herein for heteroaryl.

The term "heteroaryloxy," as used herein, refers to a structure -OR, in which R is heteroaryl. Heteroaryloxy can be optionally substituted as defined for heterocyclyl.

The term "heterocyclyl," as used herein, represents a monocyclic, bicyclic, tricyclic, or tetracyclic ring system having fused, bridging, and/or spiro 3-, 4-, 5-, 6-, 7-, or 8-membered rings, unless otherwise specified, containing one, two, three, or four heteroatoms independently selected from the group consisting of nitrogen, oxygen, and sulfur. In some embodiments, "heterocyclyl" is a monocyclic, bicyclic, tricyclic, or tetracyclic ring system having fused or bridging 5-, 6-, 7-, or 8-membered rings, unless otherwise specified, containing one, two, three, or four heteroatoms independently selected from the group consisting of nitrogen, oxygen, and sulfur. Heterocyclyl can be aromatic or non-aromatic. Non-aromatic 5-membered heterocyclyl has zero or one double bonds, non-aromatic 6- and 7-membered heterocyclyl groups have zero to two double bonds, and non-aromatic 8-membered heterocyclyl groups have zero to two double bonds and/or zero or one carbon-carbon triple bond. Heterocyclyl groups include from 1 to 16 carbon atoms unless otherwise specified. Certain heterocyclyl groups may include up to 9 carbon atoms. Non-aromatic heterocyclyl groups include pyrrolinyl, pyrrolidinyl, pyrazolinyl, pyrazolidinyl, imidazolinyl, imidazolidinyl, piperidinyl, homopiperidinyl, piperazinyl, pyridazinyl, oxazolidinyl, isoxazolidiniyl, morpholinyl, thiomorpholinyl, thiazolidinyl, isothiazolidinyl, thiazolidinyl, tetrahydrofuranyl, dihydrofuranyl, tetrahydrothienyl, dihydrothienyl, dihydroindolyl, tetrahydroquinolyl, tetrahydroisoquinolyl, pyranyl, dihydropyranyl, dithiazolyl, etc. If the heterocyclic ring system has at least one aromatic resonance structure or at least one aromatic tautomer, such structure is an aromatic heterocyclyl (i.e., heteroaryl). Non-limiting examples of heteroaryl groups include benzimidazolyl, benzofuryl, benzothiazolyl, benzothienyl, benzoxazolyl, furyl, imidazolyl, indolyl, isoindazolyl, isoquinolinyl, isothiazolyl, isothiazolyl, isoxazolyl, oxadiazolyl, oxazolyl, purinyl, pyrrolyl, pyridinyl, pyrazinyl, pyrimidinyl, qunazolinyl, quinolinyl, thiadiazolyl (e.g., 1,3,4-thiadiazole), thiazolyl, thienyl, triazolyl, tetrazolyl, etc. The term "heterocyclyl" also represents a heterocyclic compound having a bridged multicyclic structure in which one or more carbons and/or heteroatoms bridges two non-adjacent members of a monocyclic ring, e.g., quinuclidine, tropanes, or diaza-bicyclo[2.2.2]octane. The term "heterocyclyl" includes bicyclic, tricyclic, and tetracyclic groups in which any of the above heterocyclic rings is fused to one, two, or three carbocyclic rings, e.g., an aryl ring, a cyclohexane ring, a cyclohexene ring, a cyclopentane ring, a cyclopentene ring, or another monocyclic heterocyclic ring. Examples of fused heterocyclyls include 1,2,3,5,8,8a-hexahydroindolizine; 2,3-dihydrobenzofuran; 2,3-dihydroindole; and 2,3-dihydrobenzothiophene. The heterocyclyl group may be unsubstituted or substituted with one, two, three, four or five substituents independently selected from the group consisting of: alkyl; alkenyl; alkynyl; alkoxy; alkylsulfinyl; alkylsulfenyl; alkylsulfonyl; amino; aryl; aryloxy; azido; cycloalkyl; cycloalkoxy; cycloalkenyl; cycloalkynyl; halo; heteroalkyl; heterocyclyl; (heterocyclyl)oxy; hydroxy; nitro; thiol; silyl; cyano; -C(O)R or -SO₂R, where R is amino or alkyl; =O; =S; =NR', where R' is H, alkyl, aryl, or heterocyclyl. Each of the substituents may itself be unsubstituted or substituted with unsubstituted substituent(s) defined herein for each respective group.

The term "heterocyclyl alkyl," as used herein, represents an alkyl group substituted with a heterocyclyl group, each as defined herein. The heterocyclyl and alkyl portions may be optionally substituted as the individual groups described herein.

The term "heterocyclylene," as used herein, represents a divalent heterocyclyl. An optionally substituted heterocyclylene is a heterocyclylene that is optionally substituted as described herein for heterocyclyl.

The term "(heterocyclyl)oxy," as used herein, represents a chemical substituent of formula -OR, where R is a heterocyclyl group, unless otherwise specified. (Heterocyclyl)oxy can be optionally substituted in a manner described for heterocyclyl.

The terms "hydroxyl" and "hydroxy," as used interchangeably herein, represent an -OH group.

The term "isotopically enriched," as used herein, refers to the pharmaceutically active agent with the isotopic content for one isotope at a predetermined position within a molecule that is at least 100 times greater than the natural abundance of this isotope. For example, a composition that is isotopically enriched for deuterium includes an active agent with at least one hydrogen atom position having at least 100 times greater abundance of deuterium than the natural abundance of deuterium. Preferably, an isotopic enrichment for deuterium is at least 1000 times greater than the natural abundance of deuterium. More preferably, an isotopic enrichment for deuterium is at least 4000 times greater (e.g., at least 4750 times greater, e.g., up to 5000 times greater) than the natural abundance of deuterium.

The term "leukemia," as used herein, refers broadly to progressive, malignant diseases of the blood-forming organs and is generally characterized by a distorted proliferation and development of leukocytes and their precursors in the blood and bone marrow. Leukemia is generally clinically classified on the basis of (1) the duration and character of the disease-acute or chronic; (2) the type of cell involved; myeloid (myelogenous), lymphoid (lymphogenous), or monocytic; and (3) the increase or non-increase in the number abnormal cells in the blood-leukemic or aleukemic (subleukemic).

The term "lymphoma," as used herein, refers to a cancer arising from cells of immune origin.

The term "melanoma," as used herein, is taken to mean a tumor arising from the melanocytic system of the skin and other organs.

The term "Myt1," as used herein, refers to membrane-associated tyrosine and threonine-specific cdc2-inhibitory kinase (Myt1) (Gene name PKMYT1).

The term "Myt1 inhibitor," as used herein, represents a compound that upon contacting the enzyme Myt1, whether in vitro, in cell culture, or in an animal, reduces the activity of Myt1, such that the measured Myt1 IC₅₀ is 10 µM or less (e.g., 5 µM or less or 1 µM or less). For certain Myt1 inhibitors, the Myt1 IC₅₀ may be 100 nM or less (e.g., 10 nM or less, or 3 nM or less) and could be as low as 100 pM or 10 pM. Preferably, the Myt1 IC₅₀ is 1 nM to 1 µM (e.g., 1 nM to 750 nM, 1 nM to 500 nM, or 1 nM to 250 nM). Even more preferably, the Myt1 IC₅₀ is less than 20 nm (e.g., 1 nM to 20 nM).

The term "nitro," as used herein, represents an -NO₂ group.

The term "oxo," as used herein, represents a divalent oxygen atom (e.g., the structure of oxo may be shown as =O).

The term "Ph," as used herein, represents phenyl.

The term "pharmaceutical composition," as used herein, represents a composition containing a compound described herein, formulated with a pharmaceutically acceptable excipient, and manufactured or sold with the approval of a governmental regulatory agency as part of a therapeutic regimen for the treatment of disease in a mammal. Pharmaceutical compositions can be formulated, for example, for oral administration in unit dosage form (e.g., a tablet, capsule, caplet, gelcap, or syrup); for topical administration (e.g., as a cream, gel, lotion, or ointment); for intravenous administration (e.g., as a sterile solution free of particulate emboli and in a solvent system suitable for intravenous use); or in any other formulation described herein.

The term "pharmaceutically acceptable excipient" or "pharmaceutically acceptable carrier," as used interchangeably herein, refers to any ingredient other than the compounds described herein (e.g., a vehicle capable of suspending or dissolving the active compound) and having the properties of being nontoxic and non-inflammatory in a patient. Excipients may include, for example: antiadherents, antioxidants, binders, coatings, compression aids, disintegrants, dyes (colors), emollients, emulsifiers, fillers (diluents), film formers or coatings, flavors, fragrances, glidants (flow enhancers), lubricants, preservatives, printing inks, sorbents, suspending or dispersing agents, sweeteners, or waters of hydration. Exemplary excipients include, but are not limited to: butylated hydroxytoluene (BHT), calcium carbonate, calcium phosphate (dibasic), calcium stearate, croscarmellose, crosslinked polyvinyl pyrrolidone, citric acid, crospovidone, cysteine, ethylcellulose, gelatin, hydroxypropyl cellulose, hydroxypropyl methylcellulose, lactose, magnesium stearate, maltitol, mannitol, methionine, methylcellulose, methyl paraben, microcrystalline cellulose, polyethylene glycol, polyvinyl pyrrolidone, povidone, pregelatinized starch, propyl paraben, retinyl palmitate, shellac, silicon dioxide, sodium carboxymethyl cellulose, sodium citrate, sodium starch glycolate, sorbitol, starch (corn), stearic acid, stearic acid, sucrose, talc, titanium dioxide, vitamin A, vitamin E, vitamin C, and xylitol.

The term "pharmaceutically acceptable salt," as use herein, represents those salts which are, within the scope of sound medical judgment, suitable for use in contact with the tissues of humans and animals without undue toxicity, irritation, allergic response and the like and are commensurate with a reasonable benefit/risk ratio. Pharmaceutically acceptable salts are well known in the art. For example, pharmaceutically acceptable salts are described in: Berge et al., J. Pharmaceutical Sciences 66:1-19, 1977 and in Pharmaceutical Salts: Properties, Selection, and Use, (Eds. P.H. Stahl and C.G. Wermuth), Wiley-VCH, 2008. The salts can be prepared in situ during the final isolation and purification of the compounds described herein or separately by reacting the free base group with a suitable organic acid. Representative acid addition salts include acetate, adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, fumarate, glucoheptonate, glycerophosphate, hemisulfate, heptonate, hexanoate, hydrobromide, hydrochloride, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, toluenesulfonate, undecanoate, valerate salts, and the like. Representative alkali or alkaline earth metal salts include sodium, lithium, potassium, calcium, magnesium, and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like.

The term "pre-malignant" or "pre-cancerous," as used herein, refers to a condition that is not malignant but is poised to become malignant.

The term "protecting group," as used herein, represents a group intended to protect a hydroxy, an amino, or a carbonyl from participating in one or more undesirable reactions during chemical synthesis. The term "O-protecting group," as used herein, represents a group intended to protect a hydroxy or carbonyl group from participating in one or more undesirable reactions during chemical synthesis. The term "N-protecting group," as used herein, represents a group intended to protect a nitrogen containing (e.g., an amino, amido, heterocyclic N-H, or hydrazine) group from participating in one or more undesirable reactions during chemical synthesis. Commonly used O- and N-protecting groups are disclosed in Greene, "Protective Groups in Organic Synthesis," 3rd Edition (John Wiley & Sons, New York, 1999).

Exemplary O- and N-protecting groups include alkanoyl, aryloyl, or carbamy groups such as formyl, acetyl, propionyl, pivaloyl, t-butylacetyl, 2-chloroacetyl, 2-bromoacetyl, trifluoroacetyl, trichloroacetyl, phthalyl, o-nitrophenoxyacetyl, α-chlorobutyryl, benzoyl, 4-chlorobenzoyl, 4-bromobenzoyl, t-butyldimethylsilyl, tri-iso-propylsilyloxymethyl, 4,4'-dimethoxytrityl, isobutyryl, phenoxyacetyl, 4-isopropylpehenoxyacetyl, dimethylformamidino, and 4-nitrobenzoyl.

Exemplary O-protecting groups for protecting carbonyl containing groups include, but are not limited to: acetals, acylals, 1,3-dithianes, 1,3-dioxanes, 1,3-dioxolanes, and 1,3-dithiolanes.

Other O-protecting groups include, but are not limited to: substituted alkyl, aryl, and aryl-alkyl ethers (e.g., trityl; methylthiomethyl; methoxymethyl; benzyloxymethyl; siloxymethyl; 2,2,2,-trichloroethoxymethyl; tetrahydropyranyl; tetrahydrofuranyl; ethoxyethyl; 1-[2-(trimethylsilyl)ethoxy]ethyl; 2-trimethylsilylethyl; t-butyl ether; p-chlorophenyl, p-methoxyphenyl, p-nitrophenyl, benzyl, p-methoxybenzyl, and nitrobenzyl); silyl ethers (e.g., trimethylsilyl; triethylsilyl; triisopropylsilyl; dimethylisopropylsilyl; t-butyldimethylsilyl; t-butyldiphenylsilyl; tribenzylsilyl; triphenylsilyl; and diphenymethylsilyl); carbonates (e.g., methyl, methoxymethyl, 9-fluorenylmethyl; ethyl; 2,2,2-trichloroethyl; 2-(trimethylsilyl)ethyl; vinyl, allyl, nitrophenyl; benzyl; methoxybenzyl; 3,4-dimethoxybenzyl; and nitrobenzyl).

Other N-protecting groups include, but are not limited to, chiral auxiliaries such as protected or unprotected D, L or D, L-amino acids such as alanine, leucine, phenylalanine, and the like; sulfonyl-containing groups such as benzenesulfonyl, p-toluenesulfonyl, and the like; carbamate forming groups such as benzyloxycarbonyl, p-chlorobenzyloxycarbonyl, p methoxybenzyloxycarbonyl, p-nitrobenzyloxycarbonyl, 2-nitrobenzyloxycarbonyl, p bromobenzyloxycarbonyl, 3,4-dimethoxybenzyloxycarbonyl, 3,5 dimethoxybenzyl oxycarbonyl, 2,4-dimethoxybenzyloxycarbonyl, 4 methoxybenzyloxycarbonyl, 2-nitro-4,5-dimethoxybenzyloxycarbonyl, 3,4,5 trimethoxybenzyloxycarbonyl, 1-(p-biphenylyl)-1-methylethoxycarbonyl, α,α-dimethyl-3,5 dimethoxybenzyloxycarbonyl, benzhydryloxy carbonyl, t-butyloxycarbonyl, diisopropylmethoxycarbonyl, isopropyloxycarbonyl, ethoxycarbonyl, methoxycarbonyl, allyloxycarbonyl, 2,2,2,-trichloroethoxycarbonyl, phenoxycarbonyl, 4-nitrophenoxy carbonyl, fluorenyl-9-methoxycarbonyl, cyclopentyloxycarbonyl, adamantyloxycarbonyl, cyclohexyloxycarbonyl, phenylthiocarbonyl, and the like, aryl-alkyl groups such as benzyl, p-methoxybenzyl, 2,4-dimethoxybenzyl, triphenylmethyl, benzyloxymethyl, and the like, silylalkylacetal groups such as [2-(trimethylsilyl)ethoxy]methyl and silyl groups such as trimethylsilyl, and the like. Useful N-protecting groups are formyl, acetyl, benzoyl, pivaloyl, t-butylacetyl, alanyl, phenylsulfonyl, benzyl, dimethoxybenzyl, [2-(trimethylsilyl)ethoxy]methyl (SEM), tetrahydropyranyl (THP), t-butyloxycarbonyl (Boc), and benzyloxycarbonyl (Cbz).

The term "tautomer" refers to structural isomers that readily interconvert, often by relocation of a proton. Tautomers are distinct chemical species that can be identified by differing spectroscopic characteristics, but generally cannot be isolated individually. Non-limiting examples of tautomers include ketone - enol, enamine - imine, amide - imidic acid, nitroso - oxime, ketene - ynol, and amino acid - ammonium carboxylate.

The term "sarcoma" generally refers to a tumor which is made up of a substance like the embryonic connective tissue and is generally composed of closely packed cells embedded in a fibrillar or homogeneous substance.

The term "subject," as used herein, represents a human or non-human animal (e.g., a mammal) that is suffering from, or is at risk of, disease or condition, as determined by a qualified professional (e.g., a doctor or a nurse practitioner) with or without known in the art laboratory test(s) of sample(s) from the subject. Preferably, the subject is a human. Non-limiting examples of diseases and conditions include diseases having the symptom of cell hyperproliferation, e.g., a cancer.

"Treatment" and "treating," as used herein, refer to the medical management of a subject with the intent to improve, ameliorate, stabilize, prevent or cure a disease or condition. This term includes active treatment (treatment directed to improve the disease or condition); causal treatment (treatment directed to the cause of the associated disease or condition); palliative treatment (treatment designed for the relief of symptoms of the disease or condition); preventative treatment (treatment directed to minimizing or partially or completely inhibiting the development of the associated disease or condition); and supportive treatment (treatment employed to supplement another therapy).

### BRIEF DESCRIPTION OF THE DRAWINGS

FIG. 1A is a bar graph showing the CCNE1 amplifiction/overexpression across tumors sequenced from TCGA PanCancer Atlas.
FIG. 1B is a scatter plot showing the CCNE1 gene expression data from TCGA PanCancer Atlas.
FIG. 2A is a bar graph showing the FBXW7 mutations across tumors sequenced fromTCGA PanCancer Atlas.
FIG. 2B is a lollipop graph showing the frequency of FBXW7 mutations across the gene. This graph highlights three common arginine hotspot mutations (R465, R479, and R505) within the third and fourth WD40 repeats that disrupt recognition of the Cyclin E1 substrate and are classified as deleterious.
FIG. 3A is a bar graph showing the results of a proliferation assay using RPE1-hTERT Cas9 TP53-/- and CCNE1-overexpressing clones treated with different doses of compound 133.
FIG. 3B is a series of images depicting the results of a clonogenic survival assay using RPE1-hTERT Cas9 TP53-/- and CCNE1-overexpressing clones transduced with PKMYT1 sgRNAs. Infected cells were plated at low density to measure their ability to form colonies of >50 cells. After 10 days of growth, the colonies were stained, imaged, and quantified. The results are normalized to the survival of RPE1-hTERT Cas9 TP53-/- parental and CCNE1-overexpressing clones transduced with a non-targeting LacZ control sgRNA.
FIG. 3C is a line graph showing the results of a proliferation assay using RPE1-hTERT Cas9 TP54-/- and CCNE1-overexpressing clones treated with different doses of compound 133.
FIG. 4A is a bar graph showing the results of a clonogenic survival assay using FT282-hTERT TP53^{R175H} and CCNE1-overexpressing cells transduced with PKMYT1 sgRNAs. Infected cells were plated at low density to measure their ability to form colonies of >50 cells. After 10 days of growth, the colonies were stained, imaged, and quantified. The results are normalized to the survival of FT282-hTERT TP53^{R175H} and CCNE1-overexpressing cells transduced with an AAVS1 control sgRNA.
FIG. 4B is a series of images showing of stained colonies described in FIG. 4A.
FIG. 4C is a line graph showing the results of a proliferation assay using FT282-hTERT TP53^{R175H} and CCNE1-overexpressing clones treated with different doses of compound 133.

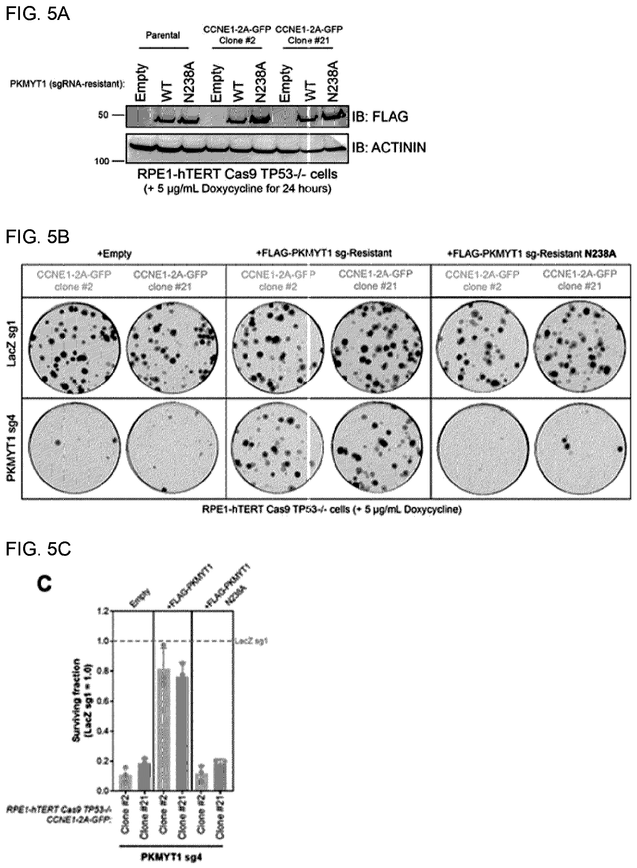

FIGS. 5A, 5B, and 5C show the results of clonogenic survival assays for stable RPE1-hTERT Cas9 TP53-/- parental and CCNE1-overexpressing clones expressing either a wild type or catalytic-dead FLAG-tagged PKMYT1 sgRNA-resistant ORF. These stable cell lines were transduced with either a LacZ non-targeting sgRNA or PKMYT1 sgRNA #4 and plated at low density to measure their ability to form colonies of >50 cells. After 10 days of growth, the colonies were stained, imaged, and quantified. The results are normalized to the survival of RPE1-hTERT Cas9 TP53-/- CCNE1-overexpressing clones transduced with a non-targeting LacZ control sgRNA and represented as a bar graph in FIG. 5C. Both clones 2 and 21 behave similarly in this study.
FIG. 6 is a chart showing the results of proliferation assays for a panel of CCNE1 wild type and CCNE1-amplified/overexpressing cancer cell lines treated with different doses of compound 28. The IC₅₀ values are plotted for each cell line and demonstrate that CCNE1-overexpressing cell lines show enhanced sensitivity to a Myt1 inhibitor compared to CCNE1 WT cell lines.
FIG. 7 is a chart showing the results of proliferation assays for a panel of FBXW7 wild type and FBXW7-mutated cancer cell lines treated with different doses of compound 95. The IC50 values are plotted for each cell line and demonstrate that FBXW7- mutated cell lines show enhanced sensitivity to a Myt1 inhibitor compared to FBXW7 WT cell lines.

### DETAILED DESCRIPTION

In general, the invention provides compounds, pharmaceutical compositions containing the same, methods of preparing the compounds, and methods of use. Compounds of the invention may be Myt1 inhibitors. These compounds may be used to inhibit Myt1 in a cell, e.g., a cell in a subject (e.g., a cell overexpressing *CCNE1* or having an inactivating mutation in the *FBXW7* gene). The subject may be in need of a treatment for a disease or condition, e.g., a disease or condition having a symptom of cell hyperproliferation, e.g., a cancer. The Myt1 inhibitory activity of the compounds disclosed herein is useful for treating a subject in need of a treatment for cancer.

Myt1 is a cell cycle regulating kinase localized predominantly in the endoplasmic reticulum and golgi complex. It is part of the Wee family of kinases that includes Wee1 and Wee1b. It is involved in the negative regulation of the CDK1-Cyclin B complex which promotes the progression of cells from G2-phase into the mitotic phase (M-phase) of the cell cycle. During DNA damage, Myt1 drives the phosphorylation on CDK1 (both Tyr15 and Thr14 of CDK1) which maintains the kinase complex in an inactive state in G2 as part of the G2 checkpoint response along with Wee1 (which mediates only Tyr15 phosphorylation) and prevents entry into mitosis until the damage has been repaired. Additionally, it has been proposed that Myt1 directly interacts with CDK1 complexes in the cytoplasm and prevents their nuclear translocation thus inhibiting cell cycle progression.

Myt1 has been implicated as a potentially important cancer target as it is essential in many cancer cells. Overexpression of Myt1 has been observed in various cancers including hepatocellular carcinoma as well as clear-cell renal-cell carcinoma. Myt1 downregulation has a minor role in unperturbed cells but has a more prominent role in cells exposed to DNA damage. Additionally, cells that exhibit high levels of replication stress in addition to defective G1 checkpoint regulation may be particularly sensitive to loss of Myt1 function, as these cells will be prone to entering mitosis prematurely with compromised genomic material leading to mitotic catastrophe.

Inhibitors of Myt1, a regulator of G2-M transition, may be particularly useful in the treatment of tumors harboring CCNE1-amplification or *FBXW7* loss-of-function mutations using a synthetic lethal therapeutic strategy.

Cyclin E1 (encoded by the *CCNE1* gene) is involved in the G1 to S phase cell cycle transition. In late G1 phase of the cell cycle, it complexes with cyclin-dependent kinase 2 (CDK2) to promote E2F transcription factor activation and entry into S-phase. Cyclin E1 levels are tightly regulated during normal cell cycles, accumulating at the G1/S transition and being completely degraded by the end of S phase. The cell cycle-dependent proteasomal degradation of Cyclin E1 is mediated by the SCF^{FBW7} ubiquitin ligase complex. Once activated in late G1, the Cyclin E1/CDK2 complex promotes the transition into S phase through phosphorylation and inactivation of RB1 and subsequent release of E2F transcription factors. S phase is promoted by E2F-mediated transcription of numerous genes involved in DNA replication including the pre-replication complex subunits ORC1, CDC6, CDT1, and the MCM helicase factors.

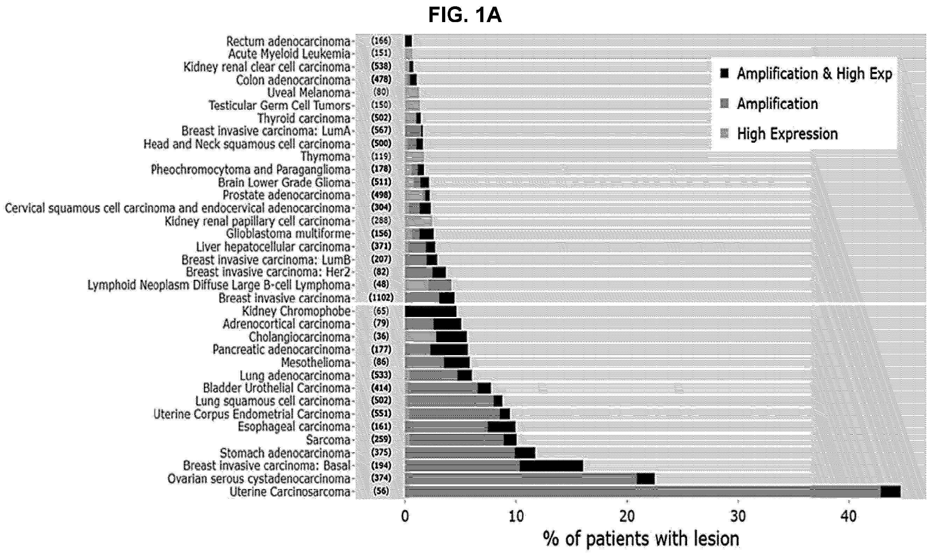

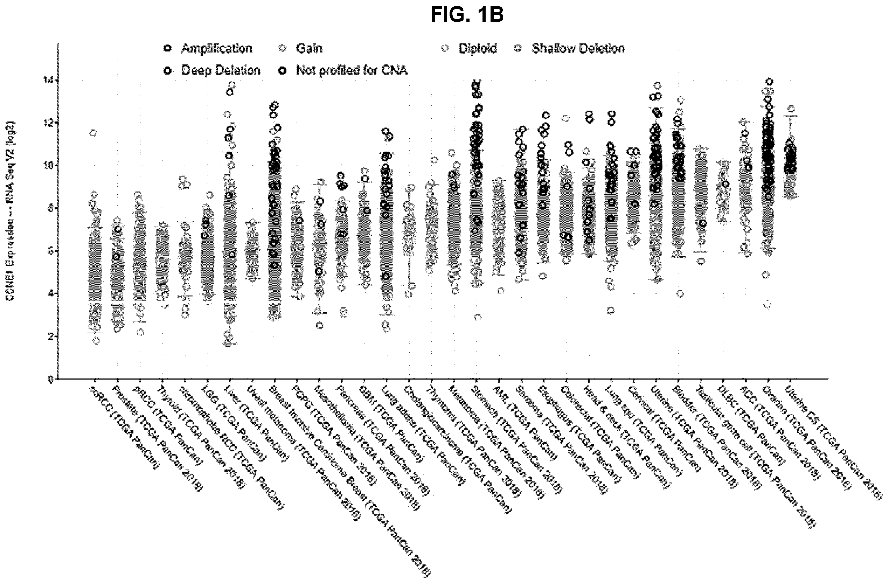

CCNE1 is frequently amplified and/or over-expressed in human cancers (FIG. 1). CCNE1 amplification has been reported in several cancer types including endometrial, ovarian, breast and gastric, ranging in frequency from 5-40%. Importantly, numerous studies have confirmed Cyclin E1 as a driver of tumorigenesis in these indications and CCNE1 amplification is observed in the more aggressive subtypes including uterine carcinosarcoma (UCS; ~40%), uterine serous carcinoma (USC; ~25%), high-grade serous ovarian carcinoma (HGSOC; ~25%), and triple-negative breast cancer (TNBC; ~8%). Patients with evidence of Cyclin E1 over-expression in tumor biopsies by immunohistochemistry and/or genomic copy number analysis have a lower overall survival compared to patients with normal Cyclin E1 levels. HGSOC patients with Cyclin E1 over-expression have a lower response rate to cisplatin, the current standard of care.

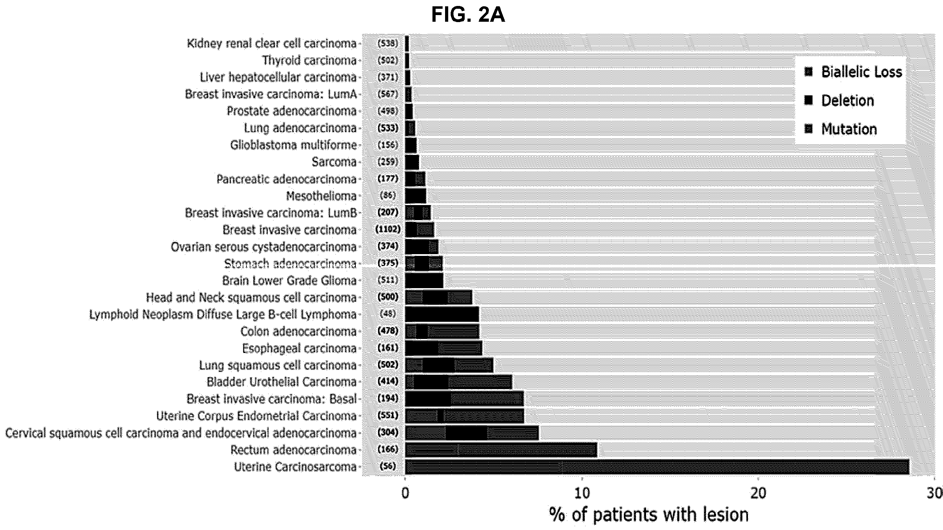

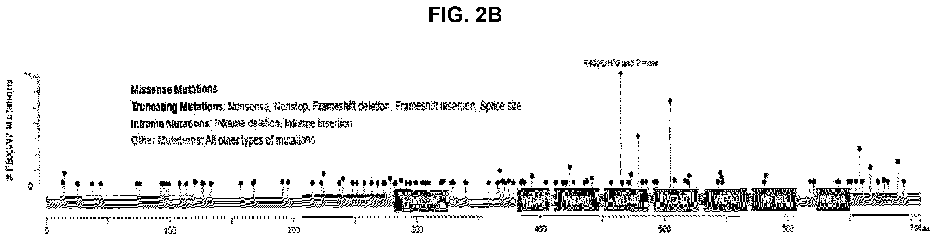

Defective cell cycle-regulated proteolysis of Cyclin E1 by the SCF^{FBW7} ubiquitin ligase complex is another mechanism of *CCNE1* over-expression observed in tumors. The F-box protein gene, *FBXW7,* is frequently mutated in several cancer types including endometrial, colorectal, and gastric, ranging in frequency from 5-35% (FIG. 2). Like *CCNE1, FBXW7* driver mutations are observed in the more aggressive subtypes of endometrial cancer including UCS (~35%) and USC *(~25%). FBXW7* has a diverse spectrum of loss-of-function mutations in cancer including truncating mutations peppered across the gene and missense mutations within the Cyclin E1 recognizing WD40 repeats. FBW7 functions as a homodimer within the SCF complex and many deleterious missense mutations within the WD40 repeats are mostly heterozygous and dominant negative. Remarkably, several recurring hotspot missense mutations are found in the WD40 repeats including R465, R479, and R505 - all of which disrupt Cyclin E1 binding and ubiquitylation.

Cyclin E1 over-expression and/or *FBXW7* loss-of-function is thought to drive tumorigenesis by inducing genome instability (e.g., increased origin firing, defective nucleotide pools, transcription-replication conflicts, and/or fork instability). Over-expression of Cyclin E1 has been shown to induce replication stress characterized by slowed or stalled replication forks and loss-of-heterozygosity at fragile sites. The primary mechanism by which Cyclin E1 over-expression causes replication stress is increased origin firing in early S-phase followed by depletion of replication factors including nucleotide pools. The decrease in overall replication proteins and nucleotides decreases fork progression and causes stalling and subsequent collapse or reversal.

The compound of the invention may be, e.g., a compound of formula (I): 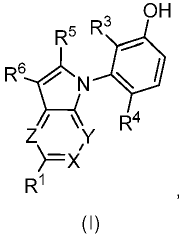 or a pharmaceutically acceptable salt thereof,
where
each of X, Y, and Z is independently N or CR²;
R¹ and each R² are independently hydrogen, optionally substituted C₁₋₆ alkyl, optionally substituted C₂₋₆ alkenyl, optionally substituted C₂₋₆ alkynyl, optionally substituted C₃₋₈ cycloalkyl, optionally substituted C₃₋₈ cycloalkenyl, optionally substituted C₂₋₉ heterocyclyl, optionally substituted C₂₋₉ heterocyclyl C₁₋₆ alkyl, optionally substituted C₆₋₁₀ aryl, optionally substituted C₁₋₉ heteroaryl, optionally substituted C₁₋₉ heteroaryl C₁₋₆ alkyl, halogen, -N(R⁷)₂, -OR⁷, -C(O)N(R⁸)₂, - SO₂N(R⁸)₂, -SO₂R^{7A}, or -Q-R^{7B}; or R¹ combines with one R² that is *vicinal* to R¹ to form an optionally substituted C₃₋₆ alkylene;
each of R³ and R⁴ is independently optionally substituted C₁₋₆ alkyl or halogen;
R⁵ is H or -N(R⁷)₂;
R⁶ is -C(O)NH(R⁸), -C(O)R^{7A}, or -SO₂R^{7A};
each R⁷ is independently hydrogen, optionally substituted C₁₋₆ alkyl, optionally substituted C₆₋₁₀ aryl C₁₋₆ alkyl, optionally substituted C₆₋₁₀ aryl, optionally substituted C₁₋₉ heteroaryl, optionally substituted C₁₋₉ heteroaryl C₁₋₆ alkyl, or -SO₂R^{7A}; or two R⁷ groups, together with the atom to which both are attached, combine to form an optionally substituted C₂₋₉ heterocyclyl;
**each** R^{7A} is independently optionally substituted C₁₋₆ alkyl, optionally substituted C₃₋₈ cycloalkyl, or optionally substituted C₆₋₁₀ aryl;
each R^{7B} is independently hydroxyl, optionally substituted C₁₋₆ alkyl, optionally substituted C₆₋₁₀ aryl, optionally substituted C₂₋₉ heterocyclyl, optionally substituted C₁₋₉ heteroaryl, -N(R⁷)₂, - C(O)N(R⁸)₂, -SO₂N(R⁸)₂, -SO₂R^{7A}, or optionally substituted alkoxy;
each R⁸ is independently hydrogen, optionally substituted C₁₋₆ alkyl, optionally substituted C₂₋₆ alkoxyalkyl, optionally substituted C₆₋₁₀ aryl C₁₋₆ alkyl, optionally substituted C₆₋₁₀ aryl, optionally substituted C₃₋₈ cycloalkyl, or optionally substituted C₁₋₉ heteroaryl; or two R⁸, together with the atom to which they are attached, combine to form an optionally substituted C₂₋₉ heterocyclyl;
Q is optionally substituted C₁₋₆ alkylene, optionally substituted C₂₋₆ alkenylene, optionally substituted C₂₋₆ alkynylene, optionally substituted C₃₋₈ cycloalkylene, optionally substituted C₃₋₈ cycloalkenylene optionally substituted C₆₋₁₀ arylene, optionally substituted C₂₋₉ heterocyclylene, or optionally substituted C₁₋₉ heteroarylene.

Preferably, the compound of formula (I) is enriched for the atropisomer of formula (IA): 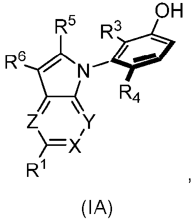 where all variables are as described herein.

The compound of the invention may be, e.g., a compound of formula (II): 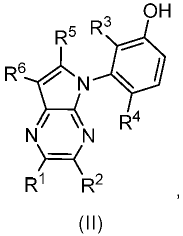 where all variables are as described herein.

Preferably, the compound of formula (II) is enriched for the atropisomer of of formula (IIA): 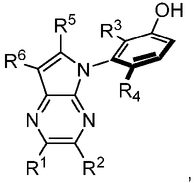 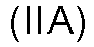 where all variables are as described herein.

The compound of the invention may be, e.g., a compound of formula (III): 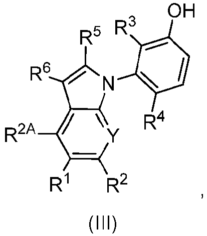 where R^{2A} is hydrogen, optionally substituted C₁₋₆ alkyl, optionally substituted C₂₋₆ alkenyl, optionally substituted C₂₋₆ alkynyl, optionally substituted C₃₋₈ cycloalkyl, optionally substituted C₃₋₈ cycloalkenyl, optionally substituted C₂₋₉ heterocyclyl, optionally substituted C₂₋₉ heterocyclyl C₁₋₆ alkyl, optionally substituted C₆₋₁₀ aryl, optionally substituted C₁₋₉ heteroaryl, optionally substituted C₁₋₉ heteroaryl C₁₋₆ alkyl, halogen, -N(R⁷)₂, -OR⁷, -C(O)N(R⁸)₂, -SO₂N(R⁸)₂, -SO₂R^{7A}, or -Q-R^{7B}.

Preferably, the compound of formula (III) is enriched for the atropisomer of formula (IIIA):

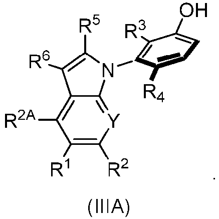

The compound of the invention may be, e.g., a compound listed in Table 1 below or a pharmaceutically acceptable salt thereof.

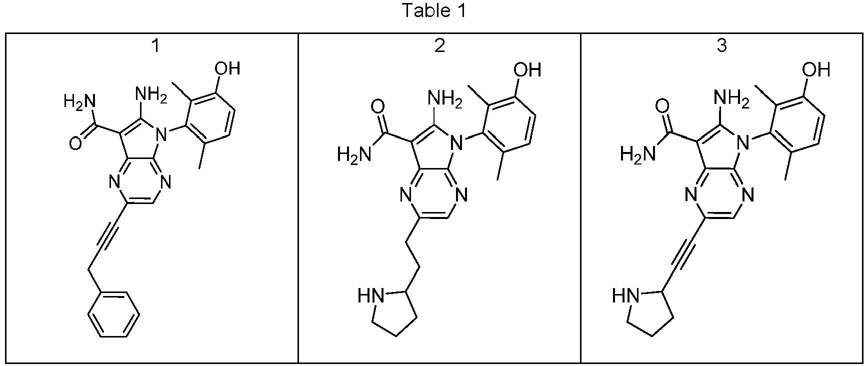

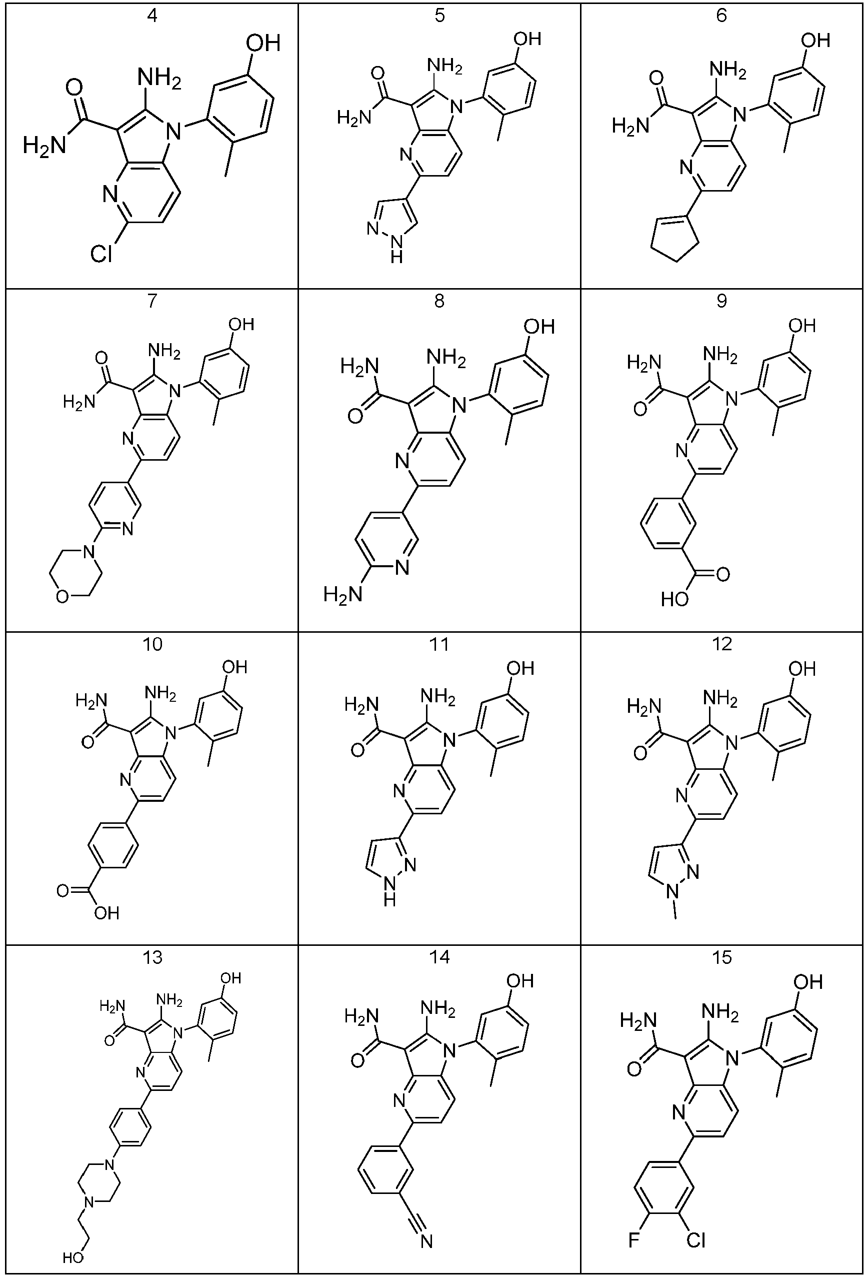

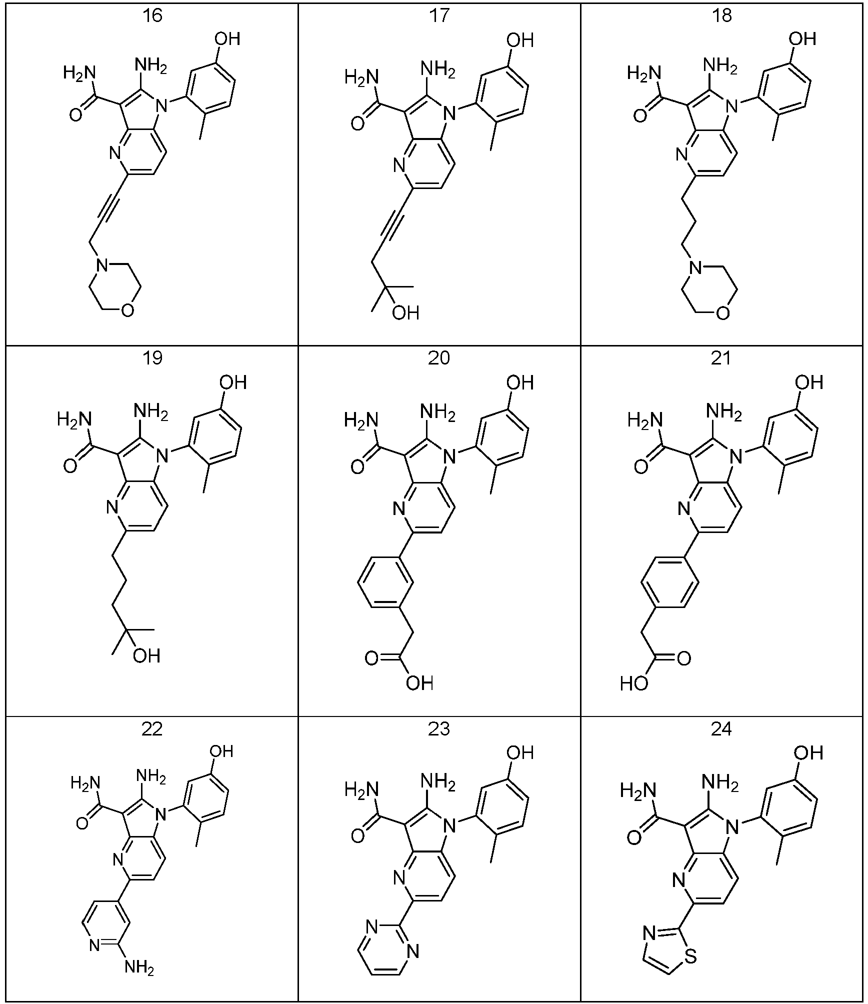

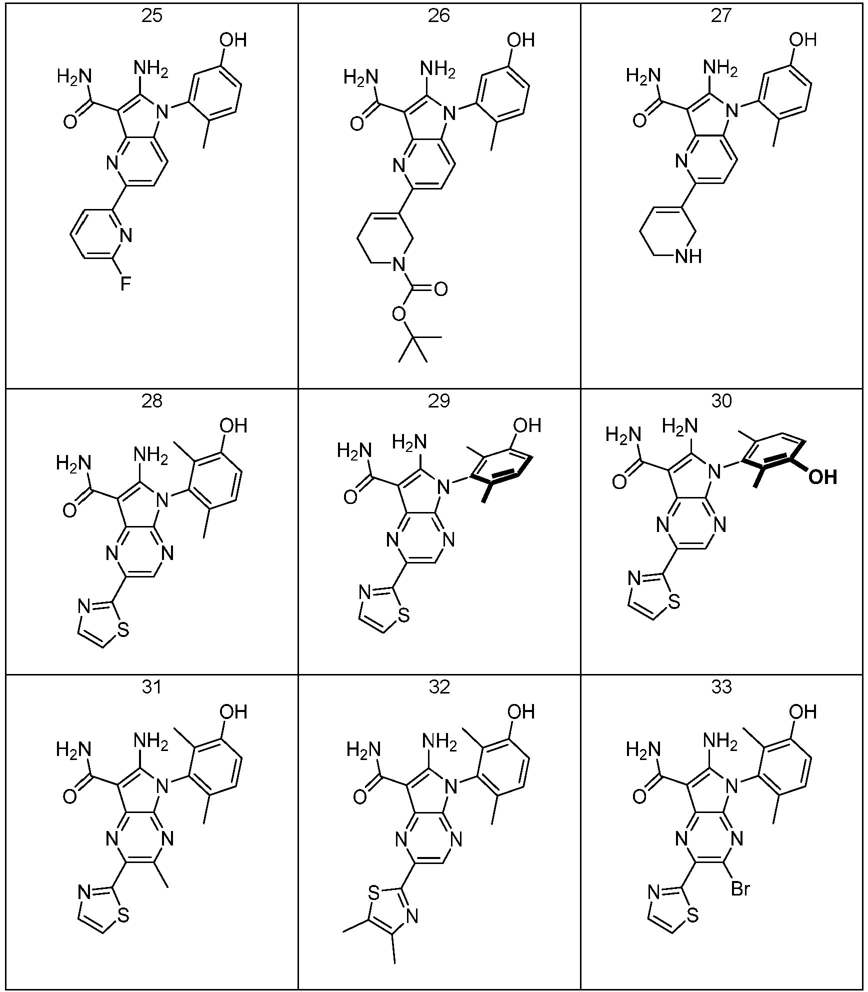

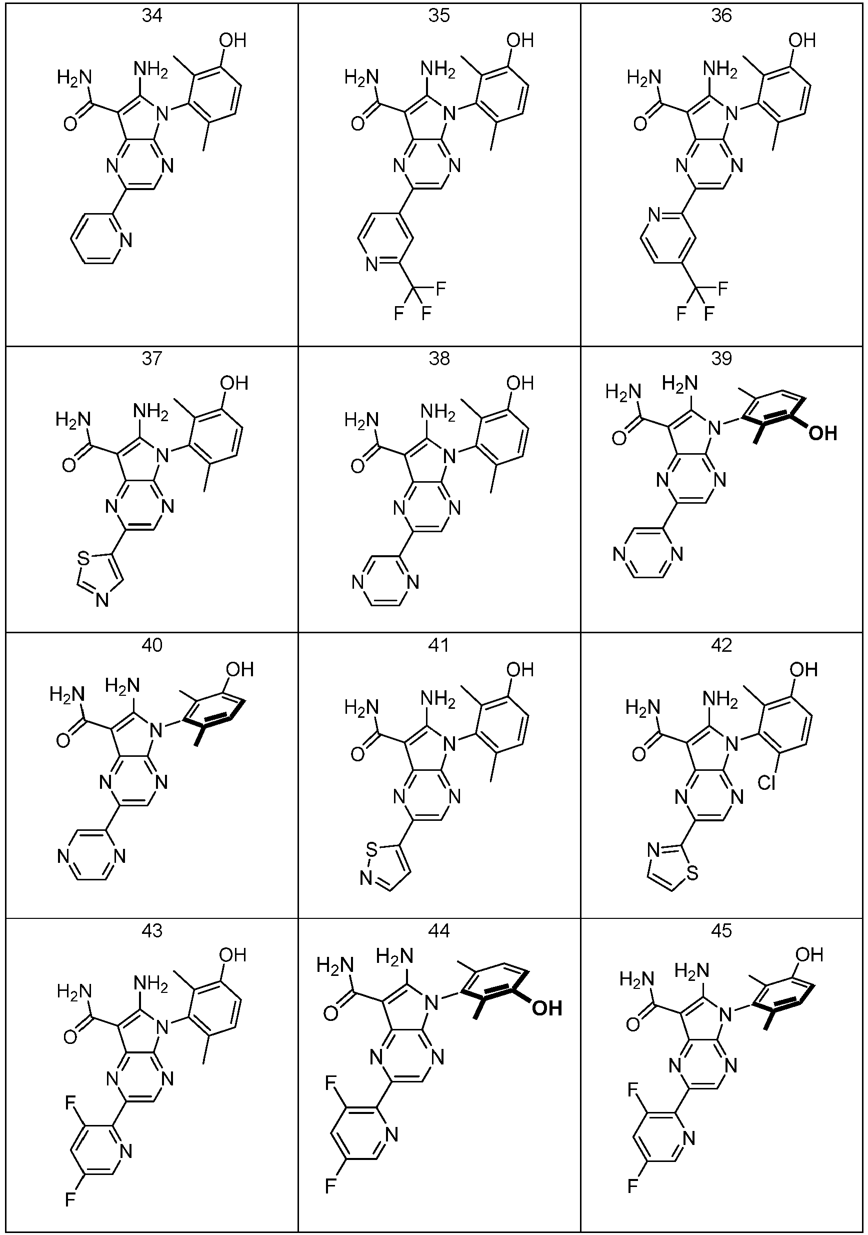

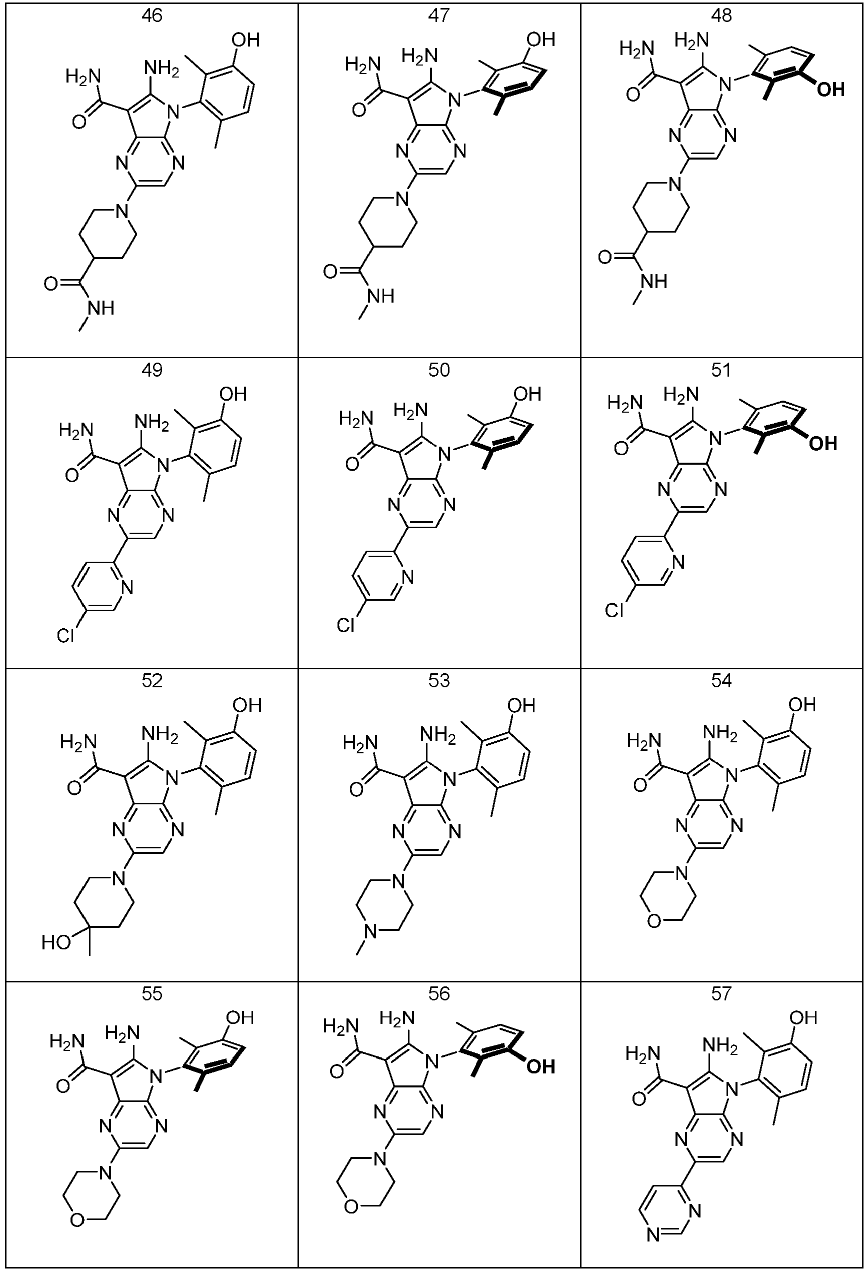

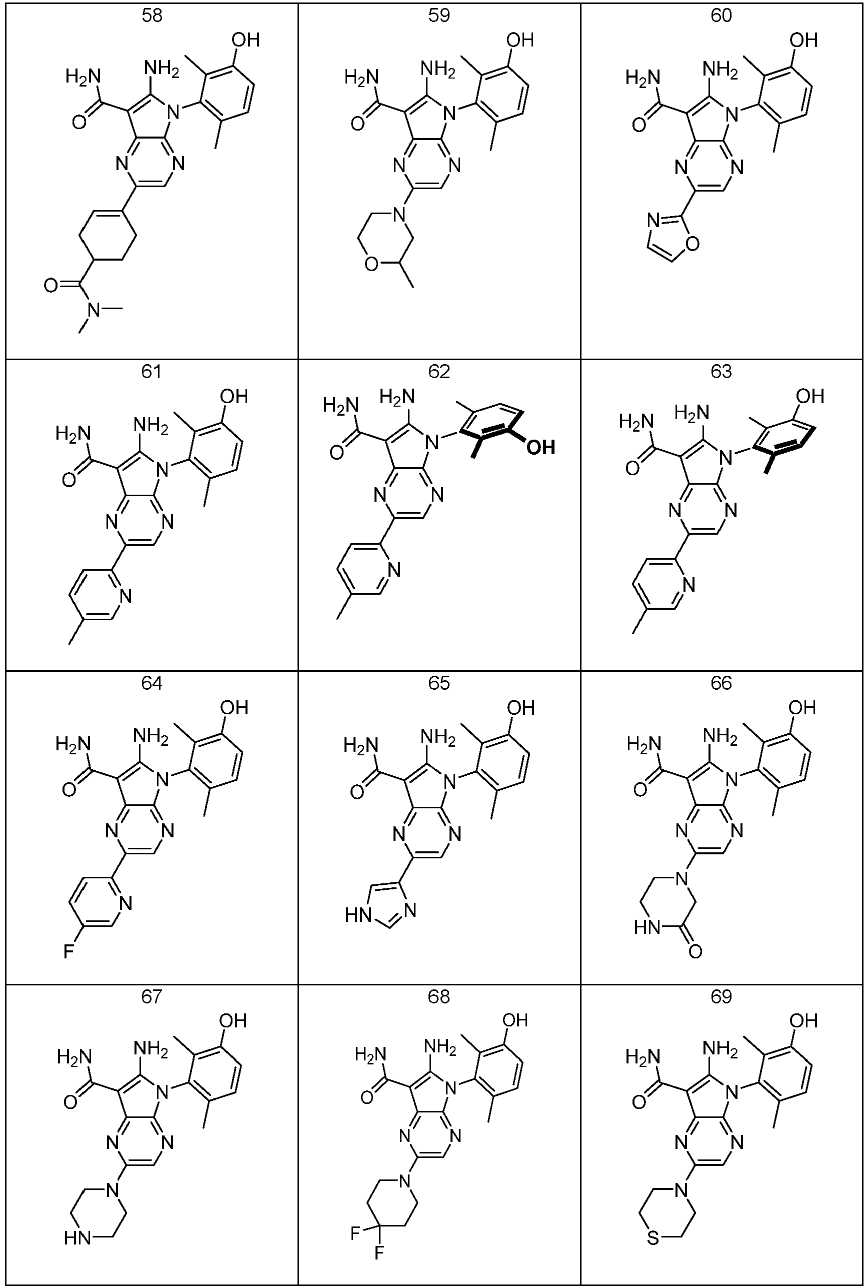

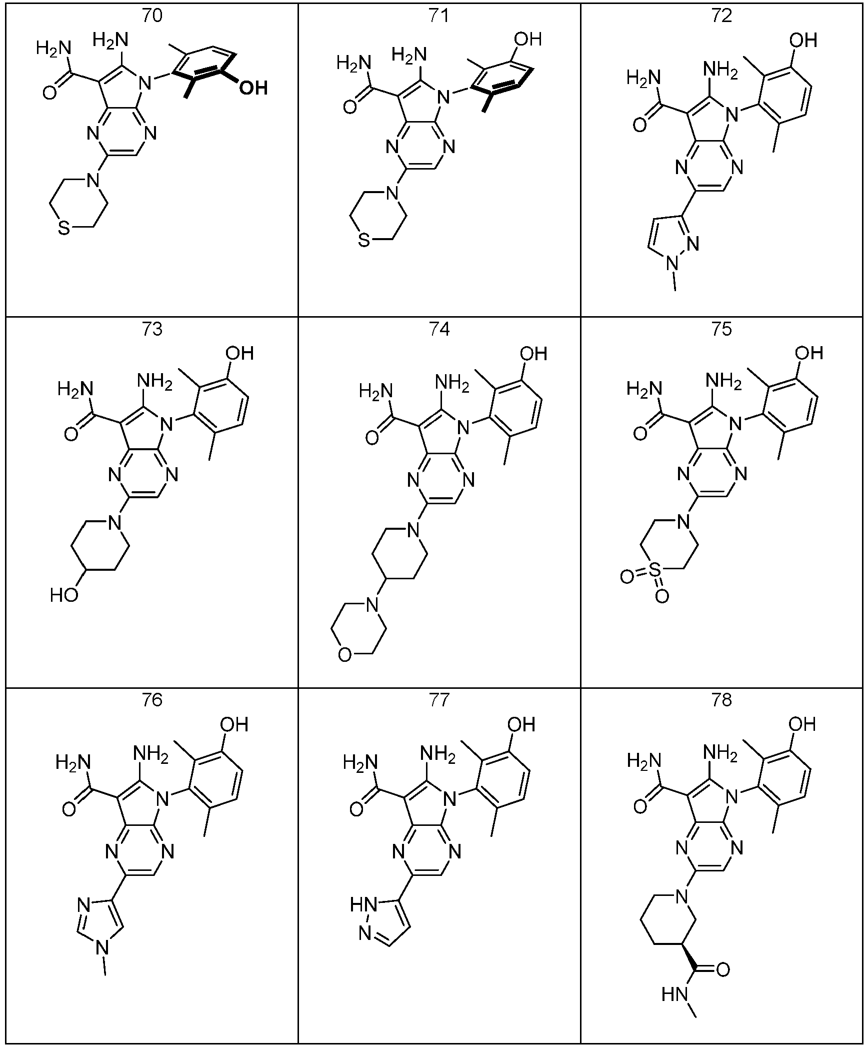

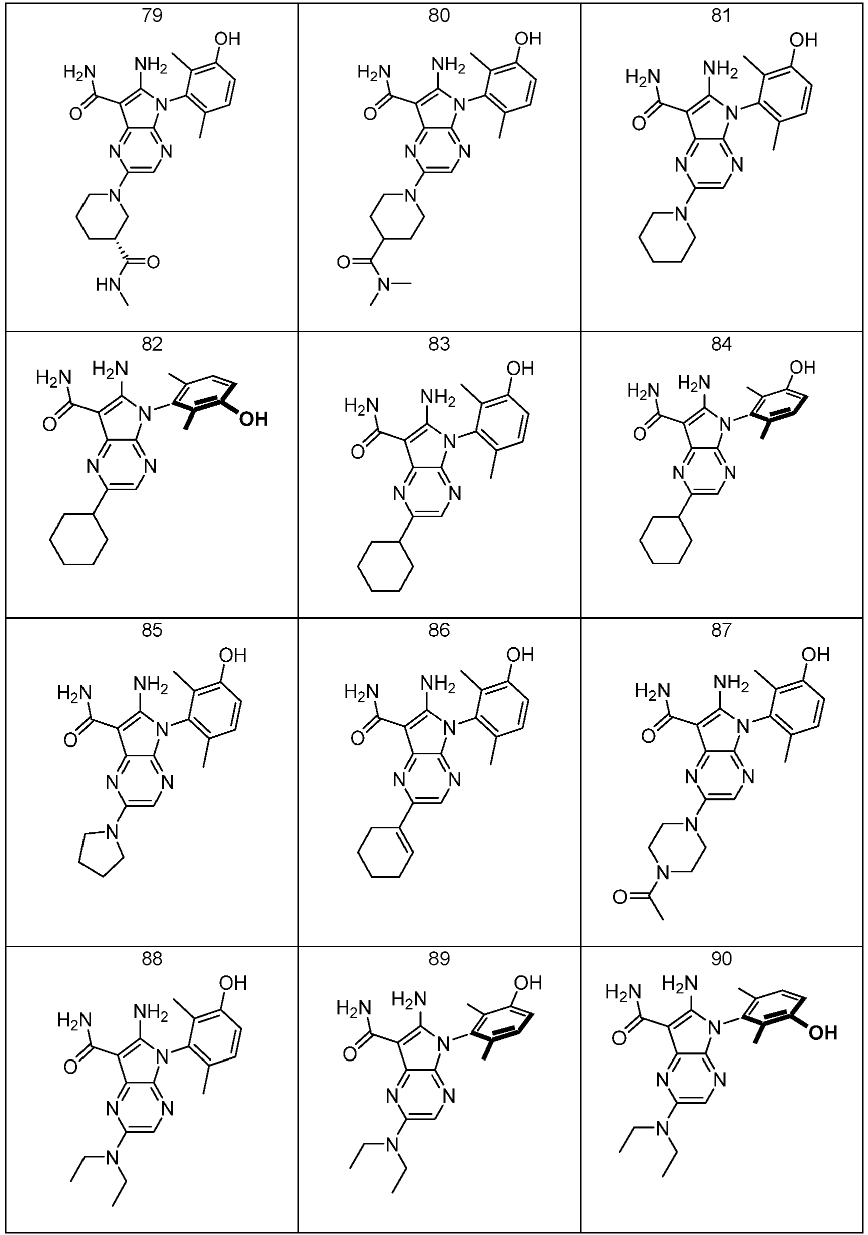

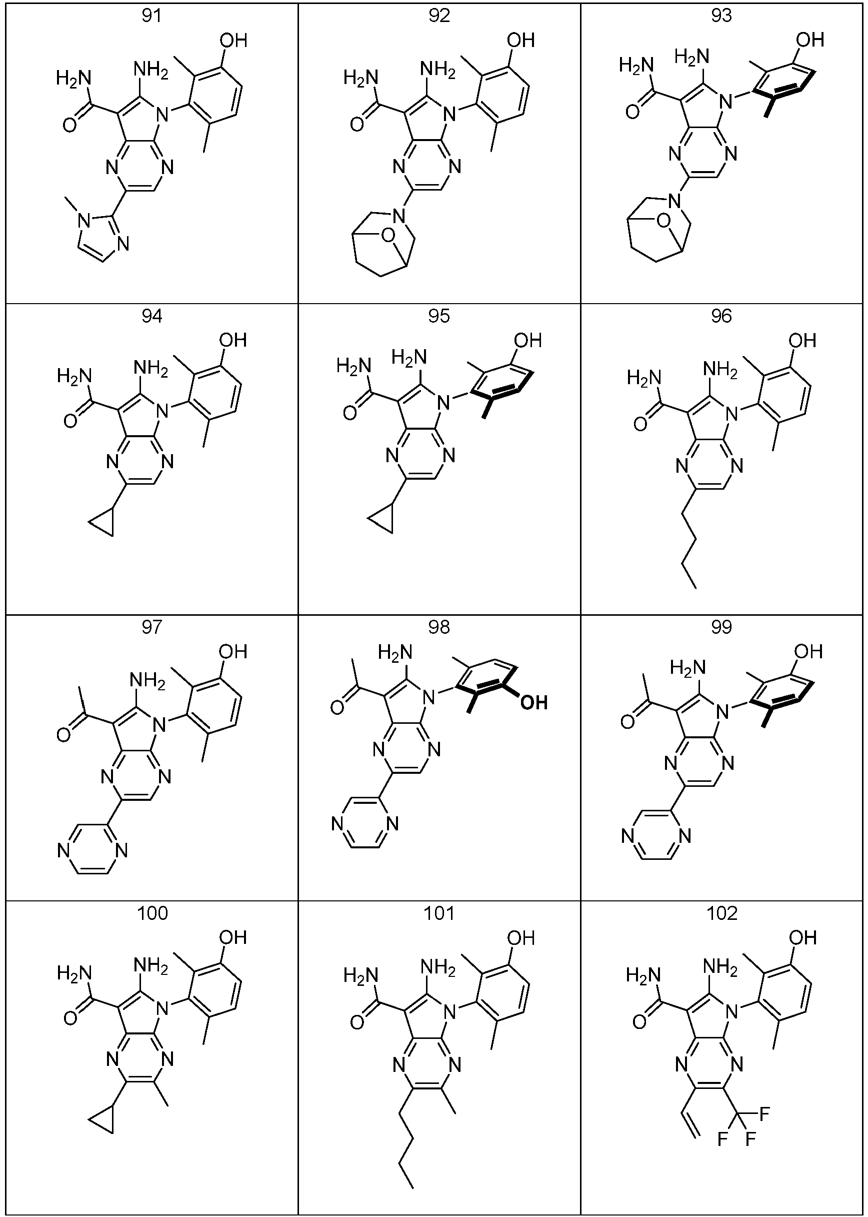

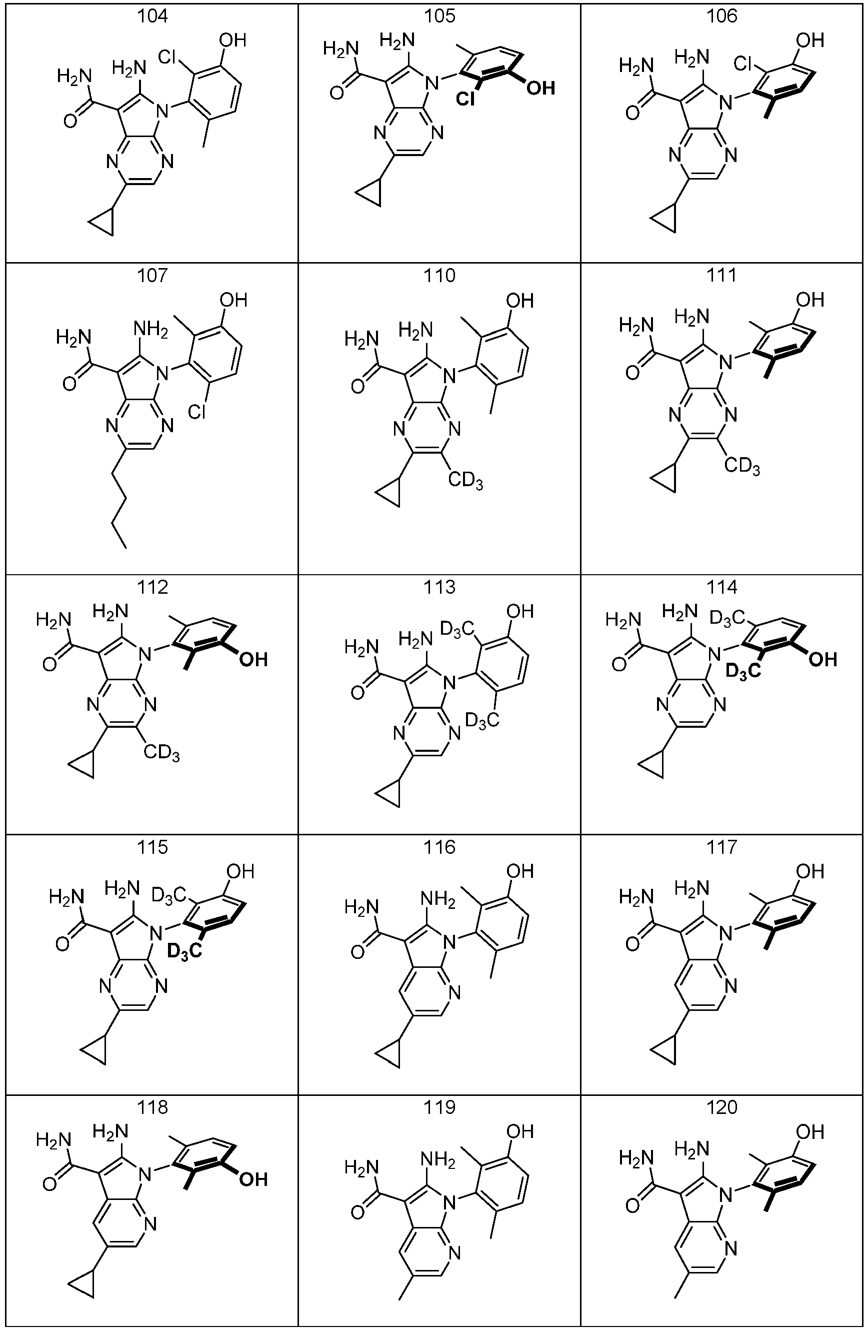

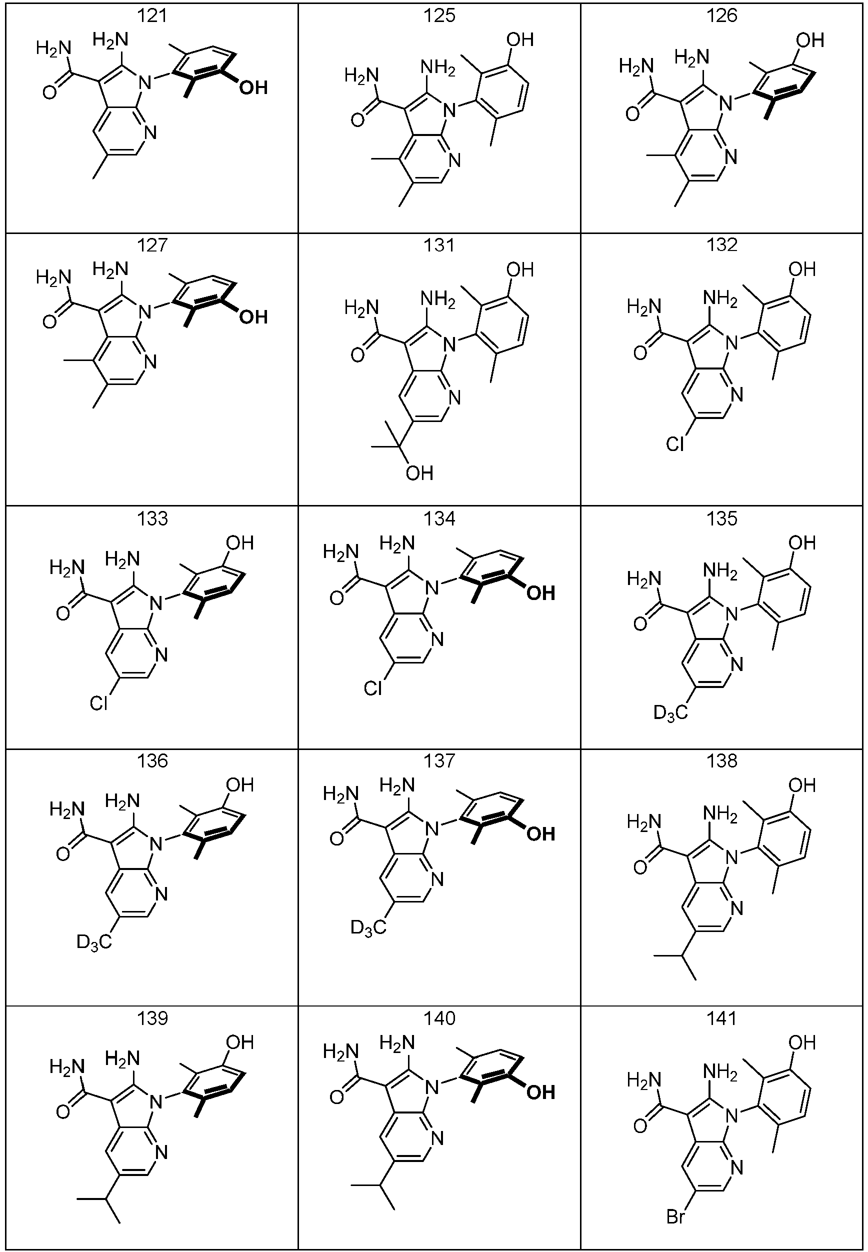

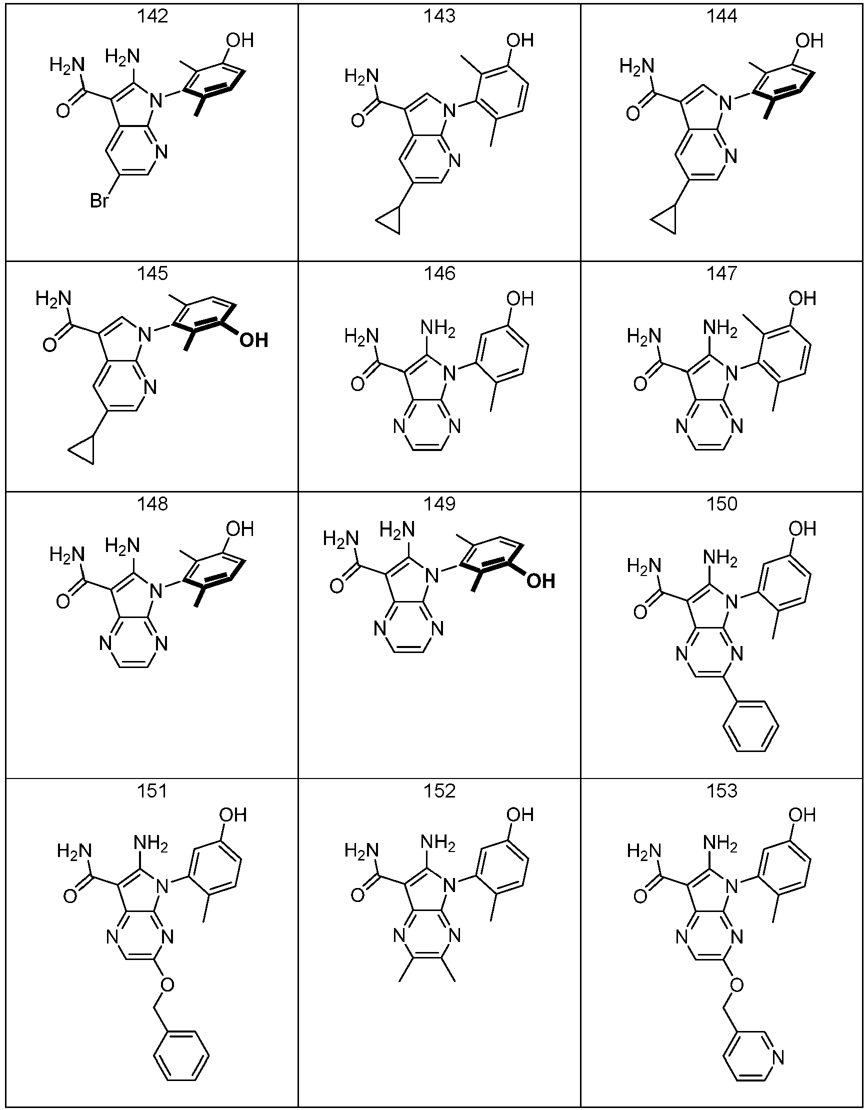

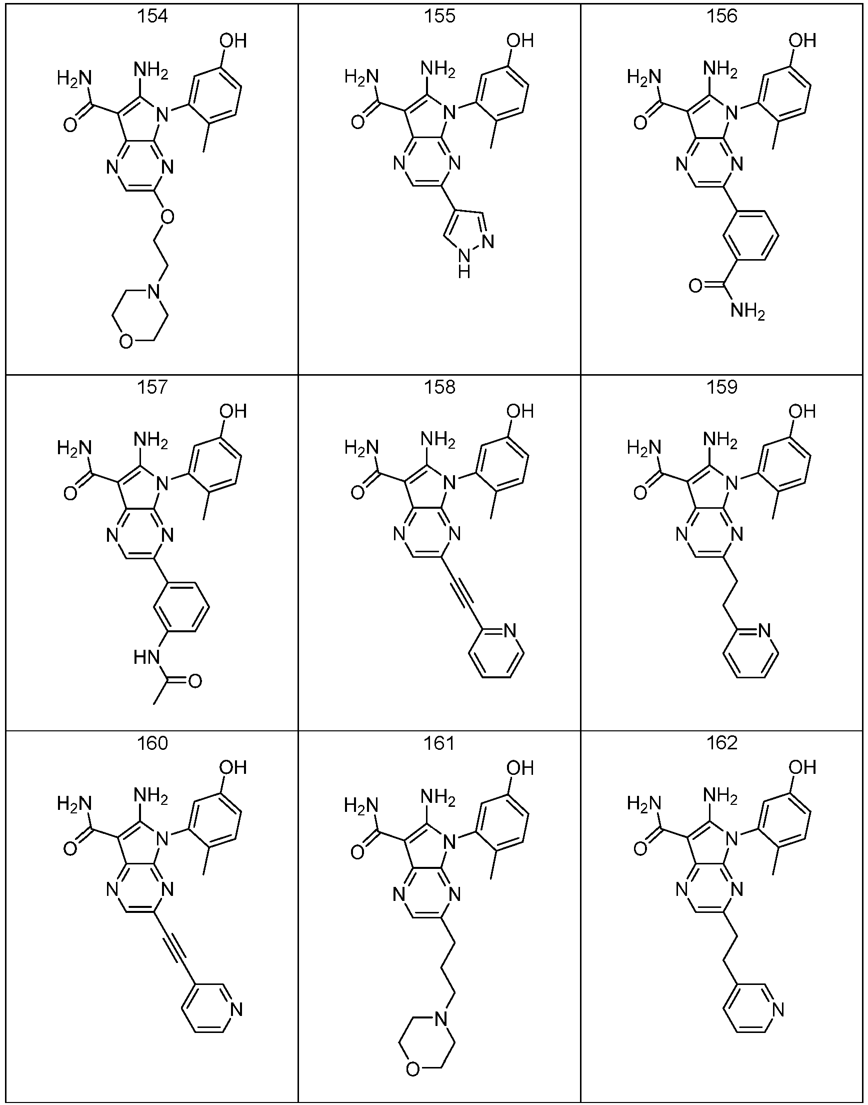

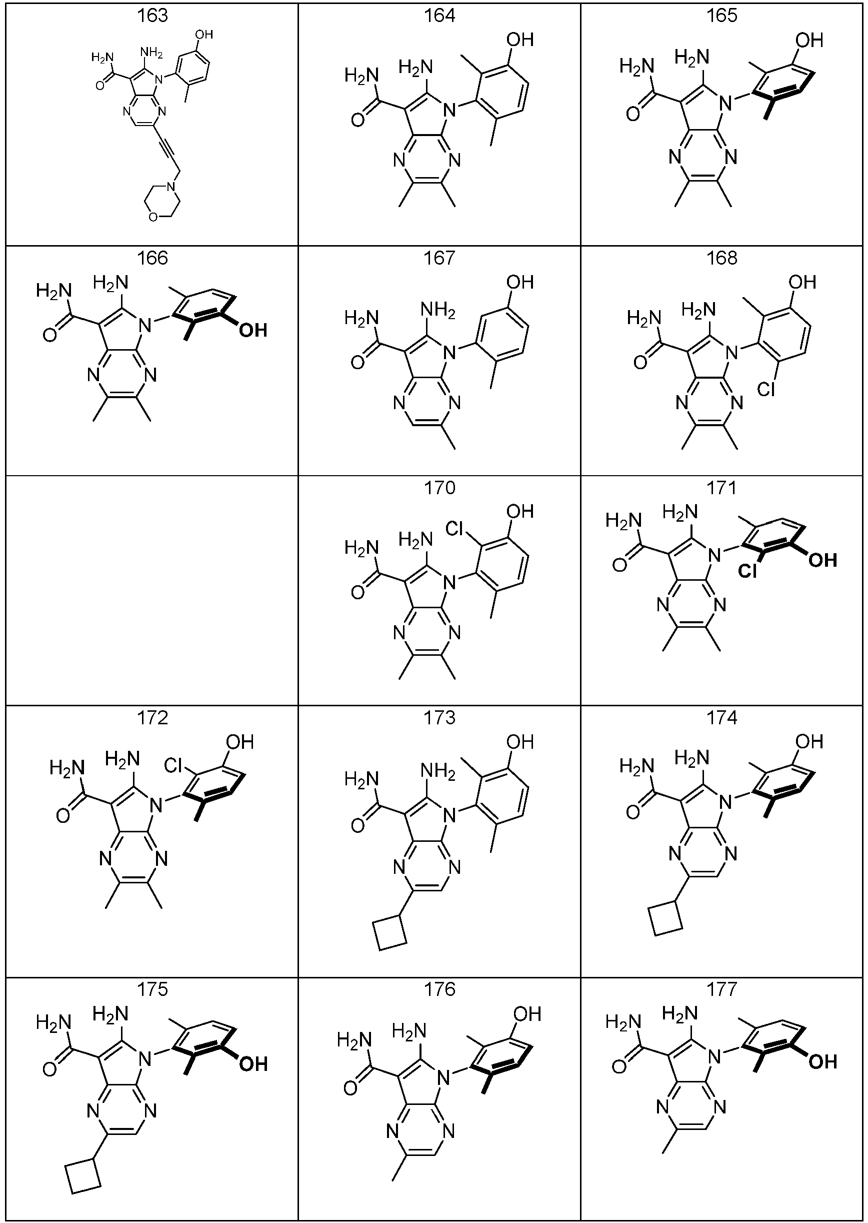

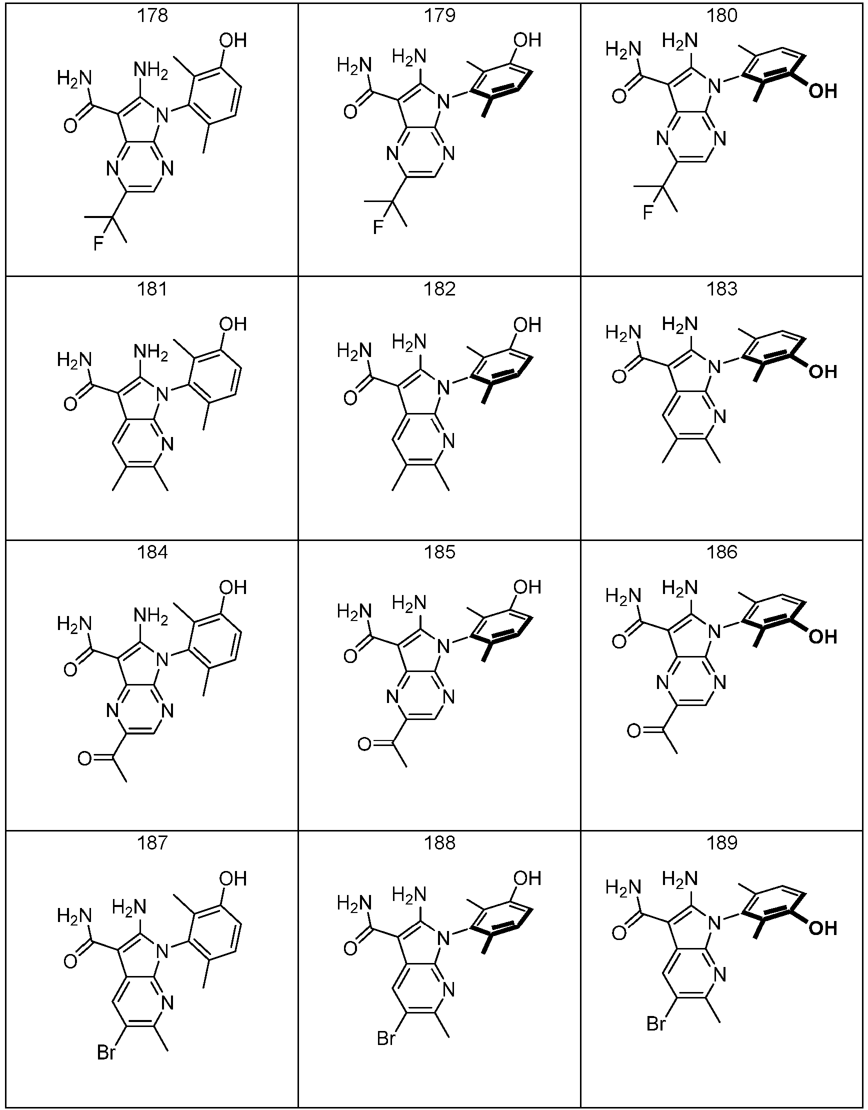

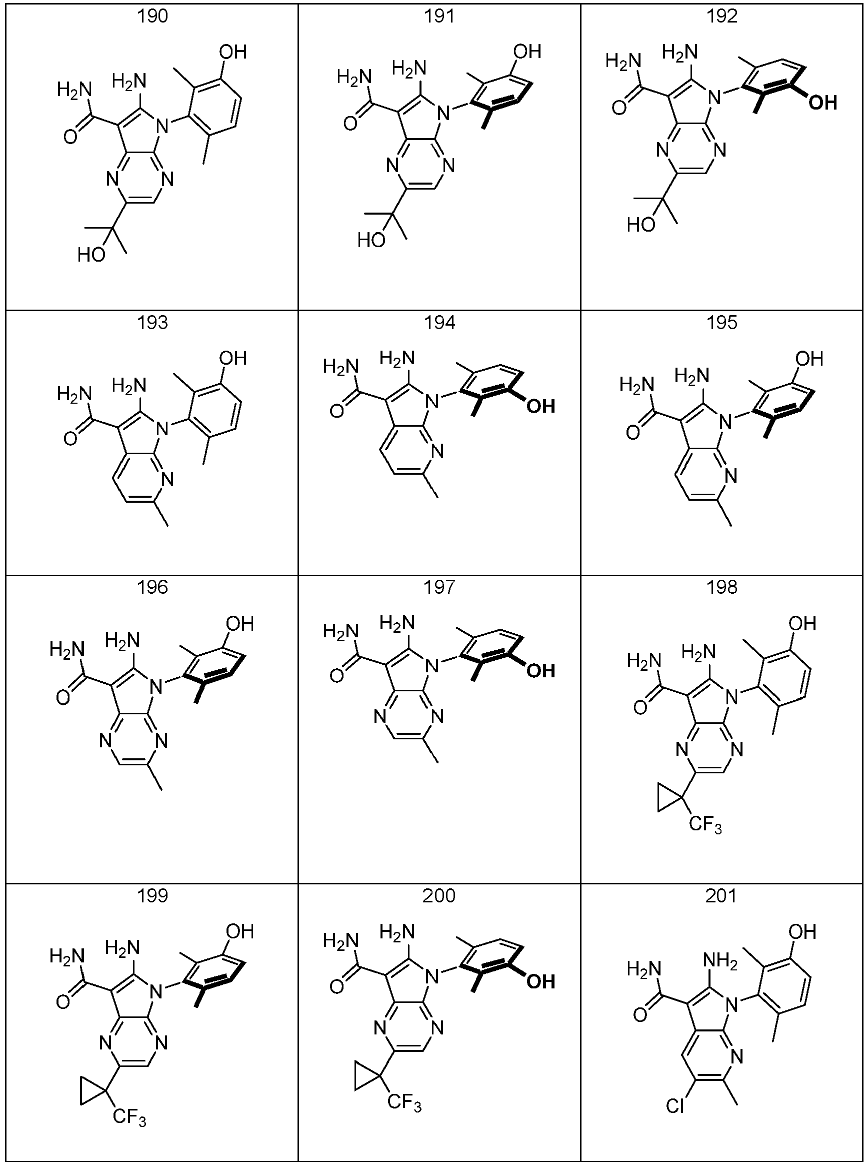

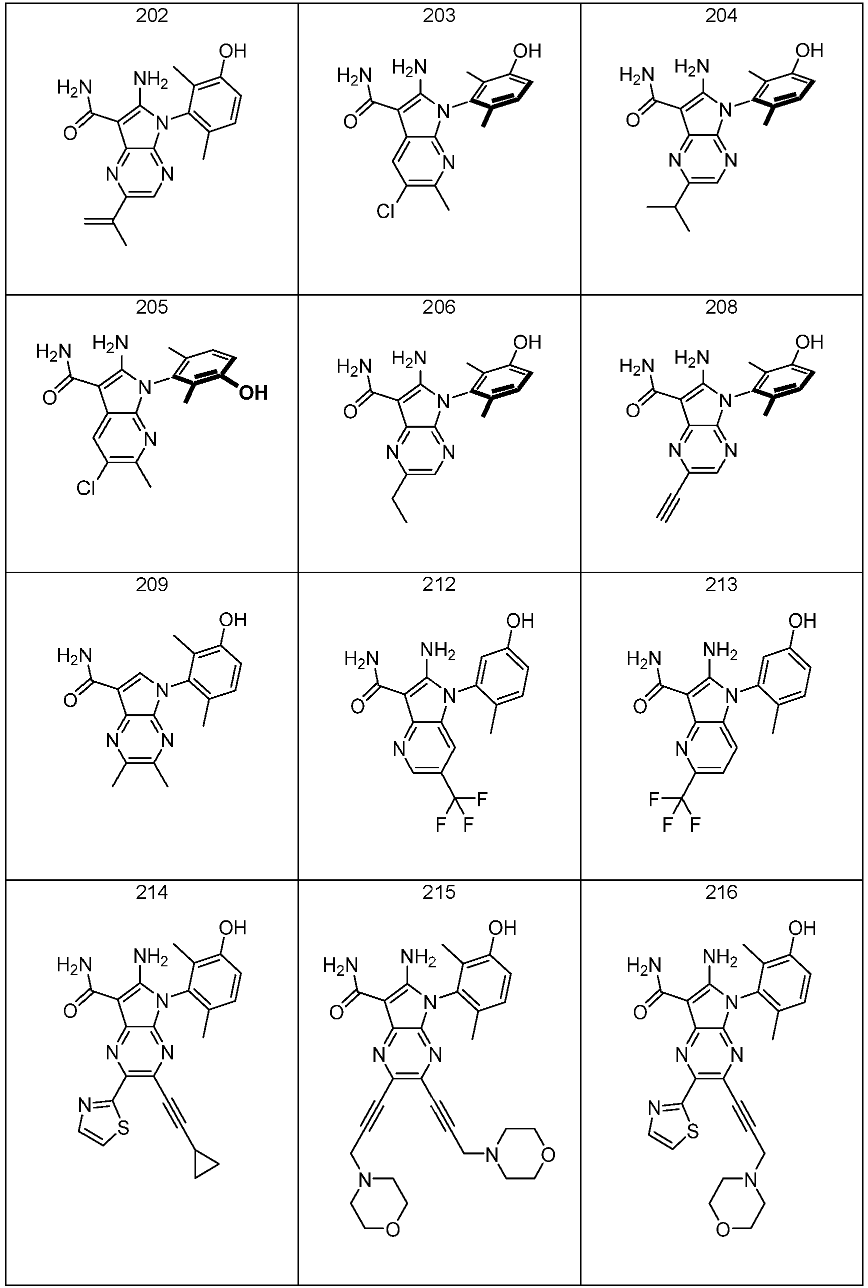

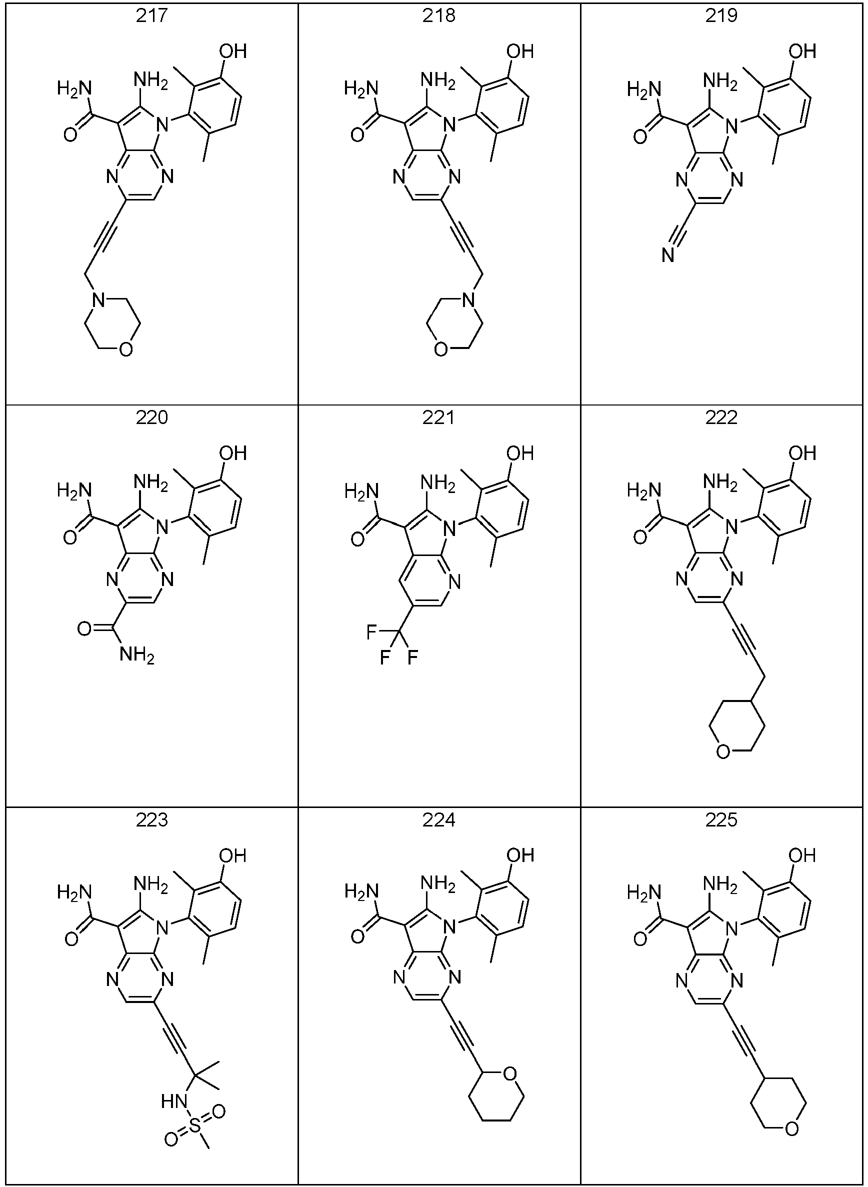

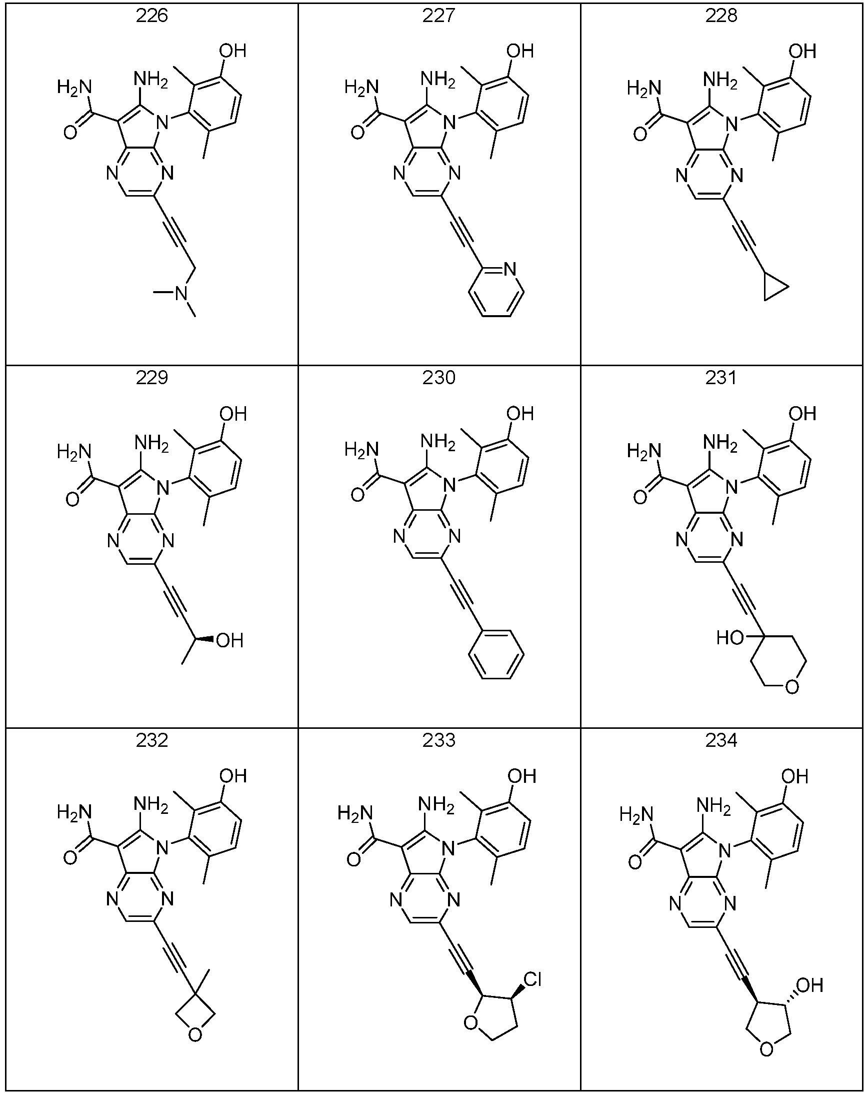

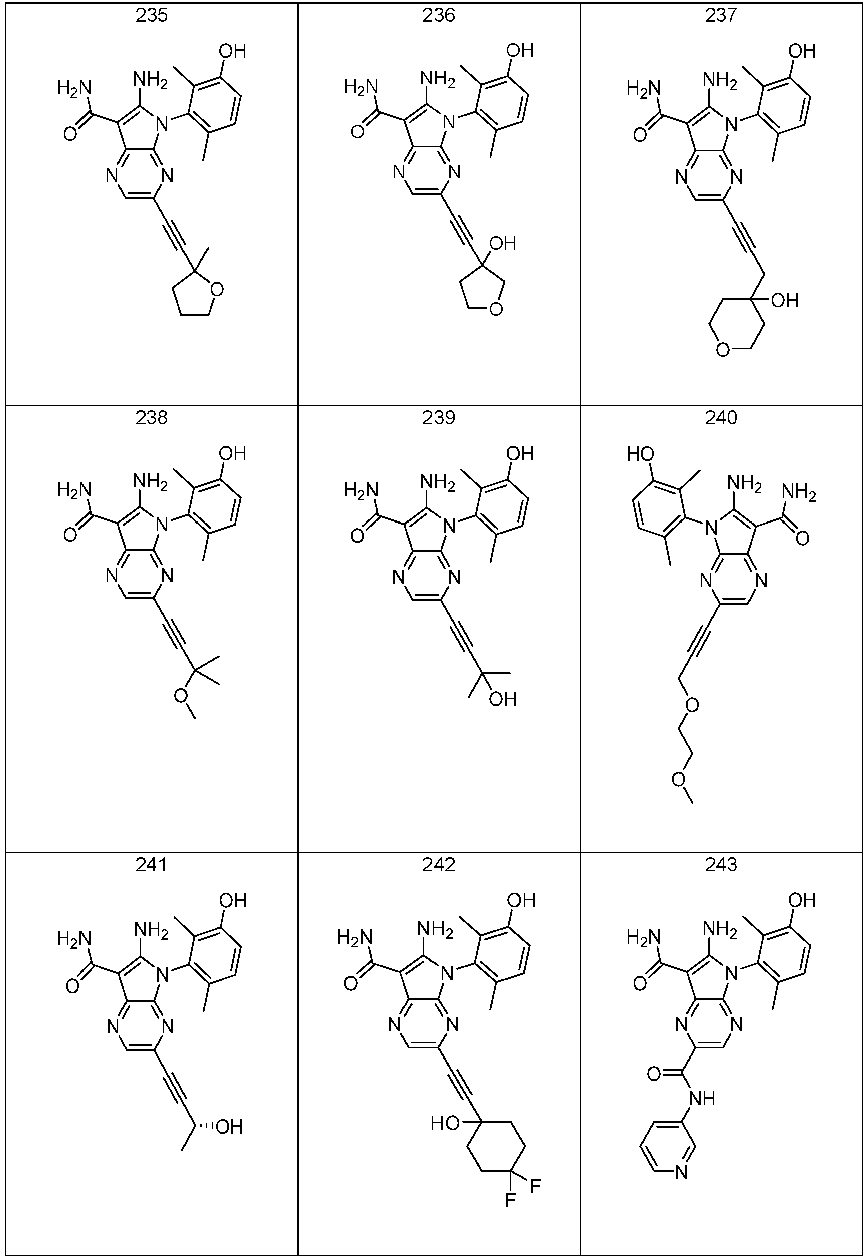

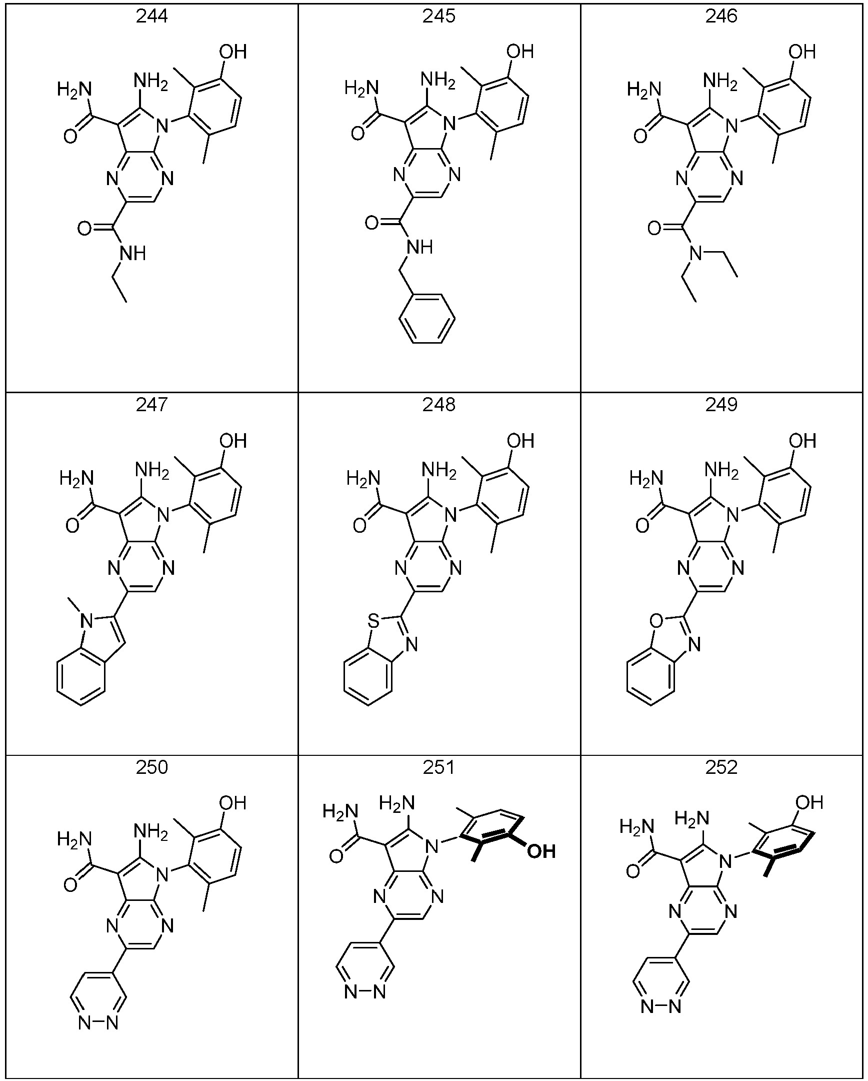

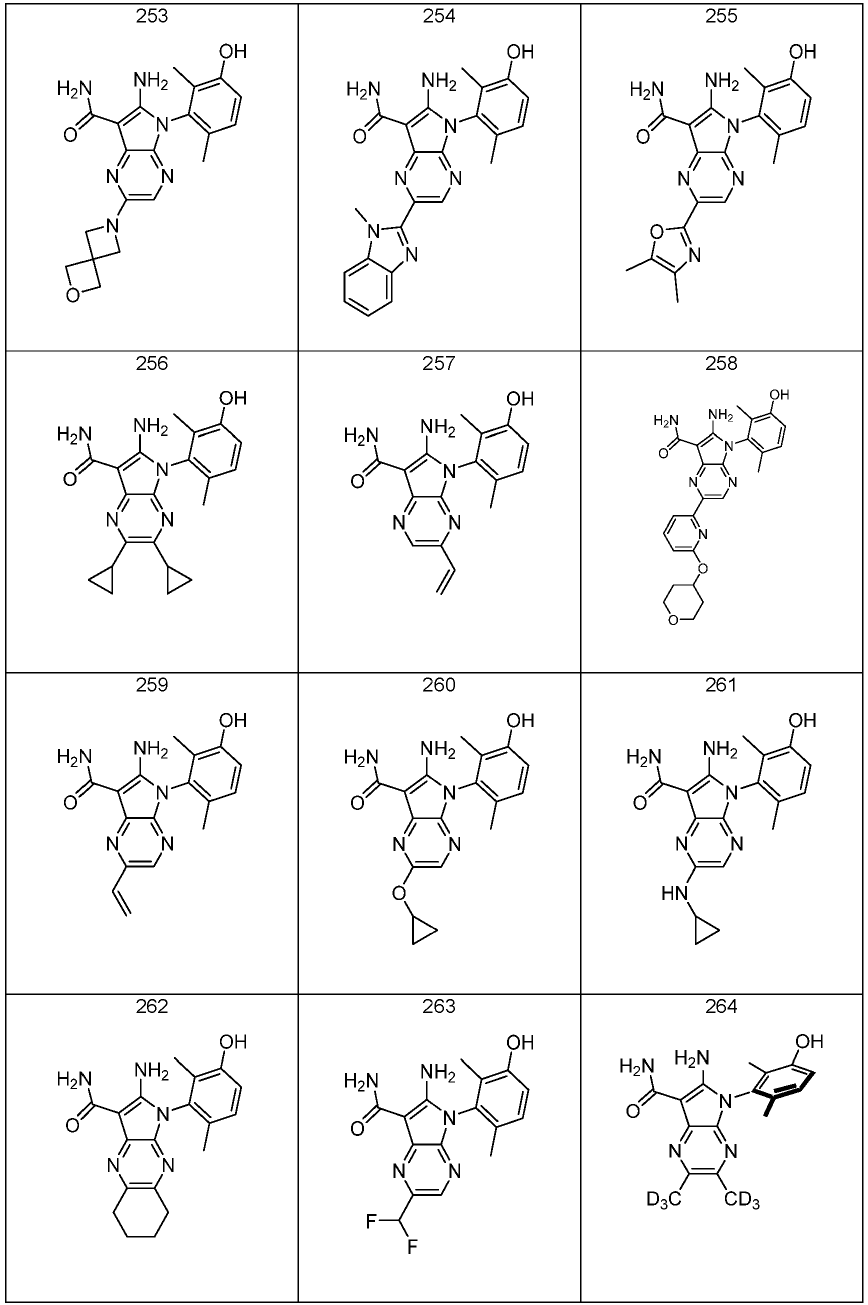

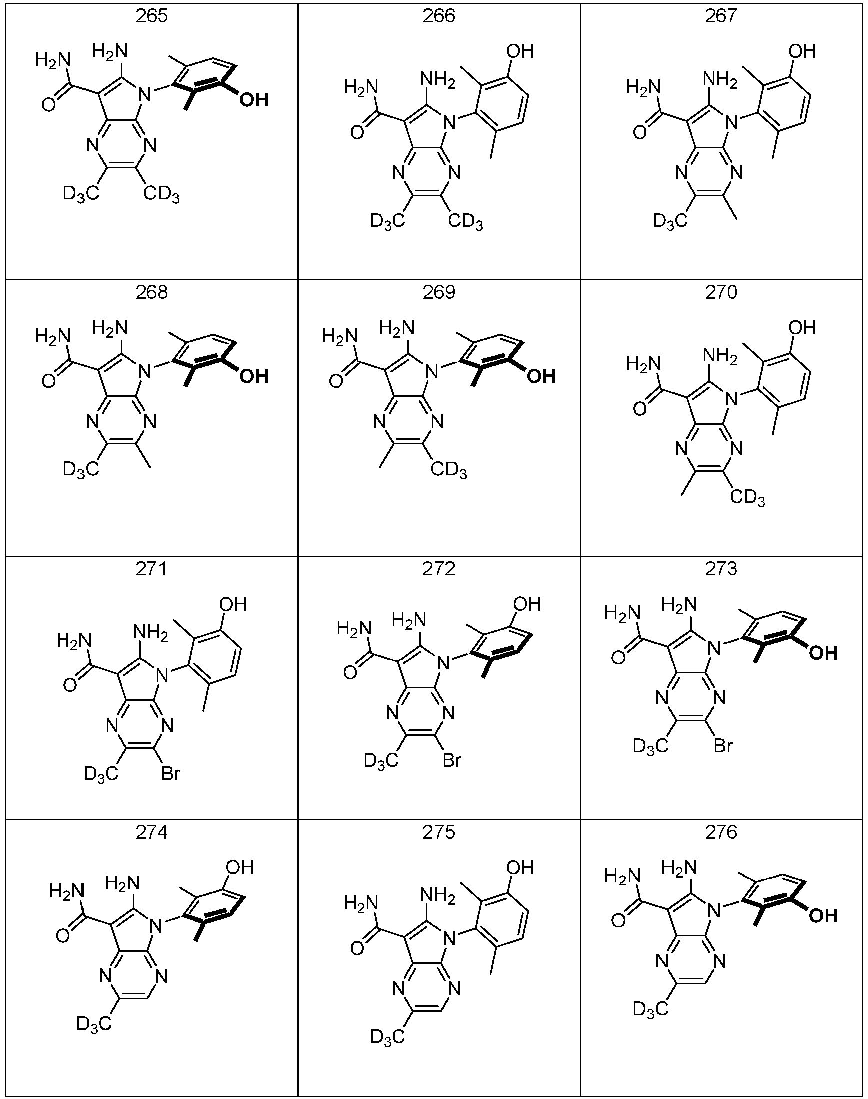

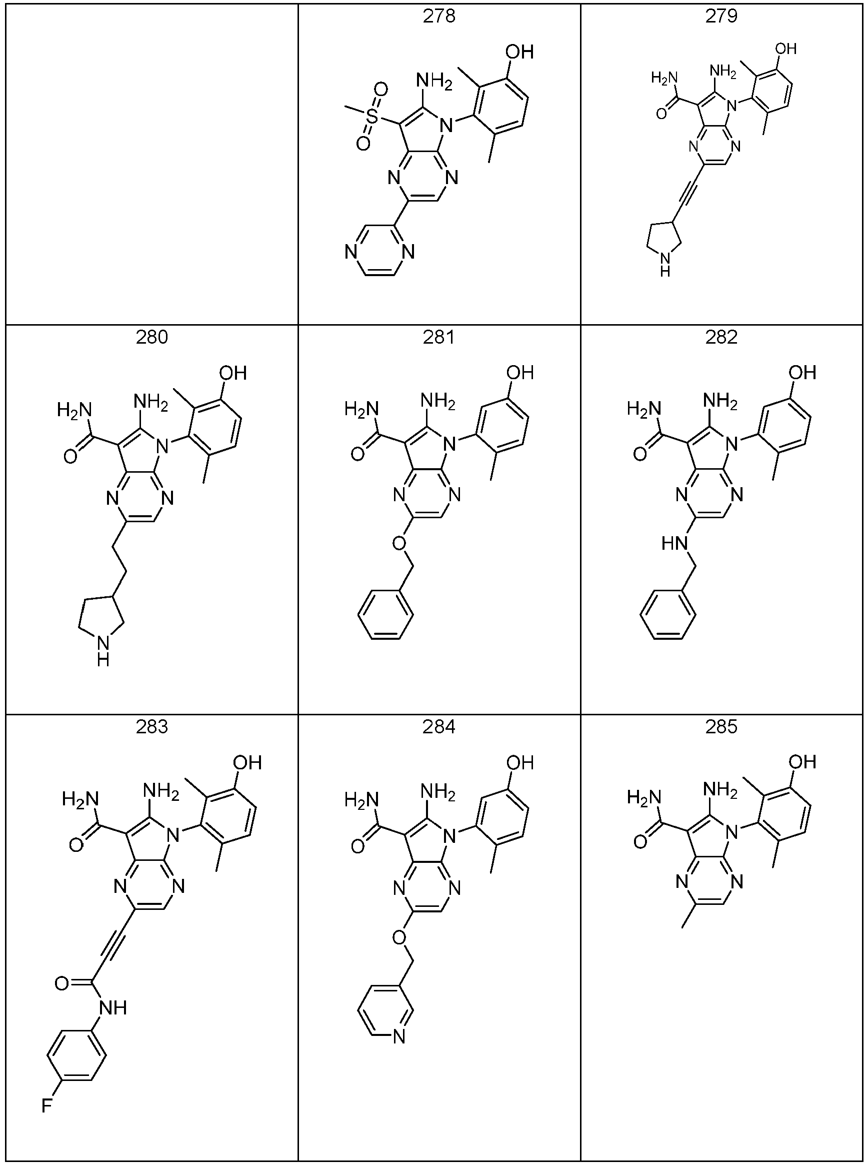

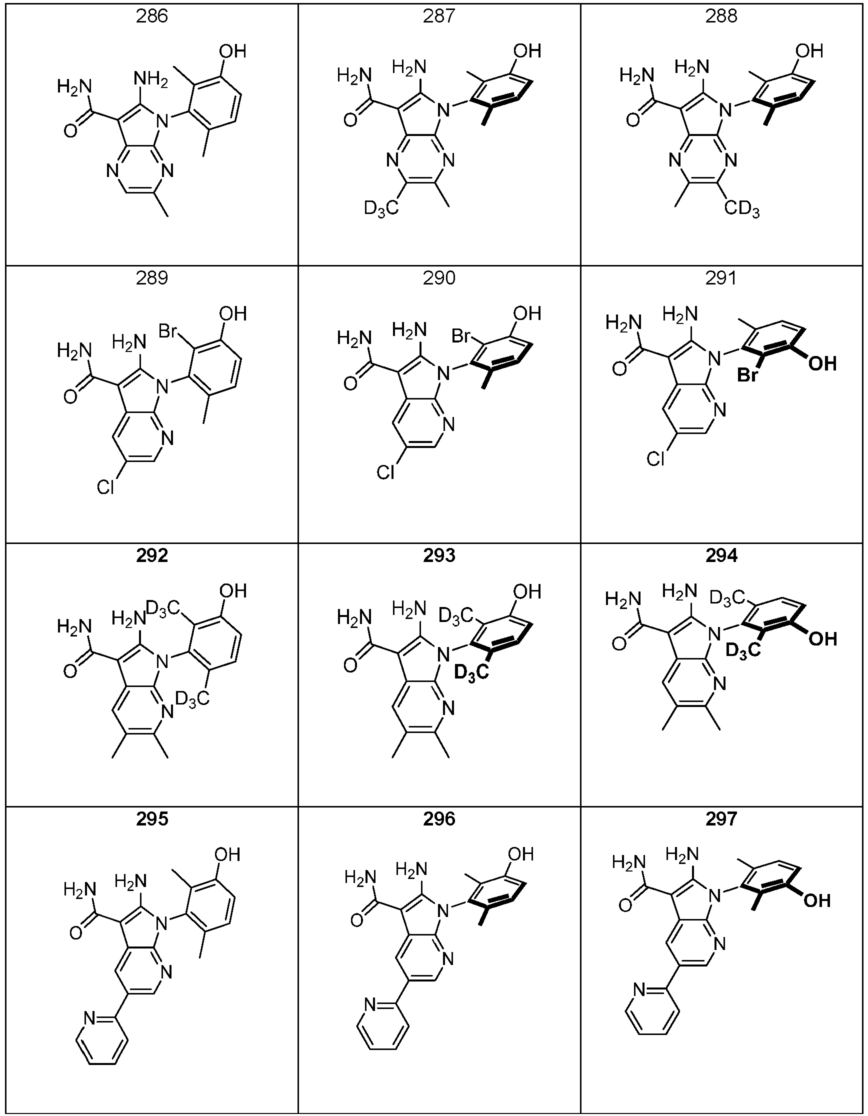

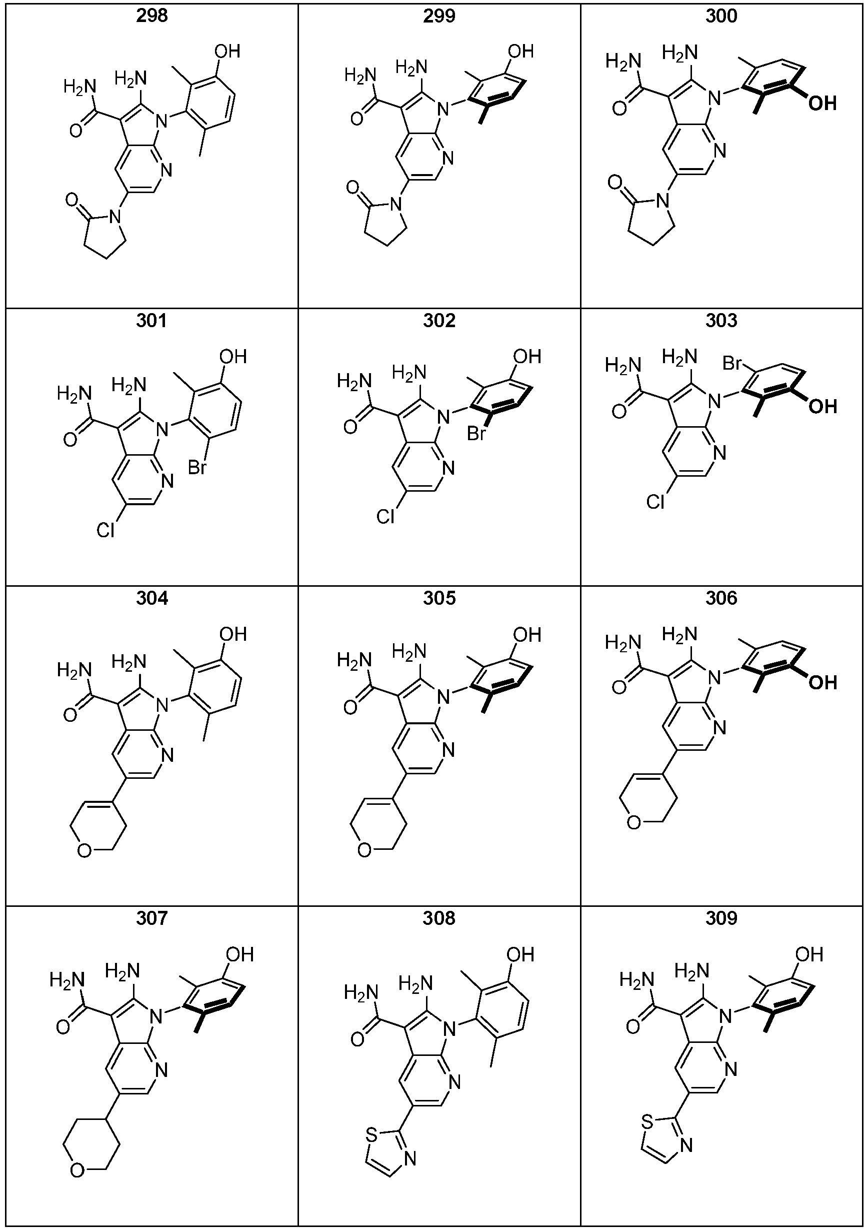

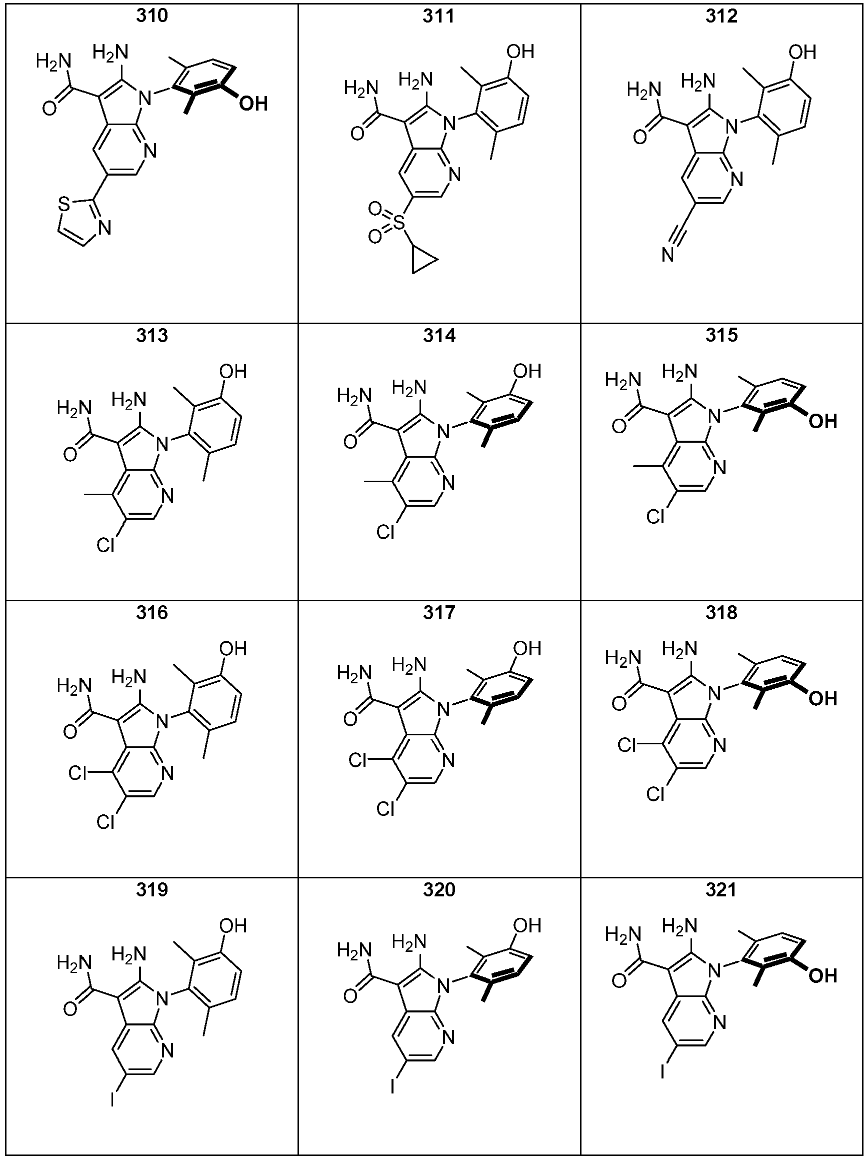

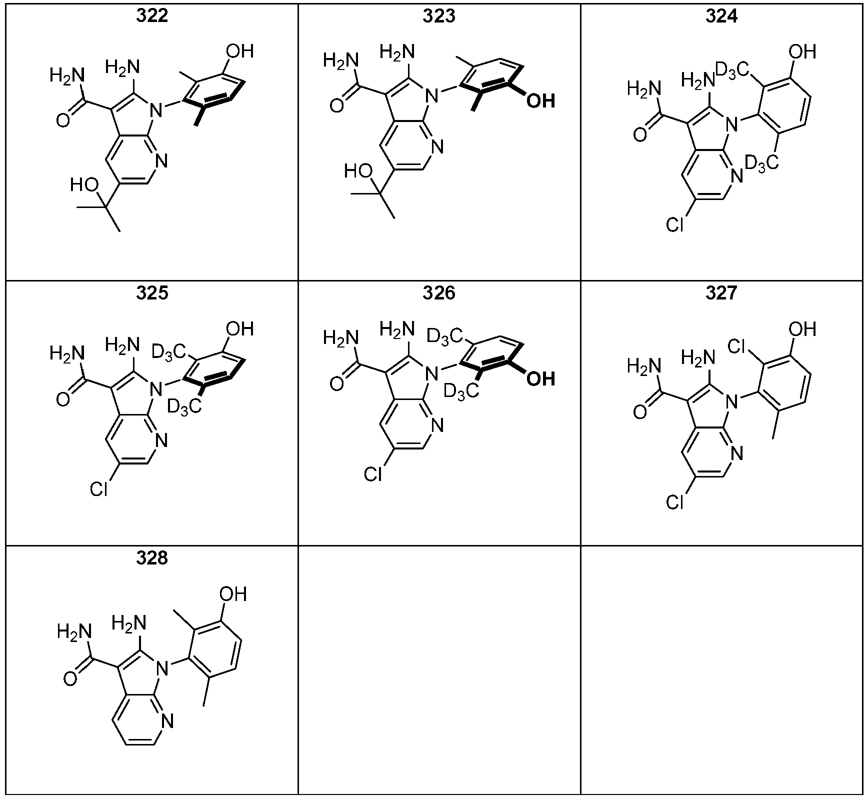

The invention includes (where possible) individual diastereomers, enantiomers, epimers, and atropisomers of the compounds disclosed herein, and mixtures of diastereomers and/or enantiomers thereof including racemic mixtures. Although the specific stereochemistries disclosed herein are preferred, other stereoisomers, including diastereomers, enantiomers, epimers, atropisomers, and mixtures of these may also have utility in treating Myt1-mediated diseases. Inactive or less active diastereoisomers and enantiomers may be useful, e.g., for scientific studies relating to the receptor and the mechanism of activation.

It is understood that certain molecules can exist in multiple tautomeric forms. This invention includes all tautomers even though only one tautomer may be indicated in the examples.

The invention also includes pharmaceutically acceptable salts of the compounds, and pharmaceutical compositions comprising the compounds and a pharmaceutically acceptable carrier. The compounds are especially useful, e.g., in certain kinds of cancer and for slowing the progression of cancer once it has developed in a patient.

The compounds disclosed herein may be used in pharmaceutical compositions comprising (a) the compound(s) or pharmaceutically acceptable salts thereof, and (b) a pharmaceutically acceptable carrier. The compounds may be used in pharmaceutical compositions that include one or more other active pharmaceutical ingredients. The compounds may also be used in pharmaceutical compositions in which the compound disclosed herein or a pharmaceutically acceptable salt thereof is the only active ingredient.

### Optical Isomers - Diastereomers - Geometric Isomers - Tautomers

Compounds disclosed herein may contain, e.g., one or more stereogenic centers and can occur as racemates, racemic mixtures, single enantiomers, individual diastereomers, and mixtures of diastereomers and/or enantiomers. The invention includes all such isomeric forms of the compounds disclosed herein. It is intended that all possible stereoisomers (e.g., enantiomers and/or diastereomers) in mixtures and as pure or partially purified compounds are included within the scope of this invention (i.e., all possible combinations of the stereogenic centers as pure compounds or in mixtures).

Some of the compounds described herein may contain bonds with hindered rotation such that two separate rotomers, or atropisomers, may be separated and found to have different biological activity which may be advantageous. It is intended that all of the possible atropisomers are included within the scope of this invention.

Some of the compounds described herein may contain olefinic double bonds, and unless specified otherwise, are meant to include both E and Z geometric isomers.

Some of the compounds described herein may exist with different points of attachment of hydrogen, referred to as tautomers. An example is a ketone and its enol form, known as keto-enol tautomers. The individual tautomers as well as mixtures thereof are encompassed by the invention.

Compounds disclosed herein having one or more asymmetric centers may be separated into diastereoisomers, enantiomers, and the like by methods well known in the art.

Alternatively, enantiomers and other compounds with chiral centers may be synthesized by stereospecific synthesis using optically pure starting materials and/or reagents of known configuration.

### Metabolites - Prodrugs

The invention includes therapeutically active metabolites, where the metabolites themselves fall within the scope of the claims. The disclosure also includes prodrugs, which are compounds that are converted to the claimed compounds as they are being administered to a patient or after they have been administered to a patient. The claimed chemical structures of this application in some cases may themselves be prodrugs.

### Isotopically Enriched Derivatives

The invention includes molecules which have been isotopically enriched at one or more position within the molecule. Thus, compounds enriched for deuterium fall within the scope of the claims.

### Methods of Preparing a Compound of the Invention

Compounds of the present invention may be prepared using reactions and techniques known in the art and those described herein. One of skill in the art will appreciate that methods of preparing compounds of the invention described herein are non-limiting and that steps within the methods may be interchangeable without affecting the structure of the end product.

### Method A

Compounds of the present invention may be prepared as shown in Scheme A and described herein. The amino group of the commercially available 5-bromo-6-chloropyrazin-2-amine can be converted to a hydroxyl that can be benzylated with benzyl bromide in presence of a base to generate key **Intermediate B.** The bromo may be substituted by an aromatic amine under metal-mediated conditions. Depending on the nature of the arylamine, a protecting group may be required to be in place prior to this reaction. The chloro may be substituted by malononitrile under metal-mediated conditions to afford the aminopyrrole **Intermediate C.** The nitrile may be hydrolyzed to the carboxamide upon treatment with an acid with concomitant cleavage of the benzyl group. The resulting hydroxyl may be converted to the triflate to generate the triflate key **Intermediate D** that can be derivatized in a number of different ways to provide compounds of the present invention. For example, a metal-mediated coupling or S_{N}Ar displacement may be used to install a group at R¹ Depending on the nature of the R¹ group, a protecting group may be required to be in place prior to the triflate derivatization reaction. In the case where the R¹ group bears an unsaturation, a hydrogenation reaction may be required to give compound of the present invention. In the case where the arylamine and/or the R¹ group bear a protecting group, a deprotection step(s) may be required using acid, base and/or fluoride to give compounds of the present invention. Depending on the nature of the arylamine, an atropisomeric mixture may be obtained. In such cases it may be necessary to isolate the atropisomer of interest to give compounds of the present invention. Alternatively, an atropisomerically pure intermediate can be isolated and may be derivatized further to give compounds of the present invention. For example, key **intermediate D** may be purified by chiral chromatography to provide an atropisomerically pure intermediate that may be manipulated similarly to **intermediate D** described above to provide compounds of the present invention.

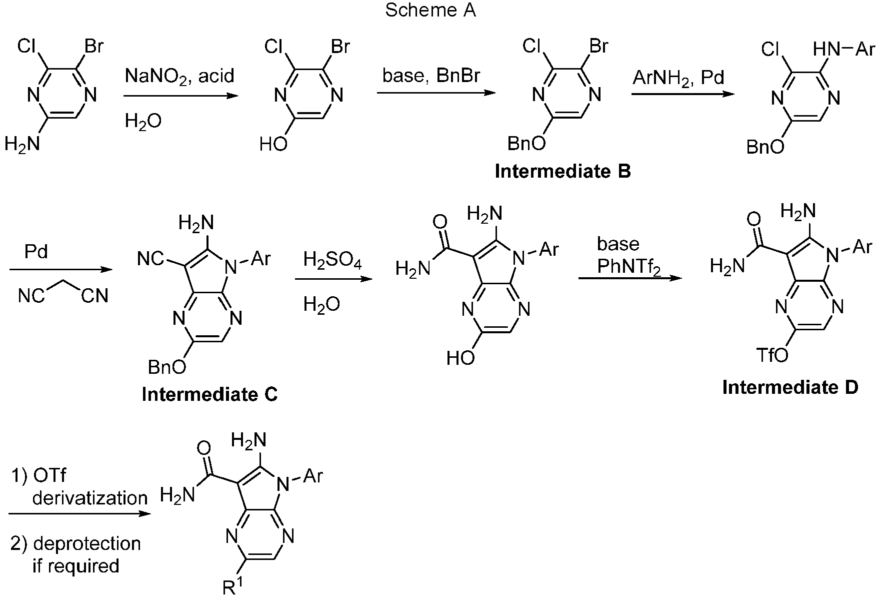

### Method B

Compounds of the present invention may be prepared as shown in Scheme B and described herein. The chloro of **Intermediate B** can be displaced by an aromatic amine under S_{N}Ar conditions. Depending on the nature of the arylamine, a protecting group may be required to be in place prior to this reaction. The bromo may then be substituted by malononitrile under metal-mediated conditions to afford an aminopyrrole. The OBn may be hydrogenolyzed to afford the key **Intermediate E** that may be derivatized in a number of different ways to give compounds of the present invention following nitrile hydrolysis. For example, Mitsunobu or alkylation conditions may be used to install a group at R². Alternatively, **Intermediate E** may be converted to the triflate key **Intermediate F** that may be derivatized in a number of different ways to give compounds of the present invention following nitrile hydrolysis. For example, a metal-mediated coupling may be used to install a group at R². Depending on the nature of the R² group, a protecting group may be required to be in place prior to the hydroxyl or triflate derivatization reaction. In the case where the R² group bears an unsaturation, a hydrogenation reaction may be required to give compound of the present invention. In the case where the arylamine and/or the R² group bear a protecting group, a deprotection step(s) may be required using acid, base and/or fluoride to give compounds of the present invention. Depending on the nature of the arylamine, an atropisomeric mixture may be obtained. In such cases it may be necessary to isolate the atropisomer of interest to give compounds of the present invention. Alternatively, an atropisomerically pure intermediate can be isolated and may be derivatized further to give compounds of the present invention.

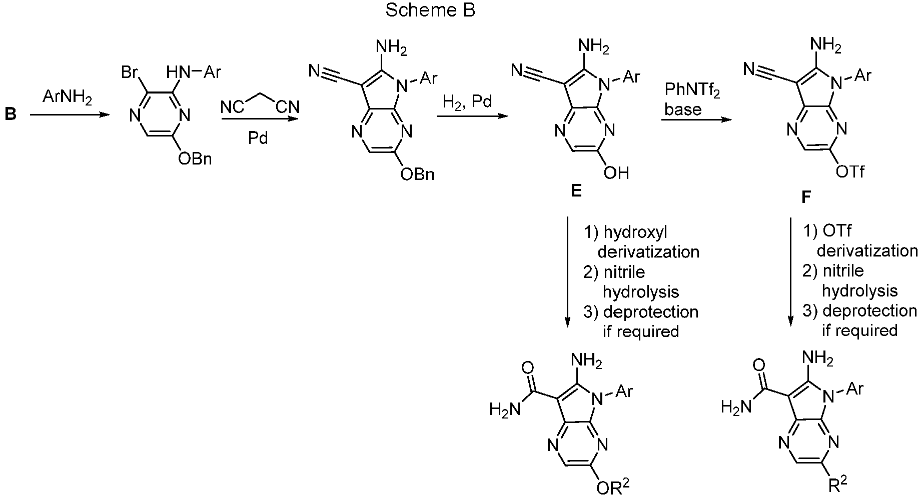

### Method C

Compounds of the present invention may be prepared as shown in Scheme C and described herein. The 2-chloro of the commercially available 3-bromo-2,6-dichloropyridine can be substituted by malononitrile under S_{N}Ar conditions with a base. The bromo may be substituted by an aromatic amine under metal-mediated conditions to afford an aminoazaindole. Depending on the nature of the arylamine, a protecting group may be required to be in place prior to this reaction. Protecting group(s) may be removed before the nitrile may be hydrolyzed to the carboxamide upon treatment with an acid to yield **Intermediate G.** The remaining chloro can be derivatized in a number of different ways to provide compounds of the present invention. For example, a metal-mediated coupling may be used to install a group at R¹ Depending on the nature of the R¹ group, a protecting group may be required to be in place prior to the chloro derivatization reaction. In the case where the R¹ group bears an unsaturation, a hydrogenation reaction may be required to give a compound of the present invention. In the case where the arylamine and/or the R¹ group bear a protecting group, a deprotection step(s) may be required using acid, base and/or fluoride to give compounds of the present invention. Depending on the nature of the arylamine, an atropisomeric mixture may be obtained. In such cases it may be necessary to isolate the atropisomer of interest to give compounds of the present invention. Alternatively, an atropisomerically pure intermediate can be isolated and may be derivatized further to give compounds of the present invention.

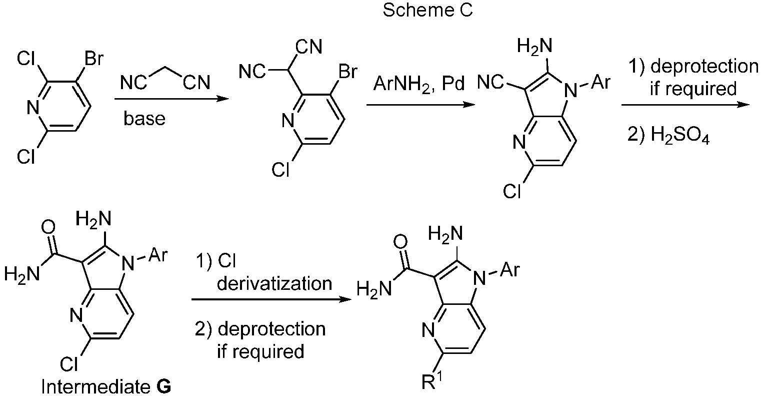

### Method D

Compounds of the present invention may be prepared as shown in Scheme D and described herein. The halogen at position 2 of an adequately substituted 5-nitropyridine may be substituted with an aromatic amine under S_{N}Ar conditions or a metal-mediated C-N coupling conditions. Depending on the nature of the arylamine, a protecting group may be required to be in place prior to this reaction. The 3-bromo may be substituted by malononitrile under palladium-mediated conditions to afford an aminoazaindole. The resulting amino group can be protected with a suitable protecting group, e.g. BOC. The nitro can be reduced and the resulting amino can be converted to a halogen under Sandmeyer conditions yielding halogenated derivatives. The aminopyrrole N-protecting group may be cleaved and the nitrile can be hydrolyzed to the carboxamide to yield **Intermediate I** that can be derivatized in a number of different ways to provide compounds of the present invention. For example, a metal-mediated coupling may be used to install a group at R¹ In the case where the R¹ group bears an unsaturation, a hydrogenation reaction may be required to give a compound of the present invention. Depending on the nature of the R¹ group, a protecting group may be required to be in place prior to the halogen derivatization reaction. In the case where the arylamine and/or the R¹ group bear a protecting group, a deprotection step(s) may be required using acid, base and/or fluoride to give compounds of the present invention. Depending on the nature of the arylamine, an atropisomeric mixture may be obtained. In such cases it may be necessary to isolate the atropisomer of interest to give compounds of the present invention. Alternatively, an atropisomerically pure intermediate can be isolated and may be derivatized further to give compounds of the present invention.

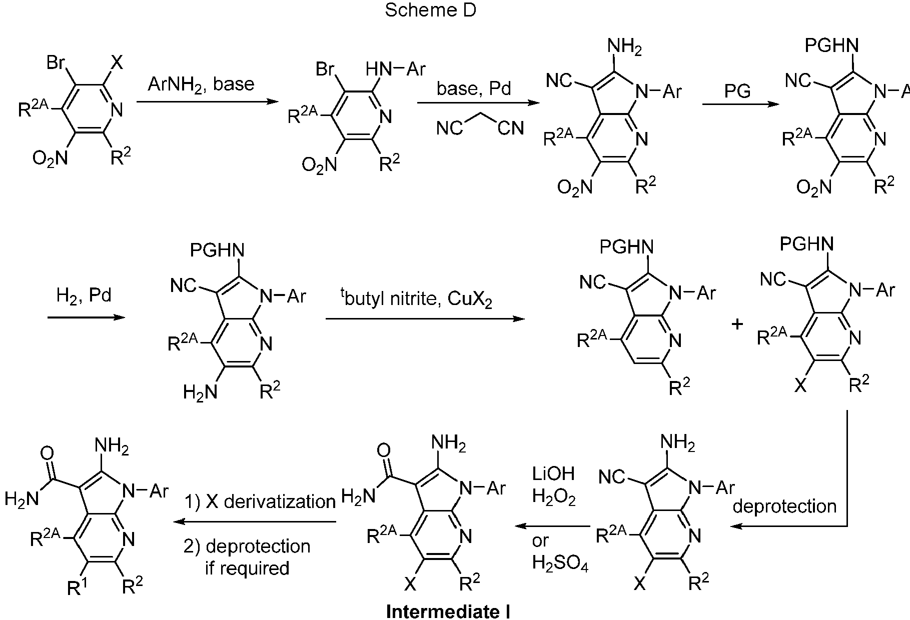

### Method E

Compounds of the present invention may be prepared as shown in Scheme E and described herein. One chloro of commercially available 2,3-dichloro-pyrazine can be substituted by malononitrile under S_{N}Ar or palladium-mediated conditions. The remaining chloro can be substituted with an aromatic amine under S_{N}Ar or palladium-mediated conditions to afford an aminopyrrole. Depending on the nature of the arylamine, a protecting group may be required to be in place prior to this reaction. Hydrolysis of the nitrile can be done under acidic or basic conditions to give compounds of the present invention. In the case where the arylamine group bear a protecting group, a deprotection step(s) may be required using acid, base and/or fluoride to give compounds of the present invention. Depending on the nature of the arylamine, an atropisomeric mixture may be obtained. In such cases it may be necessary to isolate the atropisomer of interest to give compounds of the present invention. Alternatively, an atropisomerically pure intermediate can be isolated and may be derivatized further to give compounds of the present invention.

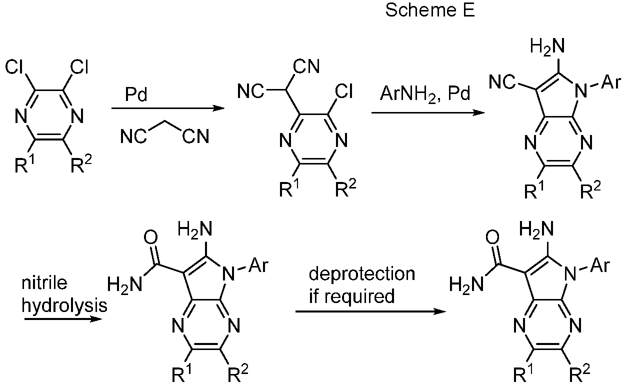

### Method F

Compounds of the present invention may be prepared as shown in Scheme F and described herein. One chloro of commercially available 2,3-dichloropyrazine can be substituted by malononitrile under S_{N}Ar or palladium-mediated conditions. The other chloro can be substituted with an aromatic amine under S_{N}Ar or palladium-mediated conditions to afford an aminopyrrole. Depending on the nature of the arylamine, a protecting group may be required to be in place prior to this reaction. The pyrazine ring can be brominated using a suitable bromination reagent such as NBS. Hydrolysis of the nitrile can be done under acidic or basic conditions and protecting group may be cleaved to give key **Intermediate H** that may be derivatized in a number of different ways to give compounds of the present invention. For example, a metal-mediated coupling may be used to install a group at R². Depending on the nature of the R² group, a protecting group may be required to be in place prior to the bromo derivatization reaction. In the case where the R² group bears an unsaturation, a hydrogenation reaction may be required to give compound of the present invention. In the case where the arylamine and/or the R² group bear a protecting group, a deprotection step(s) may be required using acid, base and/or fluoride to give compounds of the present invention. Depending on the nature of the arylamine, an atropisomeric mixture may be obtained. In such cases it may be necessary to isolate the atropisomer of interest to give compounds of the present invention. Alternatively, an atropisomerically pure intermediate can be isolated and may be derivatized further to give compounds of the present invention.

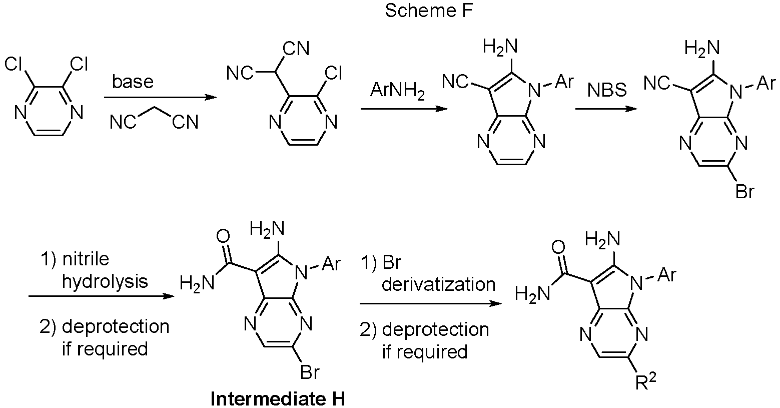

### Method G

Compounds of the present invention may be prepared as shown in Scheme G and described herein. The chloro of commercially available 2-chloro-3-bromopyridines can be substituted by malononitrile under S_{N}Ar conditions. The bromo can be substituted with an aromatic amine under S_{N}Ar or palladium-mediated conditions to afford an aminopyrrole. Depending on the nature of the arylamine, a protecting group may be required to be in place prior to this reaction. Hydrolysis of the nitrile can be done under acidic or basic conditions to give compounds of the present invention. For an arylamine group bearing a protecting group, a deprotection step(s) may be required using acid, base and/or fluoride to give compounds of the present invention. Depending on the nature of the arylamine, an atropisomeric mixture may be obtained. In such cases it may be necessary to isolate the atropisomer of interest to give compounds of the present invention. Alternatively, an atropisomerically pure intermediate can be isolated and may be derivatized further to give compounds of the present invention.

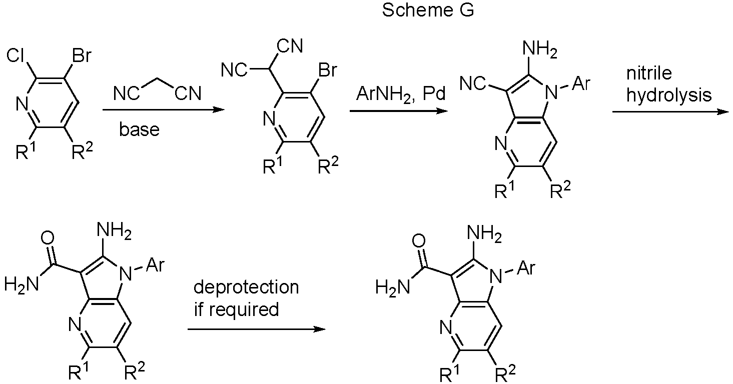

### Method H

Compounds of the present invention may be prepared as shown in Scheme H and described herein. Key **Intermediate C** may be brominated using a suitable bromination reagent such as NBS. The nitrile may be hydrolyzed to the carboxamide upon treatment with an acid with concomitant cleavage of the benzyl group. The resulting hydroxyl may be converted to the triflate. The bromo and the triflate may be derivatized sequentially with different groups or, alternatively the bromo and the triflate may be derivatized simultaneously with the same groups. The bromo and triflate may be derivatized in a number of different ways to give compounds of the present invention. For example, metal-mediated couplings may be used to install a group at R¹ and/or R² sequentially or simultaneously. Depending on the nature of the R¹ and/or R² group, a protecting group may be required to be in place prior to the bromo or triflate derivatization reaction. In the case where the arylamine, the R¹ and/or the R² group bear a protecting group, a deprotection step(s) may be required using acid, base and/or fluoride to give compounds of the present invention. Depending on the nature of the arylamine used to prepare **intermediate C,** an atropisomeric mixture may be obtained. In such cases it may be necessary to isolate the atropisomer of interest to give compounds of the present invention. Alternatively, an atropisomerically pure intermediate can be isolated and may be derivatized further to give compounds of the present invention.

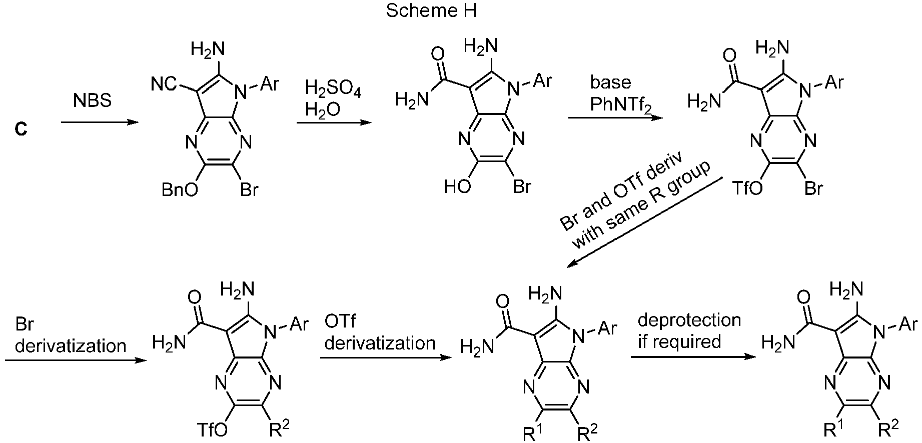

### Method I

Compounds of the present invention may be prepared as shown in Scheme I and described herein. The chloro of commercially available 3-bromo-2-chloro-5-(trifluoromethyl)pyridine can be substituted with an aromatic amine under S_{N}Ar or palladium-mediated conditions. Depending on the nature of the arylamine, a protecting group may be required to be in place prior to this reaction. The bromo can be substituted with malononitrile under palladium-mediated conditions to afford an aminopyrrole. Hydrolysis of the nitrile can be done under acidic or basic conditions to give compounds of the present invention. In the case where the arylamine group bear a protecting group, a deprotection step(s) may be required using acid, base and/or fluoride to give compounds of the present invention. Depending on the nature of the arylamine, an atropisomeric mixture may be obtained. In such cases it may be necessary to isolate the atropisomer of interest to give compounds of the present invention. Alternatively, an atropisomerically pure intermediate can be isolated and may be derivatized further to give compounds of the present invention.

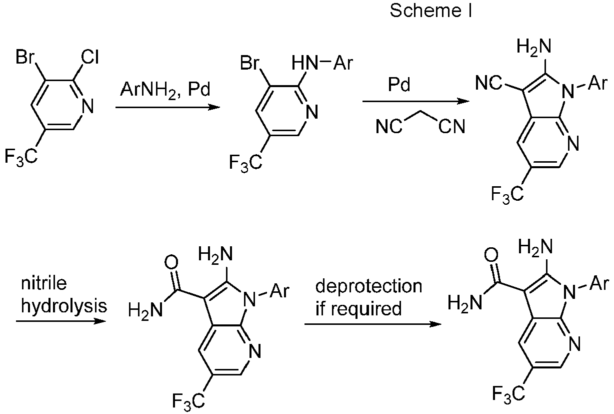

### Method J

Compounds of the present invention may be prepared as shown in Scheme J and described herein. Key **Intermediate C** may be halogenated using a suitable halogenating reagent such as NBS or NIS. The halogen can be derivatized in a number of different ways. For example, a metal-mediated coupling may be used to install a group at R². The nitrile may be hydrolyzed to the carboxamide upon treatment with an acid with concomitant cleavage of the benzyl group. The resulting hydroxyl may be converted to the triflate. The triflate may be derivatized in a number of different ways to give compounds of the present invention. For example, a metal-mediated coupling may be used to install a group at R¹. Depending on the nature of the R¹ and/or R² group, a protecting group may be required to be in place prior to the halogen and/or triflate derivatization reaction. In the case where the arylamine, the R¹ and/or the R² group bear a protecting group, a deprotection step(s) may be required using acid, base and/or fluoride to give compounds of the present invention. Depending on the nature of the arylamine used to prepared **intermediate C,** an atropisomeric mixture may be obtained. In such cases it may be necessary to isolate the atropisomer of interest to give compounds of the present invention. Alternatively, an atropisomerically pure intermediate can be isolated and may be derivatized further to give compounds of the present invention.

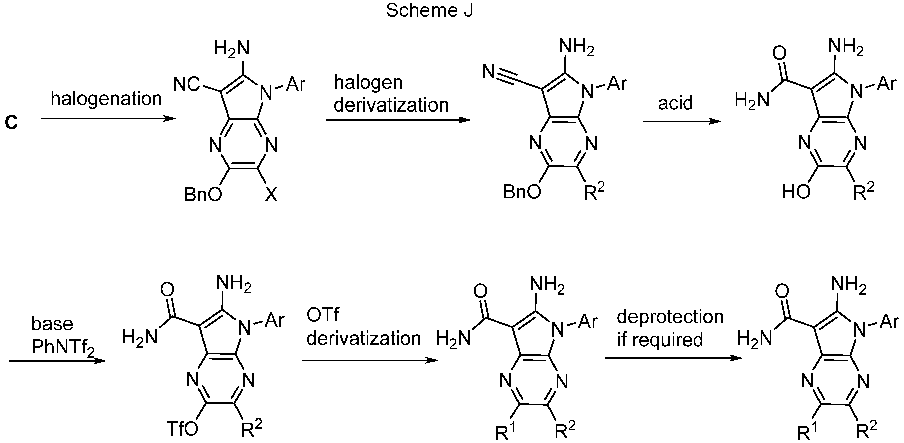

### Method K

Compounds of the present invention may be prepared as shown in Scheme K and described herein. The fluoro of a 3-bromo-2-fluoro-pyridine can be substituted with an aromatic amine under S_{N}Ar conditions. Depending on the nature of the arylamine, a protecting group may be required to be in place prior to this reaction. The bromo can be substituted with malononitrile under palladium-mediated conditions to afford an aminoazaindole. Hydrolysis of the nitrile can be done under acidic or basic conditions to give compounds of the present invention. In the case where the arylamine or R¹ group bear a protecting group, a deprotection step(s) may be required using acid, base and/or fluoride to give compounds of the present invention. Depending on the nature of the arylamine, an atropisomeric mixture may be obtained. In such cases it may be necessary to isolate the atropisomer of interest to give compounds of the present invention. Alternatively, an atropisomerically pure intermediate can be isolated and may be derivatized further to give compounds of the present invention.

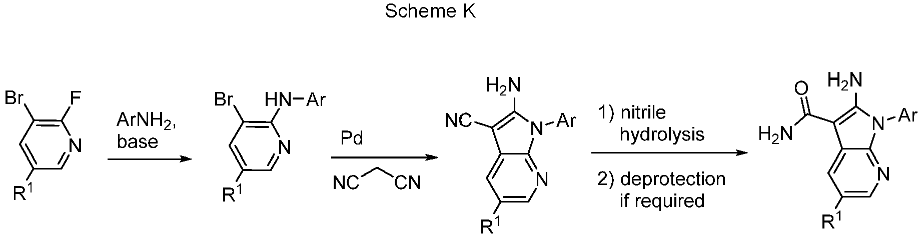

### Method L

Compounds of the present invention may be prepared as shown in Scheme L and described herein. The triflate of key **Intermediate D** may be derivatized in a number of different ways to give compounds of the present invention. For example, a metal-mediated coupling may be used to install a group at R¹ the pyrazine may be brominated using a suitable bromination reagent such as NBS. The bromo may be derivatized in a number of different ways to give compounds of the present invention. For example, a metal-mediated coupling may be used to install a group at R². Depending on the nature of the R¹ and/or R² group, a protecting group may be required to be in place prior to the triflate and/or bromo derivatization reaction. In the case where the arylamine, the R¹ and/or the R² group bear a protecting group, a deprotection step(s) may be required using acid, base and/or fluoride to give compounds of the present invention. Depending on the nature of the arylamine used to prepare **intermediate D,** an atropisomeric mixture may be obtained. In such cases it may be necessary to isolate the atropisomer of interest to give compounds of the present invention. Alternatively, an atropisomerically pure intermediate can be isolated and may be derivatized further to give compounds of the present invention.

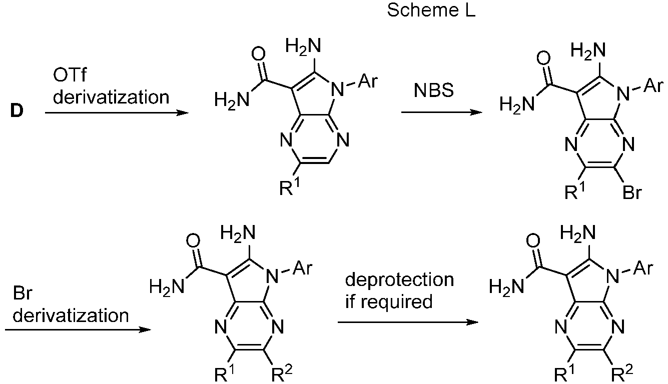

### Method M

Compounds of the present invention may be prepared as shown in Scheme M and described herein. The nitrile of key **Intermediate** C can yield a ketone upon treatment with a Grignard reagent. The benzyl group can be cleaved under acidic conditions. The resulting hydroxyl may be converted to the triflate to generate the triflate that can be derivatized in a number of different ways to provide compounds of the present invention. For example, a metal-mediated coupling may be used to install a group at R¹ Depending on the nature of the R¹ group, a protecting group may be required to be in place prior to the triflate derivatization reaction. In the case where the R¹ group bears an unsaturation, a hydrogenation reaction may be required to give compound of the present invention. In the case where the arylamine and/or the R¹ group bear a protecting group, a deprotection step(s) may be required using acid, base and/or fluoride to give compounds of the present invention. Depending on the nature of the arylamine used to prepare **intermediate C,** an atropisomeric mixture may be obtained. In such cases it may be necessary to isolate the atropisomer of interest to give compounds of the present invention. Alternatively, an atropisomerically pure intermediate can be isolated and may be derivatized further to give compounds of the present invention.

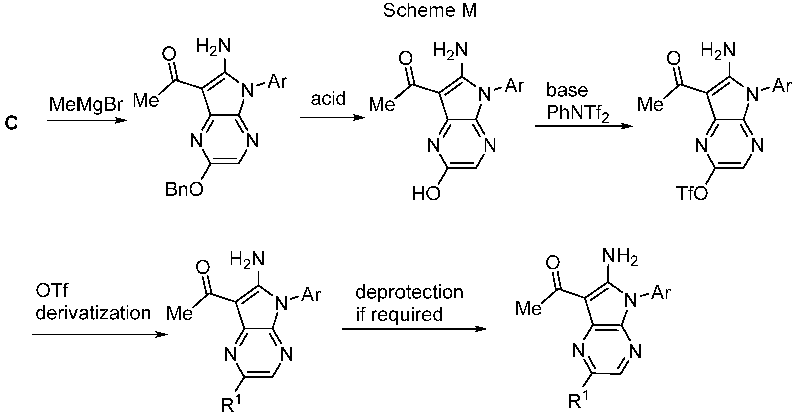

### Method N

Compounds of the present invention may be prepared as shown in Scheme N and described herein. The amino of an aminopyrrole describe herein can be substituted by a proton under diazotization conditions. The nitrile can be hydrolyzed to the carboxamide under acidic or basic conditions to give compound of the present invention. In the case where the arylamine, the R¹ and/or the R² group bear a protecting group, a deprotection step(s) may be required using acid, base and/or fluoride to give compounds of the present invention. Depending on the nature of the N-aryl group, an atropisomeric mixture may be obtained. In such cases it may be necessary to isolate the atropisomer of interest to give compounds of the present invention. Alternatively, an atropisomerically pure intermediate can be isolated and may be derivatized further to give compounds of the present invention.

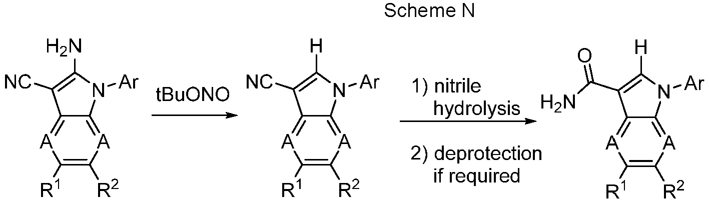

### Method O

Compounds of the present invention may be prepared as shown in Scheme O and described herein. The 2-aminopyridine can be converted to the 2-hydroxypyridine which can be converted to the 2-bromopyridine. The 2-bromo can be substituted with an aromatic amine under palladium-mediated conditions. Depending on the nature of the arylamine, a protecting group may be required to be in place prior to this reaction. The 3-bromo can be substituted with malononitrile under palladium-mediated conditions to afford an aminopyrrole. Hydrolysis of the nitrile can be done under acidic or basic conditions to give compounds of the present invention. In the case where the arylamine group bears a protecting group, a deprotection step(s) may be required using acid, base and/or fluoride to give compounds of the present invention. Depending on the nature of the arylamine, an atropisomeric mixture may be obtained. In such cases it may be necessary to perform chiral chromatography to isolate the atropisomer of interest to give compounds of the present invention. Alternatively, an atropisomerically pure intermediate can be isolated and may be derivatized further to give compounds of the present invention.

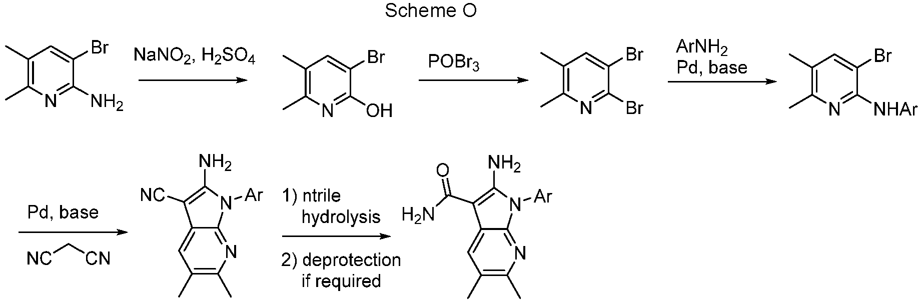

### Methods of Treatment

Compounds of the invention may be used for the treatment of a disease or condition (e.g., a cancer overexpressing CCNE1 or having an inactivating mutation in the *FBXW7* gene) which depend on the activity of Myt1 (Gene name PKMYT1).

The disease or condition may have the symptom of cell hyperproliferation. For example, the disease or condition may be a cancer (e.g., a cancer overexpressing CCNE1 or having an inactivating mutation in the *FBXW7* gene).

Cancers which have a high incidence of CCNE1 overexpression include e.g., uterine cancer, ovarian cancer, breast cancer, stomach cancer, esophageal cancer, lung cancer, and endometrial cancer.

Cancers which have a deficiency in FBXW7 include, e.g., uterine cancer, colorectal cancer, breast cancer, lung cancer, and esophageal cancer.

A compound of the invention may be administered by a route selected from the group consisting of oral, sublingual, buccal, transdermal, intradermal, intramuscular, parenteral, intravenous, intra-arterial, intracranial, subcutaneous, intraorbital, intraventricular, intraspinal, intraperitoneal, intranasal, inhalation, intratumoral, and topical administration.

### Pharmaceutical Compositions

The compounds used in the methods described herein are preferably formulated into pharmaceutical compositions for administration to human subjects in a biologically compatible form suitable for administration in vivo. Pharmaceutical compositions typically include a compound as described herein and a pharmaceutically acceptable excipient. Certain pharmaceutical compositions may include one or more additional pharmaceutically active agents described herein.

The compounds described herein can also be used in the form of the free base, in the form of salts, zwitterions, solvates, or as prodrugs, or pharmaceutical compositions thereof. All forms are within the scope of the invention. The compounds, salts, zwitterions, solvates, prodrugs, or pharmaceutical compositions thereof, may be administered to a patient in a variety of forms depending on the selected route of administration, as will be understood by those skilled in the art. The compounds used in the methods described herein may be administered, for example, by oral, parenteral, buccal, sublingual, nasal, rectal, patch, pump, or transdermal administration, and the pharmaceutical compositions formulated accordingly. Parenteral administration includes intravenous, intraperitoneal, subcutaneous, intramuscular, transepithelial, nasal, intrapulmonary, intrathecal, rectal, and topical modes of administration. Parenteral administration may be by continuous infusion over a selected period of time.

For human use, a compound of the invention can be administered alone or in admixture with a pharmaceutical carrier selected with regard to the intended route of administration and standard pharmaceutical practice. Pharmaceutical compositions for use in accordance with the present invention thus can be formulated in a conventional manner using one or more physiologically acceptable carriers including excipients and auxiliaries that facilitate processing of a compound of the invention into preparations which can be used pharmaceutically.

This invention also includes pharmaceutical compositions which can contain one or more pharmaceutically acceptable carriers. In making the pharmaceutical compositions of the invention, the active ingredient is typically mixed with an excipient, diluted by an excipient or enclosed within such a carrier in the form of, for example, a capsule, sachet, paper, or other container. When the excipient serves as a diluent, it can be a solid, semisolid, or liquid material (e.g., normal saline), which acts as a vehicle, carrier or medium for the active ingredient. Thus, the compositions can be in the form of tablets, powders, lozenges, sachets, cachets, elixirs, suspensions, emulsions, solutions, syrups, and soft and hard gelatin capsules. As is known in the art, the type of diluent can vary depending upon the intended route of administration. The resulting compositions can include additional agents, e.g., preservatives.

The excipient or carrier is selected on the basis of the mode and route of administration. Suitable pharmaceutical carriers, as well as pharmaceutical necessities for use in pharmaceutical formulations, are described in Remington: The Science and Practice of Pharmacy, 21st Ed., Gennaro, Ed., Lippincott Williams & Wilkins (2005), a well-known reference text in this field, and in the USP/NF (United States Pharmacopeia and the National Formulary). Examples of suitable excipients are lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, and methyl cellulose. The formulations can additionally include: lubricating agents, e.g., talc, magnesium stearate, and mineral oil; wetting agents; emulsifying and suspending agents; preserving agents, e.g., methyl- and propylhydroxybenzoates; sweetening agents; and flavoring agents. Other exemplary excipients are described in Handbook of Pharmaceutical Excipients, 6th Edition, Rowe et al., Eds., Pharmaceutical Press (2009).

These pharmaceutical compositions can be manufactured in a conventional manner, e.g., by conventional mixing, dissolving, granulating, dragee-making, levigating, emulsifying, encapsulating, entrapping, or lyophilizing processes. Methods well known in the art for making formulations are found, for example, in Remington: The Science and Practice of Pharmacy, 21st Ed., Gennaro, Ed., Lippincott Williams & Wilkins (2005), and Encyclopedia of Pharmaceutical Technology, eds. J. Swarbrick and J. C. Boylan, 1988-1999, Marcel Dekker, New York. Proper formulation is dependent upon the route of administration chosen. The formulation and preparation of such compositions is well-known to those skilled in the art of pharmaceutical formulation. In preparing a formulation, the active compound can be milled to provide the appropriate particle size prior to combining with the other ingredients. If the active compound is substantially insoluble, it can be milled to a particle size of less than 200 mesh. If the active compound is substantially water soluble, the particle size can be adjusted by milling to provide a substantially uniform distribution in the formulation, e.g., about 40 mesh.

### Dosages

The dosage of the compound used in the methods described herein, or pharmaceutically acceptable salts or prodrugs thereof, or pharmaceutical compositions thereof, can vary depending on many factors, e.g., the pharmacodynamic properties of the compound; the mode of administration; the age, health, and weight of the recipient; the nature and extent of the symptoms; the frequency of the treatment, and the type of concurrent treatment, if any; and the clearance rate of the compound in the animal to be treated. One of skill in the art can determine the appropriate dosage based on the above factors. The compounds used in the methods described herein may be administered initially in a suitable dosage that may be adjusted as required, depending on the clinical response. In general, a suitable daily dose of a compound of the invention will be that amount of the compound that is the lowest dose effective to produce a therapeutic effect. Such an effective dose will generally depend upon the factors described above.

A compound of the invention may be administered to the patient in a single dose or in multiple doses. When multiple doses are administered, the doses may be separated from one another by, for example, 1-24 hours, 1-7 days, 1-4 weeks, or 1-12 months. The compound may be administered according to a schedule or the compound may be administered without a predetermined schedule. An active compound may be administered, for example, 1, 2, 3, 4, 5, 6, 7, 8, 9, 10, 11, or 12 times per day, every 2nd, 3rd, 4th, 5th, or 6th day, 1, 2, 3, 4, 5, 6, or 7 times per week, 1, 2, 3, 4, 5, or 6 times per month, or 1, 2, 3, 4, 5, 6, 7, 8, 9, 10, 11, or 12 times per year. It is to be understood that, for any particular subject, specific dosage regimes should be adjusted over time according to the individual need and the professional judgment of the person administering or supervising the administration of the compositions.

While the attending physician ultimately will decide the appropriate amount and dosage regimen, an effective amount of a compound of the invention may be, for example, a total daily dosage of, e.g., between 0.05 mg and 3000 mg of any of the compounds described herein. Alternatively, the dosage amount can be calculated using the body weight of the patient. Such dose ranges may include, for example, between 10-1000 mg (e.g., 50-800 mg). In some embodiments, 50, 100, 150, 200, 250, 300, 350, 400, 450, 500, 550, 600, 650, 700, 750, 800, 850, 900, 950, or 1000 mg of the compound is administered.

In the methods of the invention, the time period during which multiple doses of a compound of the invention are administered to a patient can vary. For example, in some embodiments, doses of the compounds of the invention are administered to a patient over a time period that is 1-7 days; 1-12 weeks; or 1-3 months. In some embodiments, the compounds are administered to the patient over a time period that is, for example, 4-11 months or 1-30 years. In some embodiments, the compounds are administered to a patient at the onset of symptoms. In any of these embodiments, the amount of compound that is administered may vary during the time period of administration. When a compound is administered daily, administration may occur, for example, 1, 2, 3, 4, 5, 6, 7, 8, 9, 10, 11, or 12 times per day.

### Formulations

A compound identified as capable of treating any of the conditions described herein, using any of the methods described herein, may be administered to patients or animals with a pharmaceutically-acceptable diluent, carrier, or excipient, in unit dosage form. The chemical compounds for use in such therapies may be produced and isolated by any standard technique known to those in the field of medicinal chemistry. Conventional pharmaceutical practice may be employed to provide suitable formulations or compositions to administer the identified compound to patients suffering from a disease or condition. Administration may begin before the patient is symptomatic.

Exemplary routes of administration of the compounds (e.g., a compound of the invention), or pharmaceutical compositions thereof, used in the present invention include oral, sublingual, buccal, transdermal, intradermal, intramuscular, parenteral, intravenous, intra-arterial, intracranial, subcutaneous, intraorbital, intraventricular, intraspinal, intraperitoneal, intranasal, inhalation, and topical administration. The compounds desirably are administered with a pharmaceutically acceptable carrier. Pharmaceutical formulations of the compounds described herein formulated for treatment of the disorders described herein are also part of the present invention.

### Formulations for Oral Administration

The pharmaceutical compositions contemplated by the invention include those formulated for oral administration ("oral dosage forms"). Oral dosage forms can be, for example, in the form of tablets, capsules, a liquid solution or suspension, a powder, or liquid or solid crystals, which contain the active ingredient(s) in a mixture with non-toxic pharmaceutically acceptable excipients. These excipients may be, for example, inert diluents or fillers (e.g., sucrose, sorbitol, sugar, mannitol, microcrystalline cellulose, starches including potato starch, calcium carbonate, sodium chloride, lactose, calcium phosphate, calcium sulfate, or sodium phosphate); granulating and disintegrating agents (e.g., cellulose derivatives including microcrystalline cellulose, starches including potato starch, croscarmellose sodium, alginates, or alginic acid); binding agents (e.g., sucrose, glucose, sorbitol, acacia, alginic acid, sodium alginate, gelatin, starch, pregelatinized starch, microcrystalline cellulose, magnesium aluminum silicate, carboxymethylcellulose sodium, methylcellulose, hydroxypropyl methylcellulose, ethylcellulose, polyvinylpyrrolidone, or polyethylene glycol); and lubricating agents, glidants, and antiadhesives (e.g., magnesium stearate, zinc stearate, stearic acid, silicas, hydrogenated vegetable oils, or talc). Other pharmaceutically acceptable excipients can be colorants, flavoring agents, plasticizers, humectants, buffering agents, and the like.

Formulations for oral administration may also be presented as chewable tablets, as hard gelatin capsules where the active ingredient is mixed with an inert solid diluent (e.g., potato starch, lactose, microcrystalline cellulose, calcium carbonate, calcium phosphate or kaolin), or as soft gelatin capsules where the active ingredient is mixed with water or an oil medium, for example, peanut oil, liquid paraffin, or olive oil. Powders, granulates, and pellets may be prepared using the ingredients mentioned above under tablets and capsules in a conventional manner using, e.g., a mixer, a fluid bed apparatus or a spray drying equipment.

Controlled release compositions for oral use may be constructed to release the active drug by controlling the dissolution and/or the diffusion of the active drug substance. Any of a number of strategies can be pursued in order to obtain controlled release and the targeted plasma concentration versus time profile. In one example, controlled release is obtained by appropriate selection of various formulation parameters and ingredients, including, e.g., various types of controlled release compositions and coatings. Examples include single or multiple unit tablet or capsule compositions, oil solutions, suspensions, emulsions, microcapsules, microspheres, nanoparticles, patches, and liposomes. In some embodiments, compositions include biodegradable, pH, and/or temperature-sensitive polymer coatings.

Dissolution or diffusion-controlled release can be achieved by appropriate coating of a tablet, capsule, pellet, or granulate formulation of compounds, or by incorporating the compound into an appropriate matrix. A controlled release coating may include one or more of the coating substances mentioned above and/or, e.g., shellac, beeswax, glycowax, castor wax, carnauba wax, stearyl alcohol, glyceryl monostearate, glyceryl distearate, glycerol palmitostearate, ethylcellulose, acrylic resins, dl-polylactic acid, cellulose acetate butyrate, polyvinyl chloride, polyvinyl acetate, vinyl pyrrolidone, polyethylene, polymethacrylate, methylmethacrylate, 2-hydroxymethacrylate, methacrylate hydrogels, 1,3 butylene glycol, ethylene glycol methacrylate, and/or polyethylene glycols. In a controlled release matrix formulation, the matrix material may also include, e.g., hydrated methylcellulose, carnauba wax and stearyl alcohol, carbopol 934, silicone, glyceryl tristearate, methyl acrylate-methyl methacrylate, polyvinyl chloride, polyethylene, and/or halogenated fluorocarbon.

The liquid forms in which the compounds and compositions of the present invention can be incorporated for administration orally include aqueous solutions, suitably flavored syrups, aqueous or oil suspensions, and flavored emulsions with edible oils, e.g., cottonseed oil, sesame oil, coconut oil, or peanut oil, as well as elixirs and similar pharmaceutical vehicles.

### Formulations for Parenteral Administration

The compounds described herein for use in the methods of the invention can be administered in a pharmaceutically acceptable parenteral (e.g., intravenous or intramuscular) formulation as described herein. The pharmaceutical formulation may also be administered parenterally (intravenous, intramuscular, subcutaneous or the like) in dosage forms or formulations containing conventional, non-toxic pharmaceutically acceptable carriers and adjuvants. In particular, formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. For example, to prepare such a composition, the compounds of the invention may be dissolved or suspended in a parenterally acceptable liquid vehicle. Among acceptable vehicles and solvents that may be employed are water, water adjusted to a suitable pH by addition of an appropriate amount of hydrochloric acid, sodium hydroxide or a suitable buffer, 1,3-butanediol, Ringer's solution and isotonic sodium chloride solution. The aqueous formulation may also contain one or more preservatives, for example, methyl, ethyl, or n-propyl p-hydroxybenzoate. Additional information regarding parenteral formulations can be found, for example, in the United States Pharmacopeia-National Formulary (USP-NF).

The parenteral formulation can be any of the five general types of preparations identified by the USP-NF as suitable for parenteral administration:
(1) Drug Injection: a liquid preparation that is a drug substance (e.g., a compound of the invention), or a solution thereof;
(2) Drug for Injection: the drug substance (e.g., a compound of the invention) as a dry solid that will be combined with the appropriate sterile vehicle for parenteral administration as a drug injection;
(3) Drug Injectable Emulsion: a liquid preparation of the drug substance (e.g., a compound of the invention) that is dissolved or dispersed in a suitable emulsion medium;
(4) Drug Injectable Suspension: a liquid preparation of the drug substance (e.g., a compound of the invention) suspended in a suitable liquid medium; and
(5) Drug for Injectable Suspension: the drug substance (e.g., a compound of the invention) as a dry solid that will be combined with the appropriate sterile vehicle for parenteral administration as a drug injectable suspension.

Formulations for parenteral administration include solutions of the compound prepared in water suitably mixed with a surfactant, e.g., hydroxypropylcellulose. Dispersions can also be prepared in glycerol, liquid polyethylene glycols, DMSO and mixtures thereof with or without alcohol, and in oils. Under ordinary conditions of storage and use, these preparations may contain a preservative to prevent the growth of microorganisms. Conventional procedures and ingredients for the selection and preparation of suitable formulations are described, for example, in Remington: The Science and Practice of Pharmacy, 21st Ed., Gennaro, Ed., Lippincott Williams & Wilkins (2005) and in The United States Pharmacopeia: The National Formulary (USP 36 NF31), published in 2013.

Formulations for parenteral administration may, for example, contain excipients, sterile water, or saline, polyalkylene glycols, e.g., polyethylene glycol, oils of vegetable origin, or hydrogenated napthalenes. Biocompatible, biodegradable lactide polymer, lactide/glycolide copolymer, or polyoxyethylene-polyoxypropylene copolymers may be used to control the release of the compounds. Other potentially useful parenteral delivery systems for compounds include ethylene-vinyl acetate copolymer particles, osmotic pumps, implantable infusion systems, and liposomes. Formulations for inhalation may contain excipients, for example, lactose, or may be aqueous solutions containing, for example, polyoxyethylene-9-lauryl ether, glycocholate and deoxycholate, or may be oily solutions for administration in the form of nasal drops, or as a gel.

The parenteral formulation can be formulated for prompt release or for sustained/extended release of the compound. Exemplary formulations for parenteral release of the compound include: aqueous solutions, powders for reconstitution, cosolvent solutions, oil/water emulsions, suspensions, oil-based solutions, liposomes, microspheres, and polymeric gels.

### Combinations

Compounds of the present invention may be administered to a subject in combination with one or more additional agents, e.g.:
(a) a cytotoxic agent;
(b) an antimetabolite;
(c) an alkylating agent;
(d) an anthracycline;
(e) an antibiotic;
(f) an anti-mitotic agent;
(g) a hormone therapy;
(h) a signal transduction inhibitor;
(i) a gene expression modulator;
(j) an apoptosis inducer;
(k) an angiogenesis inhibitor;
(l) an immunotherapy agent;
(m) a DNA damage repair inhibitor;
   or
a combination thereof.

The cytotoxic agent may be, e.g., actinomycin-D, alemtuzumab, alitretinoin, allopurinol, altretamine, amifostine, amphotericin, amsacrine, arsenic trioxide, asparaginase, azacitidine, azathioprine, Bacille Calmette-Guérin (BCG), bendamustine, bexarotene, bevacuzimab, bleomycin, bortezomib, busulphan, capecitabine, carboplatin, carfilzomib, carmustine, cetuximab, cisplatin, chlorambucil, cladribine, clofarabine, colchicine, crisantaspase, cyclophosphamide, cyclosporine, cytarabine, cytochalasin B, dacarbazine, dactinomycin, darbepoetin alfa, dasatinib, daunorubicin, 1-dehydrotestosterone, denileukin, dexamethasone, dexrazoxane, dihydroxy anthracin dione, disulfiram, docetaxel, doxorubicin, emetine, epirubicin, erlotinib, epigallocatechin gallate, epoetin alfa, estramustine, ethidium bromide, etoposide, everolimus, filgrastim, finasunate, floxuridine, fludarabine, flurouracil (5-FU), fulvestrant, ganciclovir, geldanamycin, gemcitabine, glucocorticoids, gramicidin D, histrelin acetate, hydroxyurea, ibritumomab, idarubicin, ifosfamide, imatinib, irinotecan, interferons, interferon alfa-2a, interferon alfa-2b, ixabepilone, lactate dehydrogenase A (LDH-A), lenalidomide, letrozole, leucovorin, levamisole, lidocaine, lomustine, mechlorethamine, melphalan, 6-mercaptopurine, mesna, methotrexate, methoxsalen, metoprine, metronidazole, mithramycin, mitomycin-C, mitoxantrone, nandrolone, nelarabine, nilotinib, nofetumomab, oprelvekin, oxaliplatin, paclitaxel, pemetrexed, pentostatin, palifermin, pamidronate, pegademase, pegaspargase, pegfilgrastim, pemetrexed disodium, plicamycin, porfimer sodium, procaine, procarbazine, propranolol, puromycin, quinacrine, radicicol, radioactive isotopes, raltitrexed, rapamycin, rasburicase, salinosporamide A, sargramostim, sunitinib, temozolomide, teniposide, tetracaine, 6-thioguanine, thiotepa, topotecan, toremifene, trastuzumab, treosulfan, tretinoin, valrubicin, vinblastine, vincristine, vindesine, vinorelbine, zoledronate, or a combination thereof.

The antimetabolites may be, e.g., methotrexate, 6-mercaptopurine, 6-thioguanine, cytarabine, 5-fluorouracil decarbazine, cladribine, pemetrexed, gemcitabine, capecitabine, hydroxyurea, mercaptopurine, fludarabine, pralatrexate, clofarabine, cytarabine, decitabine, floxuridine, nelarabine, trimetrexate, thioguanine, pentostatin, or a combination thereof.

The alkylating agent may be, e.g., mechlorethamine, thiotepa, chlorambucil, melphalan, carmustine (BSNU), lomustine (CCNU), cyclothosphamide, busulfan, dibromomannitol, streptozotocin, mitomycin C, cis-dichlorodiamine platinum (II) (DDP) cisplatin, altretamine, cyclophosphamide, ifosfamide, hexamethylmelamine, altretamine, procarbazine, dacarbazine, temozolomide, streptozocin, carboplatin, cisplatin, oxaliplatin, uramustine, bendamustine, trabectedin, semustine, or a combination thereof.

The anthracycline may be, e.g., daunorubicin, doxorubicin, aclarubicin, aldoxorubicin, amrubicin, annamycin, carubicin, epirubicin, idarubicin, mitoxantrone, valrubicin, or a combination thereof.

The antibiotic may be, e.g., dactinomycin, bleomycin, mithramycin, anthramycin (AMC), ampicillin, bacampicillin, carbenicillin, cloxacillin, dicloxacillin, flucloxacillin, mezlocillin, nafcillin, oxacillin, piperacillin, pivampicillin, pivmecillinam, ticarcillin, aztreonam, imipenem, doripenem, ertapenem, meropenem, cephalosporins, clarithromycin, dirithromycin, roxithromycin, telithromycin, lincomycin, pristinamycin, quinupristin, amikacin, gentamicin, kanamycin, neomycin, netilmicin, paromomycin, tobramycin, streptomycin, sulfamethizole, sulfamethoxazole, sulfisoxazole, demeclocycline, minocycline, oxytetracycline, tetracycline, penicillin, amoxicillin, cephalexin, erythromycin, clarithromycin, azithromycin, ciprofloxacin, levofloxacin, ofloxacin, doxycycline, clindamycin, metronidazole, tigecycline, chloramphenicol, metronidazole, tinidazole, nitrofurantoin, vancomycin, teicoplanin, telavancin, linezolid, cycloserine, rifamycins, polymyxin B, bacitracin, viomycin, capreomycin, quinolones, daunorubicin, doxorubicin, 4'-deoxydoxorubicin, epirubicin, idarubicin, plicamycin, mitomycin-c, mitoxantrone, or a combination thereof.

The anti-mitotic agent may be, e.g., vincristine, vinblastine, vinorelbine, docetaxel, estramustine, ixabepilone, paclitaxel, maytansinoid, a dolastatin, a cryptophycin, or a combination thereof.

The signal transduction inhibitor may be, e.g., imatinib, trastuzumab, erlotinib, sorafenib, sunitinib, temsirolimus, vemurafenib, lapatinib, bortezomib, cetuximab panitumumab, matuzumab, gefitinib, STI 571, rapamycin, flavopiridol, imatinib mesylate, vatalanib, semaxinib, motesanib, axitinib, afatinib, bosutinib, crizotinib, cabozantinib, dasatinib, entrectinib, pazopanib, lapatinib, vandetanib, or a combination thereof.

The gene expression modulator may be, e.g., a siRNA, a shRNA, an antisense oligonucleotide, an HDAC inhibitor, or a combination thereof. An HDAC inhibitor may be, e.g., trichostatin A, trapoxin B, valproic acid, vorinostat, belinostat, LAQ824, panobinostat, entinostat, tacedinaline, mocetionstat, givinostat, resminostat, abexinostat, quisinostat, rocilinostat, practinostat, CHR-3996, butyric acid, phenylbutyric acid, 4SC202, romidepsin, sirtinol, cambinol, EX-527, nicotinamide, or a combination thereof. An antisense oligonucleotide may be, e.g., custirsen, apatorsen, AZD9150, trabadersen, EZN-2968, LErafAON-ETU, or a combination thereof. An siRNA may be, e.g., ALN-VSP, CALAA-01, Atu-027, SPC2996, or a combination thereof.

The hormone therapy may be, e.g., a luteinizing hormone-releasing hormone (LHRH) antagonist. The hormone therapy may be, e.g., firmagon, leuproline, goserelin, buserelin, flutamide, bicalutadmide, ketoconazole, aminoglutethimide, prednisone, hydroxyl-progesterone caproate, medroxy-progesterone acetate, megestrol acetate, diethylstil-bestrol, ethinyl estradiol, tamoxifen, testosterone propionate, fluoxymesterone, flutamide, raloxifene, droloxifene, iodoxyfene, 4-hydroxytamoxifen, trioxifene, keoxifene, LY117018, onapristone, toremifine citrate, megestrol acetate, exemestane, fadrozole, vorozole, letrozole, anastrozole, nilutamide, tripterelin, histerelin, arbiraterone, medroxyprogesterone acetate, diethylstilbestrol, premarin, fluoxymesterone, tretinoin, fenretinide, troxacitabine, or a combination thereof.

The apoptosis inducers may be, e.g., a recombinant human TNF-related apoptosis-inducing ligand (TRAIL), camptothecin, bortezomib, etoposide, tamoxifen, or a combination thereof.

The angiogenesis inhibitors may be, e.g., sorafenib, sunitinib, pazopanib, everolimus or a combination thereof.

The immunotherapy agent may be, e.g., a monoclonal antibody, cancer vaccine (e.g., a dendritic cell (DC) vaccine), oncolytic virus, cytokine, adoptive T cell therapy, Bacille Calmette-Guérin (BCG), GM-CSF, thalidomide, lenalidomide, pomalidomide, imiquimod, or a combination thereof. The monoclonal antibody may be, e.g., anti-CTLA4, anti-PD1, anti-PD-L1, anti-LAG3, anti-KIR, or a combination thereof. The monoclonal antibody may be, e.g., alemtuzumab, trastuzumab, ibritumomab tiuxetan, brentuximab vedotin, trastuzumab, ado-trastuzumab emtansine, blinatumomab, bevacizumab, cetuximab, pertuzumab, panitumumab, ramucirumab, obinutuzumab, ofatumumab, rituximab, pertuzumab, tositumomab, gemtuzumab ozogamicin, tositumomab, or a combination thereof. The cancer vaccine may be, e.g., Sipuleucel-T, BioVaxID, NeuVax, DCVax, SuVaxM, CIMAvax^{®}, Provenge,^{®}, hsp110 chaperone complex vaccine, CDX-1401, MIS416, CDX-110, GVAX Pancreas, HyperAcute^{™} Pancreas, GTOP-99 (MyVax^{®}), or Imprime PGG^{®}. The oncolytic virus may be, e.g., talimogene laherparepvec. The cytokine may be, e.g., IL-2, IFNα, or a combination thereof. The adoptive T cell therapy may be, e.g., tisagenlecleucel, axicabtagene ciloleucel, or a combination thereof.

The DNA damage repair inhibitor may be, e.g., a PARP inhibitor, a cell checkpoint kinase inhibitor, or a combination thereof. The PARP inhibitor may be, e.g., olaparib, rucaparib, veliparib (ABT-888), niraparib (ZL-2306), iniparib (BSI-201), talazoparib (BMN 673), 2X-121, CEP-9722, KU-0059436 (AZD2281), PF-01367338 or a combination thereof. The cell checkpoint kinase inhibitor may be, e.g., MK-1775 or AZD1775, AZD7762, LY2606368, PF-0477736, AZD0156, GDC-0575, ARRY-575, CCT245737, PNT-737 or a combination thereof.

### EXAMPLES

The following examples were meant to illustrate the invention. They were not meant to limit the invention in any way. Reactions were typically performed at room temperature (rt) under a nitrogen atmosphere using dry solvents (Sure/Seal^{™}) if not described otherwise in the Examples below. Reactions were monitored by TLC or by injection of a small aliquot on a Waters Acquity-H UPLC^{®} Class system using an Acquity^{®} UPLC HSS C18 2.1x30mm column eluting with a gradient (1.86 min) of acetonitrile (15% to 98%) in water (both containing 0.1% formic acid). Purifications by preparative HPLC were performed on a Teledyne Isco Combi *Flash*^{®}*EZ Prep* system using either Phenomenex Gemini^{®} 5µm NX-C18 110Å 150 x 21.2 mm column at a flow of 40 mL/min over 12 min (<100mg or multiple injections of <100mg) or HP C18 *RediSep*^{®}*Rf* gold column (>100mg) eluting with an appropriate gradient of acetonitrile in water (both containing 0.1% formic acid) unless otherwise specified. The gradient was selected based on the retention time observed by reaction monitoring on the Waters Acquity-H UPLC^{®} Class system (see above). Fractions containing the desired compounds were combined and finally lyophilized. Purifications by silica gel chromatography were performed on a Teledyne Isco Combi Flash^{®}*Rf* system using *RediSep*^{®}*Rf* silica gel columns of appropriate sizes. Purity of final **Compounds** was assessed by injection of a small aliquot on a Waters Acquity-H UPLC^{®} Class system using an Acquity^{®} UPLC BEH C18 2.1x50mm column eluting with a gradient (7 min) of acetonitrile (2% to 98%) in water (both containing 0.1% formic acid).

### Abbreviations

Abbreviations and terms that are commonly used in the fields of organic chemistry, medicinal chemistry, pharmacology, and medicine and are well known to practitioners in these fields are used herein. Representative abbreviations and definitions are provided below:
Ac is acetyl [CH₃C(O)-];
ACN is acetonitrile;
Ac₂O is acetic anhydride;
AcOH is acetic acid;
Ar is aryl;
BOC is tert-butyloxycarbonyl;
n-BuLi is n-butyl lithium;
cmpd is compound;
conc. is concentrated;
DCM is dichloromethane;
DIPEA is diisopropylethyl amine;
DMAP is 4-(Dimethylamino)pyridine;
DME is dimethoxyethane;
DMF is N,N-dimethylformamide;
DMSO is dimethyl sulfoxide;
EtOAc is ethyl acetate;
EtOH is ethanol;
h is hour;
HATU is 1-[Bis(dimethylamino)methylene]-1H-1,2,3-triazolo[4,5-b]pyridinium 3-oxide hexafluorophosphate;
HCl is hydrochloric acid;
Hex is hexanes;
HPLC is high performance liquid chromatography;
IPA is isopropanol;
LCMS is HPLC with mass spectral detection;
LiHMDS is lithium hexamethyldisilazane;
M is molar; mmol is millimole;
Me is methyl;
MeCN is acetonitrile;
MeMgBr is methylmagnesium bromide;
MeMgCl is methylmagnesium chloride;
MeOH is methanol;
MOM is methoxymethyl;
min is minute;
N is normal;
NBS is N-bromosuccinimide;
NCS is N-chlorosuccinimide;
NIS is N-iodosuccinimide;
NMP is N-methyl pyrrolidinone;
NMR is nuclear magnetic resonance spectroscopy;
PdCl₂(dppf) is [1,1-Bis(diphenylphosphino)ferrocene]dichloropalladium(II);
PdCl₂(dppf).CH₂Cl₂ is [1,1'-Bis(diphenylphosphino)ferrocene]dichloropalladium(II), complex with dichloromethane;
Pd₂(dba)₃ is tris(dibenzylideneacetone)dipalladium(0);
Pd-PEPPSI^{™}-SIPr is (1,3-Bis(2,6-diisopropylphenyl)imidazolidene) ( 3-chloropyridyl) palladium(II) dichloride;
Ph is phenyl;
PIV-Cl is pivaloyl chloride, Trimethylacetyl chloride;
Reagent alcohol is a mixture of 90% ethanol, 5% isopropanol and 5% methanol;
rt is room temperature;
sat. is saturated;
tBu is tert-butyl;
Tf is trifluoromethanesulfonate;
TFA is trifluoroacetic acid;
THF is tetrahydrofuran;
TMS is trimethylsilyl;
Ts is p-toluenesulfonyl;
Xantphos is 4,5-Bis(diphenylphosphino)-9,9-dimethylxanthene.
In the examples 1 inch = 2,54 cm and 1 atm = 1013 mbar.

### Example 1. Preparation of Compounds

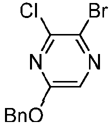

### Intermediate B (5-benzyloxy-2-bromo-3-chloro-pyrazine)

Step 1. Sodium nitrite (40 g, 580 mmol) was added portion wise to a solution of 5-bromo-6-chloro-pyrazin-2-amine (110 g, 528 mmol) in sulfuric acid (770 mL) at 0°C under mechanical stirring. The resulting thick mixture was stirred at 0°C for 1 h and was then slowly poured in 6 L of cold water containing crushed ice maintaining temperature below 30°C. The resulting precipitate was collected by filtration, washed with water then dried by co-evaporation with toluene under vacuum twice to give 5-bromo-6-chloro-pyrazin-2-ol (104.6 g, 95% yield) as a pale beige solid.

Step 2. Benzyl bromide (48 mL, 404 mmol) was added dropwise to a suspension of 5-bromo-6-chloro-pyrazin-2-ol (80 g, 382 mmol) and silver carbonate (216 g, 778 mmol) in toluene (2 L). After stirring for 3 h, the suspension was filtered on Celite. The filtrate was evaporated to dryness to provide a yellow oil that was dissolved in warm EtOH. After slow addition of water under sonication, the precipitate was collected by filtration to provide 5-benzyloxy-2-bromo-3-chloropyrazine (85.2 g, 75% yield) as a beige solid. ¹H NMR (400 MHz, DMSO-d6) δ 8.27 (s, 1H), 7.51 - 7.46 (m, 2H), 7.44 - 7.33 (m, 3H), 5.36 (s, 2H).

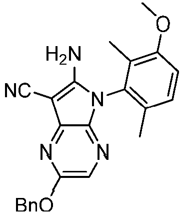

### Intermediate C (6-amino-2-benzyloxy-5-(3-methoxy-2,6-dimethyl-phenyl)pyrrolo[2,3-blpyrazine-7-carbonitrile)

Step 1. To a solution of **Intermediate B** (90 g, 300 mmol) in toluene (1350 mL) were added potassium tert-butoxide (45.0 g, 401 mmol), 3-methoxy-2,6-dimethyl-aniline (48 g, 318 mmol), Pd₂(dba)₃ (14.4 g, 15.7 mmol) and Xantphos (18.0 g, 31 mmol). The mixture was degassed in vacuo and back filled with nitrogen. The resulting mixture was stirred at 80°C for 45 min and then concentrated in vacuo. The residue was dissolved in DCM (500 mL), 200 g of silica gel was added, and the suspension was evaporated to dryness under vacuum. The residue was purified on a pad of silica gel (1 kg of silica gel) eluting with a gradient of 0 to 15% EtOAc in hexanes to provide 5-benzyloxy-3-chloro-N-(3-methoxy-2,6-dimethyl-phenyl)pyrazin-2-amine (108.4 g, 98% yield) as a pale beige solid.

Step 2. To a solution of propanedinitrile (42.1 g, 637 mmol) in DME (1800 mL) was added portion wise NaH (25.0 g, 628 mmol, 60% dispersion in mineral oil). The resulting mixture was stirred for 30 min, then 5-benzyloxy-3-chloro-N-(3-methoxy-2,6-dimethyl-phenyl)pyrazin-2-amine (115 g, 311 mmol) in DME (500 mL) and Pd(PPh₃)₄ (17.7 g, 15.3 mmol) were added. The resulting mixture was stirred at reflux for 2 h, and then concentrated in vacuo to 1 L. Water (1 L) was added slowly and the resulting biphasic mixture was stirred for 18 h with a mechanical stirrer. The resulting solid was recovered by filtration, washed with water, and dried in vacuo. Trituration in DCM provided the first batch of the desired material as a beige solid isolated by filtration. The mother liquor was concentrated in vacuo, and the residue was purified by silica gel chromatography (dry load) eluting with a gradient of 10 to 60% EtOAc in hexanes to provide a second batch of the desired material. The two batches were combined to provide 6-amino-2-benzyloxy-5-(3-methoxy-2,6-dimethyl-phenyl)pyrrolo[2,3-b]pyrazine-7-carbonitrile (103.1 g, 83% yield) as a beige solid. ¹H NMR (400 MHz, Chloroform-d) δ 7.60 (s, 1H), 7.53 - 7.47 (m, 2H), 7.42 - 7.34 (m, 2H), 7.33 - 7.27 (m, 1H), 7.21 = 7.15 (m, 1H), 6.94 (d, J = 8.5 Hz, 1H), 5.45 (s, 2H), 4.91 (s, 2H), 3.84 (s, 3H), 1.90 (d, J = 0.7 Hz, 3H), 1.83 (s, 3H). MS: [M+1]: 400.4.

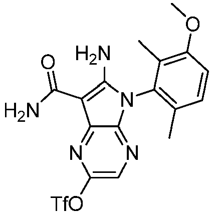

### Intermediate D ([6-amino-7-carbamoyl-5-(3-methoxy-2,6-dimethyl-phenyl)pyrrolo[2,3-b]pyrazin-2-yl] trifluoromethanesulfonate)

Step 1. A solution of **Intermediate C** (83 g, 208 mmol) in sulfuric acid (550 mL) was stirred with a mechanical stirrer for 18 h. The thick brown mixture was poured slowly in icy water (2 L) in an ice bath maintaining internal temperature below 20 °C while stirred with a mechanical stirrer. A pale-yellow solid precipitated out. The resulting suspension in an ice bath was slowly neutralized to basic pH with aqueous ammonium hydroxide (28% solution; 850 mL) while maintaining the internal temperature below 40°C. The precipitate was collected by filtration, washed with water, and dried in vacuo to provide 6-amino-2-hydroxy-5-(3-methoxy-2,6-dimethyl-phenyl)pyrrolo[2,3-b]pyrazine-7-carboxamide (65.1 g, 96% yield) as a pale beige solid.

Step 2. To a solution of 6-amino-2-hydroxy-5-(3-methoxy-2,6-dimethyl-phenyl)pyrrolo[2,3-b]pyrazine-7-carboxamide (30.5 g, 93.2 mmol) and Cs₂CO₃ (34.9 g, 107 mmol) in DMF (300 mL) was added 1,1,1-trifluoro-N-phenyl-N-(trifluoromethylsulfonyl)methanesulfonamide (36.6 g, 103 mmol). The reaction mixture was stirred for 1h, diluted with water (900 mL) and extracted with EtOAc (3x300 mL). The combined organic extracts were washed with water, brine, dried over Na₂SO₄, filtered and concentrated in vacuo. The residue was purified by silica gel chromatography eluting with a gradient of 20 to 100% EtOAc in hexanes to provide [6-amino-7-carbamoyl-5-(3-methoxy-2,6-dimethyl-phenyl)pyrrolo[2,3-b]pyrazin-2-yl] trifluoromethanesulfonate (28 g, 65% yield) as an off-white solid. ¹H NMR (400 MHz, Chloroform-d) δ 7.75 (s, 1H), 7.22 m, 2H), 6.97 (d, J = 8.5 Hz, 1H), 6.37 (s, 2H), 5.49 (s, 1H), 3.86 (s, 3H), 1.91 (s, 3H), 1.84 (s, 3H). MS: [M+1]: 528.4.

Chiral SFC separation of **Intermediate D** (7.0 g, 15 mmol) (Instrument: Waters Prep 100 SFC-MS; Column: Phenomenex Lux Cellulose-2, 30 x 250 mm, 5 µm; Conditions: Isocratic at 25% MeOH with 75% CO2; Flow Rate: 70 mL/min) provided **Intermediate D1** and **Intermediate D2.**

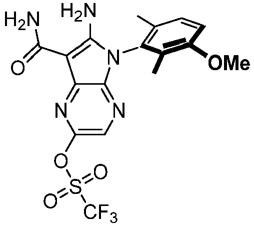

**Intermediate D1** from chiral SFC separation of **Intermediate D.** Peak 1 (retention time 4.95 min, 99.77%): *R*-6-amino-7-carbamoyl-5-(3-methoxy-2,6-dimethylphenyl)-5H-pyrrolo[2,3-b]pyrazin-2-yl trifluoromethanesulfonate (1.93 g) as a white fluffy solid. 1H NMR (400 MHz, DMSO-d6) δ 7.94 (s, 1H), 7.89 (br s, 2H), 7.50 (br s, 1H), 7.28 (dt, J = 8.4, 0.8 Hz, 1H), 7.14 (d, J = 8.5 Hz, 1H), 6.80 (br s, 1H), 3.85 (s, 3H), 1.82 (d, J = 0.7 Hz, 3H), 1.74 (s, 3H). 19F NMR (376 MHz, DMSO-d6) δ - 72.83. MS: [M+1]: 460.0.

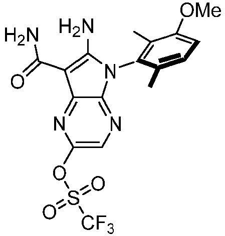

**Intermediate D2** from chiral SFC separation of **Intermediate D.** Peak 2 (retention time 6.44 min, 99.01%): S-6-amino-7-carbamoyl-5-(3-methoxy-2,6-dimethylphenyl)-5H-pyrrolo[2,3-b]pyrazin-2-yl trifluoromethanesulfonate (1.95 g) as a white fluffy solid. 1H NMR (400 MHz, DMSO-d6) δ 7.94 (s, 1H), 7.89 (br s, 2H), 7.50 (br s, 1H), 7.28 (dt, J = 8.5, 0.7 Hz, 1H), 7.14 (d, J = 8.5 Hz, 1H), 6.80 (br s, 1H), 3.85 (s, 3H), 1.82 (d, J = 0.7 Hz, 3H), 1.74 (s, 3H). 19F NMR (376 MHz, DMSO-d6) δ - 72.83. MS: [M+1]: 460.0.

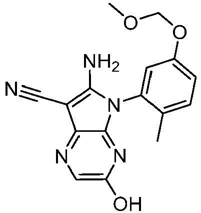

### Intermediate E (6-amino-3-hydroxy-5-[5-(methoxymethoxy)-2-methyl-phenyl]pyrrolo[2,3-b]pyrazine-7-carbonitrile)

Step 1. To a solution of 5-benzyloxy-2-bromo-3-chloro-pyrazine (5.08 g, 17.0 mmol) and 5-(methoxymethoxy)-2-methyl-aniline (5.70 g, 34.1 mmol) in THF (40 mL) at 0 °C was added dropwise potassium tert-butoxide in THF (1 M, 48 mL). After stirring for 90 min at 0 °C, the reaction mixture was quenched with saturated NH₄Cl, diluted with water, and extracted with EtOAc (3x). The combined organic extracts were washed with water, brine, dried over Na₂SO₄, filtered and concentrated in vacuo. The crude residue was purified by silica gel chromatography (dry load) eluting with a gradient of 0 to 20% EtOAc in hexanes to provide 6-benzyloxy-3-bromo-N-[5-(methoxymethoxy)-2-methyl-phenyl]pyrazin-2-amine (1.80 g, 25% yield) as a pale yellow solid.

Step 2. To a suspension of NaH (631 mg, 16.5 mmol, 60% dispersion in mineral oil) in THF (28 mL) at 0 °C, was added malononitrile (556 mg, 8.42 mmol) in THF (12 mL) dropwise. After stirring at 0 °C for 30 min, the ice bath was removed and 6-benzyloxy-3-bromo-N-[5-(methoxymethoxy)-2-methyl-phenyl]pyrazin-2-amine (1.80 g, 4.18 mmol) and Pd(PPh₃)₄ (242 mg, 209 µmol) were added. The resulting mixture was flushed with nitrogen, and stirred at 60 °C for 1 h. The resulting mixture was cooled down to rt, poured slowly in saturated aqueous NH₄Cl and then was extracted with EtOAc (2x). The combined organic extracts were washed with water, brine, dried over Na₂SO₄, filtered and concentrated in vacuo. The residue was purified by silica gel chromatography (dry load) eluting with a gradient of 0 to 100% EtOAc in hexanes to provide 6-amino-3-benzyloxy-5-[5-(methoxymethoxy)-2-methyl-phenyl]pyrrolo[2,3-b]pyrazine-7-carbonitrile (1.63 g, 94% yield) as a light tan solid.

Step 3. A mixture 6-amino-3-benzyloxy-5-[5-(methoxymethoxy)-2-methylphenyl]pyrrolo[2,3-b]pyrazine-7-carbonitrile (1.39 g, 3.35 mmol) and palladium on carbon (350 mg, 0.329 mmol, 10% w/w) was flushed with nitrogen and MeOH (40 mL) was added. The reaction mixture was flushed with hydrogen and stirred under a hydrogen atmosphere (1 atm) for 2 h, then flushed with nitrogen, filtered on a Celite pad using DCM and MeOH. The filtrate was concentrated in vacuo, then dried to afford 6-amino-3-hydroxy-5-[5-(methoxymethoxy)-2-methylphenyl]pyrrolo[2,3-b]pyrazine-7-carbonitrile (1.12 g, 100%) as an ochre solid. MS: [M+1]: 326.1.

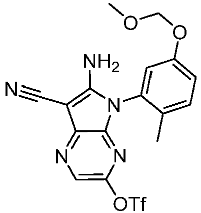

### Intermediate F ([6-amino-7-cyano-5-[5-(methoxymethoxy)-2-methyl-phenyl]pyrrolo[2,3-b]pyrazin-3-yl] trifluoromethanesulfonate)

Step 1. To a solution of 6-amino-3-hydroxy-5-[5-(methoxymethoxy)-2-methylphenyl]pyrrolo[2,3-b]pyrazine-7-carbonitrile (1.12 g, 3.44 mmol) and 1,1,1-trifluoro-N-phenyl-N-(trifluoromethylsulfonyl)methanesulfonamide (1.49 g, 4.17 mmol) in THF (45 mL) was added Et₃N (1.23 g, 12.2 mmol, 1.70 mL). The reaction mixture was stirred for 18 h, then concentrated in vacuo. The residue was purified by silica gel chromatography (dry load) eluting with a gradient of 0 to 100% EtOAc in hexanes to afford [6-amino-7-cyano-5-[5-(methoxymethoxy)-2-methylphenyl]pyrrolo[2,3-b]pyrazin-3-yl] trifluoromethanesulfonate (1.72 g, 100%) as a dark yellow solid. ¹H NMR (400 MHz, DMSO-d6) δ 8.38 (s, 1H), 8.13 (br s, 2H), 7.41 (dd, J = 8.5, 0.9 Hz, 1H), 7.19 (dd, J = 8.5, 2.6 Hz, 1H), 7.11 (d, J = 2.6 Hz, 1H), 5.22 (d, J = 6.8 Hz, 1H), 5.17 (d, J = 6.8 Hz, 1H), 3.38 (s, 3H), 1.90 (s, 3H). MS: [M+1]: 458.0.

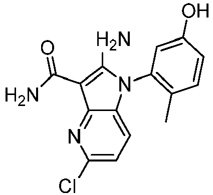

### Intermediate G (2-amino-5-chloro-1-(5-hydroxy-2-methyl-phenyl)pyrrolo[3,2-b]pyridine-3-carboxamide)

Step 1. To a solution of propanedinitrile (11.8 g, 179 mmol) in DME (200 mL) at 0 °C was added portion wise NaH (7.0 g, 175.00 mmol, 60% dispersion in mineral oil). 3-bromo-2,6-dichloro-pyridine (20 g, 88.15 mmol) was then added and the resulting mixture was stirred at 90 °C for 6h. The reaction mixture was cooled to rt, neutralized with 1M HCl, diluted with water and extracted with EtOAc (3x). The combined organic extracts were washed with brine, filtered and concentrated in vacuo. The residue was purified by preparative HPLC in multiple batches. The desired fractions were combined and concentrated to dryness to provide 2-(3-bromo-6-chloro-2-pyridyl)propanedinitrile (8.0 g, 35% yield) as an off-white solid.

Step 2. To a solution of 2-(3-bromo-6-chloro-2-pyridyl)propanedinitrile (5 g, 19.5 mmol) in DMF (75 mL) were added Pd₂(dba)₃ (1.75g, 1.91 mmol), 5-(methoxymethoxy)-2-methyl-aniline (3.6 g, 21.53 mmol), Cs₂CO₃ (12.7 g) and Xantphos (1.12 g, 1.94 mmol). The mixture was degassed in vacuo and back filled with nitrogen 3 times. The resulting mixture was stirred at 130 °C for 8 h then cooled to rt. The resulting mixture was diluted with water and extracted with EtOAc (3x). The combined organic extracts were washed with brine, dried over Na₂SO₄, filtered and concentrated in vacuo. The residue was purified by silica gel chromatography eluting with a gradient of 10 to 60% EtOAc in hexanes. The desired fractions were concentrated to dryness, and the residue was triturated with DCM to provide 2-amino-5-chloro-1-[5-(methoxymethoxy)-2-methyl-phenyl]pyrrolo[3,2-b]pyridine-3-carbonitrile (2.2 g, 33% yield) as an off-white solid.

Step 3. To the suspension of 2-amino-5-chloro-1-[5-(methoxymethoxy)-2-methylphenyl]pyrrolo[3,2-b]pyridine-3-carbonitrile (2.20 g, 6.42 mmol) in DCM (5 mL) was added hydrogen chloride 4M in dioxane (4 M, 5 mL). The mixture was stirred for 30 min. The volatiles were removed in vacuo to provide 2-amino-5-chloro-1-(5-hydroxy-2-methylphenyl)pyrrolo[3,2-b]pyridine-3-carbonitrile HCl salt (2.10 g, 98% yield) as an off-white solid.

Step 4. The solution of 2-amino-5-chloro-1-(5-hydroxy-2-methyl-phenyl)pyrrolo[3,2-b]pyridine-3-carbonitrile (2.3 g, 7.70 mmol) in conc. sulfuric acid (25 mL) was stirred at rt for 1 h. Then it was diluted with crushed ice, basified with conc. aqueous ammonia to pH 8. The suspension was filtered. The precipitate was washed with water, dried in vacuo to provide an off-white mixture mainly containing 2-amino-5-chloro-1-(5-hydroxy-2-methyl-phenyl)pyrrolo[3,2-b]pyridine-3-carboxamide (2 g, 82 % yield) which was used directly in the next step without further purification. ¹H NMR (400 MHz, DMSO-d6) δ 9.77 (s, 1H), 7.42 (s, 1H), 7.30 (d, *J* = 8.3 Hz, 1H), 7.16 (m, 3H), 6.98 - 6.91 (m, 2H), 6.87 (d, *J* = 8.1 Hz, 1H), 6.71 (d, *J* = 2.6 Hz, 1H), 1.81 (s, 3H). MS: [M+1]: 317.1.

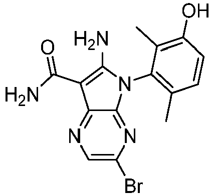

### Intermediate H (6-amino-3-bromo-5-(3-hydroxy-2,6-dimethyl-phenyl)pyrrolo[2,3-b]pyrazine-7-carboxamide)

Step 1. Propanedinitrile (26.6 g, 403 mmol) was added dropwise with vigorous stirring to a suspension of NaH 60% dispersion in mineral oil (16 g, 418 mmol) in DME (600 mL). The mixture was stirred for 30 min and then 2,3-dichloropyrazine (30 g, 201 mmol) was added. The reaction mixture was stirred for 3 h and then heated to reflux for 1 h. The DME was evaporated under vacuum and the resulting residue was treated with cold aqueous HCl 1 M to give a yellow product that was recovered by filtration, washed with water and a minimum of ethanol to afford 2-(3-chloropyrazin-2-yl)propanedinitrile (34.2 g, 95% yield) as a yellow solid.

Step 2. A microwave vial containing 2-(3-chloropyrazin-2-yl)propanedinitrile (1.00 g, 5.60 mmol), 3-methoxy-2,6-dimethyl-aniline (2.54 g, 16.8 mmol) and NMP (10 mL) was capped, stirred at 150 °C for 1h then at 200 °C for 8 h. The reaction mixture was cooled to rt, poured into saturated aqueous NaHCO₃ and diluted with water and EtOAc. The mixture was filtered through a pad of Celite and the layers were separated. The organic layer was dried over Na₂SO₄, filtered, adsorbed on silica and purified by silica gel chromatography eluting with a gradient of 0 to 100% EtOAc in hexanes. The appropriate fractions were combined, concentrated in vacuo. The residue was purified again by silica gel chromatography eluting with a gradient of 0 to 20% MeOH in DCM. The appropriate fractions were were combined, concentrated and dried in vacuo to provide 6-amino-5-(3-methoxy-2,6-dimethyl-phenyl)pyrrolo[2,3-b]pyrazine-7-carbonitrile (346 mg, 21% yield) as a beige solid.

Step 3. To a solution of 6-amino-5-(3-methoxy-2,6-dimethyl-phenyl)pyrrolo[2,3-b]pyrazine-7-carbonitrile (600 mg, 2.05 mmol) in DMF (10 mL) was added NBS (436 mg, 2.45 mmol). The mixture was stirred for 10 min, diluted with water, stirred for 20 min then filtered. The solid was washed with water and dried in vacuo. Purification by silica gel chromatography eluting with a gradient of 0 to 100% EtOAc in hexanes provided 6-amino-3-bromo-5-(3-methoxy-2,6-dimethylphenyl)pyrrolo[2,3-b]pyrazine-7-carbonitrile (350 mg, 46% yield).

Step 4. To a solution of 6-amino-3-bromo-5-(3-methoxy-2,6-dimethyl-phenyl)pyrrolo[2,3-b]pyrazine-7-carbonitrile (350 mg, 940 µmol) in DCM (10 mL) was added H₂SO₄ (1.88 mmol, 1 mL). The mixture was stirred 60 min, quenched with crushed ice and extracted with DCM. The organic phase was dried over Na₂SO₄, filtered and concentrated in vacuo. The residue was purified on silica gel using a gradient of 0 to 20% MeOH in DCM to provide 6-amino-2-bromo-5-(3-methoxy-2,6-dimethyl-phenyl)pyrrolo[2,3-b]pyrazine-7-carboxamide (300 mg, 82% yield).

Step 5. To a solution of 6-amino-3-bromo-5-(3-methoxy-2,6-dimethyl-phenyl)pyrrolo[2,3-b]pyrazine-7-carboxamide (300 mg, 769 µmol) in DCM (3 mL) was added BBr₃ solution in DCM (1 M, 2.31 mL). The mixture was stirred for 2 h. The volatiles were removed in vacuo to afford a crude mixture of 6-amino-3-bromo-5-(3-hydroxy-2,6-dimethyl-phenyl)pyrrolo[2,3-b]pyrazine-7-carboxamide (263 mg, 91% yield) that was used in the next step without further purification. ¹H NMR (400 MHz, DMSO-d6) δ 8.29 (s, 1H), 7.55 (s, 2H), 7.31 (s, 1H), 7.21 (s, 1H), 7.13 - 7.06 (m, 1H), 6.96 (d, J = 8.3 Hz, 1H), 1.78 (s, 3H), 1.70 (s, 3H). MS: [M+1]: 378.3.

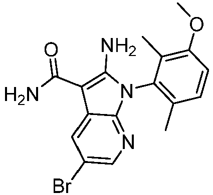

### Intermediate I (2-amino-5-bromo-1-(3-methoxy-2,6-dimethyl-phenyl)pyrrolo[2,3-b]pyridine-3-carboxamide)

Step 1. To a solution of 2,3-dibromo-5-nitro-pyridine (20 g, 63.85 mmol) in NMP (120 mL) were added 2,6-dimethylpyridine (11.08 g, 103.4 mmol, 12 mL), 3-methoxy-2,6-dimethyl-aniline (14 g, 95.23 mmol). The mixture was heated at 130 °C overnight. After it was cooled to rt, it was diluted with water dropwise, stirred at rt for 20 min, filtered. The solid was washed with water, dried in vacuo. The residue was purified using 2 x 330 g silica gel eluting with a gradient of 10 to 30% EtOAc in heptane to provide 3-bromo-N-(3-methoxy-2,6-dimethyl-phenyl)-5-nitro-pyridin-2-amine (12 g, 53% yield) as an off-white solid.

Step 2. To a solution of propanedinitrile (4.4 g, 66.6 mmol, 4.19 mL) in DME (120 mL) was added portion wise NaH (2.90 g, 66.9 mmol, 60% dispersion in mineral oil). The resulting mixture was stirred for 5 min, then 3-bromo-N-(3-methoxy-2,6-dimethyl-phenyl)-5-nitro-pyridin-2-amine (11.6 g, 32.9 mmol) and PdCl₂(dppf).CH₂Cl₂ (1.34 g, 1.65 mmol) were added. The mixture was stirred 2h at 110 °C. The mixture was cooled to rt, diluted with water and extracted twice with EtOAc. The combined organic extracts were washed with brine, dried over Na₂SO₄, filtered and concentrated in vacuo. The residue was purified by silica gel chromatography eluting with a gradient of 0 to 60% EtOAc in hexanes to provide 2-amino-1-(3-methoxy-2,6-dimethyl-phenyl)-5-nitro-pyrrolo[2,3-b]pyridine-3-carbonitrile (11 g, 99% yield) as a yellow solid.

Step 3. To a solution of 2-amino-1-(3-methoxy-2,6-dimethyl-phenyl)-5-nitro-pyrrolo[2,3-b]pyridine-3-carbonitrile (1.130 g, 3.35 mmol) in THF (15 mL) were added Et₃N (3.37 mmol, 470 uL), DMAP (45 mg, 368 µmol) and tert-butoxycarbonyl tert-butyl carbonate (1.47 g, 6.73 mmol). The mixture was stirred at 50 °C for 1h then cooled to rt. Ethylenediamine (500 µL) was added and the mixture was stirred for 2h. The resulting mixture was diluted with water, extracted with DCM (2x). The combined organic extracts were washed with brine, dried over Na₂SO₄, filtered and concentrated in vacuo. The residue was purified by silica gel chromatography eluting with a gradient of 20 to 60% EtOAc in to provide tert-butyl N-[3-cyano-1-(3-methoxy-2,6-dimethyl-phenyl)-5-nitro-pyrrolo[2,3-b]pyridin-2-yl]carbamate (1.27 g, 87% yield).

Step 4. To a solution of tert-butyl N-[3-cyano-1-(3-methoxy-2,6-dimethyl-phenyl)-5-nitro-pyrrolo[2,3-b]pyridin-2-yl]carbamate (2.94 g, 6.72 mmol) in DCM (30 mL) and MeOH (30 mL) was added palladium on carbon (10% w/w, 400 mg, 376 µmol). The mixture was stirred for 3h under 1 atm of H₂. The suspension was filtered over a pad of Celite and concentrated in vacuo to provide tert-butyl N-[5-amino-3-cyano-1-(3-methoxy-2,6-dimethyl-phenyl)pyrrolo[2,3-b]pyridin-2-yl]carbamate (2.7 g, 99% yield) as an off-white solid.

Step 5. To a solution of tert-butyl N-[5-amino-3-cyano-1-(3-methoxy-2,6-dimethylphenyl)pyrrolo[2,3-b]pyridin-2-yl]carbamate (15.1 g, 37.1 mmol) in a mixture of DMF (60 mL) and acetonitrile (80 mL) was added tert-butyl nitrite (5.72 g, 55.5 mmol, 6.6 mL) followed by Copper(II) bromide (10 g, 44.8 mmol). The mixture was stirred at 60 °C for 20 min, then it was diluted with water, treated with ammonia, extracted with EtOAc (3x). The combined organic extracts were washed with brine, dried over Na₂SO₄, filtered. The filtrate was concentrated to dryness. The residue was purified using 3 X 330 silica gel column eluting with a gradient of 0 to 5% EtOAc in DCM to provide tert-butyl N-[5-bromo-3-cyano-1-(3-methoxy-2,6-dimethyl-phenyl)pyrrolo[2,3-b]pyridin-2-yl]carbamate (9.67 g, 55% yield) as an off-white solid. MS: 471.2 (M+H)⁺. The following side product was also isolated from the purification: tert-butyl (3-cyano-1-(3-methoxy-2,6-dimethylphenyl)-1H-pyrrolo[2,3-b]pyridin-2-yl)carbamate (350 mg, 2% yield).

Step 6. To a solution of tert-butyl N-[5-bromo-3-cyano-1-(3-methoxy-2,6-dimethylphenyl)pyrrolo[2,3-b]pyridin-2-yl]carbamate (1.05 g, 2.23 mmol) in EtOH (15 mL) at 80 °C was added aqueous HCl (6 M, 6 mL). The mixture was stirred for 20 min then concentrated to dryness, coevaporated with MeOH, treated with Et₃N and then concentrated to dryness. The residue was purified by reverse phase flash chromatography on a C18 cartridge eluting with CH₃CN/water/0.1% formic acid to provide 2-amino-5-bromo-1-(3-methoxy-2,6-dimethylphenyl)pyrrolo[2,3-b]pyridine-3-carbonitrile (515 mg, 60% yield) as an off-white solid.

Step 7. To a solution of 2-amino-5-bromo-1-(3-methoxy-2,6-dimethyl-phenyl)pyrrolo[2,3-b]pyridine-3-carbonitrile (580 mg, 1.56 mmol) in a mixture of EtOH (6 mL) and water (2 mL) were added LiOH. H₂O (500 mg, 11.9 mmol) and H₂O₂ (27% w/w aq. solution, 21.02 mmol, 650 uL). The mixture was stirred at 60 °C for 20 min, cooled to rt, diluted with water and filtered. The solid was washed with water, dried in vacuo to provide 2-amino-5-bromo-1-(3-methoxy-2,6-dimethylphenyl)pyrrolo[2,3-b]pyridine-3-carboxamide (600 mg, 99% yield) as an off-white solid. ¹H NMR (400 MHz, DMSO-*d6*) δ 8.21 (d, J = 2.0 Hz, 1H), 7.77 (d, J = 2.0 Hz, 1H), 7.20 (dt, J = 8.4, 0.7 Hz, 1H), 7.13 (s, 2H), 7.05 (d, J = 8.5 Hz, 1H), 6.83 (s, 2H), 3.73 (s, 3H), 1.75 (d, J = 0.7 Hz, 3H), 1.65 (s, 3H). MS: [M+1]: 469.1.

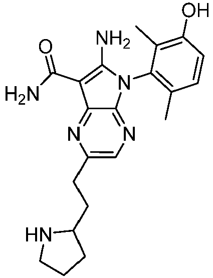

### Compound 2 (6-amino-5-(3-hydroxy-2,6-dimethylphenyl)-2-(2-(pyrrolidin-2-yl)ethyl)-5H-pyrrolo[2,3-b]pyrazine-7-carboxamide)

Step 1. To a solution of **Intermediate D** (0.25 g, 0.55 mmol) in DMF was added tert-butyl 2-ethynylpyrrolidine-1-carboxylate (0.213 g, 1.08 mmol) and Et₃N (234 µL, 1.66 mmol). Nitrogen gas was bubbled in the reaction mixture for 10 min. Cul (10 mg, 0.054 mmol) and PdCl₂(PPh₃)₂ (20 mg, 0.027 mmol) were then added and the reaction mixture was heated at 100 °C for 1.5 h. The reaction mixture was cooled to rt, diluted with cold water and extracted with EtOAc (3x). The combined organic layers were dried over Na₂SO₄ filtered and concentrated in vacuo. The residue was purified by silica gel chromatography eluting with 60% EtOAc in hexanes to afford of tert-butyl 2-((6-amino-7-carbamoyl-5-(3-methoxy-2,6-dimethylphenyl)-5H-pyrrolo[2,3-b]pyrazin-2-I)ethynyl)pyrrolidine-1-carboxylate (0.27 g, 83% yield) as a yellow solid.

Step 2. To a solution of *tert*-butyl 2-((6-amino-7-carbamoyl-5-(3-methoxy-2,6-dimethylphenyl)-5H-pyrrolo[2,3-b]pyrazin-2-I)ethynyl)pyrrolidine-1-carboxylate (0.13 g, 0.55 mmol) in methanol was added palladium on carbon (10% w/w, 50% moisture). The suspension was stirred under hydrogen atmosphere for 2 h. The reaction mixture was filtered on Celite and washed with methanol. The filtrate was concentrated in vacuo and the residue was purified by silica gel chromatography eluting with 30% EtOAc in hexanes to provide *tert*-butyl 2-(2-(6-amino-7-carbamoyl-5-(3-methoxy-2,6-dimethylphenyl)-5H-pyrrolo[2,3-b]pyrazin-2-yl)ethyl)pyrrolidine-1-carboxylate (0.105 g, 49% yield) as an off-white solid.

Step 3. For O-Me deprotection using BBr₃, the same procedure used for **Compound 35** provided a residue which was purified by preparative HPLC to provide 6-amino-5-(3-hydroxy-2,6-dimethylphenyl)-2-(2-(pyrrolidin-2-yl)ethyl)-5H-pyrrolo[2,3-b]pyrazine-7-carboxamide (2.7 mg, 3.5% yield) as a white solid. ¹H NMR (400 MHz, DMSO-*d*₆) *δ* 9.79 (bs, 1H), 8.40 (s, 1H), 7.65 (s, 1H), 7.47 (s, 1H), 7.35 (s, 2H), 7.27 (s, 1H), 7.07 (d, *J* = 8 Hz, 1H), 6.95 (d, *J* = 8.4 Hz, 1H), 3.39 (s, 2H), 3.06 (s, 1H), 2.99 (s, 1H), 2.80 (s, 2H), 2.02 (s, 3H), 1.83 (s, 1H), 1.76 (s, 3H), 1.68 (s, 3H), 1.46 (s, 1H). MS: [M+1]: 395.5.

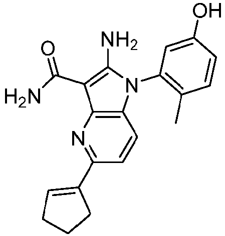

### Compound 6 (2-amino-5-(cyclopenten-1-yl)-1-(5-hydroxy-2-methyl-phenyl)pyrrolo[3,2-b]pyridine-3-carboxamide)

To a solution of **Intermediate G** (33 mg, 104 µmol) in dioxane (1.5 mL) were added 2-(cyclopenten-1-yl)-4,4,5,5-tetramethyl-1,3,2-dioxaborolane (40 mg, 206 µmol), PdCl₂(dppf).CH₂Cl₂ (8 mg, 10 µmol) and aqueous Na₂CO₃ (2 M, 200 µL). The mixture was stirred at 100 °C for 5h. The volatiles were removed in vacuo. The residue was purified by preparative HPLC to provide 2-amino-5-(cyclopenten-1-yl)-1-(5-hydroxy-2-methyl-phenyl)pyrrolo[3,2-b]pyridine-3-carboxamide (5 mg, 14% yield) as an off-white solid. ¹H NMR (400 MHz, DMSO-*d6*) δ 9.77 (s, 1H), 8.03 (s, 1H), 7.25 (d, *J* = 8.3 Hz, 1H), 6.99 (d, *J* = 8.1 Hz, 2H), 6.91 (s, 2H), 6.88 (dd, *J* = 8.3, 2.6 Hz, 1H), 6.82 (d, *J* = 8.1 Hz, 1H), 6.66 (d, *J* = 2.5 Hz, 1H), 6.41 (t, *J* = 2.1 Hz, 1H), 2.72 (m, 2H), 2.50 (m, 2H), 1.94 (m, 2H), 1.78 (s, 3H). MS: [M+1]: 349.1.

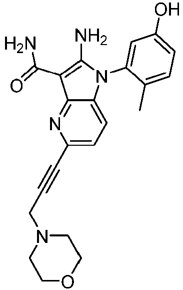

### Compound 16 (2-amino-1-(5-hydroxy-2-methyl-phenyl)-5-(3-morpholinoprop-1-ynyl)pyrrolo[3,2-b]pyridine-3-carboxamide)

A solution of 4-prop-2-ynylmorpholine (40 mg, 0.32 mmol), **Intermediate G** (50 mg, 0.16 mmol), Copper(I) iodide (3 mg, 16 µmol), Na₂CO₃ (70 mg, 0.66 mmol), tri-tert-butylphosphonium tetrafluoroborate (9 mg, 31 µmol) and PdCl₂ (3 mg, 17 µmol) in DMF (2 mL) was degassed in vacuo and back-filled with nitrogen. The mixture was stirred at 100 °C for 5 h. The mixture was purified by preparative HPLC eluting with CH₃CN/water/10 mM ammonium bicarbonate (pH 10). The desired fractions were combined and lyophilized to provide 2-amino-1-(5-hydroxy-2-methylphenyl)-5-(3-morpholinoprop-1-ynyl)pyrrolo[3,2-b]pyridine-3-carboxamide (22 mg, 35% yield) as an off-white solid. ¹H NMR (400 MHz, DMSO-d6) δ 9.76 (s, 1H), 7.74 (d, *J =* 3.6 Hz, 1H), 7.38 - 7.16 (m, 1H), 7.06 (s, 3H), 6.98 (d, *J* = 8.1 Hz, 1H), 6.88 (dd, *J* = 8.4, 2.6 Hz, 1H), 6.83 (d, *J* = 8.0 Hz, 1H), 6.66 (d, *J* = 2.5 Hz, 1H), 3.57 (m, 4H), 3.50 (s, 2H), 2.52 - 2.47 (m, 4H), 1.77 (s, 3H). MS: [M+1]: 406.2.

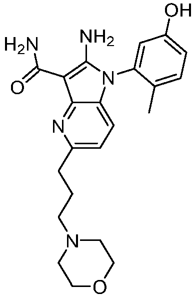

### Compound 18 (2-amino-1-(5-hydroxy-2-methyl-phenyl)-5-(3-morpholinopropyl)pyrrolo[3,2-b]pyridine-3-carboxamide)

To a solution of 2-amino-1-(5-hydroxy-2-methyl-phenyl)-5-(3-morpholinoprop-1-ynyl)pyrrolo[3,2-b]pyridine-3-carboxamide (20 mg, 49 µmol) in MeOH (2 mL) was added palladium on carbon (10% w/w, 6 mg). The mixture was stirred under a hydrogen atmosphere for 30 min. The resulting mixture was filtered and concentrated in vacuo to provide 2-amino-1-(5-hydroxy-2-methyl-phenyl)-5-(3-morpholinopropyl)pyrrolo[3,2-b]pyridine-3-carboxamide (17.8 mg, 88% yield) as a white solid. ¹H NMR (400 MHz, DMSO-d6) δ 9.74 bs, 1H), 8.01 (d, *J =* 4.0 Hz, 1H), 7.24 (dd, *J* = 8.3, 0.8 Hz, 1H), 6.97 (d, *J* = 4.0 Hz, 1H), 6.90 - 6.81 (m, 3H), 6.76 (d, *J* = 8.0 Hz, 1H), 6.67 (d, *J* = 8.1 Hz, 1H), 6.63 (d, *J* = 2.5 Hz, 1H), 3.51 (m, 4H), 2.77 - 2.58 (m, 2H), 2.28 (m, 6H), 1.91 - 1.78 (m, 2H), 1.77 (s, 3H). MS: [M+1]: 410.2.

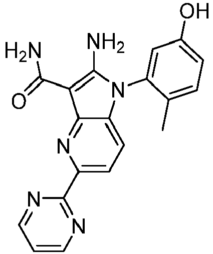

### Compound 23 (2-amino-1-(5-hydroxy-2-methyl-phenyl)-5-pyrimidin-2-yl-pyrrolo[3,2-b]pyridine-3-carboxamide)

To a solution of **Intermediate G** (50 mg, 0.158 mmol) in DMF (2 mL) were added tributyl(pyrimidin-2-yl)stannane (73 mg, 0.199 mmol, 60 µL), Cul (3 mg, 16 µmol), LiCl (7 mg, 165 µmol) and PdCl₂(dppf).CH₂Cl₂ (12 mg, 16 µmol). The mixture was degassed in vacuo and back filled with nitrogen three times, then stirred at 110 °C for 18 h. The mixture was then purified by preparative HPLC to provide 2-amino-1-(5-hydroxy-2-methyl-phenyl)-5-pyrimidin-2-yl-pyrrolo[3,2-b]pyridine-3-carboxamide (8 mg, 14% yield) as a yellowish solid. ¹H NMR (400 MHz, DMSO-d6) δ 9.79 (s, 1H), 8.90 (d, *J* = 4.8 Hz, 2H), 8.32 (d, *J* = 3.9 Hz, 1H), 8.02 (d, *J* = 8.3 Hz, 1H), 7.43 (t, *J =* 4.8 Hz, 1H), 7.28 (d, *J* = 8.3 Hz, 1H), 7.16 (d, *J* = 3.8 Hz, 1H), 7.05 (s, 2H), 7.02 (d, *J* = 8.3 Hz, 1H), 6.90 (dd, *J* = 8.4, 2.5 Hz, 1H), 6.72 (d, *J* = 2.5 Hz, 1H), 1.82 (s, 3H). MS: [M+1]: 361.2.

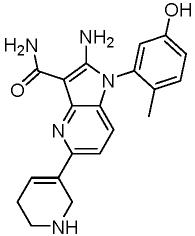

### Compound 27 (2-amino-1-(5-hydroxy-2-methyl-phenyl)-5-(1,2,3,6-tetrahydropyridin-5-yl)pyrrolo[3,2-b]pyridine-3-carboxamide HCl salt)

To a solution of tert-butyl 5-[2-amino-3-carbamoyl-1-(5-hydroxy-2-methylphenyl)pyrrolo[3,2-b]pyridin-5-yl]-3,6-dihydro-2H-pyridine-1-carboxylate (75 mg, 0.162 mmol, prepared similarly to **Compound 6)** in MeOH (1 mL) was added HCl in dioxane (4 M, 0.5 mL). The mixture was stirred for 2h. The volatiles were removed in vacuo. The residue was dissolved in water and CH₃CN then lyophilized to provide 2-amino-1-(5-hydroxy-2-methyl-phenyl)-5-(1,2,3,6-tetrahydropyridin-5-yl)pyrrolo[3,2-b]pyridine-3-carboxamide HCl salt (64 mg, 99% yield) as an off-white solid. ¹H NMR (400 MHz, DMSO-d6) δ 9.76 (s, 1H), 9.20 (s, 2H), 8.01 - 7.41 (m, 1H), 7.26 (d, *J* = 8.4, Hz, 1H), 7.12 (d, *J* = 8.3 Hz, 1H), 7.02 (s, 3H), 6.92 - 6.82 (m, 2H), 6.72 - 6.61 (m, 2H), 4.11 (s, 2H), 3.44 (m 2H), 3.19 (m, 2H), 1.77 (s, 3H). MS: [M+1]: 464.2.

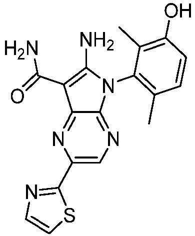

### Compound 28 (6-amino-5-(3-hydroxy-2,6-dimethyl-phenyl)-2-thiazol-2-yl-pyrrolo[2,3-b]pyrazine-7-carboxamide)

Step 1. A mixture of tributyl(thiazol-2-yl)stannane (345 mg, 0.922 mmol, 290 uL), **Intermediate D** (210 mg, 0.457 mmol), Cul (11 mg, 58 µmol), LiCl (40 mg, 0.943 mmol), PdCl₂(dppf).CH₂Cl₂ (35 mg, 45 µmol) in DMF (3 mL) was degassed in vacuo, then back filled with nitrogen. The final mixture was stirred at 120 °C for 4 h. The volatiles were removed in vacuo. The residue was purified by silica gel chromatography eluting with a gradient of 20 to 100% EtOAc in hexanes to provide a 6-amino-5-(3-methoxy-2,6-dimethyl-phenyl)-2-thiazol-2-yl-pyrrolo[2,3-b]pyrazine-7-carboxamide (136 mg, 75% yield) as an off-white solid.

Step 2. For OMe deprotection using BBr₃, the same procedure used for **Compound 35** was done on the appropriate intermediate (23 mg, 58 µmol) to provide a residue which was purified by preparative HPLC to provide 6-amino-5-(3-hydroxy-2,6-dimethyl-phenyl)-2-thiazol-2-yl-pyrrolo[2,3-b]pyrazine-7-carboxamide (10 mg, 45% yield) as an off-white solid. ¹H NMR (400 MHz, DMSO-*d6*) δ 9.63 (s, 1H), 8.49 (s, 1H), 7.91 (d, *J* = 3.2 Hz, 1H), 7.78 (d, *J* = 3.2 Hz, 1H), 7.61 (s, 2H), 7.40 (s, 1H), 7.29 (s, 1H), 7.06 (d, , *J* = 8.4 Hz, 1H), 6.92 (d, *J* = 8.3 Hz, 1H), 1.77 (s, 3H), 1.69 (s, 3H). MS: [M+1]: 381.2.

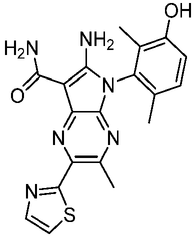

### Compound 31 (6-amino-5-(3-hydroxy-2,6-dimethyl-phenyl)-3-methyl-2-thiazol-2-yl-pyrrolo[2,3-b]pyrazine-7-carboxamide)

Step 1. To a solution of 6-amino-2-benzyloxy-5-(3-methoxy-2,6-dimethylphenyl)pyrrolo[2,3-b]pyrazine-7-carbonitrile **Intermediate C** (4 g, 10 mmol) in DMF (40 mL) was added NBS (4 g, 10 mmol). The mixture was stirred for 1 h, diluted with water, and finally stirred for 20 min. The resulting solid was filtered, washed with water and dried in vacuo then purified by silica gel chromatography eluting with a gradient of 20 to 80% EtOAc in hexanes to provide 6-amino-2-benzyloxy-3-bromo-5-(3-methoxy-2,6-dimethyl-phenyl)pyrrolo[2,3-b]pyrazine-7-carbonitrile (3.86 g, 81% yield) as an off-white solid.

Step 2. To a solution of 6-amino-2-benzyloxy-3-bromo-5-(3-methoxy-2,6-dimethylphenyl)pyrrolo[2,3-b]pyrazine-7-carbonitrile (650 mg, 1.36 mmol) in dioxane (10 mL) and water (3 mL) were added Pd(PPh₃)₄ (80 mg, 69 µmol), K₂CO₃ (800 mg, 5.79 mmol). The mixture was degassed in vacuo and then back filled with nitrogen three times. 2,4,6-trimethyl-1,3,5,2,4,6-trioxatriborinane (712 mg, 2.84 mmol, 0.8 mL) was then added and the final mixture was stirred at 100 °C for 18 h. The mixture was cooled to rt, diluted with EtOAc, washed with water, brine, dried over Na₂SO₄, filtered and concentrated in vacuo. The residue was purified by silica gel chromatography eluting with a gradient of 0 to 45% EtOAc in hexanes to provide 6-amino-2-benzyloxy-5-(3-methoxy-2,6-dimethyl-phenyl)-3-methyl-pyrrolo[2,3-b]pyrazine-7-carbonitrile (300 mg, 53% yield) as an off-white solid.

Step 3. For nitrile hydrolysis using sulfuric acid, the same procedure used for **Compound 164** was done on the appropriate intermediate (260 mg, 0.629 mmol) to provide 6-amino-2-hydroxy-5-(3-methoxy-2,6-dimethyl-phenyl)-3-methyl-pyrrolo[2,3-b]pyrazine-7-carboxamide (205 mg, 96% yield) as an off-white solid.

Step 4. To a solution of 6-amino-2-hydroxy-5-(3-methoxy-2,6-dimethyl-phenyl)-3-methyl-pyrrolo[2,3-b]pyrazine-7-carboxamide (206 mg, 0.603 mmol) and Cs₂CO₃ (390 mg, 1.20 mmol) in DMF (2 mL) was added 1,1,1-trifluoro-N-phenyl-N-(trifluoromethylsulfonyl)methanesulfonamide (235 mg, 0.658 mmol). The mixture was stirred for 1 h, then diluted with water and extracted with EtOAc (4x). The combined organic extracts were washed with brine, dried over Na₂SO₄, filtered, and concentrated in vacuo. The residue was purified by silica gel chromatography eluting with a gradient of 0 to 70% EtOAc in hexanes to provide [6-amino-7-carbamoyl-5-(3-methoxy-2,6-dimethyl-phenyl)-3-methyl-pyrrolo[2,3-b]pyrazin-2-yl] trifluoromethanesulfonate (160 mg, 56% yield) as an off-white solid.

Step 5. A mixture of tributyl(thiazol-2-yl)stannane (83 mg, 0.223 mmol, 70 uL), [6-amino-7-carbamoyl-5-(3-methoxy-2,6-dimethyl-phenyl)-3-methyl-pyrrolo[2,3-b]pyrazin-2-yl] trifluoromethanesulfonate (50 mg, 0.106 mmol), Cul (3 mg, 16 µmol), LiCl (7 mg, 0.165 mmol), PdCl₂(dppf).CH₂Cl₂ (8 mg, 11 µmol) in DMF (1.5 mL) was degassed in vacuo, then back filled with nitrogen. The reaction mixture was stirred at 110 °C for 4 h, cooled to rt and concentrated in vacuo. The residue was purified by preparative HPLC to provide 6-amino-5-(3-methoxy-2,6-dimethyl-phenyl)-3-methyl-2-thiazol-2-yl-pyrrolo[2,3-b]pyrazine-7-carboxamide (22 mg, 51% yield) as an off-white solid.

Step 6. For OMe deprotection using BBr₃, the same procedure used for **Compound 35** was done on the appropriate intermediate (22 mg, 53 µmol) to provide a residue which was purified by preparative HPLC to provide 6-amino-5-(3-hydroxy-2,6-dimethyl-phenyl)-3-methyl-2-thiazol-2-yl-pyrrolo[2,3-b]pyrazine-7-carboxamide (5 mg, 24% yield) as an off-white solid. ¹H NMR (400 MHz, DMSO-d6) δ 9.64 (s, 1H), 7.94 (d, *J* = 3.3 Hz, 1H), 7.76 (d, *J* = 3.3 Hz, 1H), 7.47 (s, 2H), 7.35 (s, 1H), 7.22 (s, 1H), 7.06 (d, *J* = 8.3 Hz, 1H), 6.92 (d, *J* = 8.3 Hz, 1H), 2.76 (s, 3H), 1.77 (s, 3H), 1.69 (s, 3H). MS: [M+1]: 395.2.

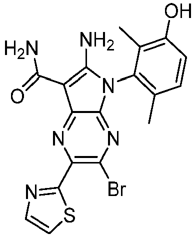

### Compound 33 (6-amino-3-bromo-5-(3-hydroxy-2,6-dimethyl-phenyl)-2-thiazol-2-yl-pyrrolo[2,3-b]pyrazine-7-carboxamide)

Step 1. To a solution of 6-amino-5-(3-methoxy-2,6-dimethyl-phenyl)-2-thiazol-2-yl-pyrrolo[2,3-b]pyrazine-7-carboxamide **(Compound 28,** 60 mg, 0.15 mmol) in DMF (1 mL) was added NBS (30 mg, 0.17 mmol). The mixture was stirred 18 h, diluted with water, treated with 20% aqueous Na₂S₂O₃, extracted with EtOAc (3x). The combined organic extracts were washed with water, brine, dried over Na₂SO₄, filtered and concentrated in vacuo. The residue was purified by silica gel chromatography eluting with a gradient of 20 to 100% EtOAc in hexanes to provide 6-amino-3-bromo-5-(3-methoxy-2,6-dimethyl-phenyl)-2-thiazol-2-yl-pyrrolo[2,3-b]pyrazine-7-carboxamide (45 mg, 63% yield) as an off-white solid.

Step 2. For OMe deprotection using BBr₃, the same procedure used for **Compound 35** was done on the appropriate intermediate (35 mg, 74 µmol) to provide a residue which was purified by preparative HPLC to provide 6-amino-3-bromo-5-(3-hydroxy-2,6-dimethyl-phenyl)-2-thiazol-2-yl-pyrrolo[2,3-b]pyrazine-7-carboxamide (10 mg, 29% yield) as an off-white solid. ¹H NMR (400 MHz, DMSO-*d6*) δ 9.71 (s, 1H), 7.97 (d, *J* = 3.3 Hz, 1H), 7.88 (d, *J* = 3.3 Hz, 1H), 7.75 (s, 2H), 7.45 (s, 1H), 7.19 - 7.00 (m, 2H), 6.94 (d, *J* = 8.3 Hz, 1H), 1.79 (s, 3H), 1.71 (s, 3H). MS: [M+1]: 460.2.

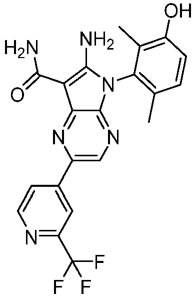

### Compound 35 (6-amino-5-(3-hydroxy-2,6-dimethyl-phenyl)-2-[2-(trifluoromethyl)-4-pyridyl]pyrrolo[2,3-b]pyrazine-7-carboxamide)

Step 1. To a solution of **Intermediate D** (50 mg, 0.109 mmol) in dioxane (1 mL) were added PdCl₂(dppf).CH₂Cl₂ (8 mg, 10 µmol). [2-(trifluoromethyl)-4-pyridyl]boronic acid (40 mg, 0.209 mmol) and aqueous Na₂CO₃ (2 M, 200 µL). The mixture was degassed in vacuo and back filled with nitrogen. The reaction mixture was stirred at 100 °C for 4 h, cooled to rt, diluted with water and then filtered. The solid was washed with water, dried in vacuo and finally purified by silica gel chromatography eluting with a gradient of 20 to 100% EtOAc in hexanes to provide 6-amino-5-(3-methoxy-2,6-dimethyl-phenyl)-2-[2-(trifluoromethyl)-4-pyridyl]pyrrolo[2,3-b]pyrazine-7-carboxamide (36 mg, 72% yield) as an off-white solid.

Step 2. **(General procedure for the OMe deprotection using BBr₃)** To a solution of 6-amino-5-(3-methoxy-2,6-dimethyl-phenyl)-2-[2-(trifluoromethyl)-4-pyridyl]pyrrolo[2,3-b]pyrazine-7-carboxamide (36 mg, 79 µmol) in DCM (1 mL) was added BBr₃ (1 M in DCM, 230 µL). The mixture was stirred for 1 h. The volatiles were removed in vacuo. The residue was dissolved in MeOH and concentrated to dryness again. Then it was dissolved in MeOH, Et₃N (100 µL) was added and the mixture was concentrated to dryness again. The residue was purified by preparative HPLC to provide 6-amino-5-(3-hydroxy-2,6-dimethyl-phenyl)-2-[2-(trifluoromethyl)-4-pyridyl]pyrrolo[2,3-b]pyrazine-7-carboxamide (16 mg, 46% yield) as an off-white solid. ¹H NMR (400 MHz, DMSO-*d6*) δ 9.63 (s, 1H), 8.81 (m, 1H), 8.62 (s, 1H), 8.48 (s, 1H), 8.41 (m, 1H), 7.63 (s, 2H), 7.45 (s, 1H), 7.33 (s, 1H), 7.07 (d, *J* = 8.3 Hz, 1H), 6.93 (d, *J* = 8.3 Hz, 1H), 1.77 (s, 3H), 1.69 (s, 3H).
MS: [M+1]: 443.2.

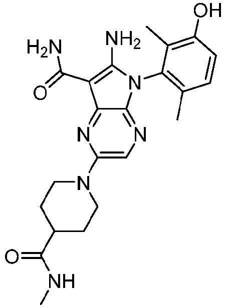

### Compound 46 (6-amino-5-(3-hydroxy-2,6-dimethyl-phenyl)-2-[4-(methylcarbamoyl)-1-piperidyl]pyrrolo[2,3-b]pyrazine-7-carboxamide)

Step 1. To a solution of **Intermediate D** (50 mg, 0.109 mmol) in DMSO (1 mL) was added N-methylpiperidine-4-carboxamide (80 mg, 0.563 mmol). The mixture was stirred at 130 °C for 2 h in a sealed vial, then cooled to rt and purified by preparative HPLC to provide 6-amino-5-(3-methoxy-2,6-dimethyl-phenyl)-2-[4-(methylcarbamoyl)-1-piperidyl]pyrrolo[2,3-b]pyrazine-7-carboxamide (14 mg, 28% yield) as an off-white solid.

Step 2. For OMe deprotection using BBr₃, the same procedure used for **Compound 35** was done on the appropriate intermediate (15 mg, 32 µmol) to provide a residue which was purified by preparative HPLC to provide 6-amino-5-(3-hydroxy-2,6-dimethyl-phenyl)-2-[4-(methylcarbamoyl)-1-piperidyl]pyrrolo[2,3-b]pyrazine-7-carboxamide (10 mg, 69% yield) as an off-white solid. ¹H NMR (400 MHz, DMSO-d6) δ 9.51 (s, 1H), 7.72 (d, *J* = 4.8 Hz, 1H), 7.35 (s, 1H), 7.27 (s, 1H), 7.14 - 6.96 (m, 4H), 6.87 (d, *J* = 8.2 Hz, 1H), 4.13 (d, *J* = 12.4 Hz, 2H), 2.85 - 2.68 (m, 2H), 2.53 (d, *J* = 4.6 Hz, 3H), 2.35 - 2.20 (m, 1H), 1.74 (m, 5H), 1.65 (s, 3H), 1.64 - 1.50 (m, 2H). MS: [M+1]: 438.2.

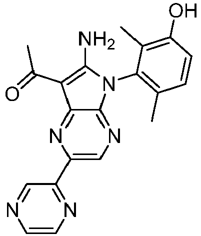

### Compound 97 (1-[6-amino-5-(3-hydroxy-2,6-dimethyl-phenyl)-2-pyrazin-2-yl-pyrrolo[2,3-b]pyrazin-7-yl]ethenone)

Step 1. To a solution of **Intermediate C** (2.5 g, 6.26 mmol) in THF (20 mL) was added MeMgBr solution in THF (3 M, 6.50 mL) at 0°C. The mixture was warmed and stirred 18 h. An additional amount of MeMgBr solution in THF (3 M, 4.00 mL) was added and the mixture was stirred an extra 5 h. The resulting mixture was quenched with sat. aqueous NH₄Cl, diluted with water, extracted with EtOAc. The organic extract was washed with water and brine, dried over Na₂SO₄, filtered and concentrated in vacuo. The residue was purified by silica gel column eluting with a gradient of 0 to 30% EtOAc in hexanes to provide 1-[6-amino-2-benzyloxy-5-(3-methoxy-2,6-dimethyl-phenyl)pyrrolo[2,3-b]pyrazin-7-yl]ethanone (120 mg, 5% yield).

Step 2. To a solution of 1-[6-amino-2-benzyloxy-5-(3-methoxy-2,6-dimethylphenyl)pyrrolo[2,3-b]pyrazin-7-yl]ethanone (100 mg, 0.240 mmol) in DCM (1 mL) was added TFA (500 µL). The mixture was stirred at 50 °C for 10h. The volatiles were removed in vacuo. The residue was purified by preparative HPLC to provide 1-[6-amino-2-hydroxy-5-(3-methoxy-2,6-dimethyl-phenyl)pyrrolo[2,3-b]pyrazin-7-yl]ethanone (43 mg, 55% yield).

Step 3. To a mixture of 1-[6-amino-2-hydroxy-5-(3-methoxy-2,6-dimethylphenyl)pyrrolo[2,3-b]pyrazin-7-yl]ethanone (43 mg, 0.132 mmol) and Cs₂CO₃ (50 mg, 0.153 mmol) in DMF (1 mL) was added 1,1,1-trifluoro-N-phenyl-N-(trifluoromethylsulfonyl)methanesulfonamide (52 mg, 0.146 mmol). The mixture was stirred for 1 h. The volatiles were removed in vacuo. The residue was purified by silica gel chromatography eluting with a gradient of 0 to 70% EtOAc in hexanes to provide [7-acetyl-6-amino-5-(3-methoxy-2,6-dimethyl-phenyl)pyrrolo[2,3-b]pyrazin-2-yl] trifluoromethanesulfonate (46 mg, 76% yield) as an off-white solid.

Step 4. To a solution of [7-acetyl-6-amino-5-(3-methoxy-2,6-dimethyl-phenyl)pyrrolo[2,3-b]pyrazin-2-yl] trifluoromethanesulfonate (46 mg, 0.100 mmol) in DMF (1 mL) were added LiCl (9 mg, 0.212 mmol), tributyl(pyrazin-2-yl)stannane (74 mg, 0.200 mmol), and PdCl₂(dppf).CH₂Cl₂ (7 mg, 9.6 µmol). The mixture was stirred at 120 °C for 10 h. The volatiles were removed in vacuo. The residue was purified by preparative HPLC to provide 1-[6-amino-5-(3-methoxy-2,6-dimethylphenyl)-2-pyrazin-2-yl-pyrrolo[2,3-b]pyrazin-7-yl]ethanone (38 mg, 97% yield) as an off-white solid.

Step 5. For OMe deprotection using BBr₃, the same procedure used for **Compound 35** was done on the appropriate intermediate (38 mg, 98 µmol) to provide a residue which was purified by preparative HPLC to provide 1-[6-amino-5-(3-hydroxy-2,6-dimethyl-phenyl)-2-pyrazin-2-yl-pyrrolo[2,3-b]pyrazin-7-yl]ethanone (18 mg, 49% yield) as an off-white solid. ¹H NMR (400 MHz, DMSO-d6) δ 9.62 (s, 1H), 9.55 (s, 1H), 8.72 (s, 1H), 8.69 - 8.62 (m, 2H), 8.13 (s, 2H), 7.07 (d, *J =* 8.3 Hz, 1H), 6.93 (d, *J* = 8.3 Hz, 1H), 2.76 (s, 3H), 1.77 (s, 3H), 1.69 (s, 3H). MS: [M+1]: 375.

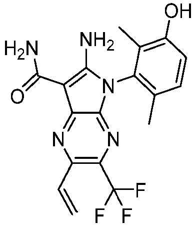

### Compound 102 (6-amino-5-(3-hydroxy-2,6-dimethyl-phenyl)-3-(trifluoromethyl)-2-vinyl-pyrrolo[2,3-b]pyrazine-7-carboxamide)

Step 1. To a solution of **Intermediate C** (800 mg, 2.00 mmol) in DMF (10 mL) was added NIS (450 mg, 2.00 mmol). The mixture was stirred for 30 min, diluted with water and stirred for 20 min. The resulting precipitate was collected by filtration and then purified by silica gel chromatography eluting with a gradient of 20 to 60% EtOAc in hexanes to provide 6-amino-2-benzyloxy-3-iodo-5-(3-methoxy-2,6-dimethyl-phenyl)pyrrolo[2,3-b]pyrazine-7-carbonitrile (820 mg, 78% yield) as an off-white solid.

Step 2. To a solution of 6-amino-2-benzyloxy-3-iodo-5-(3-methoxy-2,6-dimethylphenyl)pyrrolo[2,3-b]pyrazine-7-carbonitrile (745 mg, 1.42 mmol) in DMF (10 mL) was added (1,10-phenanthroline)(trifluoromethyl)copper(I) (900 mg, 2.88 mmol). The mixture was stirred at 70 °C for 4 h. The volatiles were removed in vacuo. The residue was purified by silica gel chromatography eluting with a gradient of 20 to 60% EtOAc in hexanes to provide 6-amino-2-benzyloxy-5-(3-methoxy-2,6-dimethyl-phenyl)-3-(trifluoromethyl)pyrrolo[2,3-b]pyrazine-7-carbonitrile (300 mg, 45% yield).

Step 3. A solution of 6-amino-2-benzyloxy-5-(3-methoxy-2,6-dimethyl-phenyl)-3-(trifluoromethyl)pyrrolo[2,3-b]pyrazine-7-carbonitrile (300 mg, 0.642 mmol) in sulfuric acid (1 mL) was stirred for 5 h, poured in crushed ice, neutralized with ammonia solution and the resulting precipitate was filtered. The precipitate was washed with water, dried in vacuo to provide 6-amino-2-hydroxy-5-(3-methoxy-2,6-dimethyl-phenyl)-3-(trifluoromethyl)pyrrolo[2,3-b]pyrazine-7-carboxamide (232 mg, 91% yield) as a yellow solid.

Step 4. To a solution of 6-amino-2-hydroxy-5-(3-methoxy-2,6-dimethyl-phenyl)-3-(trifluoromethyl)pyrrolo[2,3-b]pyrazine-7-carboxamide (232 mg, 0.587 mmol) and Cs₂CO₃ (200 mg, 0.615 mmol) in DMF (2 mL) was added 1,1,1-trifluoro-N-phenyl-N-(trifluoromethylsulfonyl)methanesulfonamide (210 mg, 0.588 mmol). The mixture was stirred for 1 h, diluted with water and stirred for 20 min. The resulting precipitate was filtered, washed with water, dried in vacuo. Further purification by silica gel chromatography eluting with a gradient of 20 to 100% EtOAc in hexanes provided [6-amino-7-carbamoyl-5-(3-methoxy-2,6-dimethyl-phenyl)-3-(trifluoromethyl)pyrrolo[2,3-b]pyrazin-2-yl] trifluoromethanesulfonate (190 mg, 61% yield) as an off-white solid.

Step 5. To the solution of [6-amino-7-carbamoyl-5-(3-methoxy-2,6-dimethyl-phenyl)-3-(trifluoromethyl)pyrrolo[2,3-b]pyrazin-2-yl] trifluoromethanesulfonate (90 mg, 0.171 mmol) in dioxane (1 mL) were added PdCl₂(dppf).CH₂Cl₂ (14 mg, 17 µmol), 4,4,5,5-tetramethyl-2-vinyl-1,3,2-dioxaborolane (30 mg, 0.195 mmol) and aqueous Na₂CO₃ (2M, 100 µL). The mixture was stirred at 120 °C for 18 h. The volatiles were removed in vacuo. The residue was purified by preparative HPLC to provide 6-amino-5-(3-methoxy-2,6-dimethyl-phenyl)-3-(trifluoromethyl)-2-vinyl-pyrrolo[2,3-b]pyrazine-7-carboxamide (10 mg, 14% yield) as an off-white solid.

Step 6. For OMe deprotection using BBr₃, , the same procedure used for **Compound 35** was done on the appropriate intermediate (10 mg, 25 µmol) to provide a residue which was purified by preparative HPLC to provide 6-amino-5-(3-hydroxy-2,6-dimethyl-phenyl)-3-(trifluoromethyl)-2-vinyl-pyrrolo[2,3-b]pyrazine-7-carboxamide (3 mg, 31% yield) as an off-white solid. ¹H NMR (400 MHz, DMSO-*d6*) δ 9.67 (s, 1H), 7.85 (s, 2H), 7.41 (m, 2H), 7.10 - 6.89 (m, 3H), 6.50 (m, 1H), 5.60 (m, 1H), 1.76 (s, 3H), 1.68 (s, 3H). MS: [M+1]: 392.2.

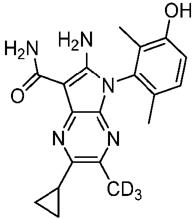

### Compound 110 (6-amino-2-cyclopropyl-5-(3-hydroxy-2,6-dimethyl-phenyl)-3-(trideuteriomethyl)pyrrolo[2,3-b]pyrazine-7-carboxamide)

Step 1. To a suspension of magnesium turnings (140 mg, 5.76 mmol) in THF (10 mL) was added iodine (13 mg, 52 µmol). The mixture was stirred for 10 min, CD₃I (5.14 mmol, 320 µL) was then added and the mixture was stirred for 18 h under nitrogen to generate an off-white suspension. To the mixture was added ZnCl₂ (0.5 M in THF, 10.5 mL) dropwise. After addition, the mixture was stirred for 20 min, then 6-amino-2-benzyloxy-3-bromo-5-(3-methoxy-2,6-dimethylphenyl)pyrrolo[2,3-b]pyrazine-7-carbonitrile (500 mg, 1.05 mmol) and Pd(PPh₃)₄ (120 mg, 0.103 mmol) were added. The final mixture was stirred at 70 °C for 6 h. The reaction was quenched with HCl 1M, diluted with water, and extracted with EtOAc (2x). The combined organic extracts were washed with water, brine, dried over Na₂SO₄, filtered and concentrated in vacuo. The residue was purified by silica gel chromatography eluting with a gradient of 0 to 60% EtOAc in hexanes to provide 6-amino-2-benzyloxy-5-(3-methoxy-2,6-dimethyl-phenyl)-3-(trideuteriomethyl)pyrrolo[2,3-b]pyrazine-7-carbonitrile (328 mg, 75% yield) as an off-white solid.

Step 2. A mixture of 6-amino-2-benzyloxy-5-(3-methoxy-2,6-dimethyl-phenyl)-3-(trideuteriomethyl)pyrrolo[2,3-b]pyrazine-7-carbonitrile (328 mg, 0.788 mmol) in H₂SO₄ (2 mL) was stirred for 4h. The mixture was cooled to 0 °C then neutralized to pH 7 using concentrated aqueous ammonia. The resulting mixture was lyophilized, and the residue was triturated with water and filtered. The solid was dried in vacuo to provide 6-amino-2-hydroxy-5-(3-methoxy-2,6-dimethyl-phenyl)-3-(trideuteriomethyl)pyrrolo[2,3-b]pyrazine-7-carboxamide (220 mg, 81% yield) as an off-white solid.

Step 3. To a solution of 6-amino-2-hydroxy-5-(3-methoxy-2,6-dimethyl-phenyl)-3-(trideuteriomethyl)pyrrolo[2,3-b]pyrazine-7-carboxamide (232 mg, 0.674mmol) in DMF (3 mL) were added Cs₂CO₃ (320 mg, 0.982 mmol) and 1,1,1-trifluoro-N-phenyl-N-(trifluoromethylsulfonyl)methanesulfonamide (360 mg, 1.01 mmol). The mixture was stirred for 1 h. The volatiles were removed in vacuo. The residue was purified by silica gel chromatography eluting with a gradient of 20 to 100% EtOAc in hexanes to provide [6-amino-7-carbamoyl-5-(3-methoxy-2,6-dimethyl-phenyl)-3-(trideuteriomethyl)pyrrolo[2,3-b]pyrazin-2-yl] trifluoromethanesulfonate (200 mg, 62% yield) as an off-white solid.

Step 4. To the solution of [6-amino-7-carbamoyl-5-(3-methoxy-2,6-dimethyl-phenyl)-3-(trideuteriomethyl)pyrrolo[2,3-b]pyrazin-2-yl] trifluoromethanesulfonate (200 mg, 0.420 µmol) in DMF (3 mL) were added lithium chloride (36 mg, 0.849 mmol) and tributyl(cyclopropyl)stannane (275 mg, 0.831 mmol). The mixture was stirred at 120 °C for 10 h. The volatiles were removed in vacuo. The residue was purified by preparative HPLC to provide 6-amino-2-cyclopropyl-5-(3-methoxy-2,6-dimethyl-phenyl)-3-(trideuteriomethyl)pyrrolo[2,3-b]pyrazine-7-carboxamide (80 mg, 52% yield) as an off-white solid.

Step 5. For OMe deprotection using BBr₃, the same procedure used for **Compound 35** was done on the appropriate intermediate (35 mg, 95 µmol) to provide a residue which was purified by preparative HPLC to provide 6-amino-2-cyclopropyl-5-(3-hydroxy-2,6-dimethyl-phenyl)-3-(trideuteriomethyl)pyrrolo[2,3-b]pyrazine-7-carboxamide (20 mg, 59% yield) as an off-white solid. ¹H NMR (400 MHz, DMSO-d6) δ 9.49 (s, 1H), 7.24 (s, 1H), 7.14 - 6.98 (m, 4H), 6.89 (d, *J* = 8.2 Hz, 1H), 2.11 (m, 1H), 1.84 - 1.68 (s, 3H), 1.64 (s, 3H), 1.04 - 0.81 (m, 4H). MS: [M+1]: 356.2. Chiral SFC separation of **Compound 110** (20 mg, 0.056 mmol) (Instrument: Waters Prep 15 SFC-MS; Column: Phenomenex Lux Cellulose-2, 10 x 250 mm, 5 µm; Conditions: Isocratic at 55% MeOH with 45% CO2; Flow Rate: 10 mL/min) provided **Compound 111** and **Compound 112.**

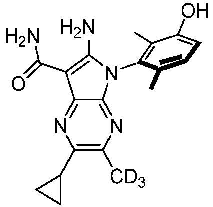

**Compound 111** from chiral SFC separation of **110.** Peak 1 (retention time 5.33 min, 99.95%): *S*-6-amino-2-cyclopropyl-5-(3-hydroxy-2,6-dimethyl-phenyl)-3-(trideuteriomethyl)pyrrolo[2,3-b]pyrazine-7-carboxamide (7.8 mg) is obtained as an off-white solid. 1H NMR (400 MHz, DMSO-d6) δ 9.49 (s, 1H), 7.24 (s, 1H), 7.14 -6.98 (m, 4H), 6.89 (d, J = 8.2 Hz, 1H), 2.11 (m, 1H), 1.84 - 1.68 (s, 3H), 1.64 (s, 3H), 1.04 - 0.81 (m, 4H). MS: [M+1]: 356.2.

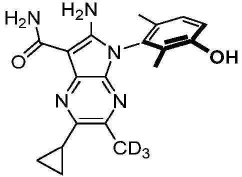

**Compound 112** from chiral SFC separation of **110.** Peak 2 (retention time 6.00 min, 99.78%): *R*-6-amino-2-cyclopropyl-5-(3-hydroxy-2,6-dimethyl-phenyl)-3-(trideuteriomethyl)pyrrolo[2,3-b]pyrazine-7-carboxamide (5.3 mg) is obtained as an off-white solid. 1H NMR (400 MHz, DMSO-d6) δ 9.49 (s, 1H), 7.24 (s, 1H), 7.14 - 6.98 (m, 4H), 6.89 (d, J = 8.2 Hz, 1H), 2.11 (m, 1H), 1.84 - 1.68 (s, 3H), 1.64 (s, 3H), 1.04 - 0.81 (m, 4H). MS: [M+1]: 356.2.

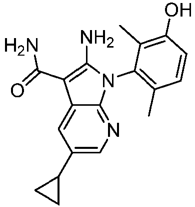

### Compound 116 (2-amino-5-cyclopropyl-1-(3-hydroxy-2,6-dimethyl-phenyl)pyrrolo[2,3-b]pyridine-3-carboxamide)

Step 1. To a solution of tert-butyl N-[5-bromo-3-cyano-1-(3-methoxy-2,6-dimethylphenyl)pyrrolo[2,3-b]pyridin-2-yl]carbamate (described in the synthesis of **intermediate I**) (110 mg, 0.233 mmol) in water (0.5 mL) and dioxane (2 mL) were added cyclopropylboronic acid (41 mg, 0.477 mmol), Cs₂CO₃ (270 mg, 0.829 mmol), PdCl₂(dppf).CH₂Cl₂ (18 mg, 22 µmol) in a sealed vial. The mixture was degassed and back filled with nitrogen 3 times. The resulting mixture was heated to 100°C and stirred 18 h. The volatiles were removed in vacuo. The residue was purified by silica gel chromatography eluting with a gradient of 0 to 60% EtOAc in hexanes to provide 2-amino-5-cyclopropyl-1-(3-methoxy-2,6-dimethyl-phenyl)pyrrolo[2,3-b]pyridine-3-carbonitrile (63 mg, 81% yield) as an off-white solid.

Step 2. To a solution of 2-amino-5-cyclopropyl-1-(3-methoxy-2,6-dimethylphenyl)pyrrolo[2,3-b]pyridine-3-carbonitrile (73 mg, 0.220 mmol) in EtOH (1.5 mL) and water (300 µL) were added LiOH.H₂O (50 mg, 1.19 mmol) and H₂O₂ (700 uL, 27% w/w aq. soln). The mixture was stirred at 60 °C for 20 min, then the volatiles were removed in vacuo. The residue was purified by silica gel chromatography eluting with a gradient of 20 to 60% EtOAc in hexanes to provide 2-amino-5-cyclopropyl-1-(3-methoxy-2,6-dimethyl-phenyl)pyrrolo[2,3-b]pyridine-3-carboxamide (22 mg, 29% yield) as an off-white solid.

Step 3. For OMe deprotection using BBr₃, the same procedure used for **Compound 35** was done on the appropriate intermediate (22 mg, 63 µmol) to provide a residue which was purified by preparative HPLC to provide 2-amino-5-cyclopropyl-1-(3-hydroxy-2,6-dimethyl-phenyl)pyrrolo[2,3-b]pyridine-3-carboxamide (15 mg, 71% yield) as an off-white solid. ¹H NMR (400 MHz, DMSO-d6) δ 9.43 (s, 1H), 7.58 (s, 1H), 7.50 (s, 1H), 7.00 (d, *J* = 8.3 Hz, 1H), 6.86 (m, 3H), 6.69 (s, 2H), 1.88 m, 1H), 1.69 (s, 3H), 1.61 (s, 3H), 0.86 (m, 2H), 0.81 - 0.68 (m, 2H). MS: [M+1]: 337.2.

Chiral SFC separation of **Compound 116** (410 mg, 1.22 mmol) (Instrument: Waters Prep 100 SFC-MS; Column: Phenomenex Lux Cellulose-2, 30 x 250 mm, 5 µm; Conditions: Isocratic at 50% ACN/EtOH 1:1 with 50% CO2; Flow Rate: 70 mL/min) provided **Compound 117** and **Compound 118.**

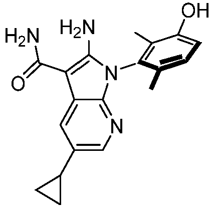

**Compound 117** from chiral SFC separation of **116.** Peak 1 (retention time 5.60 min, 99.83%): S-2-amino-5-cyclopropyl-1-(3-hydroxy-2,6-dimethyl-phenyl)pyrrolo[2,3-b]pyridine-3-carboxamide (130 mg) is obtained as an off-white solid. ¹H NMR (400 MHz, DMSO-d6) δ 9.52 (s, 1H), 8.12 (d, J = 2.2 Hz, 1H), 8.08 - 7.92 (m, 2H), 7.46 (s, 1H), 7.00 (d, J = 8.2 Hz, 2H), 6.86 (d, J = 8.3 Hz, 1H), 2.03 (m, 1H), 1.70 (s, 3H), 1.59 (s, 3H), 0.96 (m, 2H), 0.68 (m, 2H). MS: [M+1]: 337.2.

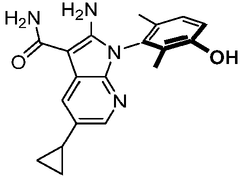

**Compound 118** from chiral SFC separation of **116.** Peak 2 (retention time 7.81 min, 98.81%): R-2-amino-5-cyclopropyl-1-(3-hydroxy-2,6-dimethyl-phenyl)pyrrolo[2,3-b]pyridine-3-carboxamide (130 mg). ¹H NMR (400 MHz, DMSO-d6) δ 9.52 (s, 1H), 8.12 (d, J = 2.2 Hz, 1H), 8.08 - 7.92 (m, 2H), 7.46 (s, 1H), 7.00 (d, J = 8.2 Hz, 2H), 6.86 (d, J = 8.3 Hz, 1H), 2.03 (m, 1H), 1.70 (s, 3H), 1.59 (s, 3H), 0.96 (m, 2H), 0.68 (m, 2H). MS: [M+1]: 337.2.

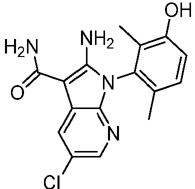

### Compound 132 (2-amino-5-chloro-1-(3-hydroxy-2,6-dimethyl-phenyl)pyrrolo[2,3-b]pyridine-3-carboxamide)

Step 1. To a solution of 3-methoxy-2,6-dimethyl-aniline (3.61 g, 23.9 mmol) and 3-bromo-5-chloro-2-fluoro-pyridine (5.02 g, 23.9 mmol) in THF (50 mL) was added LiHMDS solution in THF (1 M, 48 mL) dropwise over 18 min. A 16 °C exotherm was observed. After 30 min, the reaction mixture was diluted with saturated aqueous NH₄Cl and extracted with EtOAc. The organic layer was separated, washed with brine, dried over Na₂SO₄, filtered and concentrated. The crude product was purified by flash chromatography (dry load) eluting with a gradient of 0 to 20% EtOAc in heptane. The fractions were combined, concentrated and dried in vacuo to provide 3-bromo-5-chloro-N-(3-methoxy-2,6-dimethyl-phenyl)pyridin-2-amine (6.58 g, 81% yield) as a peach solid.

Step 2. To a suspension of NaH (1.08 g, 24.9 mmol, 60% dispersion in mineral oil) in DME (60 mL) is added propanedinitrile (1.62 g, 24.6 mmol) in DME (15 mL) dropwise. After stirring for 30 min, 3-bromo-5-chloro-N-(3-methoxy-2,6-dimethyl-phenyl)pyridin-2-amine (4.00 g, 11.7 mmol) in DME (15 mL) and PdCl₂(dppf).CH₂Cl₂ (1.08 g, 1.32 mmol) were added. The reaction mixture was flushed with nitrogen bubbling through the solution, then stirred at 100 °C for 5 h. The reaction mixture was cooled to rt and icy water (250 mL) was added dropwise. The resulting precipitate was collected by filtration and washed with water. The solid was air-dried then co-evaporated with toluene (2x), dried in vacuo to afford 4.67 g crude product. Purification by silica gel chromatography (dry load) eluting with a gradient of 0 to 100% EtOAc in heptane. The fractions were combined, concentrated and dried in vacuo, to afford 2-amino-5-chloro-1-(3-methoxy-2,6-dimethyl-phenyl)pyrrolo[2,3-b]pyridine-3-carbonitrile (3.27 g, 85% yield) as an ivory crystalline solid.

Step 3. To a suspension of 2-amino-5-chloro-1-(3-methoxy-2,6-dimethylphenyl)pyrrolo[2,3-b]pyridine-3-carbonitrile (7.50 g, 23.0 mmol) in water (60 mL) and reagent alcohol (180 mL) was added LiOH.H₂O, 98% (7.22 g, 172 mmol) and H₂O₂ (27% w/w aq. solution, 9.8 mL). The mixture was stirred at 60 °C for 30 min, then cooled to rt. Water was added dropwise (500 mL) and the solid was collected by filtration, washed with water and air-dried. The filtrate was diluted with water and a second crop of solid was obtained. Finally the filtrate was extracted with EtOAc (3x). The combined organic extracts were dried over Na₂SO₄, filtered and concentrated then dried in vacuo affording a third crop of crude product. The combined crude material was purified by silica gel chromatography using a gradient of 50 to 100% EtOAc in heptane. Pure fractions were combined and concentrated, dried in vacuo to provide 2-amino-5-chloro-1-(3-methoxy-2,6-dimethyl-phenyl)pyrrolo[2,3-b]pyridine-3-carboxamide (3.90 g, 49% yield) as a light yellow solid. Alternatively, nitrile hydrolysis could be performed under H₂SO₄ conditions (using the same procedure used for **Compound 164**) affording 2-amino-5-chloro-1-(3-methoxy-2,6-dimethylphenyl)pyrrolo[2,3-b]pyridine-3-carboxamide in quantitative yield.

Step 4. For OMe deprotection using BBr₃, the same procedure used for **Compound 35** was used on the appropriate intermediate (3.90 g, 11.3 mmol) to provide a residue which was co-evaporated with MeOH (4x), dried in vacuo, triturated with saturated aqueous NaHCO₃ and filtered. The crude product was purified by silica gel chromatography silica using a gradient of 0 to 20% MeOH in CH₂Cl₂ to provide 2-amino-5-chloro-1-(3-hydroxy-2,6-dimethyl-phenyl)pyrrolo[2,3-b]pyridine-3-carboxamide (3.54g, 95% yield) as a light beige solid. ¹H NMR (400 MHz, DMSO-*d6*) δ 954 (s, 1H), 8.14 (d, *J* = 2.2 Hz, 1H), 7.74 (d, *J* = 2.1 Hz, 1H), 7.14 (br s, 2H), 7.06 (d, *J* = 8.3 Hz, 1H), 6.91 (d, *J* = 8.3 Hz, 1H), 6.86 (br s, 2H), 1.74 (s, 3H), 1.65 (s, 3H). MS: [M+1]: 331.1.

Chiral SFC separation of **Compound 132** (3.54 g, 10.7 mmol) (Instrument: Waters Prep 100 SFC-MS; Column: Phenomenex Lux Cellulose-2, 30 x 250 mm, 5 µm; Conditions: Isocratic at 45% ACN/EtOH 1:1 with 55% CO2; Flow Rate: 70 mL/min) provided **Compound 133** and **Compound 134.**

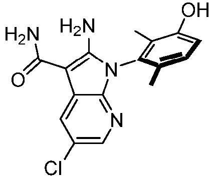

**Compound 133** from chiral SFC separation of **132.** Peak 1 (retention time 5.37 min, 99.70%): S-2-amino-5-chloro-1-(3-hydroxy-2,6-dimethyl-phenyl)pyrrolo[2,3-b]pyridine-3-carboxamide (1.26 g) is obtained as an off-white solid. ¹H NMR (400 MHz, DMSO-*d6*) δ 9.54 (s, 1H), 8.14 (d, J = 2.2 Hz, 1H), 7.74 (d, J = 2.1 Hz, 1H), 7.14 (br s, 2H), 7.06 (dt, J = 8.2, 0.7 Hz, 1H), 6.91 (d, J = 8.3 Hz, 1H), 6.86 (br s, 2H), 1.74 (d, J = 0.7 Hz, 3H), 1.65 (s, 3H). MS: [M+1]: 331.1.

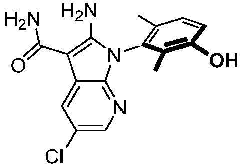

**Compound 134** from chiral SFC separation of **132.** Peak 2 (retention time 7.79 min, 99.19%): R-2-amino-5-chloro-1-(3-hydroxy-2,6-dimethyl-phenyl)pyrrolo[2,3-b]pyridine-3-carboxamide (1.26 g). ¹H NMR (400 MHz, DMSO-*d6*) δ 9.54 (s, 1H), 8.14 (d, J = 2.2 Hz, 1H), 7.74 (d, J = 2.1 Hz, 1H), 7.14 (br s, 2H), 7.06 (dt, J = 8.2, 0.7 Hz, 1H), 6.91 (d, J = 8.3 Hz, 1H), 6.86 (br s, 2H), 1.74 (d, J = 0.7 Hz, 3H), 1.65 (s, 3H). MS: [M+1]: 331.1.

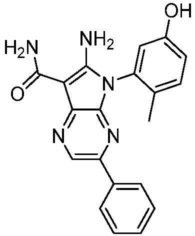

### Compound 150 (6-amino-5-(5-hydroxy-2-methyl-phenyl)-3-phenyl-pyrrolo[2,3-b]pyrazine-7-carboxamide)

Step 1. **Intermediate F** (101 mg, 0.236 mmol), phenyl boronic acid (29 mg, 0.24 mmol). Pd(PPh₃)₄ (30 mg, 0.026 mmol) and potassium phosphate tribasic anhydrous (176 mg, 0.829 mmol) were loaded in a microwave vial, which was flushed with nitrogen, then dioxane (2 mL) was added, the vial was capped and placed in a heat block set to 90 °C. After 90 min, the reaction mixture was cooled to rt, diluted with water, extracted with EtOAc (3x). The combined organic extracts were washed with brine, dried over Na₂SO₄, filtered and concentrated. The residue was purified by silica gel chromatography eluting with a gradient of 0 to 100% EtOAc in hexanes to provide 6-amino-5-(5-methoxy-2-methyl-phenyl)-3-phenyl-pyrrolo[2,3-b]pyrazine-7-carbonitrile (30 mg, 36% yield) as an orange solid.

Step 2. For nitrile hydrolysis using sulfuric acid, the same procedure used for **Compound 164** was done on the appropriate intermediate (28 mg, 0.079 mmol) to provide 6-amino-5-(5-methoxy-2-methyl-phenyl)-3-phenyl-pyrrolo[2,3-b]pyrazine-7-carboxamide (20 mg, 68% yield) as a pale yellow solid.

Step 3. For OMe deprotection using BBr₃, the same procedure used for **Compound 35** provided a residue which was purified by preparative HPLC to provide 6-amino-5-(5-hydroxy-2-methyl-phenyl)-3-phenyl-pyrrolo[2,3-b]pyrazine-7-carboxamide (11 mg, 57% yield) as a white fluffy solid. ¹H NMR (400 MHz, DMSO-d6) δ 9.71 (br s, 1H), 8.73 (s, 1H), 7.92 - 7.76 (m, 2H), 7.49 (br s, 2H), 7.45 - 7.37 (m, 3H), 7.35 - 7.28 (m, 2H), 7.26 (br s, 1H), 6.92 (dd, J = 8.3, 2.6 Hz, 1H), 6.77 (d, J = 2.5 Hz, 1H), 1.89 (s, 3H). MS: [M+1]: 360.2.

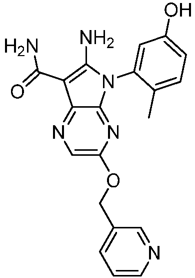

### Compound 153 (6-amino-5-(5-hydroxy-2-methyl-phenyl)-3-(3-pyridylmethoxy)pyrrolo[2,3-b]pyrazine-7-carboxamide)

Step 1. To a mixture of **Intermediate E** (51 mg, 0.16 mmol), 3-pyridylmethanol (41 mg, 0.38 mmol) and triphenylphosphine (62 mg, 0.24 mmol) in THF (2mL) was added diisopropyl azodicarboxylate (47 uL, 0.24 mmol). The resulting mixture was stirred 18 h. Additional triphenylphosphine (62 mg, 0.24 mmol), 3-pyridylmethanol (41 mg, 0.38 mmol) and diisopropyl azodicarboxylate (47 uL, 0.24 mmol) were added and the mixture was stirred for another 2.5 h and concentrated to dryness. The crude residue was purified by silica gel chromatography eluting with a gradient of 0 to 100% EtOAc in hexanes to provide 6-amino-5-[5-(methoxymethoxy)-2-methylphenyl]-3-(3-pyridylmethoxy)pyrrolo[2,3-b]pyrazine-7-carbonitrile (115 mg) as an impure amber gum (containing triphenyl phosphine oxide), that was carried to next step without further purification.

Step 2. To a solution of 6-amino-5-[5-(methoxymethoxy)-2-methyl-phenyl]-3-(3-pyridylmethoxy)pyrrolo[2,3-b]pyrazine-7-carbonitrile (65.0 mg, 0.156 mmol) in MeOH (1.5 mL) was added HCl in dioxane (4 M, 1.50 mL). After stirring for 75 min, the reaction mixture was concentrated and dried in vacuo. Crude 6-amino-5-(5-hydroxy-2-methyl-phenyl)-3-(3-pyridylmethoxy)pyrrolo[2,3-b]pyrazine-7-carbonitrile assumed bis HCl salt was carried to the next step without further purification.

Step 3. To a solution of crude 6-amino-5-(5-hydroxy-2-methyl-phenyl)-3-(3-pyridylmethoxy)pyrrolo[2,3-b]pyrazine-7-carbonitrile (70 mg, 0.156 mmol, assuming bis HCl salt) in MeOH (1.0 mL) was added aqueous NaOH solution (4 M, 1.0 mL). Reaction mixture was transferred to a preheated heat block at 90 °C and stirred for 18 h. After cooling down to rt, the mixture was neutralized with 3N HCl and diluted with water. The precipitate was collected by filtration and washed with water, then air-dried. Purification by preparative HPLC provided 6-amino-5-(5-hydroxy-2-methyl-phenyl)-3-(3-pyridylmethoxy)pyrrolo[2,3-b]pyrazine-7-carboxamide (4 mg, 7% yield) as a white fluffy solid. ¹H NMR (400 MHz, DMSO-d6) δ 9.68 (br s, 1H), 8.48 (d, J = 2.2 Hz, 1H), 8.42 (dd, J = 4.8, 1.7 Hz, 1H), 7.85 (s, 1H), 7.65 (dt, J = 7.8, 2.0 Hz, 1H), 7.26 (ddd, J = 7.9, 4.9, 0.9 Hz, 1H), 7.23 (d, J = 8.6 Hz, 1H), 7.15 (br s, 1H), 7.04 (br s, 1H), 7.00 (br s, 2H), 6.87 (dd, J = 8.3, 2.6 Hz, 1H), 6.66 (d, J = 2.5 Hz, 1H), 5.09 (d, J = 12.2 Hz, 1H), 5.08 (d, J = 12.2 Hz, 1H), 1.72 (s, 3H). MS: [M+1]: 391.2.

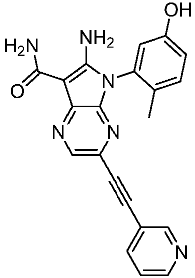

### Compound 160 (6-amino-5-(5-hydroxy-2-methyl-phenyl)-3-[2-(3-pyridyl)ethynyl]pyrrolo[2,3-b]pyrazine-7-carboxamide)

Step 1. To a microwave vial charged with **Intermediate F** (251 mg, 0.549 mmol), Pd(PPh₃)₄ (65 mg, 0.056 mmol), Cul (43 mg, 0.023 mmol) and flushed with nitrogen was added 3-ethynylpyridine (72 mg, 0.698 mmol) in DMF (2.5 mL) followed by Et₃N (610 µL, 4.39 mmol). The vial was capped then transferred to a preheated heat block (120°C). After 1 h, the reaction mixture was concentrated under vacuum, then taken in THF and adsorbed on silica. The volatiles were evaporated under vacuum and the residue was purified by silica gel chromatography eluting with a gradient of 0 to 100% EtOAc in hexanes then 0 to 20% MeOH in EtOAc, to provide 6-amino-5-[5-(methoxymethoxy)-2-methyl-phenyl]-3-[2-(3-pyridyl)ethynyl]pyrrolo[2,3-b]pyrazine-7-carbonitrile (260 mg, 99%) as a tan solid.

Step 2. To a suspension of 6-amino-5-[5-(methoxymethoxy)-2-methyl-phenyl]-3-[2-(3-pyridyl)ethynyl]pyrrolo[2,3-b]pyrazine-7-carbonitrile (225 mg, 0.548 mmol) in MeOH (3 mL) was added HCl in dioxane (4 M, 3 mL). After stirring for 30 min, the reaction mixture was concentrated to dryness then dried in vacuo, affording 6-amino-5-(5-hydroxy-2-methyl-phenyl)-3-[2-(3-pyridyl)ethynyl]pyrrolo[2,3-b]pyrazine-7-carbonitrile (319 mg, bis HCl salt) as a tan sticky solid which was used in the next step without further purification.

Step 3. 6-amino-5-(5-hydroxy-2-methyl-phenyl)-3-[2-(3-pyridyl)ethynyl]pyrrolo[2,3-b]pyrazine-7-carbonitrile bis HCl salt (241 mg, 0.548 mmol) was stirred in conc. H₂SO₄ (2 mL). After 3 days, the reaction mixture was quenched with crushed ice, placed in ice bath and made alkaline (pH about 10) with 1:1 NH₄OH/H₂O. The solids were collected by filtration and air-dried overnight, affording crude product as an ochre yellow solid (249 mg). A portion (67 mg) was purified by preparative HPLC to provide 6-amino-5-(5-hydroxy-2-methyl-phenyl)-3-[2-(3-pyridyl)ethynyl]pyrrolo[2,3-b]pyrazine-7-carboxamide (26 mg, 46% calculated yield) as a light yellow fluffy solid. ¹H NMR (400 MHz, DMSO-d6) δ 9.77 (br s, 1H), 8.75 (dd, J = 2.3, 0.9 Hz, 1H), 8.57 (dd, J = 4.9, 1.7 Hz, 1H), 8.42 (s, 1H), 7.98 (dt, J = 7.9, 1.9 Hz, 1H), 7.73 (br s, 2H), 7.44 (ddd, J = 8.0, 4.9, 0.9 Hz, 1H), 7.35 (br d, J = 4.4 Hz, 2H), 7.29 (d, J = 8.4 Hz, 1H), 6.94 (dd, J = 8.3, 2.6 Hz, 1H), 6.77 (d, J = 2.6 Hz, 1H), 1.85 (s, 3H). MS: [M+1]: 385.3.

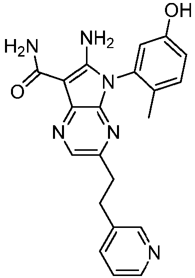

### Compound 162 (6-amino-5-(5-hydroxy-2-methyl-phenyl)-3-[2-(3-pyridyl)ethyl]pyrrolo[2,3-b]pyrazine-7-carboxamide)

Step 1. 6-amino-5-(5-hydroxy-2-methyl-phenyl)-3-[2-(3-pyridyl)ethynyl]pyrrolo[2,3-b]pyrazine-7-carboxamide (102 mg, 0.265 mmol) and palladium on carbon (30 mg, 0.028 mmol, 10% w/w) was stirred in MeOH (3 mL) under hydrogen atmosphere overnight, filtered on disk filter using MeOH, concentrated and purified by preparative HPLC to provide 6-amino-5-(5-hydroxy-2-methyl-phenyl)-3-[2-(3-pyridyl)ethyl]pyrrolo[2,3-b]pyrazine-7-carboxamide (7 mg, 7% yield) as an off-white fluffy solid. ¹H NMR (400 MHz, DMSO-*d6*) δ 8.34 (dd, J = 4.8, 1.7 Hz, 1H), 8.31 (dd, J = 2.3, 0.8 Hz, 1H), 7.95 (s, 1H), 7.55 (ddd, J = 7.8, 2.4, 1.7 Hz, 1H), 7.34 (br s, 1H), 7.30 (br s, 2H), 7.28 (dd, J = 8.4, 0.8 Hz, 1H), 7.23 (ddd, J = 7.8, 4.8, 0.9 Hz, 1H), 7.15 (br s, 1H), 6.92 (dd, J = 8.3, 2.6 Hz, 1H), 6.72 (d, J = 2.6 Hz, 1H), 3.01 - 2.93 (m, 2H), 2.92 - 2.84 (m, 2H), 1.82 (s, 3H). MS: [M+1]: 389.2.

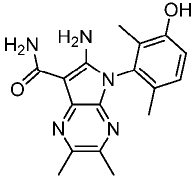

### Compound 164 (6-amino-5-(3-hydroxy-2,6-dimethyl-phenyl)-2,3-dimethyl-pyrrolo[2,3-b]pyrazine-7-carboxamide)

Step 1. To a suspension of NaH (3.54 g, 92 mmol, 60% dispersion in mineral oil) in THF (100 mL) at 0 °C was added malononitrile (3.99 g, 60.4 mmol) in THF (30 mL) dropwise via an addition funnel. The cold bath was removed at the end of the addition and the resulting mixture was allowed to stir for 45 min at rt. 2,3-dichloro-5,6-dimethyl-pyrazine (5.49 g, 31.0 mmol) and Pd(PPh₃)₄ (1.76 g, 1.52 mmol) were added, the reaction mixture was refluxed for 3.25 h, cooled to rt, poured into 200 mL 1:1 crushed ice and 1N HCl, and extracted with DCM (3x). The combined organic layers were washed with brine, dried over Na₂SO₄, filtered and concentrated. The crude product was adsorbed on silica using DCM/THF and purified by silica gel chromatography eluting with a gradient of 0 to 100% EtOAc in hexanes. Mix fractions were re-purified by a second silica gel chromatography using same conditions. Clean fractions from both columns were combined, concentrated and dried in vacuo to afford 2-(3-chloro-5,6-dimethyl-pyrazin-2-yl)propanedinitrile (5.0 g, 78% yield) as an orange solid.

Step 2. The reaction was split in 3 vials containing 1/3 of the amounts each. The microwave vials were charged with 2-(3-chloro-5,6-dimethyl-pyrazin-2-yl)propanedinitrile (3.04 g, 14.7 mmol), 3-methoxy-2,6-dimethyl-aniline (6.61 g, 43.7 mmol), potassium tert-butoxide (3.30 g, 29.4 mmol) and Pd-PEPPSI^{™}-SIPr catalyst (513 mg, 0.752 mmol), flushed with nitrogen three times, then dry NMP (30 mL) was added, flushed again with nitrogen, capped and submitted to microwave irradiation (100 °C) for 30 min. The vials were combined and diluted with EtOAc, saturated aqueous NH₄Cl and water. The layers were separated, and the aqueous layer was extracted twice with EtOAc. The combined organic extracts were washed with brine, dried over Na₂SO₄, filtered and concentrated in vacuo. The crude product was purified by silica gel chromatography eluting with a gradient of 0 to 100% EtOAc in heptane. Desired fractions were combined, concentrated and dried in vacuo to provide 6-amino-5-(3-methoxy-2,6-dimethyl-phenyl)-2,3-dimethyl-pyrrolo[2,3-b]pyrazine-7-carbonitrile (2.57 g, 54% yield) as a yellow solid.

Step 3. **(General procedure for nitrile hydrolysis using sulfuric acid)** 6-amino-5-(3-methoxy-2,6-dimethyl-phenyl)-2,3-dimethyl-pyrrolo[2,3-b]pyrazine-7-carbonitrile (7.11 g, 22.1 mmol) was dissolved in concentrated sulfuric acid (70 mL), then stirred for 45 min. The reaction mixture was slowly poured into crushed ice (500 cc), then placed in an ice bath and neutralized to pH 8-9 with concentrated NH₄OH (about 190 mL), maintaining internal temperature below 35°C. After stirring for 1 h, the precipitate was filtered, washed with water, air-dried, then further dried by co-evaporation with toluene under vacuum then dried in vacuo to afford 6-amino-5-(3-methoxy-2,6-dimethyl-phenyl)-2,3-dimethyl-pyrrolo[2,3-b]pyrazine-7-carboxamide (7.5 g, quantitative yield) as a yellow solid.

Step 4. To a suspension of 6-amino-5-(3-methoxy-2,6-dimethyl-phenyl)-2,3-dimethyl-pyrrolo[2,3-b]pyrazine-7-carboxamide (7.50 g, 22.1 mmol) in DCM (132 mL) was added BBr₃ (66.3 mmol, 6.4 mL) slowly. After stirring for 70 min, the reaction mixture was concentrated to dryness, resuspended in DCM, and MeOH was added (exotherm observed). After concentrating, the crude mixture was co-evaporated again with DCM/MeOH then carefully triturated with saturated aqueous NaHCO₃ (100 mL), diluted with water and stirred for 1.5 h. The precipitate was filtered, washed with water and air-dried then purified by silica gel chromatography (dry load) eluting with a gradient of 0 to 20% MeOH in DCM. Mixed fractions were combined and re-purified by silica gel chromatography using the same conditions. The clean material from both columns was combined, concentrated then dried in vacuo, affording 6-amino-5-(3-hydroxy-2,6-dimethyl-phenyl)-2,3-dimethyl-pyrrolo[2,3-b]pyrazine-7-carboxamide (5.3 g, 74% yield). ¹H NMR (400 MHz, DMSO-d6) δ 957 (s, 1H), 7.45 (br s, 1H), 7.18 - 7.02 (m, 4H), 6.93 (d, J = 8.3 Hz, 1H), 2.48 - 2.45 (m, 3H), 2.35 - 2.24 (m, 3H), 1.81 - 1.73 (m, 3H), 1.68 (s, 3H). MS: [M+1]: 326.4.

Chiral SFC separation of **Compound 164** (5.40 g, 16.6 mmol) (Instrument: Waters Prep 100 SFC-MS; Column: Phenomenex Lux Cellulose-2, 30 x 250 mm, 5 µm; Conditions: Isocratic at 55% ACN/EtOH 1:1 with 45% CO2; Flow Rate: 70 mL/min) provided **Compound 165** and **Compound 166.**

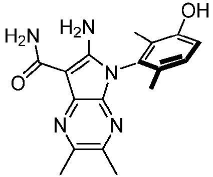

**Compound 165** from chiral SFC separation of **164.** Peak 1 (retention time 4.07 min, 99.99%): S-6-amino-5-(3-hydroxy-2,6-dimethyl-phenyl)-2,3-dimethyl-pyrrolo[2,3-b]pyrazine-7-carboxamide (1.31 g) was obtained as a light beige solid. ¹H NMR (400 MHz, DMSO-d6) δ 9.57 (s, 1H), 7.45 (br s, 1H), 7.12 (br s, 1H), 7.09 (br s, 2H), 7.06 (d, J = 8.8 Hz, 1H), 6.93 (d, J = 8.3 Hz, 1H), 2.47 (s, 3H), 2.31 (s, 3H), 1.76 (s, 3H), 1.68 (s, 3H). MS: [M+1]: 327.3.

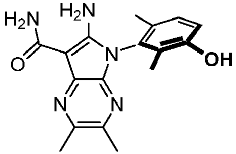

**Compound 166** from chiral SFC separation of **164.** Peak 2 (retention time 4.81 min, 99.83%): R-6-amino-5-(3-hydroxy-2,6-dimethyl-phenyl)-2,3-dimethyl-pyrrolo[2,3-b]pyrazine-7-carboxamide (1.43 g) was obtained as a light beige solid. ¹H NMR (400 MHz, DMSO-*d6*) δ 9.57 (s, 1H), 7.45 (br s, 1H), 7.12 (br s, 1H), 7.09 (br s, 2H), 7.07 (d, J = 8.4 Hz, 1H), 6.93 (d, J = 8.3 Hz, 1H), 2.47 (s, 3H), 2.31 (s, 3H), 1.76 (s, 3H), 1.68 (s, 3H). MS: [M+1]: 327.3.

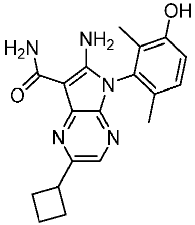

### Compound 173 (6-amino-2-cyclobutyl-5-(3-hydroxy-2,6-dimethyl-phenyl)pyrrolo[2,3-b]pyrazine-7-carboxamide)

Step 1. A microwave vial was loaded with [6-amino-7-carbamoyl-5-(3-methoxy-2,6-dimethyl-phenyl)pyrrolo[2,3-b]pyrazin-2-yl] trifluoromethanesulfonate (200 mg, 0.435 µmol) and Pd(PPh₃)₄ (56 mg, 49 µmol), flushed with nitrogen, THF (2 mL) was added, bubbled through with nitrogen, cyclobutyl zinc bromide solution (0.5 M, 4.35 mL) added, bubbled through with nitrogen, capped and transferred to a heat block preheated at 70 °C. The reaction mixture was stirred 90 min, cooled to rt, quenched with saturated aqueous NH₄Cl and extracted with EtOAc (2x). The combined organic extracts were washed with brine, dried over Na₂SO₄, filtered and concentrated. The residue was purified by silica gel chromatography eluting with a gradient of 0 to 100% EtOAc in hexanes to provide 6-amino-2-cyclobutyl-5-(3-methoxy-2,6-dimethylphenyl)pyrrolo[2,3-b]pyrazine-7-carboxamide (110 mg, 69% yield) as a white/beige solid.

Step 2. For OMe deprotection using BBr₃, the same procedure used for **Compound 35** was done on the appropriate intermediate (110 mg, 0.301 mmol) to provide a residue which was purified by preparative HPLC to provide 6-amino-2-cyclobutyl-5-(3-hydroxy-2,6-dimethylphenyl)pyrrolo[2,3-b]pyrazine-7-carboxamide (54 mg, 51% yield) as an off-white fluffy solid. ¹H NMR (400 MHz, DMSO-*d6*) δ 9.58 (s, 1H), 7.61 (s, 1H), 7.59 (br s, 1H), 7.32 (br s, 2H), 7.23 (br s, 1H), 7.07 (d, *J* = 8.3 Hz, 1H), 6.93 (d, *J* = 8.3 Hz, 1H), 3.66 (p, *J* = 8.6 Hz, 1H), 2.42 - 2.17 (m, 4H), 2.10 - 1.94 (m, 1H), 1.88 (td, J = 8.5, 4.0 Hz, 1H), 1.76 (s, 3H), 1.68 (s, 3H). MS: [M+1]: 352.4.

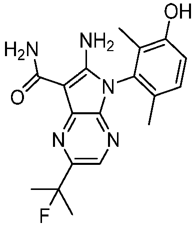

### Compound 178 (6-amino-2-(1-fluoro-1-methyl-ethyl)-5-(3-hydroxy-2,6-dimethylphenyl)pyrrolo[2,3-b]pyrazine-7-carboxamide)

Step 1. To a solution of 6-amino-5-(3-hydroxy-2,6-dimethyl-phenyl)-2-(1-hydroxy-1-methylethyl)pyrrolo[2,3-b]pyrazine-7-carboxamide (Compound **190,** 46.0 mg, 0.129 mmol) in DCM (2 mL) at -78 °C was added Deoxo-fluor^{®} solution (315 mg, 0.712 mmol, 50% in THF) dropwise. The mixture was stirred at 0 °C for 45 min, concentrated and purified by preparative HPLC to provide 6-amino-2-(1-fluoro-1-methyl-ethyl)-5-(3-hydroxy-2,6-dimethyl-phenyl)pyrrolo[2,3-b]pyrazine-7-carboxamide (23 mg, 50% yield) as an off-white fluffy solid. ¹H NMR (400 MHz, DMSO-d6) δ 9.63 (s, 1H), 7.92 (d, J = 0.6 Hz, 1H), 7.46 (br s, 2H), 7.37 (br s, 1H), 7.26 (br s, 1H), 7.08 (d, J = 8.3 Hz, 1H), 6.94 (d, J = 8.3 Hz, 1H), 1.75 (d, J = 22.4 Hz, 6H), 1.77 (s, 3H), 1.69 (s, 3H). ¹⁹F NMR (376 MHz, DMSO-d6) δ -137.78 (hept, J = 22.1 Hz). MS: [M+1]: 358.2.

Chiral SFC separation of **Compound 178** (19 mg, 0.053 mmol) (Instrument: Mettler Toledo Minigram SFC; Column: Phenomenex Lux Cellulose-2, 10 x 250 mm, 5 µm; Conditions: Isocratic at 45% ACN/EtOH 1:1 with 55% CO2; Flow Rate: 10 mL/min) provided **Compound 179** and **Compound 180.**

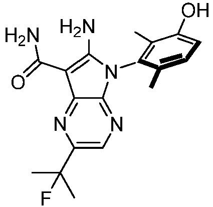

**Compound 179** from chiral SFC separation of **178.** Peak 1 (retention time 3.63 min, 99.87%): S-6-amino-2-(1-fluoro-1-methyl-ethyl)-5-(3-hydroxy-2,6-dimethyl-phenyl)pyrrolo[2,3-b]pyrazine-7-carboxamide (5 mg) as a white fluffy solid. ¹H NMR (400 MHz, DMSO-d6) δ 9.63 (s, 1H), 7.92 (s, 1H), 7.46 (br s, 2H), 7.42 - 7.32 (m, 1H), 7.30 = 7.19 (m, 1H), 7.08 (d, J = 8.3 Hz, 1H), 6.94 (d, J = 8.2 Hz, 1H), 1.77 (s, 3H), 1.75 (d, J = 22.4 Hz, 6H), 1.69 (s, 3H). ¹⁹F NMR (376 MHz, DMSO-d6) δ -137.75 (hept, J = 22.1 Hz). MS: [M+1]: 358.2.

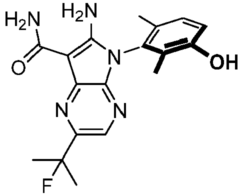

**Compound 180** from chiral SFC separation of **178.** Peak 2 (retention time 4.00 min, 99.90%): R-6-amino-2-(1-fluoro-1-methyl-ethyl)-5-(3-hydroxy-2,6-dimethyl-phenyl)pyrrolo[2,3-b]pyrazine-7-carboxamide (5 mg) as a white fluffy solid. ¹H NMR (400 MHz, DMSO-d6) δ 9.63 (s, 1H), 7.92 (s, 1H), 7.46 (br s, 2H), 7.37 (br s, 1H), 7.26 (br s, 1H), 7.08 (d, J = 8.5 Hz, 1H), 6.94 (d, J = 8.3 Hz, 1H), 1.77 (s, 3H), 1.74 (d, J = 22.4 Hz, 6H), 1.69 (s, 3H). ¹⁹F NMR (376 MHz, DMSO-*d6*) δ -137.75 (hept, J = 22.1 Hz). MS: [M+1]: 358.2.

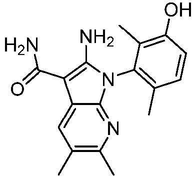

### Compound 181 2-amino-1-(3-hydroxy-2,6-dimethyl-phenyl)-5,6-dimethyl-pyrrolo[2,3-b]pyridine-3-carboxamide (from method D)

Step 1. A pressure vessel was loaded with 3-bromo-2-chloro-6-methyl-5-nitro-pyridine (10.11 g, 40.2 mmol) and 3-methoxy-2,6-dimethylaniline (Aniline A2, 9.20 g, 60.8 mmol). NMP (40 mL) and 2,6-dimethylpyridine (8.58 g, 80.1 mmol, 9.3 mL) were added and the reaction mixture was heated to 130 °C (pellet bath) for 5 days until satisfactory conversion as assessed by UPLCMS was achieved. The reaction mixture was cooled to RT and the resulting paste transferred to a conical flask and 500 mL HCl 0.5 N was added dropwise while stirring, resulting in a sticky gum. The supernatant was filtered on a Buchner funnel. The remaining gum was washed with H₂O, dissolved in DCM, and combined with the solid which had also been dissolved in DCM (200 mL total). The DCM solution was dried over Na₂SO₄, filtered and concentrated. The crude residue was purified by silica gel chromatography (dry load) eluting with a gradient of 0 to 100% DCM in heptanes to provide 3-bromo-N-(3-methoxy-2,6-dimethyl-phenyl)-6-methyl-5-nitro-pyridin-2-amine (10.5 g, 71% yield) as a light yellow solid.

Step 2. To a RBF containing sodium hydride (3.13 g, 72.2 mmol, 60% w/w in mineral oil) in DME (150 mL) was added a solution of propanedinitrile (4.75 g, 71.9 mmol) in DME (50 mL) slowly. After stirring for 1h, 3-bromo-N-(3-methoxy-2,6-dimethyl-phenyl)-6-methyl-5-nitro-pyridin-2-amine (10.5 g, 28.7 mmol) and Pd(dppf)Cl₂•DCM (2.31 g, 2.83 mmol) were added. The resulting mixture was degassed by bubbling N₂ through solution, equipped with a condenser, and heated to 95 °C for 1h. The reaction mixture was cooled to RT, poured into saturated aqueous NH₄Cl and extracted with DCM (3x). The combined organic extracts were washed with H₂O, brine, dried over Na₂SO₄, filtered and adsorbed on silica. The crude residue was purified by silica gel chromatography (dry load) eluting with a gradient of 0 to 100% EtOAc in heptanes. Appropriate fractions were combined, concentrated and the resulting solid was triturated with DCM, filtered, and dried in vacuo, affording 2-amino-1-(3-methoxy-2,6-dimethyl-phenyl)-6-methyl-5-nitro-pyrrolo[2,3-b]pyridine-3-carbonitrile (7.97 g, 79% yield) as a bright yellow solid. A second crop of material was obtained from the filtrate from the previous trituration by purification by flash chromatography and trituration in the same fashion, providing additional 2-amino-1-(3-methoxy-2,6-dimethyl-phenyl)-6-methyl-5-nitro-pyrrolo[2,3-b]pyridine-3-carbonitrile (1.04 g, 10% yield) as a dark yellow solid.

Step 3. To a solution of 2-amino-1-(3-methoxy-2,6-dimethyl-phenyl)-6-methyl-5-nitro-pyrrolo[2,3-b]pyridine-3-carbonitrile (9.0 g, 25.6 mmol) in THF (120 mL) was added triethylamine (7.99 g, 78.9 mmol, 11 mL) , DMAP (312 mg, 2.55 mmol) and tert-butoxycarbonyl tert-butyl carbonate (17.0 g, 77.9 mmol) . The mixture was stirred at 50 °C for 40 min. The heating was stopped, and ethylenediamine (6.20 g, 103 mmol, 6.90 mL) was added and the mixture was stirred at RT for 45 min, then diluted with H₂O and DCM. The layers were separated and the aqueous layer was extracted with DCM (2x). The combined organic extracts were washed with half-saturated brine, dried over Na₂SO₄, filtered and concentrated. The crude residue was purified by silica gel chromatography eluting with a gradient of 0 to 60% EtOAc in heptanes to provide tert-butyl N-[3-cyano-1-(3-methoxy-2,6-dimethyl-phenyl)-6-methyl-5-nitro-pyrrolo[2,3-b]pyridin-2-yl]carbamate (13.94 g, quantitative yield) as an off-white solid, which was contaminated with tert-butyl N-[2-(tert-butoxycarbonylamino)ethyl]carbamate (50 mol% by ¹H NMR).

Step 4. To a RBF containing tert-butyl N-[3-cyano-1-(3-methoxy-2,6-dimethyl-phenyl)-6-methyl-5-nitro-pyrrolo[2,3-b]pyridin-2-yl]carbamate (13.94 g, 25.6 mmol) (assumed quantitative from the previous step) in DCM (280 mL) and MeOH (280 mL) was added palladium on carbon (2.08 g, 1.95 mmol, 10%w/w) as a slurry in some of the solvent mixture. The reaction mixture was flushed with H₂ and stirred under H₂ atmosphere (balloon) overnight. The reaction mixture was flushed with N₂, filtered on a celite pad, rinsed with DCM and MeOH. The filtrate was concentrated and dried in vacuo, affording a light yellow solid which was purified by silica gel chromatography (dry load) eluting with a gradient of EtOAc (20 to 100%) in heptanes. Appropriate fractions were combined and concentrated in vacuo to afford tert-butyl N-[5-amino-3-cyano-1-(3-methoxy-2,6-dimethyl-phenyl)-6-methyl-pyrrolo[2,3-b]pyridin-2-yl]carbamate (9.34 g, 87% yield) as an off-white solid.

Step 5. To a solution of tert-butyl N-[5-amino-3-cyano-1-(3-methoxy-2,6-dimethyl-phenyl)-6-methyl-pyrrolo[2,3-b]pyridin-2-yl]carbamate (10.34 g, 24.5 mmol) in acetonitrile (100 mL) and DMF (60 mL) was added tert-butyl nitrite (5.20 g, 50.5 mmol, 6.0 mL), followed by copper(II) bromide (6.58 g, 29.4 mmol). The mixture was heated to 70 °C for 35 min, cooled to RT, diluted with H₂O (600 mL) and NH₄OH conc (30 mL) and extracted with EtOAc (3x). The combined organic extracts were washed with saturated NH₄Cl (2x), half-saturated brine, dried over Na₂SO₄, filtered and concentrated. The residue was purified by silica gel chromatography (dry load) eluting with a gradient of EtOAc (0 to 100%) in heptanes. Appropriate fractions were combined and concentrated in vacuo to afford tert-butyl N-[5-bromo-3-cyano-1-(3-methoxy-2,6-dimethyl-phenyl)-6-methyl-pyrrolo[2,3-b]pyridin-2-yl]carbamate (6.18 g, 52% yield) as an ivory solid.

Step 6. Tert-butyl N-[5-bromo-3-cyano-1-(3-methoxy-2,6-dimethyl-phenyl)-6-methyl-pyrrolo[2,3-b]pyridin-2-yl]carbamate (6.18 g, 12.7 mmol) in EtOH (60 mL) was treated with aqueous HCl (6M, 34 mL) and stirred for 70 min at 80 °C then cooled to RT and concentrated. The residue was dissolved in MeOH, made alkaline with excess Et₃N and concentrated again. The residue was purified by silica gel chromatography (dry load) eluting with a gradient of EtOAc (0 to 100%) in heptanes. Appropriate fractions were combined and concentrated in vacuo to afford 2-amino-5-bromo-1-(3-methoxy-2,6-dimethyl-phenyl)-6-methyl-pyrrolo[2,3-b]pyridine-3-carbonitrile (3.70 g, 75% yield) as a dark magenta solid.

Step 7. 2-Amino-5-bromo-1-(3-methoxy-2,6-dimethyl-phenyl)-6-methyl-pyrrolo[2,3-b]pyridine-3-carbonitrile (3.70 g, 9.6 mmol) was stirred in concentrated sulfuric acid (18M, 25 mL) for 55 min, then the reaction mixture was quenched with crushed ice, placed in an ice bath and made alkaline to pH 8-9 with saturated NH₄OH added slowly. The resulting solid was collected by filtration on a Buchner funnel and washed with H₂O. The material was air-dried then co-evaporated twice with toluene and dried in vacuo, then stirred in 10% MeOH/DCM and filtered on a silica plug, eluting with 10% MeOH/DCM to remove residual ammonium salts. The filtrate was concentrated then dried in vacuo, affording 2-amino-5-bromo-1-(3-methoxy-2,6-dimethylphenyl)-6-methyl-pyrrolo[2,3-b]pyridine-3-carboxamide (3.80 g, 98% yield) as a pink solid.

Step 8. To a solution of methyl magnesium chloride (3M, 18.8 mL) in THF (160 mL) in a RBF under N₂ was added a solution of zinc dichloride in THF (0.5M, 112 mL) at RT dropwise via an addition funnel. After the addition, the resulting white suspension was stirred at RT for 35 min. To the zincate solution was added 2-amino-5-bromo-1-(3-methoxy-2,6-dimethyl-phenyl)-6-methyl-pyrrolo[2,3-b]pyridine-3-carboxamide (4.48 g, 11.1 mmol), the flask was rinsed with 20 mL THF, and palladium (0) tetrakis(triphenylphosphine) (1.14 g, 0.987 mmol) was added. The mixture was bubbled through with N₂ then equipped with a condenser and refluxed (heat block set to 80 °C) for 24h. The reaction mixture was cooled to RT, then diluted with saturated aqueous NH₄Cl and extracted with EtOAc (3x). The combined organic extracts were washed with brine, dried over Na₂SO₄, filtered and concentrated. The residue was purified by silica gel chromatography (dry load) eluting with a gradient of EtOAc (0 to 100%) in heptanes then purified again by silica gel chromatography (dry load) eluting with a gradient of MeOH (1 to 15%) in DCM. Appropriate fractions from the two columns were combined and concentrated in vacuo to afford 2-amino-1-(3-methoxy-2,6-dimethyl-phenyl)-5,6-dimethyl-pyrrolo[2,3-b]pyridine-3-carboxamide (2.22 g, 59% yield, 77% purity) as a light pink solid, which contained some 2-amino-1-(3-methoxy-2,6-dimethylphenyl)-6-methyl-pyrrolo[2,3-b]pyridine-3-carboxamide side product (19% by UPLCMS).

Step 9. To a suspension of 2-amino-1-(3-methoxy-2,6-dimethyl-phenyl)-5,6-dimethyl-pyrrolo[2,3-b]pyridine-3-carboxamide (2.22 g, 6.56 mmol, 77% purity) in DCM (25 mL) was added tribromoborane in DCM (1M, 26 mmol, 26 mL) dropwise. The reaction mixture was stirred at RT for 45 min, then concentrated to dryness. The crude product was taken in DCM and placed in an ice bath and MeOH was added carefully (exotherm). The mixture was concentrated to dryness then co-evaporated twice with MeOH. The residue was triturated with saturated aqueous NaHCO₃. The solids were collected by filtration on a Buchner funnel, washed with H₂O and air-dried. The still wet solid was dissolved in DCM/MeOH, concentrated to dryness and triturated in 20% MeOH/DCM (50 mL). The solid was collected by filtration, washed with 20% MeOH/DCM, air-dried then dried in vacuo to afford 2-amino-1-(3-hydroxy-2,6-dimethyl-phenyl)-5,6-dimethyl-pyrrolo[2,3-b]pyridine-3-carboxamide (1.60g, 75% yield) as a light beige solid. MS: [M+1]: 325.1. A different batch was purified by preparative HPLC to yield 2-amino-1-(3-hydroxy-2,6-dimethylphenyl)-5,6-dimethyl-pyrrolo[2,3-b]pyridine-3-carboxamide (63% yield) as an off-white fluffy solid. ¹H NMR (400 MHz, DMSO-d6) δ 951 (s, 1H), 7.82 (s, 1H), 7.05 (d, J = 8.3 Hz, 1H), 6.90 (d, J = 8.2 Hz, 1H), 6.71 (br s, 2H), 6.64 (br s, 2H), 2.26 (s, 3H), 2.23 (s, 3H), 1.74 (s, 3H), 1.65 (s, 3H). MS: [M+1]: 325.1.

Chiral SFC separation of **Compound 181** (1.60g, 4.93 mmol) (Instrument: Waters Prep 100 SFC-MS; Column: Phenomenex Lux Cellulose-2, 30 x 250 mm, 5 µm; Conditions: isocratic at 55% IPA + 10mM Ammonium Formate with 45% CO₂; Flow Rate: 70 mL/min) provided **Compound 182** and **Compound 183.**

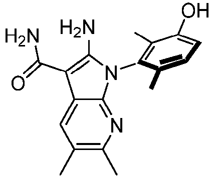

**Compound 182** from SFC separation of **181.** Peak 1 (retention time 3.94 min, 99.86%): (S)-2-amino-1-(3-hydroxy-2,6-dimethyl-phenyl)-5,6-dimethyl-pyrrolo[2,3-b]pyridine-3-carboxamide (381 mg) was obtained as an off white fluffy solid. ¹H NMR (400 MHz, DMSO-d6) δ 9.50 (s, 1H), 7.83 (s, 1H), 7.05 (d, J = 8.3 Hz, 1H), 6.90 (d, J = 8.3 Hz, 1H), 6.72 (s, 2H), 6.65 (s, 2H), 2.26 (s, 3H), 2.24 (s, 3H), 1.74 (s, 3H), 1.65 (s, 3H). MS: [M+1]: 325.1.

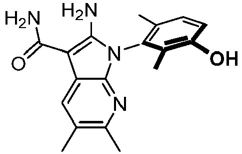

**Compound 183** from SFC separation of **181.** Peak 2 (retention time 4.35 min, 98.09%): (*R*)-2-amino-1-(3-hydroxy-2,6-dimethyl-phenyl)-5,6-dimethyl-pyrrolo[2,3-b]pyridine-3-carboxamide (495 mg) was obtained as an off white fluffy solid. ¹H NMR (400 MHz, DMSO-d6) δ 9.50 (s, 1H), 7.83 (s, 1H), 7.05 (d, J = 8.2 Hz, 1H), 6.90 (d, J = 8.2 Hz, 1H), 6.72 (s, 2H), 6.66 (s, 2H), 2.26 (s, 3H), 2.24 (s, 3H), 1.74 (s, 3H), 1.65 (s, 3H). MS: [M+1]: 325.1.

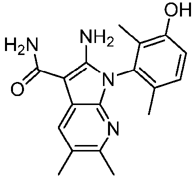

### Compound 181 2-amino-1-(3-hydroxy-2,6-dimethyl-phenyl)-5,6-dimethyl-pyrrolo[2,3-b]pyridine-3-carboxamide (From method O)

Step 1. Sulfuric acid (140.1 mL, 2575 mmol) was added slowly to water (1.15 L) and the solution was cooled at 25 °C. 2-Amino-3-bromo-5,6-dimethylpyridine (114.8 g, 571.0 mmol) was added in one portion to get a solution. The solution was cooled to 0 °C - 5 °C with an ice-water bath to give a suspension. Under strong stirring, a solution of sodium nitrite (49.25 g, 713.7 mmol) in water (175.0 mL) was added dropwise over 90 minutes. The ice-water bath was removed, and the suspension was warmed up slowly to 11 °C and stirred for 1 hour. A solution of sodium hydroxide (175 g, 4.37 mol) in 400 mL of water was added dropwise to maintain the temperature under 20 °C. The pH of the solution was adjusted to 7 with K₂HPO₄ (~58 g, 0.33 mol) in 70 mL of water. The suspension was filtered at 10 °C. The filter cake was triturated in water (250 mL) and filtered. The filter cake was washed profusely with ice-cooled water and dried by vacuum suction. The product was oven-dried under vacuum at 60 °C overnight to get 3-bromo-5,6-dimethylpyridin-2-ol as a light yellow crystalline solid (105.69 g, 91.6%) ¹H NMR (400 MHz, DMSO-*d*6) δ 11.96 (br s, 1H), 7.73 (s, 1H), 2.11 (s, 3H), 1.96 (s, 3H). MS: [M+1]: 202.0, 204.0.

Step 2. To a solution of 3-bromo-5,6-dimethylpyridin-2-ol (105.3 g, 521.2 mmol) in *N,N-*dimethylformamide (316 mL) and toluene (527 mL) at 90 °C under nitrogen was added phosphorus oxybromide (1.3:1, 56.5% (w/w) in xylenes) (278 mL, 781.7 mmol) dropwise over 90 min. After the addition was complete, the mixture was stirred at 90 °C overnight. The mixture was cooled to room temperature and was slowly added to water (2 L). The flask was washed with 500 mL of water. The combined aqueous phases were extracted with MTBE (3 x 1 L). The organic phases were combined and washed with 0.5N NaOH (1 L), water (3 x 1 L) and brine (1 L) then dried with sodium sulfate and concentrated. The solid was partially dissolved in MTBE (400 mL) and heptanes (300 mL) was added. The mixture was concentrated under reduced pressured to ~1.7 volume to provide a precipitate. The mixture was filtered and rinsed with heptanes. The residue was dried to afford 2,3-dibromo-5,6-dimethylpyridine as a beige solid (114.290 g, 82.8 %). The filtrate was further concentrated and filtered to provide a second crop of solid: (8.73 g, 6.32 %). ¹H NMR (400 MHz, DMSO-d6) δ 7.95 (s, 1H), 2.36 (s, 3H), 2.21 (s, 3H). MS: [M+1]: 264.0, 266.0, 268.0.

Step 3. A 2000 mL 4-neck round-bottomed flask was charged with **intermediate A2** (33.0 g, 218.0 mmol), degassed 1,2-dimethoxyethane (750 mL), 2,3-dibromo-5,6-dimethylpyridine (55 g, 207.6 mmol), 4,5-bis(diphenylphosphino)-9,9- dimethylxanthene (10.8 g, 18.68 mmol) and cesium carbonate (169.1 g, 519.0 mmol). The reaction mixture was sonicated for 20 minutes while sparging the suspension with nitrogen. Tris(dibenzylideneacetone)dipalladium(0) (8.55 g, 9.341 mmol) was added and the suspension was heated to reflux. After 13 hours of stirring, the reaction mixture was cooled to room temperature and was filtered over a pad of silica gel. The filter cake was washed with ethyl acetate (1.2L). The filtrate was evaporated to a volume of ~200 mL and heptanes (300 mL) was added. The solvents were evaporated to provide a suspension in ~2 volumes of solvent. The suspension was filtered and washed with heptanes to provide 3-bromo-N-(3-methoxy-2,6-dimethylphenyl)-5,6-dimethylpyridin-2-amine as a light yellow solid. (56.2 g, 80.8 %). ¹H NMR (400 MHz, DMSO-d6) δ 7.57 (s, 1H), 7.26 (s, 1H), 7.02 (d, *J* = 8.6 Hz, 1H), 6.77 (d, *J* = 8.3 Hz, 1H), 3.76 (s, 3H), 2.07 (s, 3H), 2.04 (s, 3H), 2.03 (s, 3H), 1.94 (s, 3H). MS: [M+1]: 335.2, 337.2.

Step 4. To a degassed solution of malononitrile (33.3 g, 503.5 mmol) in 1,2-dimethoxyethane (1000 mL) was added sodium tert-butoxide (46.3 g, 482.0 mmol) in 4 portions. The reaction mixture was stirred 30 minutes at room temperature to get a solution. 3-bromo-N-(3-methoxy-2,6-dimethylphenyl)-5,6-dimethylpyridin-2-amine (80 g, 238.6 mmol) and 1,1'-bis(diphenylphosphino)ferrocene palladium(II) chloride, complex with dichloromethane (14.9 g, 18.26 mmol) were added in one portion and the suspension was heated to strong reflux. After 17 hours of stirring, the reaction mixture was transferred into a 5000 mL flask and ethyl acetate (1.5 L) was added. A solution of N-acetyl-L-cysteine (12.1 g, 74 mmol, 4x Pd mol content) and Na₂CO₃ (15.7 g, 148 mmol) in water (500 mL) were added. The biphasic solution was stirred for 10 minutes at 60 °C and then cooled slowly to 40 °C over 75 minutes. Inside the 5000 ml flask, the two layers were separated at 40°C and the organic phase was washed with water (2x 250 mL) and brine (200 mL) and then filtered over a pad of silica gel (3 inches, 185 g). The filter cake was rinsed with DCM/EtOAc. The filtrate was evaporated, and the solvents were switched for EtOAc during rotavap evaporation to provide a suspension. The suspension was filtered at room temperature and the filter cake was triturated in 50 mL of ice-cooled ethyl acetate. The product was filtered and the filter cake was rinsed with 50 mL of ice-cooled ethyl acetate. The product was oven-dried under vacuum at 60 °C overnight to afford 2-amino-1-(3-methoxy-2,6-dimethylphenyl)-5,6-dimethyl-1H-pyrrolo[2,3-b]pyridine-3-carbonitrile as a light yellow solid. (65.38 g, 85.5 %, 8% (w/w) DME). ¹H NMR (400 MHz, DMSO-*d*6) δ 7.38 (s, 1H), 7.22 (d, J = 8.4 Hz, 1H), 7.07 (d, J = 8.6 Hz, 1H), 6.76 (br s, 2H), 3.84 (s, 3H), 2.26 (s, 3H), 2.23 (s, 3H), 1.78 (s, 3H), 1.69 (s, 3H). MS: [M+1]: 321.2.

Step 5. To methanesulfonic acid (600 mL, 9239 mmol) was added slowly a solution of sulfuric acid (93 mL) / water (7.0 mL) over 5 minutes at room temperature. 2-amino-1-(3-methoxy-2,6-dimethylphenyl)-5,6-dimethyl-1H-pyrrolo[2,3-b]pyridine-3-carbonitrile (80 g, 249.7 mmol) was added portion wise over 15 minutes to keep the reaction temperature below 40 °C. The resulting solution was stirred at room temperature for 90 minutes. DL-Methionine (149.028 g, 998.8 mmol) was added portion wise over 20 minutes below 40°C. The solution was stirred at 40°C. After 37 hours of stirring, the reaction mixture was cooled to room temperature and then added slowly over 1.5h to a solution of K₂HPO₄ (100 g) and NaOH (540 g) in water (5 L). EtOAc (1 L) was added and the biphasic mixture was stirred for 5 min to get a precipitate. The product was filtered. The mother liquors were extracted with EtOAc (3 x 1L). The organic phases were combined, dried over Na₂SO₄, filtered and concentrated under reduced pressure to ~ 100 mL to get a suspension. The suspension was filtered and rinse with EtOAc (50 mL). The solids were combined and triturated in water (800 mL) two times. The residue was suspended in EtOAc (500 mL), stirred for 10 minutes and filtered. The product was oven-dried under reduced pressure to get 67.8 g of crude product. The compound was suspended in DMSO (350 mL, 5 vol) and the mixture was heated to 65 °C to get a solution. The solution was cooled slowly at 28 °C with a water bath. Water (1.05 L) was added dropwise over 2 hours to get a suspension. After 5 minutes of stirring at room temperature, the product was filtered. The solid was triturated in 100 mL of water and filtered. The filter cake was washed with 2 x 100 mL of water. The product was oven dried at 60 °C under vacuum to get 2-amino-1-(3-hydroxy-2,6-dimethyl-phenyl)-5,6-dimethyl-pyrrolo[2,3-b]pyridine-3-carboxamide as a light yellow solid. 61.5 g, (75%, 3% (w/w) EtOAc and 6% (w/w) DMSO). ¹H NMR (400 MHz, DMSO-*d*6) δ 9.47 (s, 1H), 7.82 (s, 1H), 7.05 (d, *J* = 8.2 Hz, 1H), 6.90 (d, *J* = 8.2 Hz, 1H), 6.71 (br. s, 2H), 6.64 (br. s, 2H), 2.27 (s, 3H), 2.24 (s, 3H), 1.75 (s, 3H), 1.66 (s, 3H). MS: [M+1]: 325.2.

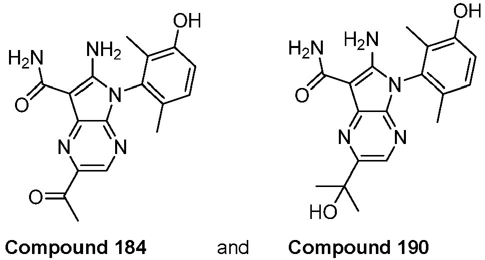

Step 1. To a solution of **Intermediate D** (1.07 g, 2.33 mmol) in DCM (9 mL) was added BBr₃ solution in DCM (1 M, 9.3 mL, 9.3 mmol). The mixture was stirred at 0 °C for 2 h, silica was added, the mixture was concentrated, then purified by silica gel chromatography (dry load) eluting with a gradient of 0 to 20% MeOH in DCM to provide [6-amino-7-carbamoyl-5-(3-hydroxy-2,6-dimethyl-phenyl)pyrrolo[2,3-b]pyrazin-2-yl] trifluoromethanesulfonate (744 mg, 72% yield) as an off-white solid.

Step 2. A solution of [6-amino-7-carbamoyl-5-(3-hydroxy-2,6-dimethyl-phenyl)pyrrolo[2,3-b]pyrazin-2-yl] trifluoromethanesulfonate (744 mg, 1.67 mmol), PdCl₂(PPh₃)₂ (117 mg, 0.167 mmol) in a mixture of DMF (8 mL) and MeOH (8 mL) and Et₃N (1.40 mL, 10.0 mmol) was heated at 70 °C under an atmosphere of carbon monoxide (balloon). The apparatus was previously flushed with carbon monoxide once. After 2 h, more PdCl₂(PPh₃)₂ (117 mg, 0.167 mmol) was added and the reaction mixture was continued for 18 h. The reaction mixture was cooled to rt, filtered through Celite, rinsing with MeOH and the filtrate was concentrated. The residue was purified by silica gel chromatography (dry load) eluting with a gradient of 0 to 20% of MeOH in DCM to provide a dark green sticky solid. The solid was dissolved in EtOAc, passed through a silica plug using EtOAc / MeOH (5%) that provided upon evaporating the volatiles methyl 6-amino-7-carbamoyl-5-(3-hydroxy-2,6-dimethyl-phenyl)pyrrolo[2,3-b]pyrazine-2-carboxylate (418 mg, 70% yield) as a light brown sticky solid.

Step 3. A solution of methyl 6-amino-7-carbamoyl-5-(3-hydroxy-2,6-dimethylphenyl)pyrrolo[2,3-b]pyrazine-2-carboxylate (322 mg, 0.906 mmol) in THF (12 mL) was cooled to - 40 °C and MeMgCl solution in THF (3 M, 4.53 mL, 13.1 mmol) was added dropwise. The mixture was left to warm to rt overnight, quenched with saturated aqueous NH₄Cl (25 mL), the pH was adjusted to 7-8 with 1N HCl and the mixture was extracted with DCM (3x). The combined organic extracts were washed with brine, dried over Na₂SO₄, filtered and concentrated. The residue was purified by silica gel chromatography eluting with a gradient of 0 to 20% MeOH in DCM to provide 6-amino-5-(3-hydroxy-2,6-dimethyl-phenyl)-2-(1-hydroxy-1-methyl-ethyl)pyrrolo[2,3-b]pyrazine-7-carboxamide **Compound 190** (103 mg, 32% yield) as a light tan solid. ¹H NMR (400 MHz, DMSO-*d6*) δ 9.61 (s, 1H), 7.99 (s, 1H), 7.50 (br s, 1H), 7.42 (br s, 2H), 7.23 (br s, 1H), 7.08 (d, J = 8.5 Hz, 1H), 6.94 (d, J = 8.3 Hz, 1H), 5.95 - 5.81 (m, 1H), 5.30 - 5.17 (m, 1H), 2.19 (s, 3H), 1.78 (s, 3H), 1.70 (s, 3H). MS: [M+1]: 338.1; and 2-acetyl-6-amino-5-(3-hydroxy-2,6-dimethylphenyl)pyrrolo[2,3-b]pyrazine-7-carboxamide **Compound 184** (31 mg, 10% yield) as a light tan solid. ¹H NMR (400 MHz, DMSO-*d*6) δ 9.83 (br s, 1H), 8.38 (d, J = 1.9 Hz, 1H), 7.68 (br s, 2H), 7.40 (s, 2H), 7.09 (d, J = 8.2 Hz, 1H), 6.97 (d, J = 8.1 Hz, 1H), 2.68 (s, 3H), 1.77 (s, 3H), 1.69 (s, 3H). MS: [M+1]: 340.1.

Chiral SFC separation of **Compound 190** (39 mg, 0.110 mmol) (Instrument: Mettler Toledo Minigram SFC; Column: Phenomenex Lux Cellulose-2, 10 x 250 mm, 5 µm; Conditions: Isocratic at 40% IPA + 10mM Ammonium Formate with 60% CO2; Flow Rate: 10 mL/min) provided **Compound 191** and **Compound 192.**

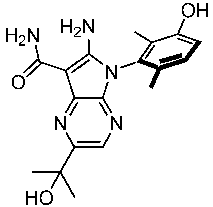

**Compound 191** from chiral SFC separation of **190.** Peak 1 (retention time 3.83 min, 100%): *S*-6-amino-5-(3-hydroxy-2,6-dimethyl-phenyl)-2-(1-hydroxy-1-methyl-ethyl)pyrrolo[2,3-b]pyrazine-7-carboxamide (10 mg) as an off-white solid. ¹H NMR (400 MHz, DMSO-d6) δ 9.61 (br s, 1H), 7.99 (s, 1H), 7.52 (br d, J = 3.0 Hz, 1H), 7.32 (br s, 2H), 7.18 (br d, J = 3.1 Hz, 1H), 7.08 (dt, J = 8.2, 0.8 Hz, 1H), 6.93 (d, J = 8.3 Hz, 1H), 5.26 (s, 1H), 1.77 (s, 3H), 1.68 (s, 3H), 1.51 (s, 6H). MS: [M+1]: 356.2.

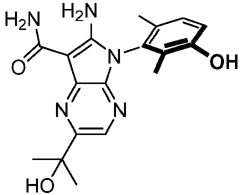

**Compound 192** from chiral SFC separation of **190.** Peak 2 (retention time 4.07 min): *R*-6-amino-5-(3-hydroxy-2,6-dimethyl-phenyl)-2-(1-hydroxy-1-methyl-ethyl)pyrrolo[2,3-b]pyrazine-7-carboxamide (10 mg). ¹H NMR (400 MHz, DMSO-d6) δ 9.62 (br s, 1H), 7.99 (s, 1H), 7.52 (br d, J = 3.2 Hz, 1H), 7.32 (br s, 2H), 7.18 (br d, J = 3.1 Hz, 1H), 7.08 (dt, J = 8.3, 0.8 Hz, 1H), 6.93 (d, J = 8.3 Hz, 1H), 5.26 (s, 1H), 1.77 (s, 3H), 1.68 (s, 3H), 1.51 (s, 6H). MS: [M+1]: 356.2.

Chiral SFC separation of **Compound 184** (103 mg, 0.290 mmol) (Instrument: Mettler Toledo Minigram SFC; Column: Phenomenex Lux Cellulose-2, 10 x 250 mm, 5 µm; Conditions: Isocratic at 40% IPA + 10mM Ammonium Formate with 60% CO2; Flow Rate: 10 mL/min) provided **Compound 185** and **Compound 186.**

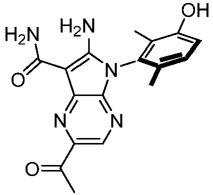

**Compound 185** from chiral SFC separation of **184.** Peak 1 (retention time 3.87 min, 100%): *S*-2-acetyl-6-amino-5-(3-hydroxy-2,6-dimethyl-phenyl)pyrrolo[2,3-b]pyrazine-7-carboxamide (9 mg) as a yellow fluffy solid. ¹H NMR (400 MHz, DMSO-*d*6) δ 9.68 (br s, 1H), 8.38 (s, 1H), 7.69 (br s, 2H), 7.41 (br s, 2H), 7.09 (dt, J = 8.3, 0.7 Hz, 1H), 6.95 (d, J = 8.3 Hz, 1H), 2.68 (s, 3H), 1.77 (d, J = 0.8 Hz, 3H), 1.69 (s, 3H). MS: [M+1]: 340.2.

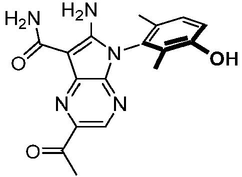

**Compound 186** from chiral SFC separation of **184.** Peak 2 (retention time 4.21 min, 99.80%): *R*-2-acetyl-6-amino-5-(3-hydroxy-2,6-dimethyl-phenyl)pyrrolo[2,3-b]pyrazine-7-carboxamide (9 mg) as a yellow fluffy solid. ¹H NMR (400 MHz, DMSO-*d*6) δ 9.69 (br s, 1H), 8.38 (s, 1H), 7.69 (br s, 2H), 7.41 (br s, 2H), 7.09 (dt, J = 8.2, 0.7 Hz, 1H), 6.95 (d, J = 8.3 Hz, 1H), 2.68 (s, 3H), 1.77 (d, J = 0.7 Hz, 3H), 1.69 (s, 3H). MS: [M+1]: 340.1.

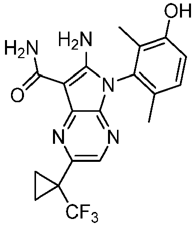

### Compound 198 (6-amino-5-(3-hydroxy-2,6-dimethyl-phenyl)-2-[1-(trifluoromethyl)cyclopropyl]pyrrolo[2,3-b]pyrazine-7-carboxamide)

Step 1. A microwave vial was loaded with **Intermediate D** (500 mg, 1.09 mmol), 4,4,6-trimethyl-2-[1-(trifluoromethyl)vinyl]-1,3,2-dioxaborinane (502 mg, 2.26 mmol), dioxane (5 mL) and aqueous Na₂CO₃ (2 M, 1.63 mL, 3.26 mmol), flushed with nitrogen (house vac then nitrogen, 3x). PdCl₂(dppf).CH₂Cl₂ (444 mg, 0.544 mmol) added, the vial was flushed again, capped and transferred to a preheated (80 °C) heat block and stirred overnight. After cooling to rt, the reaction mixture was filtered on Celite, washed with water and DCM and diluted with brine. The layers were separated (phase separator). The aqueous layer was extracted with DCM (2x). The combined organic extracts were concentrated and purified by silica gel chromatography eluting with a gradient of 0 to 50% EtOAc in DCM affording 6-amino-5-(3-methoxy-2,6-dimethyl-phenyl)-2-[1-(trifluoromethyl)vinyl]pyrrolo[2,3-b]pyrazine-7-carboxamide (169 mg, 38% yield) as a dark yellow gum.

Step 2. To a solution of 6-amino-5-(3-methoxy-2,6-dimethyl-phenyl)-2-[1-(trifluoromethyl)vinyl]pyrrolo[2,3-b]pyrazine-7-carboxamide (42.0 mg, 0.104 mmol) in DCM (2 mL) at 0 °C was added diazomethane solution in Et₂O (0.5 M, 400 µL) then warmed to rt. Another portion of diazomethane solution (0.5 M, 400 µL) was added. After reaction was deemed complete by UPLCMS, the reaction mixture was quenched with AcOH (200 µL), stirred for a few minutes and concentrated to dryness, then taken in saturated aqueous NaHCO₃ and DCM. The layers were separated (phase separator). The aqueous layer was extracted with DCM (3 x). The combined organic extracts were concentrated then dried in vacuo, affording 6-amino-5-(3-methoxy-2,6-dimethyl-phenyl)-2-[5-(trifluoromethyl)-3,4-dihydropyrazol-5-yl]pyrrolo[2,3-b]pyrazine-7-carboxamide (48 mg, quantitative yield) as a yellow gum.

Step 3. 6-amino-5-(3-methoxy-2,6-dimethyl-phenyl)-2-[5-(trifluoromethyl)-3,4-dihydropyrazol-5-yl]pyrrolo[2,3-b]pyrazine-7-carboxamide (48.0 mg, 0.107 mmol) was dissolved in xylenes (3 mL) and heated to 130 °C with a reflux condenser open to air for a total of 75 min. The reaction mixture was concentrated then purified by silica gel chromatography eluting with a gradient of 0 to 100% EtOAc in heptane, then a gradient of 0 to 20% MeOH in EtOAc to provide 6-amino-5-(3-methoxy-2,6-dimethyl-phenyl)-2-[1-(trifluoromethyl)cyclopropyl]pyrrolo[2,3-b]pyrazine-7-carboxamide (35 mg, 81% yield) as a light yellow solid.

Step 4. For OMe deprotection using BBr₃, the same procedure used for **Compound 35** provided a residue which was purified by preparative HPLC to provide 6-amino-5-(3-hydroxy-2,6-dimethyl-phenyl)-2-[1-(trifluoromethyl)cyclopropyl]pyrrolo[2,3-b]pyrazine-7-carboxamide (17 mg, 50% yield) as a white fluffy solid. ¹H NMR (400 MHz, DMSO-d6) δ 9.62 (s, 1H), 7.88 (s, 1H), 7.49 (br s, 2H), 7.36 (br s, 1H), 7.28 (br s, 1H), 7.08 (d, J = 8.2 Hz, 1H), 6.94 (d, J = 8.3 Hz, 1H), 1.76 (s, 3H), 1.69 (s, 3H), 1.46 - 1.29 (m, 4H). ¹⁹F NMR (376 MHz, DMSO-*d6*) δ -66.85. MS: [M+1]: 406.1.

Chiral SFC separation of **Compound 198** (14.5 mg, 0.358 mmol) (Instrument: Mettler Toledo Minigram SFC; Column: Phenomenex Lux Cellulose-2, 10 x 250 mm, 5 µm; Conditions: Isocratic at 40% ACN/EtOH 1:1 with 60% CO2; Flow Rate: 10 mL/min) provided **Compound 199** and **Compound 200.**

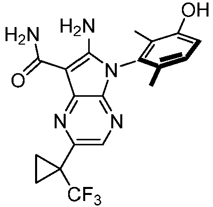

**Compound 199** from chiral SFC separation of **198.** Peak 1 (retention time 3.50 min, 99.99%): *S*-6-amino-5-(3-hydroxy-2,6-dimethyl-phenyl)-2-[1-(trifluoromethyl)cyclopropyl]pyrrolo[2,3-b]pyrazine-7-carboxamide (5 mg) as a white fluffy solid. 1H NMR (400 MHz, DMSO-*d6*) δ 9.61 (s, 1H), 7.88 (s, 1H), 7.48 (br s, 2H), 7.36 (br s, 1H), 7.27 (br s, 1H), 7.08 (d, J = 8.3 Hz, 1H), 6.94 (d, J = 8.3 Hz, 1H), 1.76 (s, 3H), 1.68 (s, 3H), 1.43 - 1.33 (m, 4H). MS: [M+1]: 406.2.

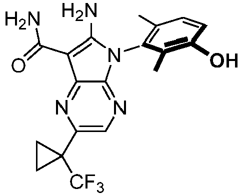

**Compound 200** from chiral SFC separation of **198.** Peak 2 ((retention time 3.81 min, 99.95%): *R-*6-amino-5-(3-hydroxy-2,6-dimethyl-phenyl)-2-[1-(trifluoromethyl)cyclopropyl]pyrrolo[2,3-b]pyrazine-7-carboxamide (5 mg) as a white fluffy solid. ¹H NMR (400 MHz, DMSO-*d6*) δ 9.61 (s, 1H), 7.87 (s, 1H), 7.48 (br s, 2H), 7.36 (br s, 1H), 7.27 (br s, 1H), 7.08 (d, J = 8.3 Hz, 1H), 6.94 (d, J = 8.3 Hz, 1H), 1.76 (s, 3H), 1.69 (s, 3H), 1.43 - 1.35 (m, 4H). MS: [M+1]: 406.2.

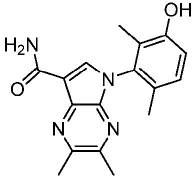

### Compound 209 (5-(3-hydroxy-2,6-dimethyl-phenyl)-2,3-dimethyl-pyrrolo[2,3-b]pyrazine-7-carboxamide)

Step 1. To a solution of 6-amino-5-(3-methoxy-2,6-dimethyl-phenyl)-2,3-dimethyl-pyrrolo[2,3-b]pyrazine-7-carbonitrile (298 mg, 0.927 mmol) in THF (7 mL) was added tert-butyl nitrite (550 µL, 4.63 mmol). After stirring 30 min, the mixture was refluxed for 3.5 h then cooled down to rt and concentrated to dryness and purified by silica gel chromatography eluting with a gradient of 0 to 100% EtOAc in hexanes. The combined pure fractions were concentrated and dried in vacuo to provide 5-(3-methoxy-2,6-dimethyl-phenyl)-2,3-dimethyl-pyrrolo[2,3-b]pyrazine-7-carbonitrile (150 mg, 53% yield) as a light yellow solid.

Step 2. For nitrile hydrolysis using sulfuric acid, the same procedure used for **Compound 164** was done on the appropriate intermediate (150 mg, 0.490 mmol) to provide 5-(3-methoxy-2,6-dimethyl-phenyl)-2,3-dimethyl-pyrrolo[2,3-b]pyrazine-7-carboxamide (144 mg, 91% yield) was obtained as an off-white solid.

Step 3. For OMe deprotection using BBr₃, the same procedure used for **Compound 35** provided a residue which was purified by preparative HPLC to provide 5-(3-hydroxy-2,6-dimethylphenyl)-2,3-dimethyl-pyrrolo[2,3-b]pyrazine-7-carboxamide (76 mg, 55% yield) as an off-white fluffy solid. ¹H NMR (400 MHz, DMSO-d6) δ 9.65 (br s, 1H), 8.13 (s, 1H), 7.86 (br s, 1H), 7.60 (br s, 1H), 7.05 (d, J = 8.2 Hz, 1H), 6.92 (d, J = 8.3 Hz, 1H), 2.64 (s, 3H), 1.75 (s, 3H), 1.64 (s, 3H). 1 Me singlet likely buried under dmso peak. MS: [M+1]: 311.1.

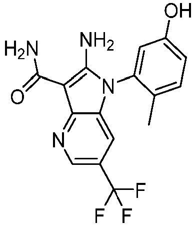

### Compound 212 (2-amino-1-(5-hydroxy-2-methyl-phenyl)-6-(trifluoromethyl)pyrrolo[3,2-b]pyridine-3-carboxamide)

Step 1. To a suspension of NaH (108 mg, 2.83 mmol, 60% dispersion in mineral oil) in DME (3 mL) was added dropwise 3-bromo-2-chloro-5-(trifluoromethyl)pyridine (300 mg, 1.15 mmol) in DME (3 mL). After the addition, the mixture was stirred for 25 min then propanedinitrile (188 mg, 2.85 mmol) was added. The resulting mixture was refluxed for 18 h, cooled to rt and concentrated under vacuum. The residue was purified by preparative HPLC to provide 2-[3-bromo-5-(trifluoromethyl)-2-pyridyl]propanedinitrile (100 mg, 30% yield).

Step 2. To a solution of 2-[3-bromo-5-(trifluoromethyl)-2-pyridyl]propanedinitrile (50 mg, 172 µmol) in DMF (2 mL) were added Pd₂dba₃ (16 mg, 17 µmol), 5-(methoxymethoxy)-2-methylaniline (33.3 mg, 199 µmol), Cs₂CO₃ (84 mg, 259 µmol) and Xantphos (10.0 mg, 17.3 µmol). The mixture was degassed in vacuo and back filled with nitrogen (3 times). The mixture was stirred at 130 °C for 8 h, cooled to rt, diluted with water and extracted with EtOAc (3x). The combined organic extracts were washed with brine, dried over Na₂SO₄, filtered and concentrated in vacuo. The residue was purified by silica gel chromatography eluting with a gradient of 5 to 100% EtOAc in hexanes to provide 2-amino-1-[5-(methoxymethoxy)-2-methyl-phenyl]-6-(trifluoromethyl)pyrrolo[3,2-b]pyridine-3-carbonitrile (30 mg, 46% yield).

Step 3. 2-amino-1-[5-(methoxymethoxy)-2-methyl-phenyl]-6-(trifluoromethyl)pyrrolo[3,2-b]pyridine-3-carbonitrile (30 mg, 135 µmol) was stirred in H₂SO₄ (1 mL). After 90 min, the reaction mixture was poured into crushed ice, placed in an ice bath and neutralized with 1:1 NH₄OH/H₂O. The precipitate was filtered, washed with water and air-dried overnight. Purification by preparative HPLC provided 2-amino-1-(5-hydroxy-2-methyl-phenyl)-6-(trifluoromethyl)pyrrolo[3,2-b]pyridine-3-carboxamide (3.2 mg, 11% yield) as an orange solid. ¹H NMR (400 MHz, DMSO-*d6*) δ 9.76 (s, 1H), 8.45 (s, 1H), 7.77 (s, 1H), 7.34 (s, 2H), 7.31 = 7.13 (m, 2H), 6.98 (s, 1H), 6.91 (d, J = 8.5 Hz, 1H), 6.71 (s, 1H), 1.78 (s, 3H). MS: [M+1]: 351.3.

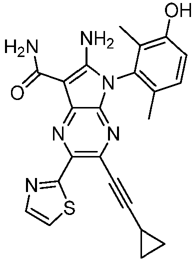

### Compound 214 (6-amino-3-(2-cyclopropylethynyl)-5-(3-hydroxy-2,6-dimethyl-phenyl)-2-thiazol-2-yl-pyrrolo[2,3-b]pyrazine-7-carboxamide)

Step 1. A solution of 6-amino-2-benzyloxy-3-bromo-5-(3-methoxy-2,6-dimethylphenyl)pyrrolo[2,3-b]pyrazine-7-carbonitrile (400 mg, 836 µmol, described in the preparation of **compound 31)** in conc. H₂SO₄ (2 mL) and DCM (2 mL) was stirred 10 min. NaOH 0.4 M was added then water and the resulting precipitate was recovered by filtration. The crude product was purified by silica gel chromatography using a gradient of 0 to 20% MeOH in DCM to afford 6-amino-3-bromo-2-hydroxy-5-(3-methoxy-2,6-dimethyl-phenyl)pyrrolo[2,3-b]pyrazine-7-carboxamide (300 mg, 88% yield).

Step 2. To a solution of 6-amino-3-bromo-2-hydroxy-5-(3-methoxy-2,6-dimethylphenyl)pyrrolo[2,3-b]pyrazine-7-carboxamide (300 mg, 739 µmol) and Cs₂CO₃ (440 mg, 1.35 mmol) in DMF (3 mL) was added 1,1,1-trifluoro-N-phenyl-N-(trifluoromethylsulfonyl)methanesulfonamide (288 mg, 807 µmol). The mixture was stirred for 1 h, diluted with water, extracted with EtOAc twice. The combined organic extracts were washed with brine, dried over Na₂SO₄, filtered and concentrated. The residue was purified by silica gel chromatography using a gradient of 0 to 100% EtOAc in hexanes to afford 6-amino-3-bromo-7-carbamoyl-5-(3-methoxy-2,6-dimethylphenyl)-5H-pyrrolo[2,3-b]pyrazin-2-yl trifluoromethanesulfonate (460 mg, 43% yield).

Step 3. A microwave vial charged with [6-amino-3-bromo-7-carbamoyl-5-(3-methoxy-2,6-dimethyl-phenyl)pyrrolo[2,3-b]pyrazin-2-yl] trifluoromethanesulfonate (35 mg, 65 µmol), ethynylcyclopropane (5.0 mg, 75 µmol), Cul (1.3 mg, 7 µmol) and PdCl₂(PPh₃)₂ (5.0 mg, 7 µmol) in DMF (1 mL) was flushed with nitrogen, then Et₃N (520 µmol, 73 uL) was added and the mixture was stirred for 1 h. The mixture was filtered and purified by preparative HPLC to provide [6-amino-7-carbamoyl-3-(2-cyclopropylethynyl)-5-(3-methoxy-2,6-dimethyl-phenyl)pyrrolo[2,3-b]pyrazin-2-yl] trifluoromethanesulfonate (10 mg, 29% yield).

Step 4. A mixture of tributyl(thiazol-2-yl)stannane (38.5 µmol, 12.1 uL), [6-amino-7-carbamoyl-3-(2-cyclopropylethynyl)-5-(3-methoxy-2,6-dimethyl-phenyl)pyrrolo[2,3-b]pyrazin-2-yl] trifluoromethanesulfonate (10.0 mg, 19.1 µmol). Cul (0.5 mg, 2.5 µmol), LiCl (1.7 mg, 40 µmol). PdCl₂(dppf).CH₂Cl₂ (1.5 mg, 2 µmol) in DMF (3 mL) was degassed in vacuo, then back filled with nitrogen. The reaction mixture was stirred at 120°C for 3 h. Water was added, and the resulting precipitate was recovered by filtration to afford crude 6-amino-3-(2-cyclopropylethynyl)-5-(3-methoxy-2,6-dimethyl-phenyl)-2-thiazol-2-yl-pyrrolo[2,3-b]pyrazine-7-carboxamide (5 mg, 57% yield).

Step 5. For OMe deprotection using BBr₃, the same procedure used for **Compound 35** provided a residue which was purified by preparative HPLC to provide 6-amino-3-(2-cyclopropylethynyl)-5-(3-hydroxy-2,6-dimethyl-phenyl)-2-thiazol-2-yl-pyrrolo[2,3-b]pyrazine-7-carboxamide (1.1 mg, 23% yield). ¹H NMR (400 MHz, Methanol-d4) δ 9.16 (d, J = 4.2 Hz, 1H), 8.47 (d, J = 4.2 Hz, 2H), 7.89 (d, J = 1.1 Hz, 1H), 7.15 (d, J = 8.3 Hz, 1H), 6.98 (d, J = 8.3 Hz, 1H), 2.55 - 2.47 (m, 1H), 1.88 (d, J = 21.7 Hz, 6H), 1.37 = 1.31 (m, 2H), 1.11 - 1.05 (m, 2H). MS:
[M+1]: 445.3.

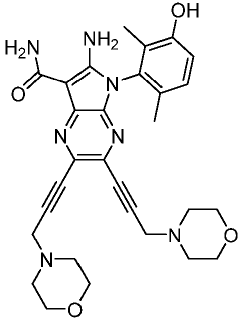

### Compound 215 (2-amino-1-(3-hydroxy-2,6-dimethyl-phenyl)-5-(trifluoromethyl)pyrrolo[2,3-b]pyridine-3-carboxamide)

Step 1. A microwave vial charged with 4-prop-2-ynylmorpholine (26 mg, 204 umol), copper (I) iodide (3.5 mg, 19 umol), and triethylamine (1.49 mmol, 207 uL) was flushed with N2, then 6-amino-3-bromo-7-carbamoyl-5-(3-methoxy-2,6-dimethylphenyl)-5H-pyrrolo[2,3-b]pyrazin-2-yl trifluoromethanesulfonate (100 mg, 186 umol) in DMF (1 mL) was added followed by PdCl₂(PPh₃)₂ (14 mg, 19 umol). The vial was capped heated to 120°C. After 1h, concentrated under vacuum then adsorbed on silica (4g) using THF. Purified by silica gel chromatography eluting with a gradient of 0 to 100% EtOAc in hexanes, then 0 to 20% MeOH in EtOAc to provide 6-amino-5-(3-methoxy-2,6-dimethyl-phenyl)-2,3-bis(3-morpholinoprop-1-ynyl)pyrrolo[2,3-b]pyrazine-7-carboxamide (16mg, 15% yield).

Step 2. For OMe deprotection using BBr₃, the same procedure used for **Compound 35** provided a residue which was purified by preparative HPLC to provide 2-amino-1-(3-hydroxy-2,6-dimethyl-phenyl)-5-(trifluoromethyl)pyrrolo[2,3-b]pyridine-3-carboxamide (7 mg, 45% yield). ¹H NMR (400 MHz, DMSO-d6) δ 9.63 (s, 1H), 7.70 (s, 2H), 7.35 - 7.13 (m, 2H), 7.05 (d, *J* = 8.3 Hz, 1H), 6.92 (d, *J* = 8.3 Hz, 1H), 3.62 - 3.51 (m, 11H), 3.49 (s, 2H), 2.52 (q, *J* = 4.3, 3.9 Hz, 4H), 1.74 (s, 3H), 1.66 (s, 3H). MS: [M+1]: 544.5.

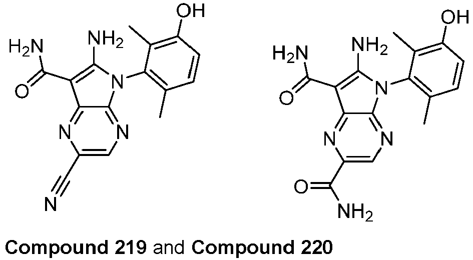

Step 1. A mixture of **Intermediate D** (150 mg, 327 µmol), Zn(CN)₂ (38.3 mg, 327 µmol) and Zn powder (4 mg, 65 µmol) in NMP (3 mL) was degassed. Pd(PPh₃)₄ (38 mg, 33 µmol) was added, and the mixture was stirred at 120°C for 24 h, cooled to rt, quenched with saturated aqueous NH₄Cl, and extracted with EtOAc (3x). The combined extracts were washed with brine, dried over Na₂SO₄, and concentrated. The residue was purified by preparative HPLC to provide 6-amino-2-cyano-5-(3-methoxy-2,6-dimethyl-phenyl)pyrrolo[2,3-b]pyrazine-7-carboxamide (50 mg, 46% yield).

Step 2. For OMe deprotection using BBr₃, the same procedure used for **Compound 35** provided a residue which was purified by preparative HPLC to provide 6-amino-2-cyano-5-(3-hydroxy-2,6-dimethyl-phenyl)pyrrolo[2,3-b]pyrazine-7-carboxamide **compound 219** (2.6 mg, 9% yield) ¹H NMR (400 MHz, DMSO-d6) δ 9.64 (s, 1H), 8.28 (s, 1H), 7.88 (s, 2H), 7.38 (s, 1H), 7.05 (d, J = 8.4 Hz, 2H), 6.92 (d, J = 8.3 Hz, 1H), 1.73 (s, 3H), 1.65 (s, 3H), MS: [M+1]: 324.2; and 6-amino-5-(3-hydroxy-2,6-dimethyl-phenyl)pyrrolo[2,3-b]pyrazine-2,7-dicarboxamide **compound 220** (10 mg, 33% yield). ¹H NMR (400 MHz, DMSO-d6) δ 9.57 (s, 1H), 8.67 (s, 1H), 8.35 (s, 1H), 7.64 (s, 3H), 7.41 (s, 1H), 7.13 (s, 1H), 7.11 - 6.97 (m, 1H), 6.91 (d, J = 8.3 Hz, 1H), 1.73 (s, 3H), 1.65 (s, 3H). MS: [M+1]: 341.4.

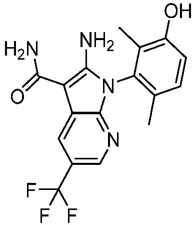

### Compound 221 (2-amino-1-(3-hydroxy-2,6-dimethyl-phenyl)-5-(trifluoromethyl)pyrrolo[2,3-b]pyridine-3-carboxamide)

Step 1. To the solution of 3-methoxy-2,6-dimethyl-aniline (2.02 g, 13.4 mmol) in toluene (15 mL) were added potassium tert-butoxide (1.64 g, 14.6 mmol), 3-bromo-2-chloro-5-(trifluoromethyl)pyridine (3.16 g, 12.1 mmol). Pd₂dba₃ (582 mg, 635 µmol) and Xantphos (723 mg, 1.26 mmol). The mixture was degassed in vacuo and back filled with nitrogen. The resulting mixture was stirred at 120°C for 2 h. The reaction mixture was cooled to rt, diluted with EtOAc, washed with water, brine, dried over Na₂SO₄, filtered and concentrated to dryness. The residue was purified by flash silica gel chromatography eluting with a gradient of 0 to 70% EtOAc in hexanes to provide 3-bromo-N-(3-methoxy-2,6-dimethyl-phenyl)-5-(trifluoromethyl)pyridin-2-amine (1.55 g, 34% yield).

Step 2. To a solution of propanedinitrile (556 mg, 8.41 mmol) in DME (20 mL) was added NaH (361 mg, 8.34 mmol, 60% dispersion in mineral oil). The mixture was stirred for 5 min then 3-bromo-N-(3-methoxy-2,6-dimethyl-phenyl)-5-(trifluoromethyl)pyridin-2-amine (1.55 g, 4.13 mmol) and Pd(PPh₃)₄ (231 mg, 200 µmol) were added. The resulting mixture was stirred at 120 °C for 17 h in a pressure vial. DME was removed under reduced pressure, then the mixture was diluted with EtOAc, washed with water, brine, dried over Na₂SO₄, filtered and concentrated to dryness. The residue was purified by flash chromatography eluting with a gradient of 0 to 100% EtOAc in hexanes to provide 2-amino-1-(3-methoxy-2,6-dimethyl-phenyl)-5-(trifluoromethyl)pyrrolo[2,3-b]pyridine-3-carbonitrile (44 mg, 3% yield).

Step 3. For nitrile hydrolysis using sulfuric acid, the same procedure used for **Compound 164** was done on the appropriate intermediate (44 mg, 0.121 mmol) to provide 2-amino-1-(3-methoxy-2,6-dimethyl-phenyl)-5-(trifluoromethyl)pyrrolo[2,3-b]pyridine-3-carboxamide (40 mg, 87% yield).

Step 4. For OMe deprotection using BBr₃, the same procedure used for **Compound 35** provided a residue which was purified by preparative HPLC to provide 2-amino-1-(3-hydroxy-2,6-dimethyl-phenyl)-5-(trifluoromethyl)pyrrolo[2,3-b]pyridine-3-carboxamide (25 mg, 59% yield). ¹H NMR (400 MHz, DMSO-d6) δ 9.66 (s, 1H), 8.47 (dt, J = 2.0, 1.0 Hz, 1H), 7.77 (s, 1H), 7.31 (s, 2H), 7.23 (s, 1H), 7.10 (dt, J = 8.3, 0.8 Hz, 1H), 7.00 - 6.80 (m, 2H), 1.82 - 1.57 (m, 6H). MS: [M+1]: 365.3.

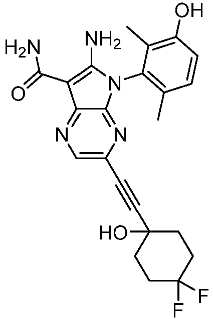

### Compound 242 (6-amino-3-[2-(4,4-difluoro-1-hydroxy-cyclohexyl)ethynyl]-5-(3-hydroxy-2,6-dimethyl-phenyl)pyrrolo[2,3-b]pyrazine-7-carboxamide)

To a microwave vial charged with **Intermediate H** (20.0 mg, 53.2 µmol), Cul (1.0 mg, 5.3 µmol), 1-ethynyl-4,4-difluoro-cyclohexanol (43 mg, 266 µmol) in DMF (1 mL) was added PdCl₂(PPh₃)₂ (4 mg, 6 µmol) and Et₃N (425 µmol, 60 uL). The vial was capped and heated to 80 °C for 3 h. After cooling to rt, the mixture was filtered and purified by preparative HPLC to provide 6-amino-3-[2-(4,4-difluoro-1-hydroxy-cyclohexyl)ethynyl]-5-(3-hydroxy-2,6-dimethylphenyl)pyrrolo[2,3-b]pyrazine-7-carboxamide (5.1 mg, 21% yield). ¹H NMR (400 MHz, DMSO-d6) δ 9.63 (s, 1H), 8.21 (s, 1H), 7.57 (d, J = 10.6 Hz, 2H), 7.28 (s, 2H), 7.05 (d, J = 8.3 Hz, 1H), 6.92 (d, J = 8.3 Hz, 1H), 5.77 (s, 1H), 2.11 - 1.74 (m, 8H), 1.73 (s, 3H), 1.65 (s, 3H). MS: [M+1]: 456.4.

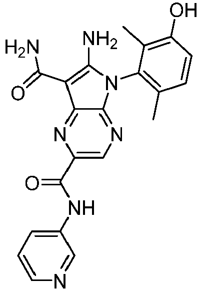

### Compound 243 (6-amino-5-(3-hydroxy-2,6-dimethyl-phenyl)-N2-(3-pyridyl)pyrrolo[2,3-b]pyrazine-2,7-dicarboxamide)

Step 1. A mixture of **Intermediate D** (1.00 g, 2.18 mmol), PdCl₂(PPh₃)₂ (319 mg, 435 □mol) in a mixture of DMF (5 mL), MeOH (5 mL) and Et₃N (1.90 mL, 13.6 mmol) was heated at 70°C under an atmosphere of carbon monoxide (balloon) for 18 h. The apparatus was previously flushed with carbon monoxide once. The volatiles were evaporated in vacuo and the residue was purified by preparative HPLC to provide methyl 6-amino-7-carbamoyl-5-(3-methoxy-2,6-dimethylphenyl)pyrrolo[2,3-b]pyrazine-2-carboxylate (689 mg, 86% yield).

Step 2. To methyl 6-amino-7-carbamoyl-5-(3-methoxy-2,6-dimethyl-phenyl)pyrrolo[2,3-b]pyrazine-2-carboxylate (689 mg, 1.87 mmol) in THF (5 mL) was added NaOH (1 M, 5.60 mL) and the mixture was stirred for 1.5 h. The pH was acidified using conc. HCl, DMSO was added, volatiles were removed under reduced pressure and the residue was purified by preparative HPLC to provide 6-amino-7-carbamoyl-5-(3-methoxy-2,6-dimethyl-phenyl)pyrrolo[2,3-b]pyrazine-2-carboxylic acid (366 mg, 55% yield) .

Step 3. To pyridin-3-amine (7.95 mg, 84.4 µmol), 6-amino-7-carbamoyl-5-(3-methoxy-2,6-dimethyl-phenyl)pyrrolo[2,3-b]pyrazine-2-carboxylic acid (25 mg, 70 µmol) and HATU (29 mg, 77 µmol) in DCM (5 mL) was added DIPEA (211 µmol, 37 µL). The reaction mixture was stirred for 18 h. Water was added to the mixture, the organic phase was separated, dried over Na₂SO₄, filtered and concentrated in vacuo to afford crude 6-amino-5-(3-methoxy-2,6-dimethyl-phenyl)-N2-(3-pyridyl)pyrrolo[2,3-b]pyrazine-2,7-dicarboxamide (21 mg, 34% yield, 49% purity).

Step 4. For OMe deprotection using BBr₃, the same procedure used for **Compound 35** provided a residue which was purified by preparative HPLC to provide 6-amino-5-(3-hydroxy-2,6-dimethyl-phenyl)-N2-(3-pyridyl)pyrrolo[2,3-b]pyrazine-2,7-dicarboxamide (3.5 mg, 12% yield). ¹H NMR (400 MHz, DMSO-*d6*) δ 10.82 (s, 1H), 9.61 (s, 1H), 8.92 (d, J = 2.5 Hz, 1H), 8.49 (s, 1H), 8.32 (dd, J = 4.8, 1.6 Hz, 1H), 8.20 - 8.10 (m, 1H), 7.75 (d, J = 9.0 Hz, 3H), 7.40 (dd, J = 8.3, 4.7 Hz, 1H), 7.30 (s, 1H), 7.07 (d, J = 8.3 Hz, 1H), 6.93 (d, J = 8.3 Hz, 1H), 1.75 (s, 3H), 1.67 (s, 3H). MS: [M+1]: 418.3.

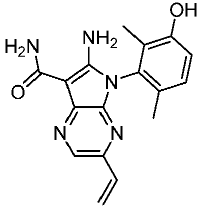

### Compound 257 (6-amino-5-(3-hydroxy-2,6-dimethyl-phenyl)-3-vinyl-pyrrolo[2,3-b]pyrazine-7-carboxamide)

Step 1. A mixture of tributyl(vinyl)stannane (37.1 mg, 117 µmol), **Intermediate H** (40.0 mg, 106 µmol), Cul (2.56 mg, 13.4 µmol), LiCl (9.30 mg, 220 µmol), PdCl₂(dppf).CH₂Cl₂ (8.1 mg, 10 µmol) in DMF (1 mL) was degassed in vacuo, then back filled with nitrogen. The final mixture was stirred at 130 °C for 3 h, cooled to rt, filtered and purified preparative HPLC to provide 6-amino-5-(3-hydroxy-2,6-dimethyl-phenyl)-3-vinyl-pyrrolo[2,3-b]pyrazine-7-carboxamide (1.4 mg, 4% yield). ¹H NMR (400 MHz, DMSO-*d6*) δ 9.59 (s, 1H), 8.29 (s, 1H), 8.19 (s, 1H), 7.38 (d, J = 24.4 Hz, 2H), 7.20 (s, 1H), 7.05 (d, J = 8.3 Hz, 1H), 6.91 (d, J = 8.3 Hz, 1H), 6.69 (dd, J = 17.3, 10.8 Hz, 1H), 5.80 (dd, J = 17.3, 1.8 Hz, 1H), 5.15 (dd, J = 10.7, 1.8 Hz, 1H), 1.75 (s, 3H), 1.67 (s, 3H). MS: [M+1]: 324.3.

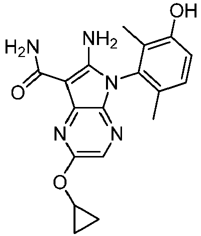

### Compound 260 (6-amino-2-(cyclopropoxy)-5-(3-hydroxy-2,6-dimethyl-phenyl)pyrrolo[2,3-b]pyrazine-7-carboxamide)

Step 1. A solution of cyclopropanol (12 mg, 202 µmol), **Intermediate D** (31 mg, 67 µmol) and Cs₂CO₃ (66 mg, 202 µmol) in NMP (1 mL) was stirred at 140 °C for 16 h. The mixture was cooled to rt, filtered and purified by preparative HPLC to provide 6-amino-2-(cyclopropoxy)-5-(3-methoxy-2,6-dimethyl-phenyl)pyrrolo[2,3-b]pyrazine-7-carboxamide (3 mg, 12% yield).

Step 2. For OMe deprotection using BBr₃, the same procedure used for **Compound 35** provided a residue which was purified by preparative HPLC to provide 6-amino-2-(cyclopropoxy)-5-(3-hydroxy-2,6-dimethyl-phenyl)pyrrolo[2,3-b]pyrazine-7-carboxamide (0.52 mg, 18% yield). ¹H NMR (400 MHz, DMSO-*d6*) δ 9.57 (s, 1H), 8.45 (s, 1H), 7.35 (s, 1H), 7.19 (d, J = 19.0 Hz, 3H), 7.02 (d, J = 8.3 Hz, 1H), 6.88 (d, J = 8.3 Hz, 1H), 4.25 - 4.14 (m, 1H), 1.73 (s, 3H), 1.65 (s, 3H), 0.73 (t, J = 5.0 Hz, 2H), 0.68 (s, 2H). MS: [M+1]: 354.4.

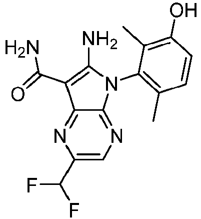

### Compound 263 (6-amino-2-(difluoromethyl)-5-(3-hydroxy-2,6-dimethyl-phenyl)pyrrolo[2,3-b]pyrazine-7-carboxamide)

Step 1. A microwave flask was charged with PdCl₂(PPh₃)₂ (79.6 mg, 109 µmol), **Intermediate D** (1.00 g, 2.18 mmol) and sodium formate (222 mg, 3.27 mmol). The flask was flushed with carbon monoxide. DMF (5 mL) was added and a slow stream of carbon monoxide was passed into the suspension. The mixture was vigorously stirred at 100 °C for 2 h under an atmosphere of carbon monoxide. The resulting mixture was cooled to rt, filtered and the supernatant was purified by preparative HPLC to provide a crude mixture of 6-amino-2-formyl-5-(3-methoxy-2,6-dimethyl-phenyl)pyrrolo[2,3-b]pyrazine-7-carboxamide (556 mg, 75% yield).

Step 2. To a solution of 6-amino-2-formyl-5-(3-methoxy-2,6-dimethyl-phenyl)pyrrolo[2,3-b]pyrazine-7-carboxamide (180 mg, 530 µmol) in DCM (2 mL) at 0 °C was added Deoxo-Fluor^{®} solution(50% in THF, 1.46 M, 2.00 mL) dropwise. The mixture was warmed to rt. After 2 h, extra Deoxo-Fluor^{®} solution (50% in THF, 1.17 g, 2.65 mmol) was added. After 1h more Deoxo-Fluor^{®} solution (50% in THF, 2.35 g, 5.30 mmol) was added and the final mixture was stirred for 4h then diluted with DCM and saturated aqueous Na₂CO₃ was added. The biphasic mixture was stirred for 1h. The organic layer was separated, dried over Na₂SO₄, filtered and concentrated. The residue was purified by preparative HPLC to provide 6-amino-2-(difluoromethyl)-5-(3-methoxy-2,6-dimethyl-phenyl)pyrrolo[2,3-b]pyrazine-7-carboxamide (10 mg, 5% yield).

Step 3. For OMe deprotection using BBr₃, the same procedure used for **Compound 35** provided a residue which was purified by preparative HPLC to provide 6-amino-2-(difluoromethyl)-5-(3-hydroxy-2,6-dimethyl-phenyl)pyrrolo[2,3-b]pyrazine-7-carboxamide (3.0 mg, 31% yield). ¹H NMR (400 MHz, DMSO-d6) δ 9.66 (s, 1H), 8.29 (s, 1H), 8.00 (s, 1H), 7.65 (s, 2H), 7.30 (d, *J* = 34.1 Hz, 2H), 7.11 - 6.98 (m, 2H), 6.99 - 6.69 (m, 1H), 1.73 (s, 3H), 1.65 (s, 3H). MS: [M+1]: 348.2.

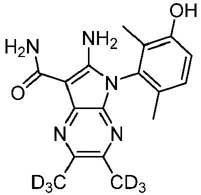

### Compound 266 (6-amino-5-(3-hydroxy-2,6-dimethyl-phenyl)-2,3-bis(trideuteriomethyl)pyrrolo[2,3-b]pyrazine-7-carboxamide)

Step 1. To a suspension of magnesium turnings (380 mg, 15.7 mmol) in Et₂O (20 mL) was added iodine (33 mg, 130 µmol). The mixture was stirred for 10 min and then CD₃I (975 µL, 15.7 mmol) was added. The mixture was stirred 18 h under nitrogen atmosphere to generate an off-white suspension. ZnCl₂ (0.5 M in THF, 1.4 mL) was added dropwise and the mixture was stirred for 20 min. **Intermediate D** (1.2 g, 2.61 mmol) and Pd(PPh₃)₄ (300 mg, 259 µmol) were added. The mixture was stirred at 70 °C for 72 h under nitrogen atmosphere. The reaction was quenched with aqueous HCl 1M, diluted with water, and extracted with EtOAc twice. The combined organic extracts were washed with water, brine, dried over Na₂SO₄, filtered and concentrated to dryness. The residue was purified by silica gel chromatography eluting with a gradient of 0 to 80% EtOAc in hexanes to provide 6-amino-5-(3-methoxy-2,6-dimethyl-phenyl)-2-(trideuteriomethyl)pyrrolo[2,3-b]pyrazine-7-carboxamide (400 mg, 46% yield).

Step 2. To a solution of 6-amino-5-(3-methoxy-2,6-dimethyl-phenyl)-2-(trideuteriomethyl)pyrrolo[2,3-b]pyrazine-7-carboxamide (400 mg, 1.22 mmol) in DMF (2 mL) was added NBS (259 mg, 1.46 mmol). The mixture was stirred for 10 min, diluted with water, stirred for 20 min and filtered. The precipitate was purified by preparative HPLC to provide 6-amino-3-bromo-5-(3-methoxy-2,6-dimethyl-phenyl)-2-(trideuteriomethyl)pyrrolo[2,3-b]pyrazine-7-carboxamide (400 mg, 81% yield).

Step 3. To a suspension of magnesium (177 mg, 7.3 mmol) in ether (10 mL) was added iodine (16 mg, 61 µmol). The mixture was stirred for 10 min and then to it was added CD₃I (455 µL, 7.31 mmol). The mixture was stirred for 18 h under nitrogen atmosphere to yield an off-white suspension. ZnCl₂ (0.5 M in THF, 14.6 mL) was added dropwise. After the addition, the mixture was stirred for 20 min. 6-amino-3-bromo-5-(3-methoxy-2,6-dimethyl-phenyl)-2-(trideuteriomethyl)pyrrolo[2,3-b]pyrazine-7-carboxamide (496 mg, 1.22 mmol), Pd₂dba₃ (111 mg, 122 µmol), and tritert-butylphosphonium tetrafluoroborate (71 mg, 244 µmol) were added and the mixture was stirred at 70 °C for 18 h under nitrogen atmosphere. The reaction was quenched with aqueous HCl 1M, diluted with water and extracted with EtOAc twice. The combined organic extracts were washed with water, brine, dried over Na₂SO₄, filtered and concentrated to dryness. The residue was purified by silica gel chromatography eluting with a gradient of 0 to 60% EtOAc in hexanes to provide 6-amino-5-(3-methoxy-2,6-dimethyl-phenyl)-2,3-bis(trideuteriomethyl)pyrrolo[2,3-b]pyrazine-7-carboxamide (400 mg, 95% yield).

Step 4. For OMe deprotection using BBr₃, the same procedure used for **Compound 35** provided a residue which was purified by preparative HPLC to provide 6-amino-5-(3-hydroxy-2,6-dimethyl-phenyl)-2,3-bis(trideuteriomethyl)pyrrolo[2,3-b]pyrazine-7-carboxamide (65 mg, 17% yield). ¹H NMR (400 MHz, DMSO-d6) δ 9.55 (s, 1H), 7.41 (s, 1H), 7.22 - 6.98 (m, 4H), 6.89 (d, *J* = 8.3 Hz, 1H), 1.74 - 1.69 (m, 3H), 1.63 (s, 3H). MS: [M+1]: 332.2.

### Examples of arylamines preparation

A variety of arylamines were used to prepare compounds of the present invention. Some of these arylamines were commercially available and some were prepared. Table 2 list some examples of such arylamines for which the preparation is described herein.

**Table 2**

| | | | |
|---|---|---|---|
| Arylamine A1 | Arylamine A2 | Arylamine A3 | Arylamine A4 |
| 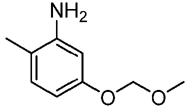 | 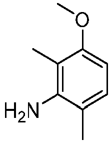 | 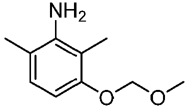 | 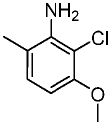 |
| Arylamine A5 | Arylamine A6 | Arylamine A7 | Arylamine A8 |
| 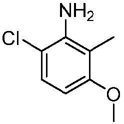 | 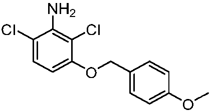 | 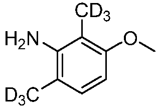 | 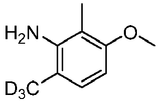 |
| Arylamine A9 | Arylamine A10 | Arylamine A11 | |
| 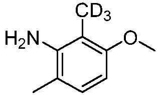 | 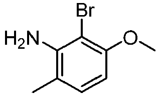 | 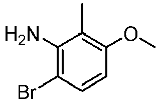 | |

### Preparation of arylamine A1

Compounds of the present invention can be prepared from arylamine A1 which can be prepared as shown in Scheme A1 and described herein. The commercially available 4-methyl-3-nitro-phenol can be O-protected with a suitable protecting group such as O-MOM. The nitro can be reduced to generate arylamine A1.

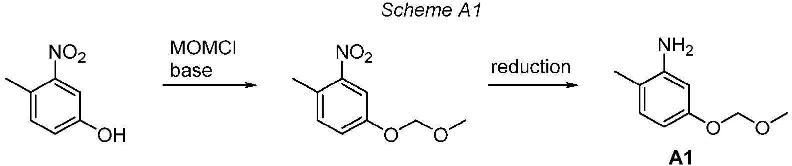

Step 1. To a suspension of 4-methyl-3-nitro-phenol (25 g, 163 mmol) in DCM (250 mL) was added DIPEA (34 mL, 195 mmol) followed by chloro(methoxy)methane (26.0 g, 323 mmol, 24.5 mL) added dropwise. After stirring 18 h, the reaction mixture was washed with water. The layers were separated. The organic layer was washed with 0.2N HCl (2x), brine, dried over MgSO₄, filtered and concentrated, then dried in vacuo affording 4-(methoxymethoxy)-1-methyl-2-nitro-benzene (31.4 g, 98% yield) as a dark red oil.

Step 2. To a suspension of 4-(methoxymethoxy)-1-methyl-2-nitro-benzene (31.4 g, 159 mmol) in EtOH (200 mL) and water (75 mL) was added ammonium chloride (43.3 g, 809 mmol) then iron powder (44.5 g, 796 mmol). The reaction mixture was heated to 80 °C for 3.5 h, then temperature was increased to 90 °C, stirring for 4 days. The reaction mixture was cooled to rt, filtered and rinsed with EtOAc. The filtrate was concentrated and diluted with EtOAc and saturated aqueous NaHCO₃. The layers were separated, and the aqueous layer was back extracted with EtOAc (2x). The combined organic extracts were washed with brine, dried over MgSO₄, filtered and concentrated, affording 26.3 g crude product as a dark brown oil which was purified on a silica gel pad, eluting with 20-30% EtOAc in hexanes. The pure fractions were combined, concentrated, then dried in vacuo, affording 5-(methoxymethoxy)-2-methyl-aniline (25.4 g, 95% yield) as a purple oil. ¹H NMR (400 MHz, Chloroform-d) δ 6.94 (dd, J = 8.0, 1.7 Hz, 1H), 6.45 - 6.30 (m, 2H), 5.12 (s, 2H), 3.47 (s, 3H), 2.10 (s, 3H). MS: [M+1]: 168.3.

### Preparation of arylamine A2

Compounds of the present invention can be prepared from arylamine **A2** which can be prepared as shown in Scheme A2 and described herein (adapted from Can J Chem 2012, 90, 75-84). Commercially available 1,3-dimethyl-2-nitrobenzene can be brominated under suitable bromination conditions. The resulting bromo can be converted to a methoxy upon treatment with sodium methoxide and copper(I) bromide. The nitro can be reduced to generate arylamine **A2.**

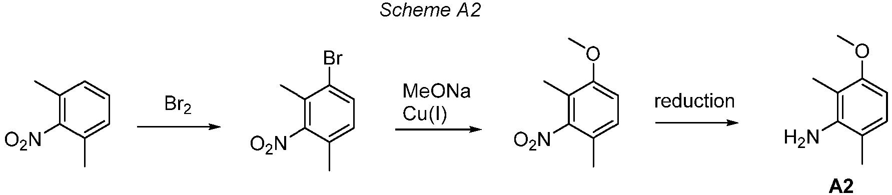

Step 1. A 3 necked 3L round-bottom flask was equipped with a mechanical stirrer, reflux condenser and addition funnel and loaded with 1,3-dimethyl-2-nitro-benzene (300 g, 1.98 mol), DCM (900 mL), iron powder (28.0 g, 501 mmol) and iron(III) bromide (11.9 g, 40.3 mmol). Bromine (112 mL, 2.19 mol) was added dropwise via an addition funnel over 45-60 min. Internal monitoring of the temperature showed an exotherm to 30 °C. 90 min after the addition of bromine was complete, more bromine (5 mL, 97.6 mmol) was added and the reaction mixture was stirred for another 45 min to complete conversion. The reaction mixture was diluted with ice water (1.5 L) and Et₂O (1.5 L). The layers were separated. The aqueous layer was back extracted with Et₂O (0.5 L). The combined organic layers were washed with aqueous 20% Na₂S₂O₃ (1 L), brine (500 mL), dried over Na₂SO₄, filtered over a silica gel pad (300 cc), concentrated then dried in vacuo to provide 1-bromo-2,4-dimethyl-3-nitro-benzene (451.5 g, 99% yield) as an off-white solid.

Step 2. A 5L 4 neck round bottom flask equipped with a mechanical stirrer and a reflux condenser was charged with 1-bromo-2,4-dimethyl-3-nitro-benzene (451.5 g, 1.96 mol) in DMF (1.6 L). CuBr (28.0 g, 195 mmol) was added followed by MeONa (1.31 L, 5.89 mol, 25% in MeOH). The reaction mixture was slowly heated to 95°C, achieving a mild reflux. After 6h, the reaction mixture was left to cool to rt overnight. The reaction mixture was diluted with Et₂O and saturated aqueous NH₄Cl (1.5L each). The layers were separated, and the aqueous layer was back extracted with Et₂O (750 mL). The combined organic extracts were washed with brine (750 mL), dried over Na₂SO₄ and filtered over a silica pad, rinsed with Et₂O and concentrated, dried in vacuo to provide 1-methoxy-2,4-dimethyl-3-nitro-benzene (352 g, 99% yield) as an ochre solid.

Step 3. To a solution of 1-methoxy-2,4-dimethyl-3-nitro-benzene (115 g, 635 mmol) in EtOH (1.5 L) in a 3 neck 3 L flask equipped with a mechanical stirrer was added iron powder (213 g, 3.81 mol), then a solution of ammonium chloride (204 g, 3.81 mol) in water (500 mL) was added portion wise. The mixture was heated to 85 °C for 8h. The mixture was cooled down to rt and filtered on Celite. The volume of the filtrate was reduced (most of the EtOH was evaporated) and the resulting mixture was diluted with Et₂O (800 mL) and water (150 mL). The layers were separated, and the aqueous layer was back extracted with Et₂O (500 mL). The combined organic extracts were washed with brine, dried over Na₂SO₄, filtered, concentrated and dried in vacuo to provide 3-methoxy-2,6-dimethyl-aniline (89.1 g, 93% yield) as a brown oil. ¹H NMR (400 MHz, Chloroform-d) δ 6.88 (dq, J = 8.3, 0.7 Hz, 1H), 6.31 (d, J = 8.2 Hz, 1H), 3.79 (s, 3H), 3.61 (br s, 2H), 2.14 (d, J = 0.7 Hz, 3H), 2.07 (s, 3H). MS: [M+1]: 152.3.

### Preparation of arylamine A3

Compounds of the present invention can be prepared from arylamine **A3** which can be prepared as shown in Scheme A3 and described herein. The methoxy of 1-methoxy-2,4-dimethyl-3-nitro-benzene described in the preparation of **Intermediate A2** can be cleaved using BBr₃ and the resulting phenol can be O-protected with a suitable protecting group such as O-MOM. The nitro can be reduced to generate arylamine **A3.**

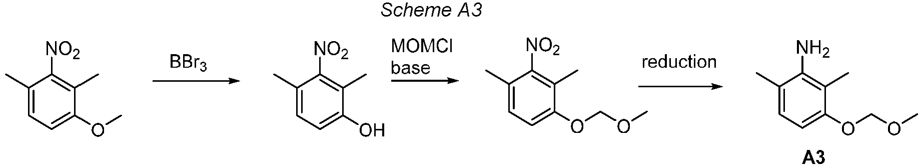

Step 1. To a solution of 1-methoxy-2,4-dimethyl-3-nitro-benzene (20 g, 110 mmol) in DCM (200 mL), cooled in a dry ice/acetonitrile bath, was added BBr₃ solution in DCM (1 M, 168 mL) dropwise via an addition funnel. The mixture was left to slowly warm to rt overnight. The reaction mixture was then poured slowly in a stirred mixture of ice, water (1 L) and KH₂PO₄ (75g). The layers were separated, and the aqueous layer was extracted with DCM (2 x 500 mL). The combined organic extracts were washed with brine (500 mL), dried over MgSO₄, filtered over a silica pad (375g), eluting with DCM, concentrated and dried in vacuo to provide 2,4-dimethyl-3-nitro-phenol (18.0 g, 98% yield) as a yellow solid.

Step 2. To a suspension of 2,4-dimethyl-3-nitro-phenol (18.96 g, 113 mmol) in DCM (200 mL) was added DIPEA (23.7 mL, 136 mmol) then chloro(methoxy)methane (9.5 mL, 125 mmol) dropwise. After stirring for 3.5 h, more chloro(methoxy)methane (2.0 mL, 26 mmol) was added and the reaction mixture was stirred overnight. The reaction mixture was quenched with aqueous saturated NH₄Cl (100 mL) and diluted with water (100 mL). The layers were separated, and the aqueous layer was back extracted with DCM (100 mL). The combined organic extracts were washed with 0.2N HCl (2 x 100 mL), 1M NaOH (100 mL), brine (100 mL), dried over MgSO₄, filtered on silica (ca 100 cc), eluting with DCM, concentrated, and dried in vacuo to provide 1-(methoxymethoxy)-2,4-dimethyl-3-nitro-benzene (22.1 g, 92% yield) as a pale yellow waxy solid.

Step 3. To a flask containing palladium on carbon (5.06 g, 4.76 mmol, 10% w/w) under nitrogen was added MeOH (300 mL) followed by 1-(methoxymethoxy)-2,4-dimethyl-3-nitrobenzene (20.1 g, 95.1 mmol). The flask was flushed with hydrogen and stirred under a hydrogen atmosphere for 2 days. The reaction mixture was flushed with nitrogen for 2 h, and Celite was added. The mixture was filtered on a Celite pad using MeOH and DCM. The filtrate was concentrated and dried in vacuo to provide 3-(methoxymethoxy)-2,6-dimethyl-aniline (17.1 g, 99% yield) as a pale orange turbid oil. ¹H NMR (400 MHz, Chloroform-d) δ 6.86 (d, J = 8.3 Hz, 1H), 6.49 (d, J = 8.3 Hz, 1H), 5.15 (s, 2H), 3.48 (s, 3H), 2.14 (s, 3H), 2.11 (s, 3H). MS: [M+1]: 182.2.

### Preparation of arylamine A4

Compounds of the present invention can be prepared from arylamine **A4** which can be prepared as shown in Scheme A4 and described herein. Commercially available 2-chloro-3-methoxy-benzoic acid can be brominated with a suitable bromination reagent and the carboxylic acid can be converted to a NHBoc under Curtius conditions. The bromo can be converted to a methyl and the NHBoc can be cleaved under acidic conditions to generate arylamine **A4.**

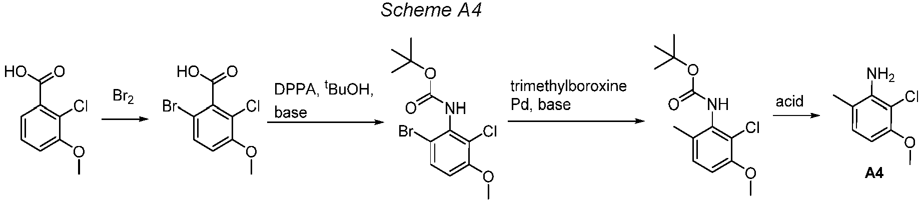

Step 1. To a solution of 2-chloro-3-methoxy-benzoic acid (50 g, 268 mmol) in AcOH (250 mL) and water (250 mL) was added bromine (27.5 mL, 537 mmol) dropwise. The mixture was stirred at 60 °C for 18 h, cooled to rt, brine was added, and the mixture was extracted twice with DCM. The combined organic extracts were dried over Na₂SO₄, filtered and concentrated in vacuo to provide 6-bromo-2-chloro-3-methoxy-benzoic acid (71 g, quantitative yield) as a brown oil which solidified upon standing under vacuum over the weekend.

Step 2. To a solution of 6-bromo-2-chloro-3-methoxy-benzoic acid (23.6 g, 88.9 mmol). Et₃N (38 mL, 271 mmol) and tert-butanol (42.5 mL, 450 mmol) in toluene (500 mL) was added [azido(phenoxy)phosphoryl]oxybenzene (29.5 mL, 136 mmol). The mixture was heated at 100 °C for 16 h, cooled down to rt then the volatiles were removed in vacuo. The residue was diluted with EtOAc (100 mL), and the organic layer was washed with 5% citric acid, water, saturated aqueous NaHCO₃, brine, dried over Na₂SO₄, filtered and concentrated to dryness. The residue was purified by silica gel chromatography eluting with a gradient of 0 to 20% EtOAc in hexanes to provide tert-butyl N-(6-bromo-2-chloro-3-methoxy-phenyl)carbamate (16.2 g, 54% yield) as a yellowish solid.

Step 3. To a solution of tert-butyl N-(6-bromo-2-chloro-3-methoxy-phenyl)carbamate (25 g, 74.3 mmol) in dioxane (500 mL) was added trimethylboroxine (50% w/w in THF, 20.51 g, 81.7 mmol). PdCl₂(dppf).CH₂Cl₂ (5.22 g, 7.43 mmol) and aqueous Na₂CO₃ (2 M, 111 mL, 223 mmol). The mixture was heated at 100 °C for 16 h, cooled down to rt then volatiles were removed in vacuo. EtOAc and water were added. The organic layer was separated and washed with brine, dried over Na₂SO₄, filtered and concentrated to dryness. The residue was purified by silica gel chromatography eluting with a gradient of 0 to 30% EtOAc in heptane to provide tert-butyl N-(2-chloro-3-methoxy-6-methyl-phenyl)carbamate (13.8 g, 68% yield) as a yellowish solid.

Step 4. HCl in dioxane (4 M, 100 mL) was added to a solution of tert-butyl N-(2-chloro-3-methoxy-6-methyl-phenyl)carbamate (13.8 g, 50.8 mmol) in MeOH (100 mL). After 3 h, the volatiles were evaporated to dryness under vacuum to provide a white solid to which was added under vigorous stirring 250 mL of EtOAc and 250 mL of aqueous saturated NaHCO₃. The organic layer was separated. The aqueous layer was back extracted with EtOAc. The combined organic layers were washed with brine, dried over Na₂SO₄, filtered and concentrated in vacuo. The residue was purified by silica gel chromatography eluting with a gradient of 0 to 30% EtOAc in heptane to provide 2-chloro-3-methoxy-6-methyl-aniline (7.9 g, 91% yield) as a clear oil which solidified upon standing. ¹H NMR (400 MHz, Chloroform-d) δ 6.95 - 6.79 (m, 1H), 6.27 (dd, J = 8.3, 1.5 Hz, 1H), 4.06 (br s, 2H), 3.83 (d, J = 1.6 Hz, 3H), 2.12 (d, J = 0.8 Hz, 3H). MS: [M+1]: 172.2.

### Preparation of arylamine A5

Compounds of the present invention can be prepared from arylamine **A5** which can be prepared as shown in Scheme A5 and described herein. Commercially available 3-methoxy-2-methyl-aniline can be chlorinated with a chlorination reagent to generate arylamine **A5.**

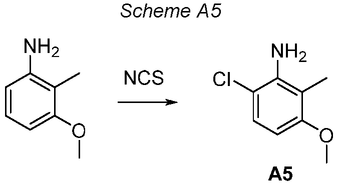

Step 1. NCS (98 g, 734 mmol) was added in 4 portions (15 minutes between each addition) to a solution of 3-methoxy-2-methyl-aniline (100 g, 729 mmol) in DCM (500 mL) at 0 °C. 30 min after the last addition, 100 g of silica gel was added, the mixture was evaporated under vacuum and the black residue was purified by silica gel chromatography (dry load) in eluting with a gradient of 0 to 10% EtOAc in hexanes to provide 6-chloro-3-methoxy-2-methyl-aniline (55.6 g, 44% yield) as an orange solid. ¹H NMR (400 MHz, Chloroform-d) δ 7.08 (d, J = 8.8 Hz, 1H), 6.28 (d, J = 8.8 Hz, 1H), 4.02 (br s, 2H), 3.78 (s, 3H), 2.07 (s, 3H). MS: [M+1]: 172.3.

### Preparation of arylamine A6

Compounds of the present invention can be prepared from arylamine **A6** which can be prepared as shown in Scheme A6 and described herein. Commercially available 3-amino-2,4-dichloro-phenol can be O-protected with a suitable protecting group such as O-PMB to generate arylamine **A6.**

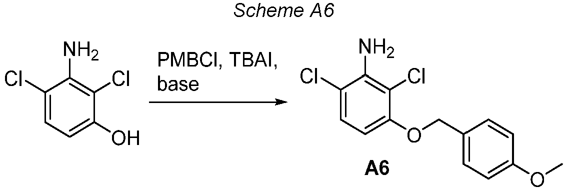

To a suspension of 3-amino-2,4-dichloro-phenol.HCl salt (20 g, 93.3 mmol) in DMF (150 mL) was added 1-(chloromethyl)-4-methoxy-benzene (14.0 mL, 103 mmol), tetrabutylammonium iodide (1 g, 3.00 mmol) and Cs₂CO₃ (64.0 g, 196 mmol). The mixture was stirred at 40 °C overnight, then it was diluted with water, stirred for 20 min, and filtered. The precipitate was washed with water and dried in vacuo. The resulting crude product was purified by silica gel chromatography eluting with a gradient of 0 to 100% DCM in hexanes to provide 2,6-dichloro-3-[(4-methoxyphenyl)methoxy]aniline (20 g, 72% yield) as an off-white solid. ¹H NMR (400 MHz, Chloroform-d) δ 7.38 - 7.30 (m, 2H), 7.05 (d, J = 8.9 Hz, 1H), 6.92 - 6.77 (m, 2H), 6.32 (s, 1H), 5.01 (s, 2H), 4.46 (s, 2H), 3.79 (s, 3H). MS: [M+1]: 298.0.

### Preparation of arylamine A7.

Compounds of the present invention can be prepared from key **Intermediate A7, A8 or A9** which can be prepared as shown in Scheme A7 and described herein (adapted from J. AM. CHEM. SOC. 2004, 126, 1150-1160). The commercially available 3-methoxyaniline can be N-protected with a suitable protecting group such as NH-PIV. A directed ortho metalation approach can be used to introduce a suitable R¹ such as CH₃ or CD₃. The NH-PIV protecting group can be cleaved under acidic conditions and the remaining ortho-to-nitrogen can be brominated with a suitable bromination reagent such as NBS. At this point, the bromine can be substituted by a boronate ester under metal-mediated conditions and further derivatized by a suitable R² such as CH₃ or CD₃ to generate key **Intermediate A7, A8** or **A9.** Alternatively, the bromoaniline can be N-protected with a suitable protecting group such as NH-Boc prior to the bromo substitution. In this case, the NH-Boc can be cleaved under acidic conditions to generate key **Intermediate A7, A8 or A9.**

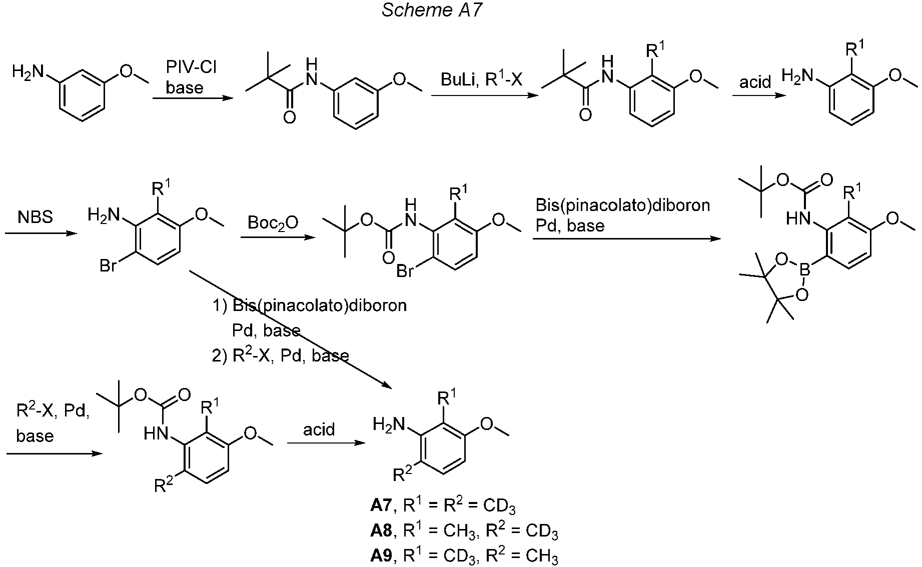

### Arylamine A7

Step 1. 2,2-dimethylpropanoyl chloride (51 mL, 416 mmol) was slowly added to a solution of 3-methoxyaniline (50 g, 406 mmol, 45.5 mL), pyridine (66 mL, 816 mmol) and DMAP (500 mg, 4.1 mmol) in DCM (500 mL). After 1 h, aqueous 1 N HCl was added and the layers were separated. The aqueous layer was back extracted with CH₂Cl₂. The organic layers were combined, washed with aqueous 1 N HCl, brine, then dried over Na₂SO₄, filtered and evaporated to dryness to provide N-(3-methoxyphenyl)-2,2-dimethyl-propanamide (84 g, quantitative yield).

Step 2. To a solution of N-(3-methoxyphenyl)-2,2-dimethyl-propanamide (82 g, 396 mmol) in THF (820 mL) at 0 °C was added nBuLi (2.5 M, 325 mL, 813 mmol) dropwise. After 2 h at 0 °C, the solution was cooled to -78 °C and CD₃I (27 mL, 434 mmol) was added dropwise. The mixture was stirred for 16 h at rt. The mixture was poured into aqueous 1 N HCl and extracted with EtOAc twice. The combined organic layers were dried over Na₂SO₄, filtered and concentrated to dryness to provide N-[3-methoxy-2-(trideuteriomethyl)phenyl]-2,2-dimethylpropanamide (82 g, 92% yield) as a white solid.

Step 3. N-[3-methoxy-2-(trideuteriomethyl)phenyl]-2,2-dimethyl-propanamide (81.5 g, 363 mmol) in dioxane (300 mL) and HCl conc (12 M, 300 mL) was heated to reflux for 24 h. The dark mixture was cooled to 0 °C in an ice bath, neutralized with aqueous 2N NaOH, extracted with EtOAc twice. The combined organic extracts were washed with brine, dried over Na₂SO₄, filtered and concentrated to dryness to provide a dark residue that was purified by silica gel chromatography eluting with a gradient of 0 to 50% EtOAc in heptane to provide 3-methoxy-2-(trideuteriomethyl)aniline (36 g, 71% yield) as a clear oil.

Step 4. To a solution of 3-methoxy-2-(trideuteriomethyl)aniline (35 g, 250 mmol) in DCM (500 mL) at 0°C was added NBS (45 g, 253 mmol). The mixture was stirred at 0 °C for 3 h and concentrated to approximately 75 mL and filtered. The filtrate was evaporated to dryness and the residue was purified by silica gel chromatography eluting with a gradient of 0% to 50% EtOAc in heptane to provide 6-bromo-3-methoxy-2-(trideuteriomethyl)aniline (35 g, 64% yield).

Step 5. tert-butoxycarbonyl tert-butyl carbonate (87.2 g, 400 mmol) was added to a solution of 6-bromo-3-methoxy-2-(trideuteriomethyl)aniline (35 g, 160 mmol), DMAP (3.90 g, 32 mmol) and DIPEA (415 mmol, 72.3 mL) in THF (500 mL). The mixture was heated to reflux for 18 h. The volatiles were removed under vacuum and the residue was filtered trough silica gel eluting with 50% EtOAc in heptane to provide a mixture of tert-butyl N-[6-bromo-3-methoxy-2-(trideuteriomethyl)phenyl]carbamate and tert-butyl N-[6-bromo-3-methoxy-2-(trideuteriomethyl)phenyl]-N-tert-butoxycarbonyl-carbamate (64 g) as a clear oil that was dissolved in methanol (500 mL). K₂CO₃ (110 g, 796 mmol) was added and the mixture was stirred at 60 °C for 48 h. The volatiles were removed under vacuum. EtOAc and water were added to the residue. The organic layer was separated, washed with brine, dried over Na₂SO₄, filtered and concentrated. The residue was purified by silica gel chromatography eluting with a gradient of 0 to 40% EtOAc to provide tert-butyl N-[6-bromo-3-methoxy-2-trideuteriomethyl)phenyl]carbamate (50 g, quantitative yield) as a clear oil.

Step 6. To a solution of tert-butyl N-[6-bromo-3-methoxy-2-(trideuteriomethyl)phenyl]carbamate (33 g, 103 mmol) in dioxane (700 mL) were added Bis(pinacolato)diboron (51 g, 201 mmol), KOAc (35.5 g, 362 mmol) and PdCl₂(dppf).CH₂Cl₂ (7.6 g, 10.4 mmol). The mixture was degassed in vacuo, back filled with nitrogen and stirred at reflux for 18 h. The mixture was cooled to rt and concentrated to a smaller volume. The black residue was diluted with EtOAc and filtered trough a silica gel pad (250 g) eluting with 2L of 50% EtOAc in heptane. The filtrate was evaporated, and the residue was purified by silica gel chromatography eluting with a gradient of 0 to 20% EtOAc in heptane to provide tert-butyl N-[3-methoxy-6-(4,4,5,5-tetramethyl-1,3,2-dioxaborolan-2-yl)-2-(trideuteriomethyl)phenyl]carbamate (22.5 g, 59% yield), which solidified upon standing under vacuum.

Step 7. To a solution of PdCl₂(dppf).CH₂Cl₂ (5.3 g, 7.24 mmol) in DMF (500 mL) was quickly added consecutively CD₃I (60.6 g, 418 mmol, 26 mL), tert-butyl N-[3-methoxy-6-(4,4,5,5-tetramethyl-1,3,2-dioxaborolan-2-yl)-2-(trideuteriomethyl)phenyl]carbamate (52 g, 142 mmol) and aqueous potassium phosphate tribasic (2 M, 350 mL). Nitrogen was bubbled in the solution for 2 min, then the mixture was stirred at 80 °C for 30 min under a nitrogen atmosphere then cooled to rt, and EtOAc was added. The organic layer was washed with water, brine, dried over Na₂SO₄, filtered and concentrated to dryness. The residue was purified by silica gel chromatography eluting with a gradient of 0 to 20% EtOAc in heptane to provide tert-butyl N-[3-methoxy-2,6-bis(trideuteriomethyl)phenyl]carbamate (15 g, 41% yield) as a thick clear oil.

Step 8. HCl in dioxane (4 M, 100 mL) was added to a solution of tert-butyl N-[3-methoxy-2,6-bis(trideuteriomethyl)phenyl]carbamate (22.5 g, 87.4 mmol) in MeOH (100 mL). After 3 h the volatiles were removed under vacuum to provide a white solid. EtOAc and water were added followed by a saturated NaHCO3 aqueous solution until basic pH. The organic layer was washed with brine, dried over Na₂SO₄, filtered and evaporated to dryness. The residue was purified by silica gel chromatography eluting with a gradient of 0 to 40% EtOAc in heptane to provide 3-methoxy-2,6-bis(trideuteriomethyl)aniline (7.5 g, 55% yield) as a clear oil. ¹H NMR (400 MHz, Chloroform-d) δ 6.96 (dd, J = 8.3, 2.6 Hz, 1H), 6.38 (dd, J = 8.3, 2.5 Hz, 1H), 3.86 (d, J = 2.4 Hz, 3H), 3.64 (s, 2H). MS: [M+1]: 158.3.

### Alternative route used for preparation arylamine A8 (without Boc)

Step 1. In a sealed tube, 6-bromo-3-methoxy-2-methylaniline (3.4 g, 15.7 mmol), Bis(pinacolato)diboron (5.58 g, 21.98 mmol) and Cs₂CO₃ (15.4 g, 47.1 mmol) was taken in anhydrous 1,4-dioxane (68 mL) and nitrogen gas was purged into the reaction mixture for 15 min. Then, PdCl₂(dppf) (1.92 g, 2.36 mmol) was added into the reaction mixture and heated at 100 °C for 2 h. After completion, the reaction mixture was quenched with ice water and extracted using EtOAc (3 x 100 mL). The combined organic layer was dried over Na₂SO₄, filtered and concentrated to give the crude product which was purified by silica gel chromatography eluting with a gradient of 10 to 12% EtOAc in hexanes) to provide 3-methoxy-2-methyl-6-(4,4,5,5-tetramethyl-1,3,2-dioxaborolan-2-yl)aniline (2.50 g, 60% yield).

Step 2. In a sealed tube, 3-methoxy-2-methyl-6-(4,4,5,5-tetramethyl-1,3,2-dioxaborolan-2-yl)aniline (2.5 g, 9.39 mmol), iodomethane-*d*₃ (4.08 g, 28.19 mmol) and potassium phosphate tribasic (9.95 g, 46.9 mmol) was taken in anhydrous DMF (50 mL) and nitrogen gas was purged into the reaction mixture for 15 min. Then, PdCl₂(dppf) (0.766 g, 0.939 mmol) was added and the reaction mixture was heated at 80 °C for 2h. After completion, the reaction mixture was quenched using ice water and extracted using EtOAc (3 x 50 mL). The combined organic layer was dried over Na₂SO₄, filtered and concentrated to give the crude product which was purified by silica gel chromatography eluting with a gradient of 6 to 8 % EtOAc in hexanes) to get pure 3-methoxy-2-methyl-6-(methyl-*d*₃) aniline as colorless liquid (0.75 g, 51% yield). ¹H NMR (400 MHz, DMSO-*d*₆) *δ* 6.68 (d, *J* = 7.2 Hz, 1H), 6.31 (d, *J* = 7.4 Hz, 1H), 4.63 (s, 2H), 3.55 (s, 3H), 1.94 (s, 3H). MS: [M+1]: 155.3.

### Preparation of Arylamine A10

Compounds of the present invention can be prepared from key **Intermediate A10** which can be prepared as shown in Scheme A8 and described herein. The commercially available 3-methoxy-2-nitrobenzoic acid can be brominated. The acid can then be esterified, the bromo converted to a methyl and the nitro reduced to the amino. The resulting amino can be converted to a bromo under Sandmeyer conditions and the ester may then be saponified. The resulting acid can then be converted to the NHBoc under Curtius conditions. In this case, the NH-Boc can be cleaved under acidic conditions to generate key **Intermediate A10.**

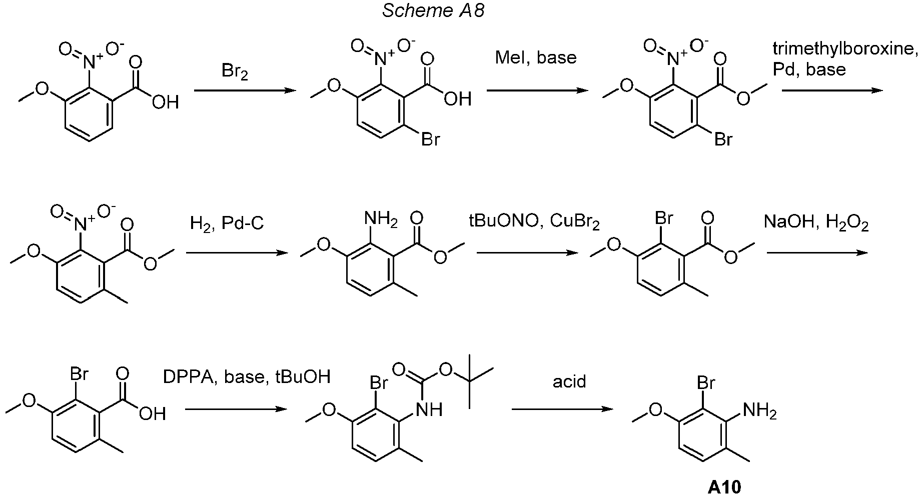

Step 1. To 3-methoxy-2-nitro-benzoic acid (10.04 g, 50.93 mmol) and Ag₂SO₄ (8.10 g, 26.0 mmol) in the dark was added conc. sulfuric acid (200 mL) and molecular bromine (9.4 g, 58.6 mmol, 3.0 mL) dropwise. The mixture was stirred in the dark for 3.5h, then quenched by adding crushed ice, cooled in an ice bath and stirred. The solids were collected by filtration, washed with H₂O and air-dried. The resulting solid was taken in acetone (300 mL), filtered, residue (silver salts) washed with acetone. The filtrate was dried over MgSO₄, filtered and concentrated, affording 6-bromo-3-methoxy-2-nitro-benzoic acid (14.64 g, 100% yield) as a purple solid.

Step 2. To a solution of 6-bromo-3-methoxy-2-nitro-benzoic acid (14.64 g, 53.0 mmol) in DMF (140 mL) was added anhydrous potassium carbonate (14.66 g, 106.1 mmol) followed by methyl iodide (11.4 g, 80.3 mmol, 5.0 mL). After stirring the reaction mixture for 2h, H₂O was added dropwise (420 mL). The solid was collected by filtration and washed with H₂O, air-dried then dried in vacuo, affording methyl 6-bromo-3-methoxy-2-nitro-benzoate (12.89 g, 84% yield) as a light beige solid.

Step 3. In a pressure vessel, a solution of methyl 6-bromo-3-methoxy-2-nitro-benzoate (6.0 g, 20.7 mmol) in dioxane (100 mL) and aqueous Na₂CO₃ solution (2 M, 31 mL, 62.3 mmol) was bubbled through with N₂ then Pd(dppf)Cl₂ (1.64 g, 2.01 mmol) and trimethylboroxine (6.74 g, 26.8 mmol, 7.5 mL) were added. The solution was bubbled through with N₂. The vessel was capped stirred at 100 °C overnight. The reaction mixture was cooled to rt, poured in H₂O and extracted with EtOAc (3x). The combined organic layers were washed with brine, dried over Na₂SO₄, filtered over a silica plug and concentrated in vacuo. The residue was purified by silica gel chromatography eluting with a gradient of EtOAc (0 to 100%) in Hep. Appropriate fractions were combined and concentrated in vacuo to afford methyl 3-methoxy-6-methyl-2-nitro-benzoate (3.06 g, 66% yield) as a light beige waxy solid.

Step 4. To a solution of methyl 3-methoxy-6-methyl-2-nitro-benzoate (3.06 g, 13.6 mmol) in MeOH (225 mL) was added palladium on carbon (10% w/w, 1.42 g, 1.33 mmol) slurried in some of the MeOH. The mixture was flushed with H₂ and stirred under a hydrogen atmosphere for 2h. The suspension was filtered through Celite and the filtrate was concentrated then dried in vacuo, affording methyl 2-amino-3-methoxy-6-methyl-benzoate (2.56 g, 97% yield) as a light amber oil.

Step 5. To a solution of methyl 2-amino-3-methoxy-6-methyl-benzoate (2.13 g, 10.9 mmol) in DMF (12 mL) and MeCN (18 mL) was added tert-butyl nitrite (2.0 mL, 17 mmol) followed by copper (II) bromide (2.86 g, 12.8 mmol). The reaction mixture was stirred at 55 °C for 9 min, then cooled to RT, diluted with H₂O and extracted with EtOAc (3x). The combined organic extracts washed with saturated aqueous NH₄Cl, brine, dried over Na₂SO₄, filtered and concentrated. The residue was purified by silica gel chromatography (dry-load) eluting with a gradient of EtOAc (0 to 70%) in Hep. Appropriate fractions were combined and concentrated in vacuo to afford methyl 2-bromo-3-methoxy-6-methyl-benzoate (1.52 g, 54% yield) as a yellow oil. ¹H NMR (400 MHz, Chloroform-d) δ 7.15 - 7.05 (m, 1H), 6.84 (d, J = 8.4 Hz, 1H), 3.95 (s, 3H), 3.88 (s, 3H), 2.26 (d, J = 0.7 Hz, 3H).

Step 6. To a solution of methyl 2-bromo-3-methoxy-6-methyl-benzoate (1.52 g, 5.87 mmol) in MeOH (15 mL) and THF (15 mL) was added NaOH aqueous (4 M, 15 mL, 60.0 mmol) and hydrogen peroxide solution 30% (1.5 mL). The reaction mixture was stirred at 70 °C overnight, then at 90 °C for 4 days. The reaction mixture was cooled to RT and the volatiles were removed in vacuo. The residue was diluted with 3N HCl (20 mL) and extracted with CHCl₃/iPrOH (4:1, 4x). The combined organic extracts were concentrated then dried in vacuo and the crude product was purified by silica gel chromatography (dry load) eluting with a gradient of MeOH (0 to 10%) in CH₂Cl₂ with 1% AcOH modifier. Appropriate fractions were combined and concentrated in vacuo to afford 2-bromo-3-methoxy-6-methyl-benzoic acid (896 mg, 62% yield) as a white solid.

Step 7. To a solution of 2-bromo-3-methoxy-6-methyl-benzoic acid (1.15 g, 4.68 mmol), triethylamine (1.42 g, 14.1 mmol, 2.0 mL) , and tert-butanol (1.73 g, 23.4 mmol, 2.25 mL) in toluene (8 mL) was added DPPA (1.93 g, 7.01 mmol, 1.52 mL) and the mixture was heated to reflux for 1h and cooled to rt. The volatiles were removed in vacuo. The residue was diluted with aq. citric acid 15% and extracted with EtOAc (3x). The combined organic layers were washed with aqueous 1N NaOH, brine, dried over Na₂SO₄, filtered and concentrated in vacuo. The crude product was purified by silica gel chromatography (dry load) eluting with a gradient of EtOAc (0 to 30%) in Hep. Appropriate fractions were combined and concentrated in vacuo to afford tert-butyl N-(2-bromo-3-methoxy-6-methyl-phenyl)carbamate (1.34 g, 91% yield) as a colorless oil.

Step 8. To a solution of tert-butyl N-(2-bromo-3-methoxy-6-methyl-phenyl)carbamate (1.34 g, 4.24 mmol) in MeOH (10 mL) was added HCl in dioxane (4 M, 10.5 mL, 42.0 mmol). The reaction mixture was stirred at RT for 75 min. The solution was evaporated to dryness in vacuo then suspended in saturated aqueous NaHCO₃ and extracted with DCM (3 x, phase separator). The combined organic extracts were concentrated and the crude product was purified by silica gel chromatography eluting with a gradient of EtOAc (0 to 50%) in Hep. Appropriate fractions were combined and concentrated in vacuo to afford 2-bromo-3-methoxy-6-methyl-aniline (807 mg, 88% yield) as an off-white waxy solid. MS: [M+1]: 218.0.

### Preparation of Arylamine A11

Compounds of the present invention can be prepared from key **Intermediate A11** which can be prepared as shown in Scheme A9 and described herein. The commercially available 6-bromo-3-methoxy-2-methylbenzoic acid can be converted to the N-Boc under Curtius rearrangement conditions. The NH-Boc can be cleaved under acidic conditions to generate key **Intermediate A11.**

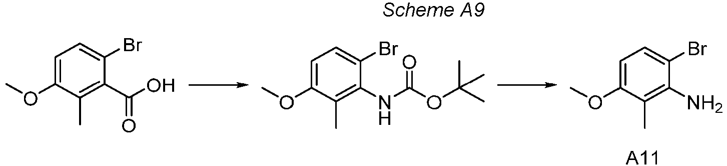

Step 1. To a solution of 6-bromo-3-methoxy-2-methyl-benzoic acid (1 g, 4.08 mmol), triethylamine (1.23 g, 12.2 mmol, 1.70 mL), and tert-butanol (1.55 g, 20.9 mmol) in toluene (7 mL) was added [azido(phenoxy)phosphoryl]oxybenzene (1.72 g, 6.25 mmol, 1.35 mL). The mixture was heated to reflux for 5h and cooled to rt. The volatiles were removed in vacuo. The residue was diluted with aq. citric acid 15% and extracted with EtOAc (3x). The combined organic layers were washed with brine, dried over Na₂SO₄, filtered, and concentrated in vacuo. The residue was purified by silica gel chromatography (dry load) eluting with a gradient of EtOAc (0 to 30%) in Hep. Appropriate fractions were combined and concentrated in vacuo to afford tert-butyl N-(6-bromo-3-methoxy-2-methyl-phenyl)carbamate (1.41 g, quantitative yield) as a colorless oil, which was not pure but used as is in the next step.

Step 2. To a solution of tert-butyl N-(6-bromo-3-methoxy-2-methyl-phenyl)carbamate (1.41 g, 4.46 mmol) in MeOH (22 mL) was added HCl in dioxane (4 M, 22 mL). The reaction mixture was stirred at RT for 80 min. The solution was evaporated to dryness in vacuo, then suspended in saturated aqueous NaHCO₃ and extracted with DCM (3 x). The combined organic extracts were dried over Na₂SO₄, filtered and concentrated. The residue was purified by silica gel chromatography (dry load) eluting with a gradient of EtOAc (0 to 50%) in Hep. Appropriate fractions were combined and concentrated in vacuo to afford 6-bromo-3-methoxy-2-methyl-aniline (586 mg, 61% yield) as a white solid. ¹H NMR (400 MHz, Chloroform-d) δ 7.23 (dd, J = 8.8, 0.6 Hz, 1H), 6.26 (d, J = 8.8 Hz, 1H), 4.06 (br s, 2H), 3.78 (s, 3H), 2.09 (t, J = 0.5 Hz, 3H). MS: [M+1]: 218.0.

### Chiral separation of selected compounds

Racemic mixtures of atropisomers wwere separated using chiral SFC methods on a Mettler Toledo Minigram SFC (MTM), a Waters Prep 15 SFC-MS (WP15) or a Waters Prep 100 SFC-MS (WP100) (Table 3). An appropriate column was selected to achieve a satisfactory resolution of the peaks. The appropriate fractions for each peak were combined, concentrated and usually taken in a mixture of water and a suitable water miscible organic solvent such as EtOH, IPA, CH₃CN or a mixture thereof and freeze-dried. The separated products were reanalyzed by chiral SFC to assess chiral purity.

C1A is Phenomenex Lux Cellulose-2, 10 x 250 mm, 5 µm; C1B is Phenomenex Lux Cellulose-2, 30 x 250 mm, 5 µm; C2 is Chiral Technologies IA, 10 x 250 mm, 5 µm; C3 is Chiral Technologies IC, 10 x 250 mm, 5 µm; C4 is Chiral Technologies ID, 10 x 250 mm, 5 µm; C5 is Chiral Technologies IG, 10 x 250 mm, 5 µm; C6 is Chiral Technologies AS, 10 x 250 mm, 5 µm; C7 is Phenomenex Lux Cellulose-4, 10 x 250 mm, 5 µm.

Structural assignments of the separated atropisomers were confirmed by biological activity where the biologically active enantiomer was assigned to have the (S) configuration, which was confirmed by X-ray crystallography of key compounds.

**Table 3**

| **Mixture Cmpd #** | **Peak 1 Cmpd #** | **Peak 2 Cmpd #** | **Instrument** | **Column** | **Eluent (%, Flow Rate (mL/min))** |
|---|---|---|---|---|---|
| 28 | 29 | 30 | WP15 | C4 | IPA + 10mM Ammonium Formate (40%,10) |
| 38 | 39 | 40 | MTM | C2 | IPA + 10mM Ammonium Formate (40%,10) |
| 43 | 44 | 45 | MTM | C4 | IPA + 10mM Ammonium Formate (40%,10) |
| 46 | 47 | 48 | MTM | C1A | 1:1 ACN/EtOH + 0.1% Formic Acid (55%,10) |
| 49 | 50 | 51 | MTM | C4 | MeOH + 10 mM Ammonium Formate (25%,10) |
| 54 | 55 | 56 | MTM | C1A | MeOH + 10 mM Ammonium Formate (55%,10) |
| 61 | 62 | 63 | MTM | C4 | IPA + 10mM Ammonium Formate (40%,10) |
| 69 | 70 | 71 | MTM | C4 | MeOH (20%,10) |
| 83 | 84 | 82 | MTM | C1A | 1:1 ACN/EtOH (55%,10) |
| 97 | 98 | 99 | MTM | C5 | IPA (30%,10) |
| 104 | 105 | 106 | MTM | C1A | MeOH + 10 mM Ammonium Formate (35%,10) |
| 110 | 111 | 112 | WP15 | C1A | MeOH (55%,10) |
| 113 | 114 | 115 | MTM | C3 | MeOH (20%,10) |
| 116 | 117 | 118 | WP100 | C1B | 1:1 ACN/EtOH (50%,70) |
| 119 | 120 | 121 | WP100 | C1B | 1:1 ACN/EtOH (45%,70) |
| 125 | 126 | 127 | WP100 | C1B | 1:1 ACN/EtOH (50%,70) |
| 132 | 133 | 134 | WP100 | C1B | 1:1 ACN/EtOH (45%,70) |
| 135 | 136 | 137 | MTM | C1A | 1:1 ACN/EtOH (45%,10) |
| 138 | 139 | 140 | MTM | C1A | 1:1 ACN/EtOH (40%,10) |
| 141 | 142 | nd | WP100 | C1B | 1:1 ACN/EtOH (50%,70) |
| 143 | 144 | 145 | MTM | C5 | IPA (30%,10) |
| 147 | 148 | 149 | MTM | C1A | 1:1 ACN/EtOH (55%,10) |
| 164 | 165 | 166 | WP100 | C1B | 1:1 ACN/EtOH (55%,70) |
| 170 | 171 | 172 | MTM | C3 | MeOH (45%,10) |
| 173 | 174 | 175 | MTM | C1A | 1:1 ACN/EtOH (55%,10) |
| 285 | 176 | 177 | MTM | C1A | IPA (50%,10) |
| 178 | 179 | 180 | MTM | C1A | ACN/EtOH (45%,10) |
| 181 | 182 | 183 | WP-100 | C1B | IPA + 10mM Ammonium Formate (55%,70) |
| 184 | 185 | 186 | MTM | C1A | IPA + 10mM Ammonium Formate (40%,10) |
| 187 | 188 | 189 | MTM | C1A | IPA + 10mM Ammonium Formate (40%,10) |
| 190 | 191 | 192 | MTM | C1A | IPA + 10mM Ammonium Formate (40%,10) |
| 193 | 194 | 195 | MTM | C5 | 1:1 ACN/EtOH + 0.1% Formic Acid (30%,10) |
| 201 | 203 | 205 | WP100 | C1B | ACN/EtOH (45%, 70) |
| 286 | 196 | 197 | MTM | C1A | IPA (50%,10) |
| 198 | 199 | 200 | MTM | C1A | 1:1 ACN/EtOH (40%,10) |
| 266 | 264 | 265 | WP100 | C1B | ACN/EtOH (55%,70) |
| 271 | 272 | 273 | MTM | C1A | IPA + 10mM Ammonium Formate (45%,10) |
| 275 | 274 | 276 | MTM | C1A | IPA + 10mM Ammonium Formate (50%,10) |
| Int. D | Int. D1 | Int. D2 | WP100 | C1B | MeOH (25%,70) |
| 270 | 288 | 269 | MTM | C1A | IPA + 10mM Ammonium Formate (55%,10) |
| 267 | 287 | 268 | MTM | C1A | IPA + 10mM Ammonium Formate (55%,10) |
| 289 | 290 | 291 | MTM | C3 | MeOH + 10mM Ammonium Formate (30%,10) |
| 292 | 293 | 294 | WP15 | C7 | MeOH + 10mM Ammonium Formate (55%,10) |
| 295 | 296 | 297 | MTM | C1A | MeOH (55%, 10) |
| 298 | 299 | 300 | MTM | C1A | MeOH (55%, 10) |
| 301 | 302 | 303 | MTM | C1A | MeOH (35%, 10) |
| 304 | 305 | 306 | MTM | C1A | 1:1 ACN/EtOH (55%,10) |
| 308 | 309 | 310 | WP15 | C1A | MeOH (55%, 10) |
| 313 | 314 | 315 | MTM | C1A | 1:1 ACN/EtOH (40%,10) |
| 316 | 317 | 318 | MTM | C1A | 1:1 ACN/EtOH (40%,10) |
| 319 | 320 | 321 | MTM | C1A | 1:1 ACN/EtOH (50%,10) |
| 131 | 322 | 323 | MTM | C1A | 1:1 ACN/EtOH (35%,10) |
| 324 | 325 | 326 | MTM | C1A | 1:1 ACN/EtOH (45%,10) |

### Example 2. Enzymatic assay

Detection of Myt1 kinase activity utilized a recombinant human Myt1 kinase assay measuring the hydrolysis of ATP using a commercially available ADP-Glo Assay (ADP-Glo^{™} Kinase Assay from Promega, 10 000 assays, #V9102). Briefly, 5 µL recombinant human Myt1 (full length PKMYT1 recombinant human protein expressed in insect cells from Thermo Fisher #A33387; ~80% purity) was prepared in reaction buffer (70 mM HEPES, 3 mM MgCl₂, 3 mM MnCl₂, 50 µg/ml PEG 20000, 3µM Na-orthovanadate, 1.2 mM DTT) and added to 384 well white polystyrene, flat bottom well, non-treated, microplate (Corning #3572). After this, 5 µL of compounds (diluted in reaction buffer to 0.5% DMSO) was added to the microplate and the plate was spun briefly and incubated at 22 °C for 15 minutes. Ultra-Pure Adenosine Triphosphate (ATP) solution (ADP-Glo kit from Promega) was diluted in reaction buffer and 5 µL was added to the microplate, spun down briefly and incubated for 60 minutes at 30 °C. The final Myt1 enzyme concentration was 18 nM and the final ATP concentration was 10 µM. After the 60-minute incubation, 15 µL of ADP-Glo reagent was added and the plate was spun briefly and sealed and incubated in the dark for 40 minutes at 22 °C. Following this, 30 µL of kinase detection reagent was added per well and the plate was spun briefly, sealed and incubated for 45-60 minutes at 22 °C in the dark. Luminescence was read using the Envision (250 ms integration). The IC₅₀ and the % max inhibition were calculated for each inhibitor compound tested.

Exemplary prepared compounds and their activities were shown in Table 4 below.

**Table 4**

| Compound | Method | Myt1 IC₅₀ (nM) | MS (+ESI) [M+1] |
|---|---|---|---|
| 1 | A | 50 | m/z 412.4 |
| 2 | A | 231 | m/z 395.5 |
| 3 | A | 462 | m/z 391.5 |
| 4 | C | 922 | m/z 317.2 |
| 5 | C | 277 | m/z 349.4 |
| 6 | C | 1240 | m/z 349.2 |
| 7 | C | 605 | m/z 445.2 |
| 8 | C | 164 | m/z 375.4 |
| 9 | C | 363 | m/z 403.2 |
| 10 | C | 187 | m/z 403.4 |
| 11 | C | 252 | m/z 349.2 |
| 12 | C | 988 | m/z 363.2 |
| 13 | C | 190 | m/z 487.5 |
| 14 | C | 480 | m/z 384.2 |
| 15 | C | 1090 | m/z 411.9 |
| 16 | C | 9630 | m/z 505.6 |
| 17 | C | 9060 | m/z 379.2 |
| 18 | C | 1940 | m/z 410.4 |
| 19 | C | 527 | m/z 383.2 |
| 20 | C | 182 | m/z 417.2 |
| 21 | C | 283 | m/z 417.2 |
| 22 | C | 79 | m/z 375.4 |
| 23 | C | 2470 | m/z 361.2 |
| 24 | C | 29 | m/z 366.2 |
| 25 | C | 399 | m/z 378.2 |
| 26 | C | 2000 | m/z 464.4 |
| 27 | C | 604 | m/z 365.3 |
| 28 | A | <10 | m/z 381.2 |
| 29 | A | <10 | m/z 381.2 |
| 30 | A | 1830 | m/z 381.2 |
| 31 | J | <10 | m/z 395.2 |
| 32 | A | <10 | m/z 409.4 |
| 33 | L | <10 | m/z 460.2 |
| 34 | A | <10 | m/z 375.4 |
| 35 | A | <10 | m/z 443.4 |
| 36 | A | 14 | m/z 443.4 |
| 37 | A | <10 | m/z 381.2 |
| 38 | A | <10 | m/z 376.2 |
| 39 | A | > 5000 | m/z 376.2 |
| 40 | A | <10 | m/z 376.2 |
| 41 | A | <10 | m/z 381.2 |
| 42 | A | <10 | m/z 401.4 |
| 43 | A | <10 | m/z 4112 |
| 44 | A | 3020 | m/z 411.2 |
| 45 | A | <10 | m/z 411.2 |
| 46 | A | <10 | m/z 437.4 |
| 47 | A | <10 | m/z 437.4 |
| 48 | A | > 5000 | m/z 437.4 |
| 49 | A | <10 | m/z 409.7 |
| 50 | A | <10 | m/z 409.7 |
| 51 | A | 87 | m/z 409.7 |
| 52 | A | 10 | m/z 411.2 |
| 53 | A | 16 | m/z 396.2 |
| 54 | A | <10 | m/z 383.2 |
| 55 | A | <10 | m/z 383.2 |
| 56 | A | 549 | m/z 383.2 |
| 57 | A | <10 | m/z 376.2 |
| 58 | A | <10 | m/z 449.2 |
| 59 | A | 12 | m/z 397.4 |
| 60 | A | 10 | m/z 365.3 |
| 61 | A | <10 | m/z 389.2 |
| 62 | A | > 5000 | m/z 389.2 |
| 63 | A | <10 | m/z 389.2 |
| 64 | A | <10 | m/z 393.2 |
| 65 | A | <10 | m/z 364.2 |
| 66 | A | 10 | m/z 396.2 |
| 67 | A | 14 | m/z 382.2 |
| 68 | A | 10 | m/z 416.2 |
| 69 | A | <10 | m/z 398.4 |
| 70 | A | 2920 | m/z 398.4 |
| 71 | A | <10 | m/z 399.3 |
| 72 | A | 11 | m/z 378.2 |
| 73 | A | 10 | m/z 396.2 |
| 74 | A | 14 | m/z 466.5 |
| 75 | A | 11 | m/z 431.5 |
| 76 | A | <10 | m/z 378.2 |
| 77 | A | <10 | m/z 363.2 |
| 78 | A | 18 | m/z 437.4 |
| 79 | A | <10 | m/z 437.4 |
| 80 | A | <10 | m/z 452.5 |
| 81 | A | <10 | m/z 381.2 |
| 82 | A | > 5000 | m/z 380.2 |
| 83 | A | <10 | m/z 380.2 |
| 84 | A | <10 | m/z 380.2 |
| 85 | A | <10 | m/z 367.2 |
| 86 | A | <10 | m/z 378.2 |
| 87 | A | <10 | m/z 424.5 |
| 88 | A | <10 | m/z 369.2 |
| 89 | A | <10 | m/z 369.2 |
| 90 | A | 2800 | m/z 369.2 |
| 91 | A | <10 | m/z 369.2 |
| 92 | A | <10 | m/z 409.2 |
| 93 | A (from int D2) | <10 | m/z 409.2 |
| 94 | A | <10 | m/z 338.2 |
| 95 | A (from int D2) | <10 | m/z 338.2 |
| 96 | A | 10 | m/z 354.2 |
| 97 | M | 12 | m/z 375.2 |
| 98 | M | > 5000 | m/z 375.2 |
| 99 | M | <10 | m/z 375.2 |
| 100 | J | <10 | m/z 352.2 |
| 101 | J | <10 | m/z 367.2 |
| 102 | J | 13 | m/z 392.2 |
| 104 | A | <10 | m/z 358.5 |
| 105 | A | 3060 | m/z 358.5 |
| 106 | A | <10 | m/z 358.5 |
| 107 | A | 12 | m/z 373.8 |
| 110 | J | Nd | m/z 355.2 |
| 111 | J | <10 | m/z 355.2 |
| 112 | J | 1380 | m/z 355.2 |
| 113 | A | <10 | m/z 344.2 |
| 114 | A | 4680 | m/z 344.2 |
| 115 | A | <10 | m/z 344.2 |
| 116 | D | <10 | m/z 337.2 |
| 117 | D | <10 | m/z 337.3 |
| 118 | D | 611 | m/z 337.3 |
| 119 | D | <10 | m/z 311.2 |
| 120 | D | <10 | m/z 311.2 |
| 121 | D | 1020 | m/z 311.2 |
| 125 | D | 16 | m/z 325.2 |
| 126 | D | <10 | m/z 325.2 |
| 127 | D | 271 | m/z 325.2 |
| 131 | D | <10 | m/z 355.2 |
| 132 | D or K | <10 | m/z 331.2 |
| 133 | D or K | <10 | m/z 331.2 |
| 134 | D or K | 653 | m/z 331.2 |
| 135 | D | <10 | m/z 314.2 |
| 136 | D | <10 | m/z 314.2 |
| 137 | D | 476 | m/z 314.2 |
| 138 | D | <10 | m/z 339.2 |
| 139 | D | <10 | m/z 339.2 |
| 140 | D | 526 | m/z 339.2 |
| 141 | D | <10 | m/z 375.2 |
| 142 | D | <10 | m/z 375.2 |
| 143 | O | <10 | m/z 322.2 |
| 144 | O | <10 | m/z 322.2 |
| 145 | O | > 2000 | m/z 322.2 |
| 146 | E | 1420 | m/z 284.2 |
| 147 | E | 55 | m/z 299.3 |
| 148 | E | 32 | m/z 299.3 |
| 149 | E | > 5000 | m/z 299.3 |
| 150 | B | 1350 | m/z 360.2 |
| 151 | B | 2750 | m/z 390.2 |
| 152 | E | 84 | m/z 312.1 |
| 153 | B | 3550 | m/z 391.2 |
| 154 | B | 6350 | m/z 413.2 |
| 155 | B | 1020 | m/z 350.2 |
| 156 | B | 465 | m/z 403.3 |
| 157 | B | 1240 | m/z 417.2 |
| 158 | B | 953 | m/z 385.2 |
| 159 | B | 1720 | m/z 389.2 |
| 160 | B | 554 | m/z 385.3 |
| 161 | B | 671 | m/z 411.3 |
| 162 | B | 596 | m/z 389.2 |
| 163 | B | 334 | m/z 407.2 |
| 164 | E | <10 | m/z 326.1 |
| 165 | E | <10 | m/z 326.1 |
| 166 | E | 3140 | m/z 326.1 |
| 167 | B | 361 | m/z 298.2 |
| 168 | E | <10 | m/z 346.4 |
| 170 | E | Nd | m/z 346.3 |
| 171 | E | 6140 | m/z 346.3 |
| 172 | E | <10 | m/z 346.3 |
| 173 | A | <10 | m/z 352.4 |
| 174 | A | <10 | m/z 352.4 |
| 175 | A | > 5000 | m/z 352.4 |
| 176 | A | <10 | m/z 312.1 |
| 177 | A | > 2000 | m/z 312.2 |
| 178 | A | <10 | m/z 358.2 |
| 179 | A | <10 | m/z 358.2 |
| 180 | A | 89 | m/z 358.2 |
| 181 | D or O | <10 | m/z 325.1 |
| 182 | D or O | <10 | m/z 325.2 |
| 183 | D or O | 1360 | m/z 325.2 |
| 184 | A | 10 | m/z 340.1 |
| 185 | A | <10 | m/z 340.2 |
| 186 | A | 1750 | m/z 340.1 |
| 187 | D | <10 | m/z 391.1 |
| 188 | D | <10 | m/z 391.1 |
| 189 | D | 1010 | m/z 391.1 |
| 190 | A | 12 | m/z 356.2 |
| 191 | A | <10 | m/z 356.2 |
| 192 | A | > 2000 | m/z 356.2 |
| 193 | D | <10 | m/z 311.1 |
| 194 | D | > 2000 | m/z 311.1 |
| 195 | D | <10 | m/z 311.1 |
| 196 | F | <10 | m/z 312.1 |
| 197 | F | 1700 | m/z 312.1 |
| 198 | A | <10 | m/z 406.1 |
| 199 | A | <10 | m/z 406.2 |
| 200 | A | > 2000 | m/z 406.2 |
| 201 | D | <10 | m/z 345.1 |
| 202 | A | <10 | m/z 338.1 |
| 203 | D | <10 | m/z 345.1 |
| 204 | A (from int D2) | <10 | m/z 340.1 |
| 205 | D | 168 | m/z 345.1 |
| 206 | A (from int D2) | <10 | m/z 326.1 |
| 208 | A (from int D2) | <10 | m/z 322.1 |
| 209 | N | 28 | m/z 311.1 |
| 212 | G | 3440 | m/z 351.3 |
| 213 | G | 195 | m/z 351.3 |
| 214 | H | 136 | m/z 445.3 |
| 215 | H | 24 | m/z 544.5 |
| 216 | H | 76 | m/z 504.5 |
| 217 | A | 168 | m/z 421.4 |
| 218 | F | 22 | m/z 421.4 |
| 219 | A | 46 | m/z 324.2 |
| 220 | A | <10 | m/z 341.4 |
| 221 | I | 81 | m/z 365.3 |
| 222 | F | <10 | m/z 420.4 |
| 223 | F | 15 | m/z 457.4 |
| 224 | F | 13 | m/z 406.4 |
| 225 | F | 34 | m/z 406.4 |
| 226 | F | 12 | m/z 379.4 |
| 227 | F | 50 | m/z 399.3 |
| 228 | F | 21 | m/z 362.4 |
| 229 | F | 52 | m/z 366.4 |
| 230 | F | 368 | m/z 398.4 |
| 231 | F | <10 | m/z 422.4 |
| 232 | F | 11 | m/z 392.4 |
| 233 | F | 10 | m/z 426.3 |
| 234 | F | 14 | m/z 408.3 |
| 235 | F | 13 | m/z 406.4 |
| 236 | F | 16 | m/z 408.4 |
| 237 | F | 19 | m/z 436.4 |
| 238 | F | 10 | m/z 394.3 |
| 239 | F | 15 | m/z 380.4 |
| 240 | F | 191 | m/z 410.4 |
| 241 | F | 24 | m/z 366.4 |
| 242 | F | <10 | m/z 456.4 |
| 243 | A | 69 | m/z 418.3 |
| 244 | A | 16 | m/z 369.4 |
| 245 | A | 11 | m/z 431.4 |
| 246 | A | 84 | m/z 397.4 |
| 247 | A | 17 | m/z 427.4 |
| 248 | A | <10 | m/z 431.3 |
| 249 | A | 20 | m/z 415.4 |
| 250 | A | <10 | m/z 376.3 |
| 251 | A | 1420 | m/z 376.3 |
| 252 | A | <10 | m/z 376.3 |
| 253 | A | 24 | m/z 395.4 |
| 254 | A | 10 | m/z 428.4 |
| 255 | A | 109 | m/z 393.4 |
| 256 | H | 50 | m/z 378.4 |
| 257 | F | 43 | m/z 324.3 |
| 258 | A | 3130 | m/z 475.5 |
| 259 | A | <10 | m/z 324.3 |
| 260 | A | 27 | m/z 354.4 |
| 261 | A | 45 | m/z 376.3 |
| 262 | E | <10 | m/z 352.4 |
| 263 | A | 16 | m/z 348.2 |
| 264 | L | <10 | m/z 332.2 |
| 265 | L | > 2000 | m/z 332.2 |
| 266 | L | nd | m/z 332.2 |
| 267 | L | nd | m/z 329.2 |
| 268 | L | > 2000 | m/z 329.2 |
| 269 | L | > 2000 | m/z 329.3 |
| 270 | L | nd | m/z 329.2 |
| 271 | L | nd | m/z 395.1 |
| 272 | L | <10 | m/z 395.1 |
| 273 | L | 585 | m/z 395.1 |
| 274 | A | <10 | m/z 315.3 |
| 275 | A | nd | m/z 315.3 |
| 276 | A | > 2000 | m/z 315.3 |
| 278 | A with 2-(methylsulfonyl)acetonitrile instead of malononitirle | > 5000 | m/z 411.3 |
| 279 | A | 250 | m/z 391.4 |
| 280 | A | 80 | m/z 395.4 |
| 281 | A | 784 | m/z 390.5 |
| 282 | A | 625 | m/z 389.4 |
| 283 | A | 32 | m/z 459.2 |
| 284 | A | 238 | m/z 391.5 |
| 285 | A | 11 | m/z 312.0 |
| 286 | F | nd | m/z 312.1 |
| 287 | L | <10 | m/z 329.2 |
| 288 | L | <10 | m/z 329.2 |
| 289 | K | <10 | m/z 397.0 |
| 290 | K | <10 | m/z 397.0 |
| 291 | K | > 2000 | m/z 397.0 |
| 292 | D or O | <10 | m/z 331.3 |
| 293 | D or O | <10 | m/z 331.3 |
| 294 | D or O | > 2000 | m/z 331.3 |
| 295 | D | <10 | m/z 374.2 |
| 296 | D | <10 | m/z 374.2 |
| 297 | D | > 2000 | m/z 374.2 |
| 298 | D | <10 | m/z 380.2 |
| 299 | D | <10 | m/z 380.2 |
| 300 | D | > 2000 | m/z 380.2 |
| 301 | K | 13 | m/z 397.0 |
| 302 | K | <10 | m/z 397.0 |
| 303 | K | > 2000 | m/z 397.0 |
| 304 | D | <10 | m/z 379.2 |
| 305 | D | <10 | m/z 379.2 |
| 306 | D | > 2000 | m/z 379.2 |
| 307 | D | <10 | m/z 381.2 |
| 308 | D | <10 | m/z 380.2 |
| 309 | D | <10 | m/z 380.2 |
| 310 | D | > 2000 | m/z 380.2 |
| 311 | D | <10 | m/z 401.2 |
| 312 | D | <10 | m/z 322.2 |
| 313 | K | <10 | m/z 345.1 |
| 314 | K | <10 | m/z 345.1 |
| 315 | K | > 2000 | m/z 345.1 |
| 316 | K | <10 | m/z 365.2 |
| 317 | K | <10 | m/z 365.2 |
| 318 | K | > 2000 | m/z 365.2 |
| 319 | D | <10 | m/z 423.2 |
| 320 | D | <10 | m/z 423.2 |
| 321 | D | > 2000 | m/z 423.2 |
| 322 | D | <10 | m/z 355.2 |
| 323 | D | > 2000 | m/z 355.2 |
| 324 | D | <10 | m/z 337.2 |
| 325 | D | <10 | m/z 337.2 |
| 326 | D | > 2000 | m/z 337.2 |
| 327 | D | <10 | m/z 351.1 |
| 328 | Hydrogenolysis of 142 | <10 | m/z 297.3 |

In Table 4, the Method column indicates a preparatory method described above used in the preparation of the compounds.

### Example 3. Genetic Validation

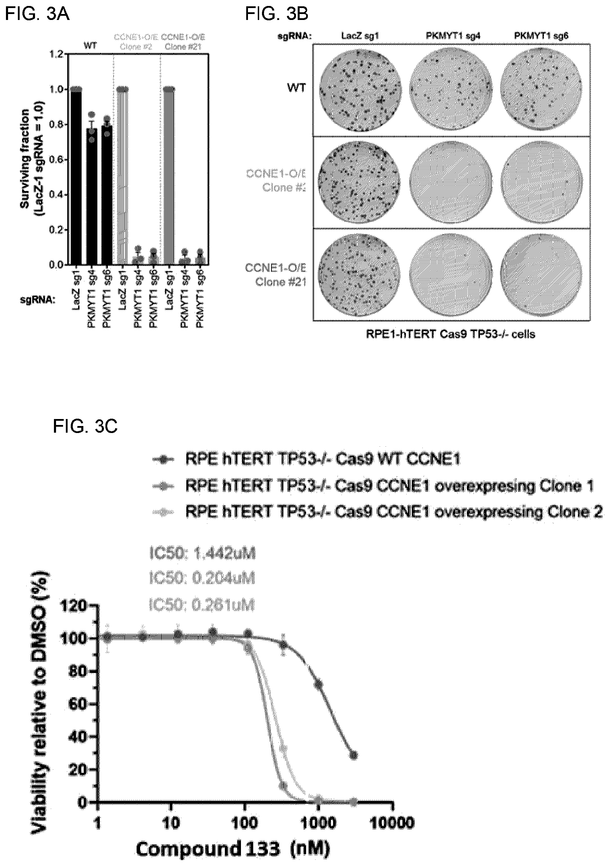

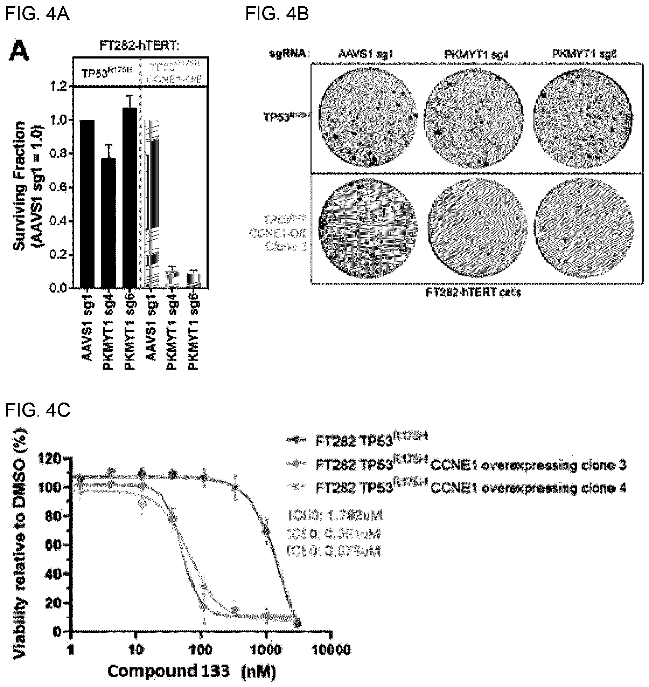

Two sgRNAs for *PKMYT1* and one sgRNA for LacZ (control) were transduced into the RPE1-hTERT Cas9 TP53-/- parental (WT) and CCNE1-overexpressing clones. Infected cells were plated at low density to measure their ability to form colonies of <50 cells. After 10 days of growth, the colonies were stained, imaged, and quantified. Using clonogenic survival assays, we observed a profound cellular fitness defect in CCNE1-overexpressing cells compared to parental cells transduced with *PKMYT1* sgRNAs (FIGS. 3A and 3B). This experiment was repeated using FT282-hTERT TP53-/- parental (WT) and CCNE1-overexpressing clones and similar results were observed (FIGS. 4A and 4B).

To determine if the kinase activity of PKMYT1 was responsible for maintaining the viability of CCNE1-overexpressing RPE1-hTERT Cas9 TP53-/- cells, the *PKMYT1* open reading frame (ORF) was cloned into an inducible mammalian expression vector. sgRNA-resistant silent mutations in the *PKMYT1* ORF sequence were then created by PCR mutagenesis. A single point mutation was generated that resulted in an asparagine (N) to alanine (A) amino acid change at residue 238. The N238A amino acid change in the kinase domain resulted in a catalytically inactive *PKMYT1* mutant. Stable cell lines in the RPE1-hTERT Cas9 TP53-/- parental and *CCNE1-*overexpressing clones were generated that either expressed the wild type *PKMYT1* ORF or the kinase-dead N238A mutant (FIG. 5A). These stable cell lines were transduced with either a LacZ non-targeting sgRNA or PKMYT1 sgRNA #4. The cells were then plated at low density to measure their ability to form colonies of >50 cells. After 10 days of growth, the colonies were stained, imaged, and quantified. Expression of an sgRNA-resistant *PKMYT1* ORF but not the catalytic-dead version rescued the fitness defect induced by transduction of sgRNA #4 into both *CCNE1-*overexpressing clones (FIGS. 5B and 5C). This result demonstrated that targeting the kinase activity of PKMYT1 selectively kills CCNE1-overexpressing cells.

### Example 4. Pharmacological validation

RPE1-hTERT Cas9 TP53-/- parental (WT) and CCNE1-overexpressing clones were treated with compound 133 in a dose titration and cell viability was determined. The CCNE1-overexpressing cells were found to be more sensitive to compound 133 than the corresponding WT cells (FIG. 3C). A similar effect was seen in FT282-hTERT TP53^{R175H} WT and CCNE1-overexpressing clones (FIG. 4C). For dose-response proliferation assays using RPE1-hTERT and FT282-hTERT cell lines, cells were seeded in 96-well plates and dosed with serially diluted Myt1 inhibitor. Cells were imaged once per day using the IncuCyte S3 microscope and percent well confluency was calculated over time. Once cells reached four population doublings the experiment was ended and IC₅₀ curves were plotted for the final time point. Percentage confluency was calculated relative to the cell confluency in the untreated wells.

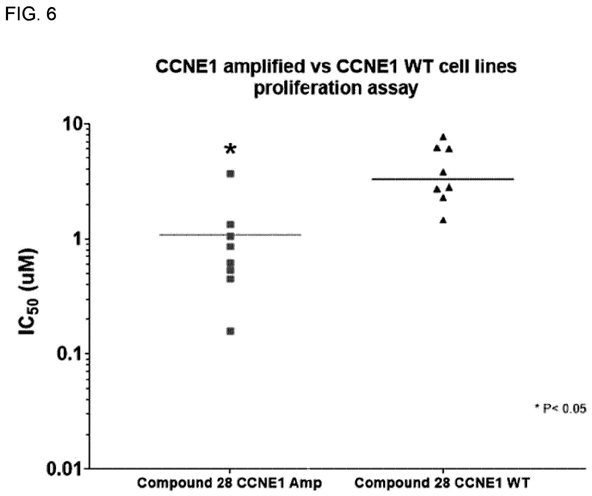

A panel of 16 cancer cell lines with either normal (n=8) or elevated levels of CCNE1 (n=8) was evaluated for their sensitivity to compound 28 in a cell proliferation assay (FIG. 6). Dose-response curves in these cancer cell line proliferation assays were generated as follows. Cells were seeded in 96-well plates and dosed with serially diluted compound 28. After 7 days, Cell Titer Glo (CTG) was used to assess the proliferation status of these cells and the IC₅₀ values were plotted.

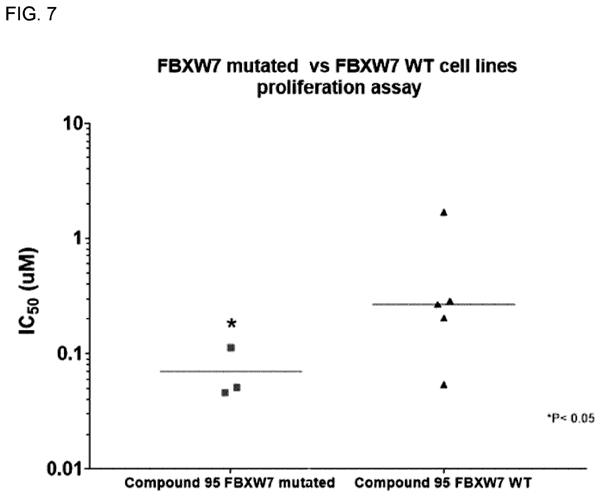

A similar experiment was conducted in a panel of 8 cancer cell lines with either wild-type FBXW7 (n=5) or FBXW7-mutations (n=3) in which these cells were evaluated for their sensitivity to compound 95 in a cell proliferation assay (FIG. 7). Dose-response curves in these cancer cell line proliferation assays were generated as follows. Cells were seeded in 96-well plates and dosed with serially diluted Myt1 inhibitor. Cells were imaged once per day using the IncuCyte S3 microscope and percent well confluency was calculated over time. Once cells reached four population doublings the experiment was ended and IC₅₀ curves were plotted for the final time point. Percentage confluency was calculated relative to the cell confluency in the untreated wells.

## Claims

1. A compound of formula (I): 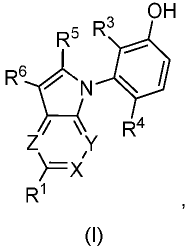 or a pharmaceutically acceptable salt thereof,
wherein
each of X, Y, and Z is independently N or CR²;
R¹ and each R² are independently hydrogen, optionally substituted C₁₋₆ alkyl, optionally substituted C₂₋₆ alkenyl, optionally substituted C₂₋₆ alkynyl, optionally substituted C₃₋₈ cycloalkyl, optionally substituted C₃₋₈ cycloalkenyl, optionally substituted C₂₋₉ heterocyclyl, optionally substituted C₂₋₉ heterocyclyl C₁₋₆ alkyl, optionally substituted C₆₋₁₀ aryl, optionally substituted C₁₋₉ heteroaryl, optionally substituted C₁₋₉ heteroaryl C₁₋₆ alkyl, halogen, cyano, -N(R⁷)₂, -OR⁷, - C(O)N(R⁸)₂, -SO₂N(R⁸)₂, -SO₂R^{7A}, or -Q-R^{7B}; or R¹ combines with one R² that is *vicinal* to R¹ to form an optionally substituted C₃₋₆ alkylene;
each of R³ and R⁴ is independently optionally substituted C₁₋₆ alkyl or halogen;
R⁵ is H or -N(R⁷)₂;
R⁶ is -C(O)NH(R⁸), -C(O)R^{7A}, or -SO₂R^{7A};
each R⁷ is independently hydrogen, optionally substituted C₁₋₆ alkyl, optionally substituted C₆₋₁₀ aryl C₁₋₆ alkyl, optionally substituted C₃₋₈ cycloalkyl, optionally substituted C₆₋₁₀ aryl, optionally substituted C₂₋₉ heterocyclyl, optionally substituted C₁₋₉ heteroaryl, optionally substituted C₁₋₉ heteroaryl C₁₋₆ alkyl, or -SO₂R^{7A}; or two R⁷ groups, together with the atom to which both are attached, combine to form an optionally substituted C₂₋₉ heterocyclyl;
each R^{7A} is independently optionally substituted C₁₋₆ alkyl, optionally substituted C₃₋₈ cycloalkyl, or optionally substituted C₆₋₁₀ aryl;
each R^{7B} is independently hydroxyl, optionally substituted C₁₋₆ alkyl, optionally substituted C₆₋₁₀ aryl, optionally substituted C₂₋₉ heterocyclyl, optionally substituted C₁₋₉ heteroaryl, -N(R⁷)₂, -C(O)N(R⁸)₂, -SO₂N(R⁸)₂, -SO₂R^{7A}, or optionally substituted alkoxy;
each R⁸ is independently hydrogen, optionally substituted C₁₋₆ alkyl, optionally substituted C₂₋₆ alkoxyalkyl, optionally substituted C₆₋₁₀ aryl C₁₋₆ alkyl, optionally substituted C₆₋₁₀ aryl, optionally substituted C₃₋₈ cycloalkyl, or optionally substituted C₁₋₉ heteroaryl; or two R⁸, together with the atom to which they are attached, combine to form an optionally substituted C₂₋₉ heterocyclyl;
Q is optionally substituted C₁₋₆ alkylene, optionally substituted C₂₋₆ alkenylene, optionally substituted C₂₋₆ alkynylene, optionally substituted C₃₋₈ cycloalkylene, optionally substituted C₃₋₈ cycloalkenylene optionally substituted C₆₋₁₀ arylene, optionally substituted C₂₋₉ heterocyclylene, or optionally substituted C₁₋₉ heteroarylene.

2. The compound of claim 1, or a pharmaceutically acceptable salt thereof, wherein the compound is enriched for the atropisomer of formula (IA):

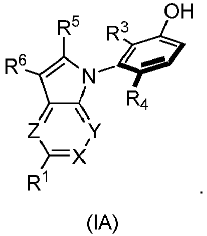

3. The compound of claim 1 or 2, or a pharmaceutically acceptable salt thereof, wherein X is CR².

4. The compound of claim 1, or a pharmaceutically acceptable salt thereof, wherein the compound is of formula (II):

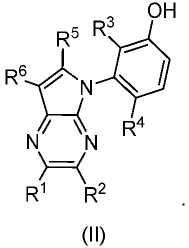

5. The compound of claim 4, or a pharmaceutically acceptable salt thereof, wherein the compound is enriched for the atropisomer of formula (IIA):

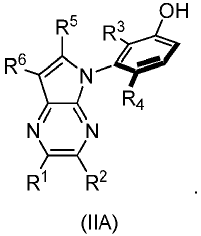

6. The compound of claim 1, or a pharmaceutically acceptable salt thereof, wherein the compound is of formula (III): 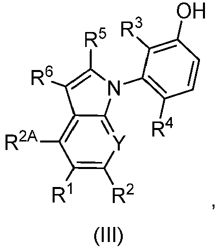 wherein R^{2A} is hydrogen, optionally substituted C₁₋₆ alkyl, optionally substituted C₂₋₆ alkenyl, optionally substituted C₂₋₆ alkynyl, optionally substituted C₃₋₈ cycloalkyl, optionally substituted C₃₋₈ cycloalkenyl, optionally substituted C₂₋₉ heterocyclyl, optionally substituted C₂₋₉ heterocyclyl C₁₋₆ alkyl, optionally substituted C₆₋₁₀ aryl, optionally substituted C₁₋₉ heteroaryl, optionally substituted C₁₋₉ heteroaryl C₁₋₆ alkyl, halogen, -N(R⁷)₂, -OR⁷, -C(O)N(R⁸)₂, -SO₂N(R⁸)₂, -SO₂R^{7A}, or -Q-R^{7B}.

7. The compound of claim 6, or a pharmaceutically acceptable salt thereof, wherein the compound is enriched for the atropisomer of formula (IIIA):

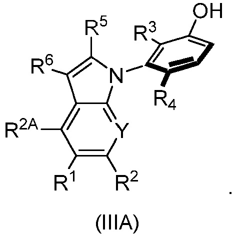

8. The compound of claim 6 or 7, or a pharmaceutically acceptable salt thereof, wherein R^{2A} is hydrogen, optionally substituted C₁₋₆ alkyl, or halogen.

9. The compound of any one of claims 1 to 8, or a pharmaceutically acceptable salt thereof, wherein R³ is:
(a) optionally substituted C₁₋₆ alkyl; or
(b) halogen, optionally wherein halogen is chlorine.

10. The compound of any one of claims 1 to 9, or a pharmaceutically acceptable salt thereof, wherein R⁴ is:
(a) optionally substituted C₁₋₆ alkyl; or
(b) halogen, optionally wherein halogen is chlorine.

11. The compound of any one of claims 1 to 10, or a pharmaceutically acceptable salt thereof, wherein R² is:
(a) hydrogen; or
(b) optionally substituted C₁₋₆ alkyl, optionally wherein C₁₋₆ alkyl is optionally substituted methyl or optionally substituted isopropyl; or
(c) halogen.

12. The compound of any one of claims 1 to 11, or a pharmaceutically acceptable salt thereof, wherein R¹ is:
(a) hydrogen; or
(b) halogen; optionally chlorine or bromine; or
(c) optionally substituted C₁₋₆ alkyl; optionally wherein optionally substituted C₁₋₆ alkyl is optionally substituted methyl, optionally substituted ethyl, optionally substituted isopropyl, optionally substituted butyl; or
(d) optionally substituted C₁₋₉ heteroaryl; optionally wherein optionally substituted C₁₋₉ heteroaryl is optionally substituted 1,3-thiazolyl, optionally substituted 1,2-thiazolyl, optionally substituted 1,3-oxazolyl, optionally substituted benzo-1,3-thiazolyl, optionally substituted benzo-1,3-oxazolyl, optionally substituted indolyl, optionally substituted benzimidazolyl, optionally substituted pyridyl, optionally substituted imidazolyl, optionally substituted pyrimidyl, optionally substituted pyrazinyl, optionally substituted pyridazinyl, optionally substituted pyrazolyl; or
(e) optionally substituted C₃₋₈ cycloalkyl; optionally wherein optionally substituted C₃₋₈ cycloalkyl is optionally substituted cyclopropyl, optionally substituted cyclobutyl, optionally substituted cyclopentyl, optionally substituted cyclohexyl; or
(f) optionally substituted C₆₋₁₀ aryl; optionally wherein optionally substituted C₆₋₁₀ aryl is optionally substituted phenyl; or
(g) optionally substituted C₂₋₉ heterocyclyl; optionally wherein optionally substituted C₂₋₉ heterocyclyl is optionally substituted 1,2,3,6-tetrahydropyridinyl, optionally substituted piperidinyl, optionally substituted morpholinyl, optionally substituted piperazinyl, optionally substituted thiomorpholinyl, optionally substituted oxa-aza-spiro[3,3]heptane, optionally substituted oxa-aza-bicyclo[3.2.1]octane; or
(h) optionally substituted cycloalkenyl; or
(i) -Q-R^{7B}; optionally wherein Q is optionally substituted C₂₋₆ alkynylene, C₁₋₆ alkylene, or C₆₋₁₀ arylene;
(j) -Q-R^{7B}; optionally wherein R^{7B} is optionally substituted C₂₋₉ heterocyclyl, or optionally substituted C₆₋₁₀ aryl; and/or
(j) -N(R⁷)₂; optionally wherein -N(R⁷)₂ is diethylamino;
optionally wherein, when R₁ is optionally substituted, the substituent is one, two, or three groups independently selected from the group consisting of methyl, difluoromethyl, trifluoromethyl, fluorine, chlorine, bromine, amino, hydroxyl, cyano, oxo, -C(O)NH₂, - C(O)NH(Me), -C(O)N(Me)₂, -(CH₂)ₙ-C(O)OH, and -(CH₂)ₙ-C(O)Ot-Bu, wherein n is 0 or 1.

13. The compound of any one of claims 1 to 12, or a pharmaceutically acceptable salt thereof, wherein R⁵ is
(a) hydrogen; or
(b) -N(R⁷), optionally wherein -NR⁷ is -NH₂.

14. The compound of any one of claims 1 to 13, or a pharmaceutically acceptable salt thereof, wherein R⁶ is
(a) -C(O)NH(R⁸); optionally wherein -C(O)NH(R⁸) is -C(O)NH_{2_}or -C(O)NH(Me); or
(b) -SO₂R^{7A}, optionally wherein -SO₂R^{7A} is -SO₂Me.

15. The compound of claim 1, wherein the compound has the structure: 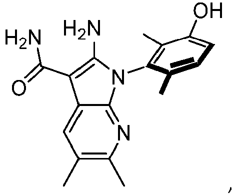 or a pharmaceutically acceptable salt thereof.

16. A pharmaceutical composition comprising the compound of any one of claims 1 to 15, or a pharmaceutically acceptable salt thereof, and a pharmaceutically acceptable excipient, optionally wherein the composition is isotopically enriched in deuterium.

## Patentansprüche

1. Verbindung der Formel (I): 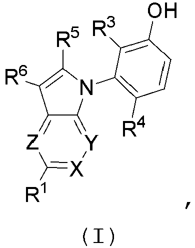 oder ein pharmazeutisch akzeptables Salz davon,
worin
jedes von X, Y und Z unabhängig N oder CR² ist;
R¹ und jedes R² unabhängig Wasserstoff, optional substituiertes C₁₋₆-Alkyl, optional substituiertes C₂₋₆-Alkenyl, optional substituiertes C₂₋₆-Alkinyl, optional substituiertes C₃₋₈-Cycloalkyl, optional substituiertes C₃₋₈-Cycloalkenyl, optional substituiertes C₂₋₉-Heterocyclyl, optional substituiertes C₂₋₉-Heterocyclyl-C₁₋₆-alkyl, optional substituiertes C₆₋₁₀-Aryl, optional substituiertes C₁₋₉-Heteroaryl, optional substituiertes C₁₋₉-Heteroaryl-C₁₋₆-alkyl, Halogen, Cyano, -N(R⁷)₂, -OR⁷, -C(O)N(R⁸)₂, -SO₂N(R⁸)₂, -SO₂R^{7A} oder -Q-R^{7B} ist; oder R¹ sich mit einem R², das zu R¹ *vicinal* ist, verbindet, um optional substituiertes C₃₋₆-Alkylen zu bilden;
jedes von R³ und R⁴ unabhängig optional substituiertes C₁₋₆-Alkyl oder Halogen ist;
R⁵ H oder -N(R⁷)₂ ist;
R⁶ -C(O)NH(R⁸), -C(O)R^{7A} oder -SO₂R^{7A} ist;
jedes R⁷ unabhängig Wasserstoff, optional substituiertes C₁₋₆-Alkyl, optional substituiertes C₆₋₁₀-Aryl-C₁₋₆-alkyl, optional substituiertes C₃₋₈-Cycloalkyl, optional substituiertes C₆₋₁₀-Aryl, optional substituiertes C₂₋₉-Heterocyclyl, optional substituiertes C₁₋₉-Heteroaryl, optional substituiertes C₁₋₉-Heteroaryl-C₁₋₆-alkyl oder -SO₂R^{7A} ist; oder zwei R⁷-Gruppen zusammen mit dem Atom, an das beide gebunden sind, optional substituiertes C₂₋₉-Heterocyclyl bilden;
jedes R^{7A} unabhängig optional substituiertes C₁₋₆-Alkyl, optional substituiertes C₃₋₈-Cycloalkyl oder optional substituiertes C₆₋₁₀-Aryl ist;
jedes R^{7B} unabhängig Hydroxyl, optional substituiertes C₁₋₆-Alkyl, optional substituiertes C₆₋₁₀-Aryl, optional substituiertes C₂₋₉-Heterocyclyl, optional substituiertes C₁₋₉-Heteroaryl, -N(R⁷)₂, -C(O)N(R⁸)₂, -SO₂N(R⁸)₂, -SO₂R^{7A} oder optional substituiertes Alkoxy ist;
jedes R⁸ unabhängig Wasserstoff, optional substituiertes C₁₋₆-Alkyl, optional substituiertes C₂₋₆-Alkoxyalkyl, optional substituiertes C₆₋₁₀-Aryl-C₁₋₆-alkyl, optional substituiertes C₆₋₁₀-Aryl, optional substituiertes C₃₋₈-Cycloalkyl oder optional substituiertes C₁₋₉-Heteroaryl ist; oder zwei R⁸ zusammen mit dem Atom, an das sie gebunden sind, optional substituiertes C₂₋₉-Heterocyclyl bilden;
Q optional substituiertes C₁₋₆-Alkylen, optional substituiertes C₂₋₆-Alkenylen, optional substituiertes C₂₋₆-Alkinylen, optional substituiertes C₃₋₈-Cycloalkylen, optional substituiertes C₃₋₈-Cycloalkenylen, optional substituiertes C₆₋₁₀-Arylen, optional substituiertes C₂₋₉-Heterocyclylen oder optional substituiertes C₁₋₉-Heteroarylen ist.

2. Verbindung gemäß Anspruch 1 oder ein pharmazeutisch akzeptables Salz davon, wobei die Verbindung in Bezug auf das Atropisomer der Formel (IA) angereichert ist:

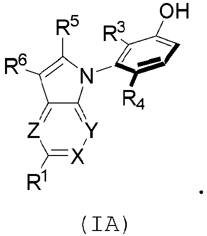

3. Verbindung gemäß Anspruch 1 oder 2 oder ein pharmazeutisch akzeptables Salz davon, worin X CR² ist.

4. Verbindung gemäß Anspruch 1 oder ein pharmazeutisch akzeptables Salz davon, wobei die Verbindung die Formel (II) aufweist:

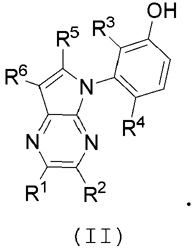

5. Verbindung gemäß Anspruch 4 oder ein pharmazeutisch akzeptables Salz davon, wobei die Verbindung in Bezug auf das Atropisomer der Formel (IIA) angereichert ist:

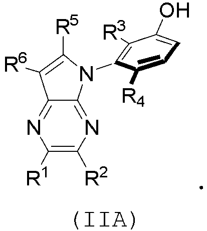

6. Verbindung gemäß Anspruch 1 oder ein pharmazeutisch akzeptables Salz davon, wobei die Verbindung die Formel (III) aufweist: 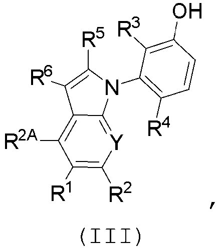 worin R^{2A} Wasserstoff, optional substituiertes C₁₋₆-Alkyl, optional substituiertes C₂₋₆-Alkenyl, optional substituiertes C₂₋₆-Alkinyl, optional substituiertes C₃₋₈-Cycloalkyl, optional substituiertes C₃₋₈-Cycloalkenyl, optional substituiertes C₂₋₉-Heterocyclyl, optional substituiertes C₂₋₉-Heterocyclyl-C₁₋₆-alkyl, optional substituiertes C₆₋₁₀-Aryl, optional substituiertes C₁₋₉-Heteroaryl, optional substituiertes C₁₋₉-Heteroaryl-C₁₋₆-alkyl, Halogen, -N(R⁷)₂, -OR⁷, -C(O)N(R⁸)₂, -SO₂N(R⁸)₂, -SO₂R^{7A} oder -Q-R^{7B} ist.

7. Verbindung gemäß Anspruch 6 oder ein pharmazeutisch akzeptables Salz davon, wobei die Verbindung in Bezug auf das Atropisomer der Formel (IIIA) angereichert ist:

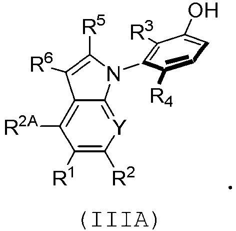

8. Verbindung gemäß Anspruch 6 oder 7 oder ein pharmazeutisch akzeptables Salz davon, worin R^{2A} Wasserstoff, optional substituiertes C₁₋₆-Alkyl oder Halogen ist.

9. Verbindung gemäß mindestens einem der Ansprüche 1 bis 8 oder ein pharmazeutisch akzeptables Salz davon, worin R³ ist:
(a) optional substituiertes C₁₋₆-Alkyl; oder
(b) Halogen, wobei das Halogen optional Chlor ist.

10. Verbindung gemäß mindestens einem der Ansprüche 1 bis 9 oder ein pharmazeutisch akzeptables Salz davon, worin R⁴ ist:
(a) optional substituiertes C₁₋₆-Alkyl; oder
(b) Halogen, wobei das Halogen optional Chlor ist.

11. Verbindung gemäß mindestens einem der Ansprüche 1 bis 10 oder ein pharmazeutisch akzeptables Salz davon, worin R² ist:
(a) Wasserstoff; oder
(b) optional substituiertes C₁₋₆-Alkyl, wobei optional das C₁₋₆-Alkyl optional substituiertes Methyl oder optional substituiertes Isopropyl ist; oder
(c) Halogen.

12. Verbindung gemäß mindestens einem der Ansprüche 1 bis 11 oder ein pharmazeutisch akzeptables Salz davon, worin R¹ ist:
(a) Wasserstoff; oder
(b) Halogen; optional Chlor oder Brom; oder
(c) optional substituiertes C₁₋₆-Alkyl; wobei optional das optional substituierte C₁₋₆-Alkyl optional substituiertes Methyl, optional substituiertes Ethyl, optional substituiertes Isopropyl, optional substituiertes Butyl ist; oder
(d) optional substituiertes C₁₋₉-Heteroaryl; wobei optional das optional substituierte C₁₋₉-Heteroaryl optional substituiertes 1,3-Thiazolyl, optional substituiertes 1,2-Thiazolyl, optional substituiertes 1,3-Oxazolyl, optional substituiertes Benzo-1,3-thiazolyl, optional substituiertes Benzo-1,3-oxazolyl, optional substituiertes Indolyl, optional substituiertes Benzimidazolyl, optional substituiertes Pyridyl, optional substituiertes Imidazolyl, optional substituiertes Pyrimidyl, optional substituiertes Pyrazinyl, optional substituiertes Pyridazinyl, optional substituiertes Pyrazolyl ist; oder
(e) optional substituiertes C₃₋₈-Cycloalkyl; wobei optional das optional substituierte C₃₋₈-Cycloalkyl optional substituiertes Cyclopropyl, optional substituiertes Cyclobutyl, optional substituiertes Cyclopentyl, optional substituiertes Cyclohexyl ist; oder
(f) optional substituiertes C₆₋₁₀-Aryl; wobei optional das optional substituierte C₆₋₁₀-Aryl optional substituiertes Phenyl ist; oder
(g) optional substituiertes C₂₋₉-Heterocyclyl; wobei optional das optional substituierte C₂₋₉-Heterocyclyl optional substituiertes 1,2,3,6-Tetrahydropyridinyl, optional substituiertes Piperidinyl, optional substituiertes Morpholinyl, optional substituiertes Piperazinyl, optional substituiertes Thiomorpholinyl, optional substituiertes Oxa-aza-spiro[3,3]heptan, optional substituiertes Oxa-aza-bicyclo[3.2.1]octan ist; oder
(h) optional substituiertes Cycloalkenyl; oder
(i) -Q-R^{7B}; wobei optional Q optional substituiertes C₂₋₆-Alkinylen, C₁₋₆-Alkylen oder C₆₋₁₀-Arylen ist;
(j) -Q-R^{7B}; wobei optional R^{7B} optional substituiertes C₂₋₉-Heterocyclyl oder optional substituiertes C₆₋₁₀-Aryl ist; und/oder
(j) -N(R⁷)₂; wobei -N(R⁷)₂ optional Diethylamino ist;
wobei optional, wenn R₁ optional substituiert ist, der Substituent eine, zwei oder drei Gruppen ist, die unabhängig ausgewählt sind aus der Gruppe bestehend aus Methyl, Difluormethyl, Trifluormethyl, Fluor, Chlor, Brom, Amino, Hydroxyl, Cyano, Oxo, -C(O)NH₂, -C(O)NH(Me), -C(O)N(Me)₂, - (CH₂)ₙ-C(O)OH und -(CH₂)ₙ-C(O)Ot-Bu, wobei n 0 oder 1 ist.

13. Verbindung gemäß mindestens einem der Ansprüche 1 bis 12 oder ein pharmazeutisch akzeptables Salz davon, worin R⁵ ist
(a) Wasserstoff; oder
(b) -N(R⁷), wobei -NR⁷ optional -NH₂ ist.

14. Verbindung gemäß mindestens einem der Ansprüche 1 bis 13 oder ein pharmazeutisch akzeptables Salz davon, worin R⁶ ist
(a) -C(O)NH(R⁸); wobei -C(O)NH(R⁸) optional -C(O)NH₂ oder -C(O)NH(Me) ist; oder
(b) -SO₂R^{7A}, wobei -SO₂R^{7A} optional -SO₂Me ist.

15. Verbindung gemäß Anspruch 1, wobei die Verbindung die folgende Struktur aufweist: 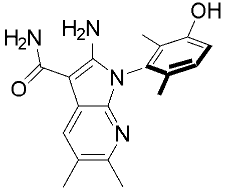 oder ein pharmazeutisch akzeptables Salz davon.

16. Pharmazeutische Zusammensetzung, umfassend die Verbindung gemäß mindestens einem der Ansprüche 1 bis 15 oder ein pharmazeutisch akzeptables Salz davon und einen pharmazeutisch akzeptablen Hilfsstoff, wobei die Zusammensetzung optional mit Deuterium isotopisch angereichert ist.

## Revendications

1. Composé de formule (I) : 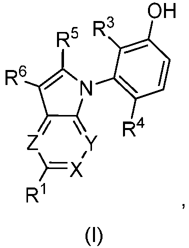
ou un sel pharmaceutiquement acceptable de celui-ci,
dans lequel
chacun parmi X, Y et Z est indépendamment N ou CR² ;
R¹ et chaque R² sont indépendamment un hydrogène, un alkyle en C₁₋₆ facultativement substitué, un alcényle en C₂₋₆ facultativement substitué, un alcynyle en C₂₋₆ facultativement substitué, un cycloalkyle en C₃₋₈ facultativement substitué, un cycloalcényle en C₃₋₈ facultativement substitué, un hétérocyclyle en C₂₋₉ facultativement substitué, un hétérocyclyle en C₂₋₉-alkyle en C₁₋₆ facultativement substitué, un aryle en C₆₋₁₀ facultativement substitué, un hétéroaryle en C₁₋₉ facultativement substitué, un hétéroaryle en C₁₋₉-alkyle en C₁₋₆ facultativement substitué, un halogène, un cyano, -N(R⁷)₂, -OR⁷, -C(O)N(R⁸)₂, -SO₂N(R⁸)₂, -SO₂R^{7A} ou -Q-R^{7B} ; ou R¹ se combine avec un R² qui est vicinal à R¹ pour former un alkylène en C₃₋₆ facultativement substitué ;
chacun parmi R³ et R⁴ peut être indépendamment un alkyle en C₁₋₆ facultativement substitué ou un halogène ;
R⁵ est H ou -N(R⁷)₂ ;
R⁶ est -C(O)NH(R⁸), -C(O)R^{7A} ou -SO₂R^{7A} ;
chaque R⁷ est indépendamment un hydrogène, un alkyle en C₁₋₆ facultativement substitué, un aryle en C₆₋₁₀-alkyle en C₁₋₆ facultativement substitué, un cycloalkyle en C₃₋₈ facultativement substitué, un aryle en C₆₋₁₀ facultativement substitué, un hétérocyclyle en C₂₋₉ facultativement substitué, un hétéroaryle en C₁₋₉ facultativement substitué, un hétéroaryle en C₁₋₉-alkyle en C₁₋₆ facultativement substitué ou -SO₂R^{7A} ; ou deux groupes R⁷, associés à l'atome auquel ils sont tous les deux liés, se combinent pour former un hétérocycle en C₂₋₉ facultativement substitué ;
Chaque R^{7A} est un alkyle en C₁₋₆ facultativement substitué, un cycloalkyle en C₃₋₈ facultativement substitué ou un aryle en C₆₋₁₀ facultativement substitué ;
chaque R^{7B} est indépendamment un hydroxyle, un alkyle en C₁₋₆ facultativement substitué, un aryle en C₆₋₁₀ facultativement substitué, un hétérocyclyle en C₂₋₉ facultativement substitué, un hétéroaryle en C₁₋₉ facultativement substitué, -N(R⁷)₂, -C(O)N(R⁸)₂, -SO₂ N(R⁸)₂, -SO₂R^{7A} ou un alcoxy facultativement substitué ;
Chaque R⁸ est indépendamment un hydrogène, un alkyle en C₁₋₆ facultativement substitué, un alcoxyalkyle en C₂₋₆ facultativement substitué, un aryle en C₆₋₁₀-alkyle en C₁₋₆ facultativement substitué, un aryle en C₆₋₁₀ facultativement substitué, un cycloalkyle en C₃₋₈ facultativement substitué ou un hétéroaryle en C₁₋₉ facultativement substitué ; ou deux R⁸, conjointement avec l'atome auquel ils sont liés, se combinent pour former un hétérocyclyle en C₂₋₉ facultativement substitué ;
Q est un alkylène en C₁₋₆ facultativement substitué, un alcénylène en C₂₋₆ facultativement substitué, un alcynylène en C₂₋₆ facultativement substitué, un cycloalkylène en C₃₋₈ facultativement substitué, un cycloalcénylène en C₃₋₈ facultativement substitué, un arylène en C₆₋₁₀ facultativement substitué, un hétérocyclylène en C₂₋₉ facultativement substitué ou un hétéroarylène en C₁₋₉ facultativement substitué.

2. Composé selon la revendication 1 ou un sel pharmaceutiquement acceptable de celui-ci, dans lequel le composé est enrichi en atropisomère de formule (IA) :

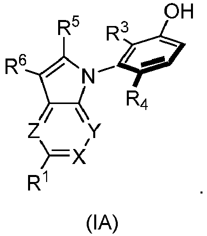

3. Composé selon la revendication 1 ou la revendication 2 ou un sel pharmaceutiquement acceptable de celui-ci, dans lequel X est CR².

4. Composé selon la revendication 1 ou un sel pharmaceutiquement acceptable de celui-ci, dans lequel le composé est de formule (II) :

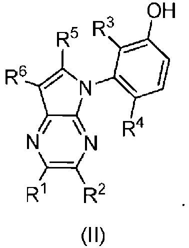

5. Composé selon la revendication 4 ou un sel pharmaceutiquement acceptable de celui-ci, dans lequel le composé est enrichi en atropisomère de formule (IIA) :

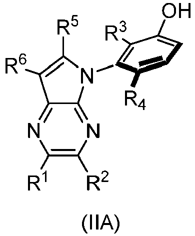

6. Composé selon la revendication 1 ou un sel pharmaceutiquement acceptable de celui-ci, dans lequel le composé est de formule (III) : 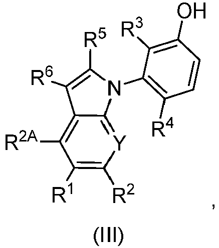 **dans lequel** R^{2A} est un hydrogène, un alkyle en C₁₋₆ facultativement substitué, un alcényle en C₂₋₆ facultativement substitué, un alcynyle en C₂₋₆ facultativement substitué, un cycloalkyle en C₃₋₈ facultativement substitué, un cycloalcényle en C₃₋₈ facultativement substitué, un hétérocyclyle en C₂₋₉ facultativement substitué, un hétérocyclyle en C₂₋₉-alkyle en C₁₋₆ facultativement substitué, un aryle en C₆₋₁₀ facultativement substitué, un hétéroaryle en C₁₋₉ facultativement substitué, un hétéroaryle en C₁₋₉-alkyle en C₁₋₆ facultativement substitué, un halogène, -N(R⁷)₂, -OR⁷, -C(O)N(R⁸)₂, -SO₂N(R⁸)₂, -SO₂R^{7A} ou -Q-R^{7B}.

7. Composé selon la revendication 6 ou un sel pharmaceutiquement acceptable de celui-ci, dans lequel le composé est enrichi en atropisomère de formule (IIIA) :

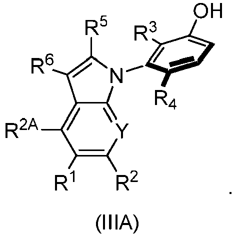

8. Composé selon la revendication 6 ou la revendication 7 ou un sel pharmaceutiquement acceptable de celui-ci, dans lequel R^{2A} est un hydrogène, un alkyle en C₁₋₆ facultativement substitué ou un halogène.

9. Composé selon l'une quelconque des revendications 1 à 8 ou un sel pharmaceutiquement acceptable de celui-ci, dans lequel R³ est :
(a) un alkyle en C₁₋₆ facultativement substitué ; ou
(b) un halogène, facultativement dans lequel l'halogène est un chlore.

10. Composé selon l'une quelconque des revendications 1 à 9 ou un sel pharmaceutiquement acceptable de celui-ci, dans lequel R⁴ est :
(a) un alkyle en C₁₋₆ facultativement substitué ; ou
(b) un halogène, facultativement dans lequel l'halogène est un chlore.

11. Composé selon l'une quelconque des revendications 1 à 10 ou un sel pharmaceutiquement acceptable de celui-ci, dans lequel R² est :
(a) un hydrogène ; ou
(b) un alkyle en C₁₋₆ facultativement substitué, facultativement dans lequel l'alkyle en C₁₋₆ est un méthyle facultativement substitué ou un isopropyle facultativement substitué ; ou
(c) un halogène.

12. Composé selon l'une quelconque des revendications 1 à 11 ou un sel pharmaceutiquement acceptable de celui-ci, dans lequel R¹ est :
(a) un hydrogène ; ou
(b) un halogène ; facultativement un chlore ou un brome ; ou
(c) un alkyle en C₁₋₆ facultativement substitué ; facultativement dans lequel l'alkyle en C₁₋₆ facultativement substitué est un méthyle facultativement substitué, un éthyle facultativement substitué, un isopropyle facultativement substitué, un butyle facultativement substitué ; ou
(d) un hétéroaryle en C₁₋₉ facultativement substitué ; facultativement dans lequel l'hétéroaryle en C₁₋₉ facultativement substitué est un 1,3-thiazolyle facultativement substitué, un 1,2-thiazolyle facultativement substitué, un 1,3-oxazolyle facultativement substitué, un benzo-1,3-thiazolyle facultativement substitué, un benzo-1,3-oxazolyle facultativement substitué, un indolyle facultativement substitué, un benzimidazolyle facultativement substitué, un pyridyle facultativement substitué, un imidazolyle facultativement substitué, un pyrimidyle facultativement substitué, un pyrazinyle facultativement substitué, un pyridazinyle facultativement substitué, un pyrazolyle facultativement substitué ; ou
(e) un cycloalkyle en C₃₋₈ facultativement substitué ; facultativement dans lequel le cycloalkyle en C₃₋₈ facultativement substitué est un cyclopropyle facultativement substitué, un cyclobutyle facultativement substitué, un cyclopentyle facultativement substitué, un cyclohexyle facultativement substitué ; ou
(f) un aryle en C₆₋₁₀ facultativement substitué ; facultativement dans lequel l'aryle en C₆₋₁₀ facultativement substitué est un phényle facultativement substitué ; ou
(g) un hétérocyclyle en C₂₋₉ facultativement substitué ; facultativement dans lequel l'hétérocyclyle en C₂₋₉ facultativement substitué est un 1,2,3,6-tétrahydropyridinyle facultativement substitué, un pipéridinyle facultativement substitué, un morpholinyle facultativement substitué, un pipérazinyle facultativement substitué, un thiomorpholinyle facultativement substitué, un oxa-aza-spiro[3,3]heptane facultativement substitué, un oxa-aza-bicyclo[3.2.1]octane facultativement substitué ; ou
(h) un cycloalcényle facultativement substitué ; ou
(i) -Q-R^{7B} ; facultativement dans lequel Q est un alcynylène en C₂₋₆, alkylène en C₁₋₆ ou arylène en C₆₋₁₀ facultativement substitué ;
(j) -Q-R^{7B} ; facultativement dans lequel R^{7B} est un hétérocycle en C₂₋₉ facultativement substitué ou un aryle en C₆₋₁₀ facultativement substitué ; et/ou
(j) -N(R⁷)₂ ; facultativement dans lequel -N(R⁷)₂ est un diéthylamino ;
facultativement dans lequel, lorsque R1 est facultativement substitué, le substituant est un, deux ou trois groupes sélectionnés indépendamment dans le groupe consistant en un méthyle, un difluorométhyle, un trifluorométhyle, un fluor, un chlore, un brome, un amino, un hydroxyle, un cyano, un oxo, -C(O)NH₂, -C(O)NH(Me), -C(O)N(Me)₂, -(CH₂)ₙ-C(O)OH, et -(CH₂)ₙ-C(O)Ot-Bu, dans lequel n est 0 ou 1.

13. Composé selon l'une quelconque des revendications 1 à 12 ou un sel pharmaceutiquement acceptable de celui-ci, dans lequel R⁵ est :
(a) un hydrogène ; ou
(b) -N(R⁷), facultativement dans lequel -NR⁷ est -NH₂.

14. Composé selon l'une quelconque des revendications 1 à 13 ou un sel pharmaceutiquement acceptable de celui-ci, dans lequel R⁶ est :
(a) -C(O)NH(R⁸) ; facultativement dans lequel -C(O)NH(R⁸) est -C(O)NH₂ ou -C(O)NH(Me) ; ou
(b) -SO₂R^{7A}, facultativement dans lequel -SO₂R^{7A} est -SO₂Me.

15. Composé selon la revendication 1, dans lequel le composé présente la structure : 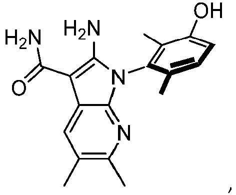 ou un sel pharmaceutiquement acceptable de celui-ci.

16. Composition pharmaceutique comprenant le composé selon l'une quelconque des revendications 1 à 15 ou un sel pharmaceutiquement acceptable de celui-ci, et un excipient pharmaceutiquement acceptable, facultativement dans laquelle la composition est enrichie isotopiquement en deutérium.
